# EUROPÄISCHE PATENTSCHRIFT

(11) **EP 3 642 258 B1**
(45) Veröffentlichungstag und Bekanntmachung des Hinweises auf die Patenterteilung: **03.04.2024**
(21) Anmeldenummer: 18730006.6
(22) Anmeldetag: 18.06.2018
(51) Int. Cl.: C08G 61/12, C07C 211/54, C07C 211/58, C07D 209/44, C07D 307/87, C07D 307/91, C07D 405/04, C07D 307/79, C07D 317/58, C07D 407/04, C07D 493/08

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 21.06.2017 EP 17177211
(43) Veröffentlichungstag der Anmeldung: 29.04.2020
(73) Patentinhaber: Merck Patent GmbH, 64293 Darmstadt (DE)
(72) Erfinder: BURKHART, Beate, 64293 Darmstadt (DE); SCHEIBLE, Katja, 64287 Darmstadt (DE); KOENEN, Nils, 64347 Griesheim (DE); HEIL, Holger, 60316 Frankfurt am Main (DE)
(74) Vertreter: Merck Patent Association
(86) Internationale Anmeldenummer: PCT/EP2018/066074
(87) Internationale Veröffentlichungsnummer: WO 2018/234220

(56) Entgegenhaltungen:
- WO-A1-2013/156130
- JP-A- 2005 208 111
- JP-A- 2012 188 637
- JP-A- 2013 124 271
- JP-A- 2013 155 294
- JP-B2- 4 276 959

## Beschreibung

Die vorliegende Anmeldung betrifft ein Polymer enthaltend mindestens eine Struktureinheit einer Formel (I), wie unten definiert. Das Polymer eignet sich zur Verwendung in einer elektronischen Vorrichtung.

Unter elektronischen Vorrichtungen im Sinne dieser Anmeldung werden sogenannte organische elektronische Vorrichtungen verstanden (organic electronic devices), die organische Halbleitermaterialien als Funktionsmaterialien enthalten. Insbesondere werden darunter OLEDs verstanden. Unter der Bezeichnung OLEDs werden elektronische Vorrichtungen verstanden, welche eine oder mehrere Schichten enthaltend organische Verbindungen aufweisen und welche unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Daher werden weiterhin neue Materialien, insbesondere Polymere, zur Verwendung in OLEDs gesucht.

Bei OLEDs sind zwei wichtige Verfahren zur Aufbringung der Materialien in Schichtform bekannt: die Aufbringung aus der Gasphase, durch Sublimation, sowie die Aufbringung aus Lösung. Für letzteres Verfahren sind als Materialien unter anderem Polymere geeignet.

Für die Herstellung derartiger Polymere ist es wichtig, dass die Polymere und die eingesetzten Monomere gut löslich sind, da sonst keine Polymere mit großen Kettenlängen erhalten werden können.

Wenn die Polymere bei der Herstellung der OLEDs aus Lösung aufgebracht werden, ist es wichtig, dass sie in den verwendeten Lösungsmitteln gut löslich sind. Weiterhin ist es wichtig, dass sie sich rasch in den verwendeten Lösungsmitteln auflösen. Weiterhin ist es wichtig, dass sie gute Filmbildungseigenschaften haben.

Von besonderer Bedeutung bei der Verwendung von Polymeren in OLEDs ist, dass diese eine hohe Lebensdauer und Effizienz der Vorrichtung bewirken. Dies gilt insbesondere bei der Verwendung von Polymeren in der lochtransportierenden Schicht der OLED, in Kombination mit einer folgenden emittierenden Schicht, die ebenfalls aus Lösung aufgetragen wird.

Weiterhin ist es wichtig, dass die Polymere chemisch möglichst stabil sind und sich nicht zersetzen.

Es wurde nun gefunden, dass wenigstens eine, bevorzugt mehrere der oben genannten technischen Aufgaben durch die Bereitstellung eines neuen Polymers enthaltend bestimmte Struktureinheiten, wie unten definiert, gelöst werden kann.

Gegenstand der vorliegenden Anmeldung ist damit ein Polymer, enthaltend mindestens eine Struktureinheit der Formel (I) in einem Anteil von 1 bis 100 mol%, bezogen auf 100 mol% aller copolymerisierbaren Monomere, die im Polymer als Struktureinheiten enthalten sind 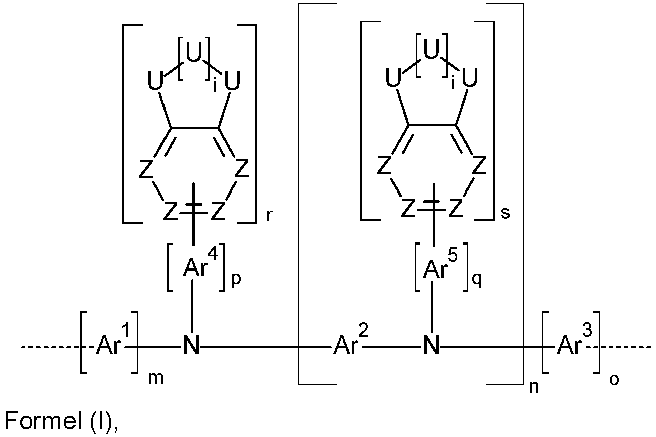 wobei für die auftretenden Variablen gilt:
U ist bei jedem Auftreten gleich oder verschieden C(R¹)₂, CR¹=CR¹, Si(R¹)₂, O oder S, wobei Gruppen gewählt aus CR¹=CR¹, O und S nicht direkt miteinander verbunden sind;
Z ist gleich CR², wenn keine Gruppe daran gebunden ist, und ist gleich C, wenn eine Gruppe daran gebunden ist;
Ar¹, Ar², Ar³, Ar⁴ und Ar⁵ sind gleich oder verschieden gewählt aus heteroaromatischen Ringsystemen mit 5 bis 40 aromatischen Ringatomen, die mit einem oder mehreren Resten R³ substituiert sein können, und aus aromatischen Ringsystemen mit 6 bis 40 aromatischen Ringatomen, die mit einem oder mehreren Resten R³ substituiert sein können;
R¹ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R², R³ sind bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C≡C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁵ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
r ist gleich 1, 2, oder 3, wenn p gleich 1 ist, und ist gleich 1, wenn p gleich 0 ist;
s ist gleich 0, 1, 2, oder 3, wenn q gleich 1 ist, und ist gleich 1, wenn q gleich 0 ist;
p ist gleich 0 oder 1; wobei wenn p gleich 0 ist, die Gruppen, die an die in eckigen Klammern mit Index p stehende Einheit gebunden sind, direkt miteinander verbunden sind;
q ist gleich 0 oder 1; wobei wenn q gleich 0 ist, die Gruppen, die an die in eckigen Klammern mit Index q stehende Einheit gebunden sind, direkt miteinander verbunden sind;
n ist gleich 0 oder 1, wobei wenn n gleich 0 ist, die Gruppen, die an die in eckigen Klammern mit Index n stehende Einheit gebunden sind, direkt miteinander verbunden sind;
m ist gleich 0 oder 1, wobei wenn m gleich 0 ist, die Gruppen, die an die in eckigen Klammern mit Index m stehende Einheit gebunden sind, direkt miteinander verbunden sind;
o ist gleich 0 oder 1, wobei wenn o gleich 0 ist, die Gruppen, die an die in eckigen Klammern mit Index o stehende Einheit gebunden sind, direkt miteinander verbunden sind;
i ist bei jedem Auftreten gleich oder verschieden 1 oder 2;
wobei mindestens eine Gruppe U vorhanden ist, die eine oder mehrere Gruppen R¹ enthält, die gewählt sind aus geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können; und
wobei diejenigen beiden Gruppen U, die sich direkt benachbart zum Brückenkopf-C-Atom, das heißt in benzylischer Position befinden, Reste R¹ tragen, die gewählt sind aus F, CN, Si(R⁴)₃, OR⁴, geradkettigen Alkyl- und Alkoxygruppen mit 1 bis 10 C-Atomen, verzweigten oder cyclischen Alkyl- und Alkoxygruppen mit 3 bis 10 C-Atomen, und aromatischen Ringsystemen mit 6 bis 20 aromatischen Ringatomen, wobei zwei oder mehr Reste R¹ bzw. R² bzw. R³ miteinander verknüpft sein können und einen Ring bilden können; und wobei die genannten Alkyl- und Alkoxygruppen und die genannten aromatischen Ringsysteme jeweils mit einem oder mehreren Resten R⁴ substituiert sein können.

In den Formeln für Struktureinheiten kennzeichnen die gestrichelten Linien die Bindungen zu benachbarten Struktureinheiten des Polymers.

In der vorliegenden Anmeldung umfasst der Begriff Polymer polymere Verbindungen, oligomere Verbindungen und Dendrimere. Die erfindungsgemäßen Polymere weisen vorzugsweise 10 bis 10000, besonders bevorzugt 10 bis 5000 und ganz besonders bevorzugt 10 bis 2000 Struktureinheiten (d.h. Wiederholungseinheiten) auf. Die erfindungsgemäßen oligomeren Verbindungen weisen vorzugsweise 3 bis 9 Struktureinheiten auf. Der Verzweigungs-Faktor der Polymere liegt dabei zwischen 0 (lineares Polymer, ohne Verzweigungsstellen) und 1 (vollständig verzweigtes Dendrimer).

Die erfindungsgemäßen Polymere weisen vorzugsweise ein Molekulargewicht M_{w} im Bereich von 10.000 bis 1.000.000 g/mol, besonders bevorzugt ein Molekulargewicht M_{w} im Bereich von 20.000 bis 500.000 g/mol und ganz besonders bevorzugt ein Molekulargewicht M_{w} im Bereich von 25.000 bis 200.000 g/mol auf. Die Bestimmung des Molekulargewichts M_{w} erfolgt mittels GPC (= Gelpermeationschromatographie) gegen einen internen Polystyrolstandard.

Bei den erfindungsgemäßen Polymeren handelt es sich entweder um konjugierte, teilkonjugierte oder nicht-konjugierte Polymere. Bevorzugt sind konjugierte oder teilkonjugierte Polymere.

Die Struktureinheiten der Formel (I) können erfindungsgemäß in die Haupt- oder in die Seitenkette des Polymeren eingebaut werden. Vorzugsweise werden die Struktureinheiten der Formel (I) jedoch in die Hauptkette des Polymeren eingebaut. Bei Einbau in die Seitenkette des Polymeren können die Struktureinheiten der Formel (I) entweder mono- oder bivalent sein, d.h. sie weisen entweder eine oder zwei Bindungen zu benachbarten Struktureinheiten im Polymer auf.

"Konjugierte Polymere" im Sinne der vorliegenden Anmeldung sind Polymere, die in der Hauptkette hauptsächlich sp²-hybridisierte (bzw. gegebenenfalls auch sp-hybridisierte) Kohlenstoffatome enthalten, die auch durch entsprechend hybridisierte Heteroatome ersetzt sein können. Dies bedeutet im einfachsten Fall abwechselndes Vorliegen von Doppel- und Einfachbindungen in der Hauptkette, aber auch Polymere mit Einheiten wie beispielsweise einem meta-verknüpften Phenylen sollen im Sinne dieser Anmeldung als konjugierte Polymere gelten. "Hauptsächlich" meint, dass natürlich (unwillkürlich) auftretende Defekte, die zu Konjugationsunterbrechungen führen, den Begriff "konjugiertes Polymer" nicht entwerten. Als konjugierte Polymere gelten ebenfalls Polymere mit einer konjugierten Hauptkette und nicht-konjugierten Seitenketten. Des Weiteren wird in der vorliegenden Anmeldung ebenfalls als konjugiert bezeichnet, wenn sich in der Hauptkette beispielsweise Arylamineinheiten, Arylphosphineinheiten, bestimmte Heterocyclen (d.h. Konjugation über N-, O- oder S-Atome) und/oder metallorganische Komplexe (d.h. Konjugation über das Metallatom) befinden. Analoges gilt für konjugierte Dendrimere. Hingegen werden Einheiten wie beispielsweise einfache Alkylbrücken, (Thio)Ether-, Ester-, Amid- oder Imidverknüpfungen eindeutig als nicht-konjugierte Segmente definiert.

Unter einem teilkonjugierten Polymer soll in der vorliegenden Anmeldung ein Polymer verstanden werden, das konjugierte Regionen enthält, die durch nicht-konjugierte Abschnitte, gezielte Konjugationsunterbrecher (z.B. Abstandsgruppen) oder Verzweigungen voneinander getrennt sind, z.B. in dem längere konjugierte Abschnitte in der Hauptkette durch nicht-konjugierte Abschnitte unterbrochen sind, bzw. das längere konjugierte Abschnitte in den Seitenketten eines in der Hauptkette nicht-konjugierten Polymers enthält. Konjugierte und teilkonjugierte Polymere können auch konjugierte, teilkonjugierte oder nicht-konjugierte Dendrimere enthalten.

Unter dem Begriff "Dendrimer" soll in der vorliegenden Anmeldung eine hochverzweigte Verbindung verstanden werden, die aus einem multifunktionellen Zentrum (core) aufgebaut ist, an das in einem regelmäßigen Aufbau verzweigte Monomere gebunden werden, so dass eine baumartige Struktur erhalten wird. Dabei können sowohl das Zentrum als auch die Monomere beliebige verzweigte Strukturen annehmen, die sowohl aus rein organischen Einheiten als auch Organometallverbindungen oder Koordinationsverbindungen bestehen. "Dendrimer" soll hier allgemein so verstanden werden, wie dies z.B. von M. Fischer und F. Vögtle (Angew. Chem., Int. Ed. 1999, 38, 885) beschrieben ist.

Unter dem Begriff Struktureinheit wird in der vorliegenden Anmeldung eine Einheit verstanden, die im Polymer mehrfach mit der angegebenen Struktur auftritt. Sie kann dabei mehrfach direkt hintereinander, und/oder vereinzelt im Polymer auftreten. Bevorzugt treten eine Vielzahl Struktureinheiten mit der angegebenen Struktur im Polymer auf, besonders bevorzugt 10 bis 1000, ganz besonders bevorzugt 50 bis 500.

Weiterhin bevorzugt ist eine Struktureinheit im Sinne der vorliegenden Anmeldung von einem in der Polymerisation eingesetzten Monomer dadurch abgeleitet, dass die reaktiven Gruppen des Monomers entsprechend ihrer chemischen Reaktivität und Bestimmung reagiert haben. Beispielsweise ist bei einem Monomer enthaltend zwei Bromatome als reaktive Gruppen in einer Suzuki-Polymerisationsreaktion die entstehende Struktureinheit im Polymer dadurch gekennzeichnet, dass sie der Monomerstruktur entspricht, mit dem Unterschied, dass die Bromatome fehlen und die Bindungen zu den Bromatomen nun Bindungen zu den benachbarten Struktureinheiten sind. Im Fall von Monomeren enthaltend Vernetzergruppen oder Vorläufergruppen für Vernetzergruppen können dabei eine oder mehrere weitere Reaktionen der Vernetzergruppe bzw. der entsprechenden Vorläufergruppen der Vernetzergruppe erfolgen, bis die entsprechende endgültige Struktureinheit des Polymers erhalten wird.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Bevorzugt wird unter einem aromatischen Ringsystem eine chemische Gruppe verstanden, in der die darin enthaltenen Arylgruppen miteinander konjugiert sind. Dies bedeutet, dass die enthaltenen Arylgruppen miteinander über Einfachbindungen oder über verbindende Einheiten, die ein freies pi-Elektronenpaar aufweisen, das an der Konjugation teilnehmen kann, verbunden sein müssen. Verbindende Einheiten sind dabei bevorzugt gewählt aus Stickstoffatomen, einzelnen C=C-Einheiten, einzelnen C=C-Einheiten, mehreren miteinander konjugierten C=C-Einheiten oder/oder C=C-Einheiten, -O-, und -S-.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Bevorzugte Alkylgruppen mit 1 bis 20 C-Atomen sind in der folgenden Tabelle abgebildet:

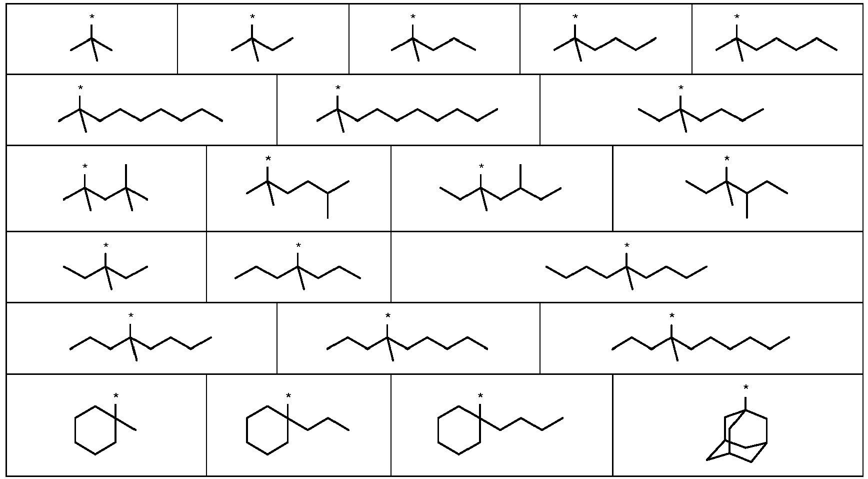

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt ist U bei jedem Auftreten gleich oder verschieden gewählt aus C(R¹)₂, O und S, besonders bevorzugt ist U gleich C(R¹)₂.

Erfindungsgemäß ist Z gleich CR², wenn keine Gruppe daran gebunden ist, und es ist gleich C, wenn eine Gruppe daran gebunden ist.

Bevorzugt sind Ar¹, Ar², Ar³, Ar⁴ und Ar⁵ bei jedem Auftreten gleich oder verschieden gewählt aus aromatischen Ringsystemen mit 6 bis 25 aromatischen Ringatomen, die mit einem oder mehreren Resten R¹ substituiert sein können, und aus heteroaromatischen Ringsystemen mit 5 bis 25 aromatischen Ringatomen, die mit einem oder mehreren Resten R² substituiert sein können. Besonders bevorzugt sind Ar¹, Ar², Ar³, Ar⁴ und Ar⁵ bei jedem Auftreten gleich oder verschieden gewählt aus Benzol, Biphenyl, Terphenyl, Fluoren, Naphthalin, Phenanthren, Indenofluoren, Spirobifluoren, Dibenzofuran, Dibenzothiophen, Carbazol, Indenocarbazol und Indolocarbazol, die jeweils mit einem oder mehreren Resten R¹ substituiert sein können. Ganz besonders bevorzugt sind Ar⁴ und Ar⁵ gleich Benzol, das mit einem oder mehreren Resten R¹ substituiert sein kann. Ganz besonders bevorzugt sind Ar¹, Ar² und Ar³ bei jedem Auftreten gleich oder verschieden gewählt aus Benzol, Biphenyl, Fluoren, Phenanthren, Indenofluoren und Spirobifluoren, die mit einem oder mehreren Resten R¹ substituiert sein können. Am stärksten bevorzugt sind Ar¹ und Ar³ gewählt aus Benzol, das mit einem oder mehreren Resten R¹ substituiert sein kann.

Bevorzugte Gruppen Ar¹ bis Ar⁵ sind gewählt aus den folgenden Gruppen

| | | |
|---|---|---|
| 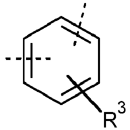 | 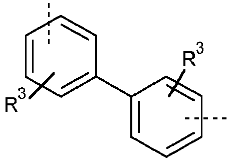 | 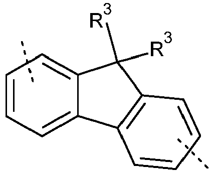 |
| A1 | A2 | A3 |
| 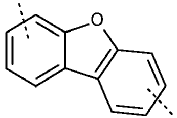 | 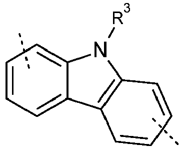 | 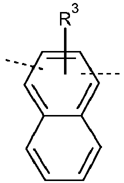 |
| A4 | A5 | A6 |
| 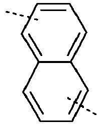 | 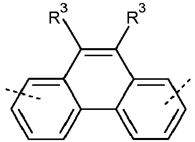 | 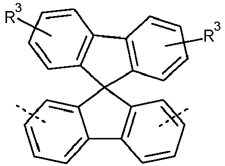 |
| A7 | A8 | A9 |
| 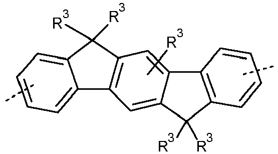 | | |
| A10 | | |

wobei die gestrichelten Linien die Anbindungspositionen darstellen, und
wobei eine als unspezifisch an einen aromatischen Ring gebunden gezeichnete Gruppe R³ bedeutet, dass an den betreffenden Ring in allen freien Positionen jeweils eine Gruppe R³ gebunden sein kann.

Bevorzugte Ausführungsformen der oben genannten Gruppen A1 bis A10 sind im Folgenden dargestellt:

| | | |
|---|---|---|
| 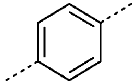 | 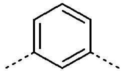 | 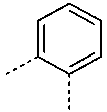 |
| A1a | A1b | A1c |
| 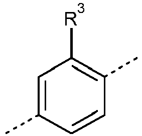 | 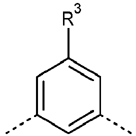 | 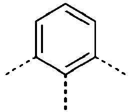 |
| A1d | A1e | A1f |
| 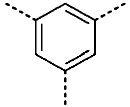 | 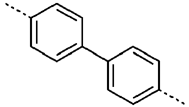 | |
| A1₈ | A2a | |
| 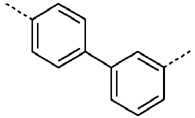 | 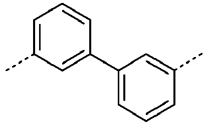 | 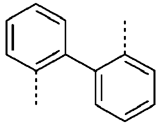 |
| A2b | A2c | A2d |
| 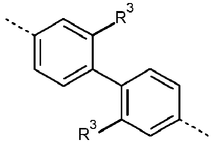 | 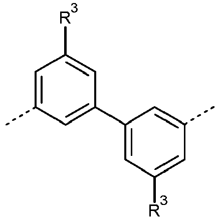 | 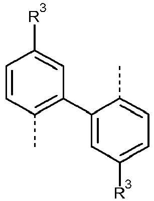 |
| A2e | A2f | A2g |
| 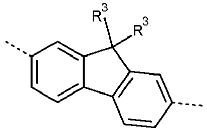 | 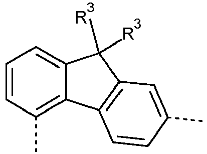 | 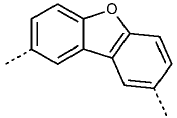 |
| A3a | A3b | A4a |
| 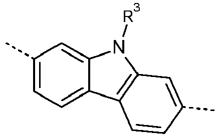 | 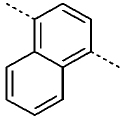 | 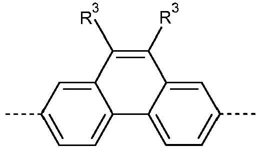 |
| A5a | A6a | A8a |
| 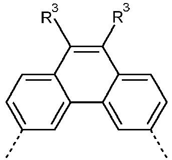 | 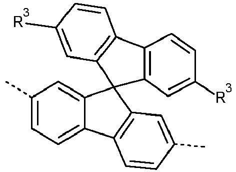 | 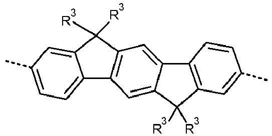 |
| A8b | A9a | A10a |

wobei die gestrichelten Linien die Anbindungspositionen darstellen.
R¹ ist bevorzugt bei jedem Auftreten gleich oder verschieden gewählt aus H, D, F, CN, Si(R⁴)₃, OR⁴, geradkettigen Alkyl- und Alkoxygruppen mit 1 bis 10 C-Atomen, verzweigten oder cyclischen Alkyl- und Alkoxygruppen mit 3 bis 10 C-Atomen, und aromatischen Ringsystemen mit 6 bis 20 aromatischen Ringatomen, wobei zwei oder mehr Reste R¹ bzw. R² bzw. R³ miteinander verknüpft sein können und einen Ring bilden können; und wobei die genannten Alkyl- und Alkoxygruppen und die genannten aromatischen Ringsysteme jeweils mit einem oder mehreren Resten R⁴ substituiert sein können. Besonders bevorzugt ist R¹ bei jedem Auftreten gleich oder verschieden gewählt aus H, D, F, geradkettigen Alkylgruppen mit 1 bis 10 C-Atomen und verzweigten oder cyclischen Alkylgruppen mit 3 bis 10 C-Atomen. Ganz besonders bevorzugt ist R¹ gewählt aus geradkettigen Alkylgruppen mit 1 bis 10 C-Atomen und verzweigten Alkylgruppen mit 3 bis 10 C-Atomen.

Es ist erfindungsgemäß, dass diejenigen beiden Gruppen U, die sich direkt benachbart zum Brückenkopf-C-Atom, das heißt in benzylischer Position befinden, Reste R¹ tragen, die ungleich H und D sind, bevorzugt Reste R¹ tragen, die gewählt sind aus F, CN, Si(R⁴)s, OR⁴, geradkettigen Alkyl- und Alkoxygruppen mit 1 bis 10 C-Atomen, verzweigten oder cyclischen Alkyl- und Alkoxygruppen mit 3 bis 10 C-Atomen, und aromatischen Ringsystemen mit 6 bis 20 aromatischen Ringatomen, wobei zwei oder mehr Reste R¹ bzw. R² bzw. R³ miteinander verknüpft sein können und einen Ring bilden können; und wobei die genannten Alkyl- und Alkoxygruppen und die genannten aromatischen Ringsysteme jeweils mit einem oder mehreren Resten R⁴ substituiert sein können.

Gemäß einer bevorzugten Ausführungsform enthält die Struktureinheit der Formel (I) mindestens eine Gruppe U, die zwei Gruppen R¹ aufweist, die miteinander verknüpft sind und einen Ring bilden, so dass die Gruppe U ein Spiroatom darstellt. Bevorzugt sind die von zwei Gruppen R¹ an einer Gruppe U gebildeten Ringe gewählt aus Cyclopropan, Cyclobutan, Cyclopentan, Cyclohexan, Fluoren, Dibenzopyran, Dihydroacridin und Pyran.

Bevorzugte Einheiten 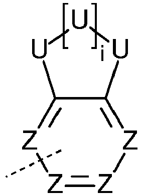 in der Struktureinheit der Formel (I) sind gewählt aus Einheiten der folgenden Formel 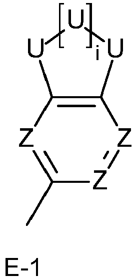 wobei die freie Bindung die Bindung an den Rest der Struktureinheit der Formel (I) darstellt.

Weiterhin ist es bevorzugt, dass oben genannte Einheit gewählt ist aus Einheiten der folgenden Formeln

| | | |
|---|---|---|
| 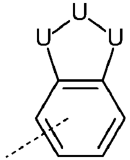 | 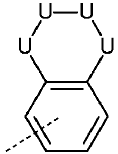 | 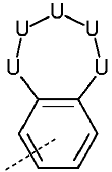 |
| E-a | E-b | E-c |

wobei die entsprechenden freien Positionen jeweils mit einem Rest R² substituiert sein können, und wobei die gestrichelte Linie die Bindung an den Rest der Struktureinheit der Formel (I) darstellt.

Insbesondere bevorzugt ist es, dass die oben genannten Einheiten der Formeln E-a bis E-c gewählt sind aus den folgenden Einheiten

| | | |
|---|---|---|
| 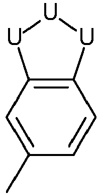 | 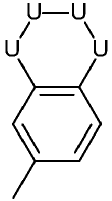 | 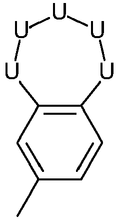 |
| E-a-1 | E-b-1 | E-c-1 |

wobei die freie Bindung die Bindung an den Rest der Struktureinheit der Formel (I) darstellt, und wobei Einheiten E-a-1 und E-b-1 unter den genannten Einheiten besonders bevorzugt sind, und am stärksten bevorzugt Einheit E-a-1 ist.

Bevorzugte Ausführungsformen der Einheiten E-a, E-b und E-c sind die folgenden Einheiten:

| | | |
|---|---|---|
| 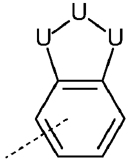 | 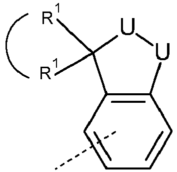 | 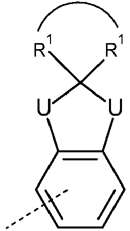 |
| E-a-1 | E-a-2 | E-a-3 |
| 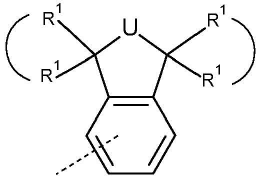 | 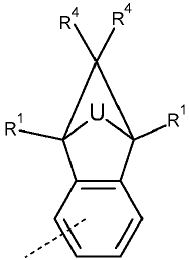 | |
| E-a-4 | E-a-5 | |
| 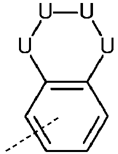 | 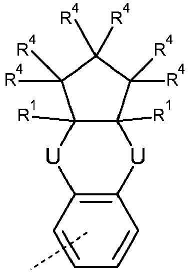 | 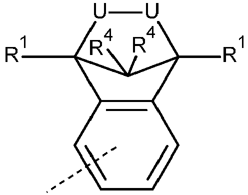 |
| E-b-1 | E-b-2 | E-b-3 |
| 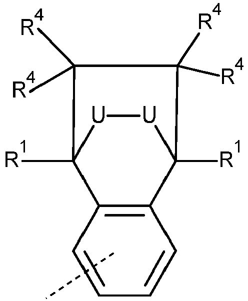 | 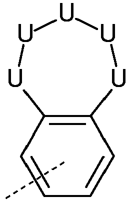 | 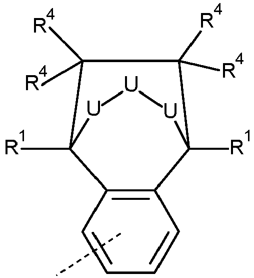 |
| E-b-4 | E-c-1 | E-c-2 |

wobei die gestrichelte Linie die Bindung an den Rest der Struktureinheit der Formel (I) darstellt, und wobei die halbkreisförmige Bindung bedeutet, dass die beiden daran beteiligten Gruppen R¹ miteinander verknüpft sind und einen Ring bilden.

Besonders bevorzugte Einheiten 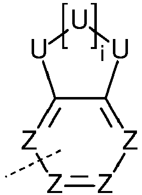 sind gewählt aus den folgenden Formeln

| | | |
|---|---|---|
| 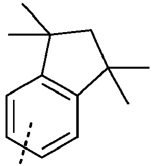 | 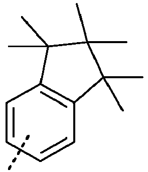 | 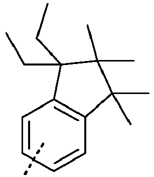 |
| a | b | c |
| 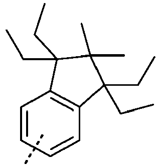 | 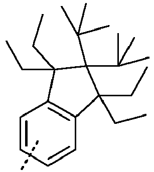 | 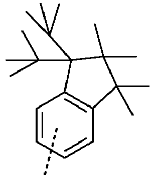 |
| d | e | f |
| 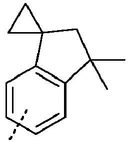 | 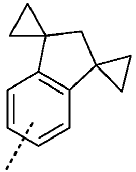 | 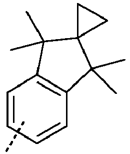 |
| g | h | i |
| 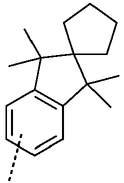 | 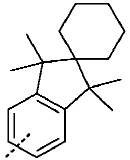 | 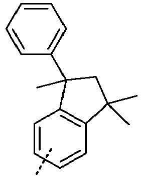 |
| j | k | l |
| 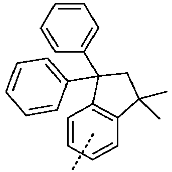 | 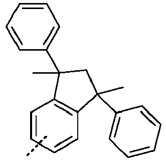 | 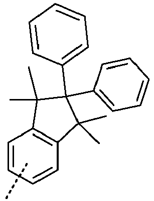 |
| m | n | o |
| 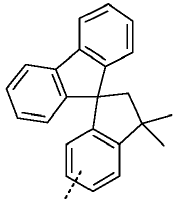 | 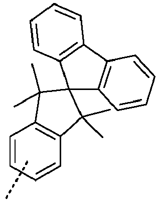 | 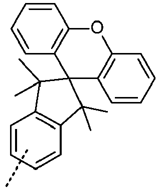 |
| p | q | r |
| 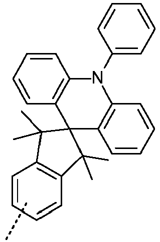 | 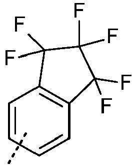 | 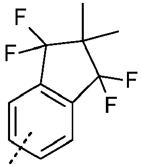 |
| s | t | u |
| 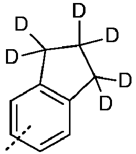 | 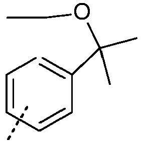 | 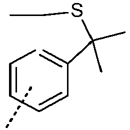 |
| v | w | X |
| 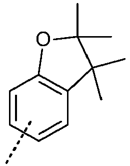 | 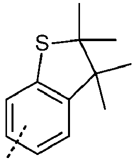 | 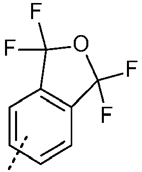 |
| y | z | aa |
| 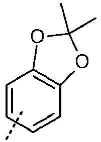 | 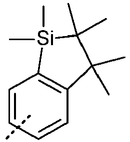 | 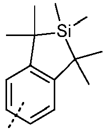 |
| ab | ac | ad |
| 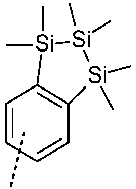 | | |
| ae | | |
| | 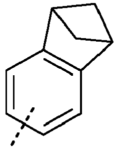 | 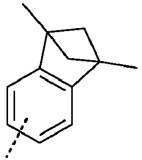 |
| | ai | ai |
| 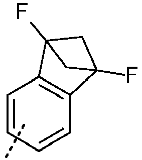 | 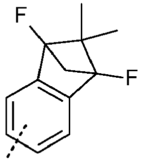 | 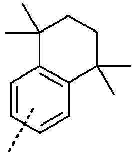 |
| ak | al | am |
| 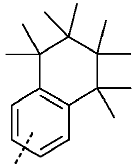 | 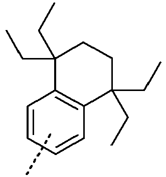 | 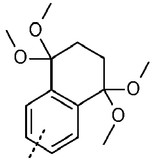 |
| an | ao | ap |
| 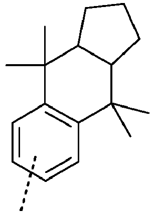 | 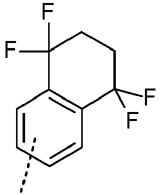 | 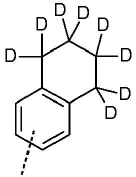 |
| aq | ar | as |
| 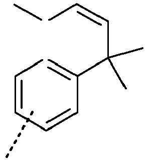 | 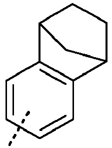 | 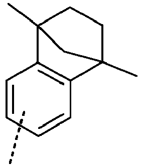 |
| at | au | av |
| 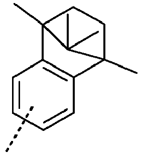 | 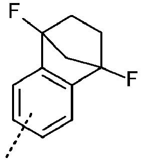 | 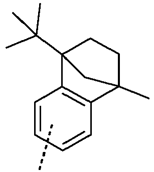 |
| aw | ax | ay |
| 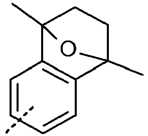 | 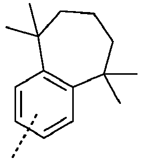 | 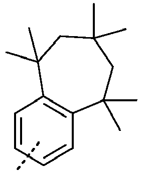 |
| az | ba | bb |
| 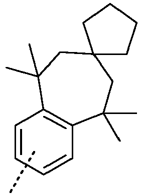 | 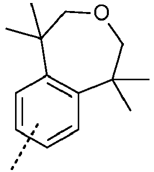 | |
| bc | bd | |

wobei die gestrichelte Linie die Bindung an den Rest der Struktureinheit der Formel (I) darstellt. Bevorzugt ist in den oben genannten Formeln die Bindung an den Rest der Struktureinheit in der Position meta und para zu den beiden Gruppen U lokalisiert, wie in der Einheit E-1 gezeigt.

Am stärksten bevorzugt unter der oben gezeigten Formeln ist Formel b.

R² ist bevorzugt bei jeden Auftreten gleich oder verschieden gewählt aus H, D, F, geradkettigen Alkylgruppen mit 1 bis 10 C-Atomen, verzweigten Alkylgruppen mit 3 bis 10 C-Atomen, aromatischen Ringsystemen mit 6 bis 20 aromatischen Ringatomen, und heteroaromatischen Ringsystemen mit 5 bis 20 aromatischen Ringatomen, wobei die genannten Alkylgruppen, aromatischen Ringsysteme und heteroaromatischen Ringsysteme jeweils mit einem oder mehreren Resten R⁴ substituiert sein können. Besonders bevorzugt ist R² bei jedem Auftreten gleich oder verschieden gewählt aus H und geradkettigen Alkylgruppen mit 1 bis 10 C-Atomen, und verzweigten Alkylgruppen mit 3 bis 10 C-Atomen.

R³ ist bevorzugt bei jeden Auftreten gleich oder verschieden gewählt aus H, D, F, geradkettigen Alkylgruppen mit 1 bis 10 C-Atomen, verzweigten Alkylgruppen mit 3 bis 10 C-Atomen, aromatischen Ringsystemen mit 6 bis 20 aromatischen Ringatomen, und heteroaromatischen Ringsystemen mit 5 bis 20 aromatischen Ringatomen, wobei die genannten Alkylgruppen, aromatischen Ringsysteme und heteroaromatischen Ringsysteme jeweils mit einem oder mehreren Resten R⁴ substituiert sein können. Besonders bevorzugt ist R³ bei jedem Auftreten gleich oder verschieden gewählt aus H und geradkettigen Alkylgruppen mit 1 bis 10 C-Atomen, und verzweigten Alkylgruppen mit 3 bis 10 C-Atomen.

R⁴ ist bevorzugt bei jedem Auftreten gleich oder verschieden gewählt aus H, D, F, CN, Si(R⁵)₃, OR⁵, geradkettigen Alkyl- und Alkoxygruppen mit 1 bis 10 C-Atomen, verzweigten Alkyl- und Alkoxygruppen mit 3 bis 10 C-Atomen, aromatischen Ringsystemen mit 6 bis 20 aromatischen Ringatomen, und heteroaromatischen Ringsystemen mit 5 bis 20 aromatischen Ringatomen, wobei zwei oder mehr Reste R⁴ miteinander verknüpft sein können und einen Ring bilden können; und wobei die genannten Alkyl- und Alkoxygruppen und die genannten aromatischen und heteroaromatischen Ringsysteme jeweils mit einem oder mehreren Resten R⁵ substituiert sein können.

Index r ist bevorzugt gleich 1 oder 2, besonders bevorzugt gleich 1.

Index s ist bevorzugt gleich 1 oder 2, besonders bevorzugt gleich 1.

Index p ist bevorzugt gleich 1.

Index q ist bevorzugt gleich 1.

Index n ist bevorzugt gleich 0.

Index m ist bevorzugt gleich 1.

Index o ist bevorzugt gleich 1.

Index i ist gleich 1 oder 2, und bevorzugt gleich 1.

Bevorzugte Ausführungsformen des Strukturelements der Formel (I) sind gewählt aus den Strukturelementen der Formeln (I-1) bis (I-6)

| |
|---|
| 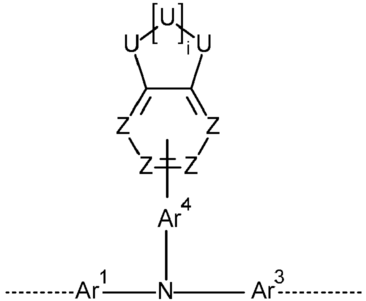 |
| (I-1) |
| 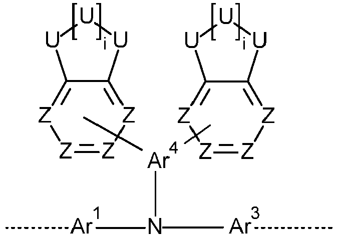 |
| (I-2) |
| 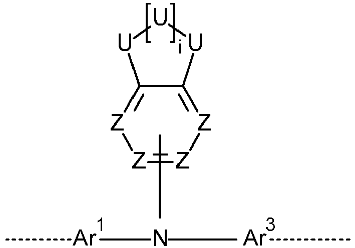 |
| (I-3) |
| 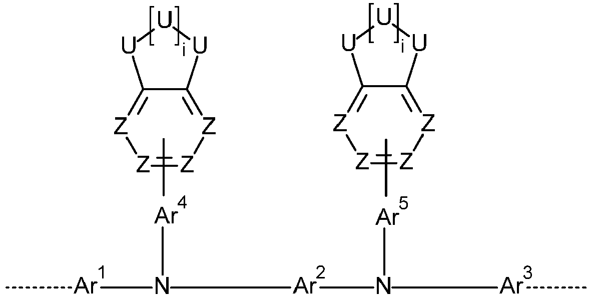 |
| (I-4) |
| 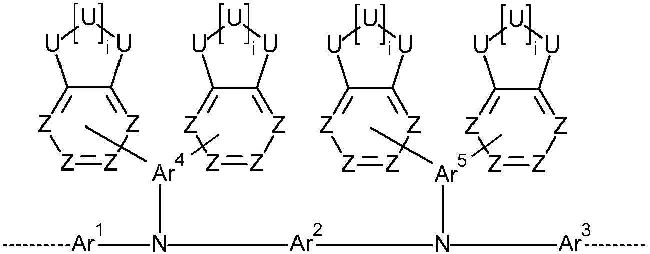 |
| (I-5) |
| 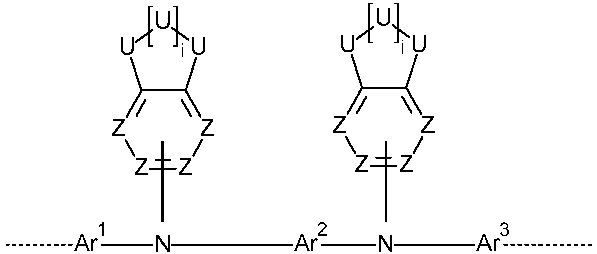 |
| (I-6) |

Unter den oben genannten Formeln ist die Formel (I-1) besonders bevorzugt.

Bevorzugte Struktureinheiten der Formel (I) sind die in der folgenden Tabelle gezeigten Struktureinheiten, in denen die in Formel (I) auftretenden Variablen Ar¹ bis Ar⁵, m, n, o, p, q, r und s wie unten gezeigt gewählt sind, und in der die Struktureinheit 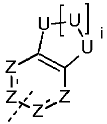 gewählt ist aus einer der unten angegebenen Formeln.

| **Struktureinheit** | **Ar¹** | **Ar²** | **Ar³** | **Ar⁴** | **Ar⁵** | 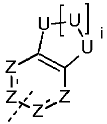 **entspricht Formel** | **m** | **n** | **o** | **p** | **q** | **r** | **s** |
|---|---|---|---|---|---|---|---|---|---|---|---|---|---|
| M1 | A1 | | A1 | | | a | 1 | 0 | 1 | 0 | 0 | 1 | 0 |
| M2 | A1 | | A1 | | | b | 1 | 0 | 1 | 0 | 0 | 1 | 0 |
| M3 | A1 | | A1 | | | c | 1 | 0 | 1 | 0 | 0 | 1 | 0 |
| M4 | A1 | | A1 | | | d | 1 | 0 | 1 | 0 | 0 | 1 | 0 |
| M5 | A1 | | A1 | | | e | 1 | 0 | 1 | 0 | 0 | 1 | 0 |
| M6 | A1 | | A1 | | | f | 1 | 0 | 1 | 0 | 0 | 1 | 0 |
| M7 | A1 | | A1 | | | q | 1 | 0 | 1 | 0 | 0 | 1 | 0 |
| M8 | A1 | | A1 | | | h | 1 | 0 | 1 | 0 | 0 | 1 | 0 |
| M9 | A1 | | A1 | | | i | 1 | 0 | 1 | 0 | 0 | 1 | 0 |
| M10 | A1 | | A1 | | | j | 1 | 0 | 1 | 0 | 0 | 1 | 0 |
| M11 | A1 | | A1 | | | k | 1 | 0 | 1 | 0 | 0 | 1 | 0 |
| M12 | A1 | | A1 | | | l | 1 | 0 | 1 | 0 | 0 | 1 | 0 |
| M13 | A1 | | A1 | | | m | 1 | 0 | 1 | 0 | 0 | 1 | 0 |
| M14 | A1 | | A1 | | | n | 1 | 0 | 1 | 0 | 0 | 1 | 0 |
| M15 | A1 | | A1 | | | o | 1 | 0 | 1 | 0 | 0 | 1 | 0 |
| M16 | A1 | | A1 | | | p | 1 | 0 | 1 | 0 | 0 | 1 | 0 |
| M17 | A1 | | A1 | | | q | 1 | 0 | 1 | 0 | 0 | 1 | 0 |
| M18 | A1 | | A1 | | | r | 1 | 0 | 1 | 0 | 0 | 1 | 0 |
| M19 | A1 | | A1 | | | s | 1 | 0 | 1 | 0 | 0 | 1 | 0 |
| M20 | A1 | | A1 | | | t | 1 | 0 | 1 | 0 | 0 | 1 | 0 |
| M21 | A1 | | A1 | | | u | 1 | 0 | 1 | 0 | 0 | 1 | 0 |
| M22 | A1 | | A1 | | | v | 1 | 0 | 1 | 0 | 0 | 1 | 0 |
| M23 | A1 | | A1 | | | w | 1 | 0 | 1 | 0 | 0 | 1 | 0 |
| M24 | A1 | | A1 | | | x | 1 | 0 | 1 | 0 | 0 | 1 | 0 |
| M25 | A1 | | A1 | | | y | 1 | 0 | 1 | 0 | 0 | 1 | 0 |
| M26 | A1 | | A1 | | | Z | 1 | 0 | 1 | 0 | 0 | 1 | 0 |
| M27 | A1 | | A1 | | | aa | 1 | 0 | 1 | 0 | 0 | 1 | 0 |
| M28 | A1 | | A1 | | | ab | 1 | 0 | 1 | 0 | 0 | 1 | 0 |
| M29 | A1 | | A1 | | | ac | 1 | 0 | 1 | 0 | 0 | 1 | 0 |
| M30 | A1 | | A1 | | | ad | 1 | 0 | 1 | 0 | 0 | 1 | 0 |
| M31 | A1 | | A1 | | | ae | 1 | 0 | 1 | 0 | 0 | 1 | 0 |
| M32 | | | | | | | | | | | | | |
| M33 | | | | | | | | | | | | | |
| M34 | | | | | | | | | | | | | |
| M35 | A1 | | A1 | | | ai | 1 | 0 | 1 | 0 | 0 | 1 | 0 |
| M36 | A1 | | A1 | | | ai | 1 | 0 | 1 | 0 | 0 | 1 | 0 |
| M37 | A1 | | A1 | | | ak | 1 | 0 | 1 | 0 | 0 | 1 | 0 |
| M38 | A1 | | A1 | | | al | 1 | 0 | 1 | 0 | 0 | 1 | 0 |
| M39 | A1 | | A1 | | | am | 1 | 0 | 1 | 0 | 0 | 1 | 0 |
| M40 | A1 | | A1 | | | an | 1 | 0 | 1 | 0 | 0 | 1 | 0 |
| M41 | A1 | | A1 | | | ao | 1 | 0 | 1 | 0 | 0 | 1 | 0 |
| M42 | A1 | | A1 | | | ap | 1 | 0 | 1 | 0 | 0 | 1 | 0 |
| M43 | A1 | | A1 | | | aq | 1 | 0 | 1 | 0 | 0 | 1 | 0 |
| M44 | A1 | | A1 | | | ar | 1 | 0 | 1 | 0 | 0 | 1 | 0 |
| M45 | A1 | | A1 | | | as | 1 | 0 | 1 | 0 | 0 | 1 | 0 |
| M46 | A1 | | A1 | | | at | 1 | 0 | 1 | 0 | 0 | 1 | 0 |
| M47 | A1 | | A1 | | | au | 1 | 0 | 1 | 0 | 0 | 1 | 0 |
| M48 | A1 | | A1 | | | av | 1 | 0 | 1 | 0 | 0 | 1 | 0 |
| M49 | A1 | | A1 | | | aw | 1 | 0 | 1 | 0 | 0 | 1 | 0 |
| M50 | A1 | | A1 | | | ax | 1 | 0 | 1 | 0 | 0 | 1 | 0 |
| M51 | A1 | | A1 | | | ay | 1 | 0 | 1 | 0 | 0 | 1 | 0 |
| M52 | A1 | | A1 | | | Z | 1 | 0 | 1 | 0 | 0 | 1 | 0 |
| M53 | A1 | | A1 | | | ba | 1 | 0 | 1 | 0 | 0 | 1 | 0 |
| M54 | A1 | | A1 | | | bb | 1 | 0 | 1 | 0 | 0 | 1 | 0 |
| M55 | A1 | | A1 | | | ba | 1 | 0 | 1 | 0 | 0 | 1 | 0 |
| M56 | A1 | | A1 | | | bd | 1 | 0 | 1 | 0 | 0 | 1 | 0 |
| M57 | A2 | | A2 | | | a | 1 | 0 | 1 | 0 | 0 | 1 | 0 |
| M58 | A2 | | A2 | | | b | 1 | 0 | 1 | 0 | 0 | 1 | 0 |
| M59 | A2 | | A2 | | | c | 1 | 0 | 1 | 0 | 0 | 1 | 0 |
| M60 | A2 | | A2 | | | d | 1 | 0 | 1 | 0 | 0 | 1 | 0 |
| M61 | A2 | | A2 | | | e | 1 | 0 | 1 | 0 | 0 | 1 | 0 |
| M62 | A2 | | A2 | | | f | 1 | 0 | 1 | 0 | 0 | 1 | 0 |
| M63 | A2 | | A2 | | | q | 1 | 0 | 1 | 0 | 0 | 1 | 0 |
| M64 | A2 | | A2 | | | h | 1 | 0 | 1 | 0 | 0 | 1 | 0 |
| M65 | A2 | | A2 | | | i | 1 | 0 | 1 | 0 | 0 | 1 | 0 |
| M66 | A2 | | A2 | | | j | 1 | 0 | 1 | 0 | 0 | 1 | 0 |
| M67 | A2 | | A2 | | | k | 1 | 0 | 1 | 0 | 0 | 1 | 0 |
| M68 | A2 | | A2 | | | l | 1 | 0 | 1 | 0 | 0 | 1 | 0 |
| M69 | A2 | | A2 | | | m | 1 | 0 | 1 | 0 | 0 | 1 | 0 |
| M70 | A2 | | A2 | | | n | 1 | 0 | 1 | 0 | 0 | 1 | 0 |
| M71 | A2 | | A2 | | | o | 1 | 0 | 1 | 0 | 0 | 1 | 0 |
| M72 | A2 | | A2 | | | p | 1 | 0 | 1 | 0 | 0 | 1 | 0 |
| M73 | A2 | | A2 | | | q | 1 | 0 | 1 | 0 | 0 | 1 | 0 |
| M74 | A2 | | A2 | | | r | 1 | 0 | 1 | 0 | 0 | 1 | 0 |
| M75 | A2 | | A2 | | | s | 1 | 0 | 1 | 0 | 0 | 1 | 0 |
| M76 | A2 | | A2 | | | t | 1 | 0 | 1 | 0 | 0 | 1 | 0 |
| M77 | A2 | | A2 | | | u | 1 | 0 | 1 | 0 | 0 | 1 | 0 |
| M78 | A2 | | A2 | | | v | 1 | 0 | 1 | 0 | 0 | 1 | 0 |
| M79 | A2 | | A2 | | | w | 1 | 0 | 1 | 0 | 0 | 1 | 0 |
| M80 | A2 | | A2 | | | x | 1 | 0 | 1 | 0 | 0 | 1 | 0 |
| M81 | A2 | | A2 | | | y | 1 | 0 | 1 | 0 | 0 | 1 | 0 |
| M82 | A2 | | A2 | | | Z | 1 | 0 | 1 | 0 | 0 | 1 | 0 |
| M83 | A2 | | A2 | | | aa | 1 | 0 | 1 | 0 | 0 | 1 | 0 |
| M84 | A2 | | A2 | | | ab | 1 | 0 | 1 | 0 | 0 | 1 | 0 |
| M85 | A2 | | A2 | | | ac | 1 | 0 | 1 | 0 | 0 | 1 | 0 |
| M86 | A2 | | A2 | | | ad | 1 | 0 | 1 | 0 | 0 | 1 | 0 |
| M87 | A2 | | A2 | | | ae | 1 | 0 | 1 | 0 | 0 | 1 | 0 |
| M88 | | | | | | | | | | | | | |
| M89 | | | | | | | | | | | | | |
| M90 | | | | | | | | | | | | | |
| M91 | A2 | | A2 | | | ai | 1 | 0 | 1 | 0 | 0 | 1 | 0 |
| M92 | A2 | | A2 | | | ai | 1 | 0 | 1 | 0 | 0 | 1 | 0 |
| M93 | A2 | | A2 | | | ak | 1 | 0 | 1 | 0 | 0 | 1 | 0 |
| M94 | A2 | | A2 | | | al | 1 | 0 | 1 | 0 | 0 | 1 | 0 |
| M95 | A2 | | A2 | | | am | 1 | 0 | 1 | 0 | 0 | 1 | 0 |
| M96 | A2 | | A2 | | | an | 1 | 0 | 1 | 0 | 0 | 1 | 0 |
| M97 | A2 | | A2 | | | ao | 1 | 0 | 1 | 0 | 0 | 1 | 0 |
| M98 | A2 | | A2 | | | ap | 1 | 0 | 1 | 0 | 0 | 1 | 0 |
| M99 | A2 | | A2 | | | aq | 1 | 0 | 1 | 0 | 0 | 1 | 0 |
| M100 | A2 | | A2 | | | ar | 1 | 0 | 1 | 0 | 0 | 1 | 0 |
| M101 | A2 | | A2 | | | as | 1 | 0 | 1 | 0 | 0 | 1 | 0 |
| M102 | A2 | | A2 | | | at | 1 | 0 | 1 | 0 | 0 | 1 | 0 |
| M103 | A2 | | A2 | | | au | 1 | 0 | 1 | 0 | 0 | 1 | 0 |
| M104 | A2 | | A2 | | | av | 1 | 0 | 1 | 0 | 0 | 1 | 0 |
| M105 | A2 | | A2 | | | aw | 1 | 0 | 1 | 0 | 0 | 1 | 0 |
| M106 | A2 | | A2 | | | ax | 1 | 0 | 1 | 0 | 0 | 1 | 0 |
| M107 | A2 | | A2 | | | ay | 1 | 0 | 1 | 0 | 0 | 1 | 0 |
| M108 | A2 | | A2 | | | Z | 1 | 0 | 1 | 0 | 0 | 1 | 0 |
| M109 | A2 | | A2 | | | ba | 1 | 0 | 1 | 0 | 0 | 1 | 0 |
| M110 | A2 | | A2 | | | bb | 1 | 0 | 1 | 0 | 0 | 1 | 0 |
| M111 | A2 | | A2 | | | ba | 1 | 0 | 1 | 0 | 0 | 1 | 0 |
| M112 | A2 | | A2 | | | bd | 1 | 0 | 1 | 0 | 0 | 1 | 0 |
| M113 | A3 | | A3 | | | a | 1 | 0 | 1 | 0 | 0 | 1 | 0 |
| M114 | A3 | | A3 | | | b | 1 | 0 | 1 | 0 | 0 | 1 | 0 |
| M115 | A3 | | A3 | | | c | 1 | 0 | 1 | 0 | 0 | 1 | 0 |
| M116 | A3 | | A3 | | | d | 1 | 0 | 1 | 0 | 0 | 1 | 0 |
| M117 | A3 | | A3 | | | e | 1 | 0 | 1 | 0 | 0 | 1 | 0 |
| M118 | A3 | | A3 | | | f | 1 | 0 | 1 | 0 | 0 | 1 | 0 |
| M119 | A3 | | A3 | | | q | 1 | 0 | 1 | 0 | 0 | 1 | 0 |
| M120 | A3 | | A3 | | | h | 1 | 0 | 1 | 0 | 0 | 1 | 0 |
| M121 | A3 | | A3 | | | i | 1 | 0 | 1 | 0 | 0 | 1 | 0 |
| M122 | A3 | | A3 | | | j | 1 | 0 | 1 | 0 | 0 | 1 | 0 |
| M123 | A3 | | A3 | | | k | 1 | 0 | 1 | 0 | 0 | 1 | 0 |
| M124 | A3 | | A3 | | | l | 1 | 0 | 1 | 0 | 0 | 1 | 0 |
| M125 | A3 | | A3 | | | m | 1 | 0 | 1 | 0 | 0 | 1 | 0 |
| M126 | A3 | | A3 | | | n | 1 | 0 | 1 | 0 | 0 | 1 | 0 |
| M127 | A3 | | A3 | | | o | 1 | 0 | 1 | 0 | 0 | 1 | 0 |
| M128 | A3 | | A3 | | | p | 1 | 0 | 1 | 0 | 0 | 1 | 0 |
| M129 | A3 | | A3 | | | q | 1 | 0 | 1 | 0 | 0 | 1 | 0 |
| M130 | A3 | | A3 | | | r | 1 | 0 | 1 | 0 | 0 | 1 | 0 |
| M131 | A3 | | A3 | | | s | 1 | 0 | 1 | 0 | 0 | 1 | 0 |
| M132 | A3 | | A3 | | | t | 1 | 0 | 1 | 0 | 0 | 1 | 0 |
| M133 | A3 | | A3 | | | u | 1 | 0 | 1 | 0 | 0 | 1 | 0 |
| M134 | A3 | | A3 | | | v | 1 | 0 | 1 | 0 | 0 | 1 | 0 |
| M135 | A3 | | A3 | | | w | 1 | 0 | 1 | 0 | 0 | 1 | 0 |
| M136 | A3 | | A3 | | | x | 1 | 0 | 1 | 0 | 0 | 1 | 0 |
| M137 | A3 | | A3 | | | y | 1 | 0 | 1 | 0 | 0 | 1 | 0 |
| M138 | A3 | | A3 | | | Z | 1 | 0 | 1 | 0 | 0 | 1 | 0 |
| M139 | A3 | | A3 | | | aa | 1 | 0 | 1 | 0 | 0 | 1 | 0 |
| M140 | A3 | | A3 | | | ab | 1 | 0 | 1 | 0 | 0 | 1 | 0 |
| M141 | A3 | | A3 | | | ac | 1 | 0 | 1 | 0 | 0 | 1 | 0 |
| M142 | A3 | | A3 | | | ad | 1 | 0 | 1 | 0 | 0 | 1 | 0 |
| M143 | A3 | | A3 | | | ae | 1 | 0 | 1 | 0 | 0 | 1 | 0 |
| M144 | | | | | | | | | | | | | |
| M145 | | | | | | | | | | | | | |
| M146 | | | | | | | | | | | | | |
| M147 | A3 | | A3 | | | ai | 1 | 0 | 1 | 0 | 0 | 1 | 0 |
| M148 | A3 | | A3 | | | ai | 1 | 0 | 1 | 0 | 0 | 1 | 0 |
| M149 | A3 | | A3 | | | ak | 1 | 0 | 1 | 0 | 0 | 1 | 0 |
| M150 | A3 | | A3 | | | al | 1 | 0 | 1 | 0 | 0 | 1 | 0 |
| M151 | A3 | | A3 | | | am | 1 | 0 | 1 | 0 | 0 | 1 | 0 |
| M152 | A3 | | A3 | | | an | 1 | 0 | 1 | 0 | 0 | 1 | 0 |
| M153 | A3 | | A3 | | | ao | 1 | 0 | 1 | 0 | 0 | 1 | 0 |
| M154 | A3 | | A3 | | | ap | 1 | 0 | 1 | 0 | 0 | 1 | 0 |
| M155 | A3 | | A3 | | | aq | 1 | 0 | 1 | 0 | 0 | 1 | 0 |
| M156 | A3 | | A3 | | | ar | 1 | 0 | 1 | 0 | 0 | 1 | 0 |
| M157 | A3 | | A3 | | | as | 1 | 0 | 1 | 0 | 0 | 1 | 0 |
| M158 | A3 | | A3 | | | at | 1 | 0 | 1 | 0 | 0 | 1 | 0 |
| M159 | A3 | | A3 | | | au | 1 | 0 | 1 | 0 | 0 | 1 | 0 |
| M160 | A3 | | A3 | | | av | 1 | 0 | 1 | 0 | 0 | 1 | 0 |
| M161 | A3 | | A3 | | | aw | 1 | 0 | 1 | 0 | 0 | 1 | 0 |
| M162 | A3 | | A3 | | | ax | 1 | 0 | 1 | 0 | 0 | 1 | 0 |
| M163 | A3 | | A3 | | | ay | 1 | 0 | 1 | 0 | 0 | 1 | 0 |
| M164 | A3 | | A3 | | | Z | 1 | 0 | 1 | 0 | 0 | 1 | 0 |
| M165 | A3 | | A3 | | | ba | 1 | 0 | 1 | 0 | 0 | 1 | 0 |
| M166 | A3 | | A3 | | | bb | 1 | 0 | 1 | 0 | 0 | 1 | 0 |
| M167 | A3 | | A3 | | | ba | 1 | 0 | 1 | 0 | 0 | 1 | 0 |
| M168 | A3 | | A3 | | | bd | 1 | 0 | 1 | 0 | 0 | 1 | 0 |
| M169 | A4 | | A4 | | | a | 1 | 0 | 1 | 0 | 0 | 1 | 0 |
| M170 | A4 | | A4 | | | b | 1 | 0 | 1 | 0 | 0 | 1 | 0 |
| M171 | A4 | | A4 | | | c | 1 | 0 | 1 | 0 | 0 | 1 | 0 |
| M172 | A4 | | A4 | | | d | 1 | 0 | 1 | 0 | 0 | 1 | 0 |
| M173 | A4 | | A4 | | | e | 1 | 0 | 1 | 0 | 0 | 1 | 0 |
| M174 | A4 | | A4 | | | f | 1 | 0 | 1 | 0 | 0 | 1 | 0 |
| M175 | A4 | | A4 | | | q | 1 | 0 | 1 | 0 | 0 | 1 | 0 |
| M176 | A4 | | A4 | | | h | 1 | 0 | 1 | 0 | 0 | 1 | 0 |
| M177 | A4 | | A4 | | | i | 1 | 0 | 1 | 0 | 0 | 1 | 0 |
| M178 | A4 | | A4 | | | j | 1 | 0 | 1 | 0 | 0 | 1 | 0 |
| M179 | A4 | | A4 | | | k | 1 | 0 | 1 | 0 | 0 | 1 | 0 |
| M180 | A4 | | A4 | | | l | 1 | 0 | 1 | 0 | 0 | 1 | 0 |
| M181 | A4 | | A4 | | | m | 1 | 0 | 1 | 0 | 0 | 1 | 0 |
| M182 | A4 | | A4 | | | n | 1 | 0 | 1 | 0 | 0 | 1 | 0 |
| M183 | A4 | | A4 | | | o | 1 | 0 | 1 | 0 | 0 | 1 | 0 |
| M184 | A4 | | A4 | | | p | 1 | 0 | 1 | 0 | 0 | 1 | 0 |
| M185 | A4 | | A4 | | | q | 1 | 0 | 1 | 0 | 0 | 1 | 0 |
| M186 | A4 | | A4 | | | r | 1 | 0 | 1 | 0 | 0 | 1 | 0 |
| M187 | A4 | | A4 | | | s | 1 | 0 | 1 | 0 | 0 | 1 | 0 |
| M188 | A4 | | A4 | | | t | 1 | 0 | 1 | 0 | 0 | 1 | 0 |
| M189 | A4 | | A4 | | | u | 1 | 0 | 1 | 0 | 0 | 1 | 0 |
| M190 | A4 | | A4 | | | v | 1 | 0 | 1 | 0 | 0 | 1 | 0 |
| M191 | A4 | | A4 | | | w | 1 | 0 | 1 | 0 | 0 | 1 | 0 |
| M192 | A4 | | A4 | | | x | 1 | 0 | 1 | 0 | 0 | 1 | 0 |
| M193 | A4 | | A4 | | | y | 1 | 0 | 1 | 0 | 0 | 1 | 0 |
| M194 | A4 | | A4 | | | Z | 1 | 0 | 1 | 0 | 0 | 1 | 0 |
| M195 | A4 | | A4 | | | aa | 1 | 0 | 1 | 0 | 0 | 1 | 0 |
| M196 | A4 | | A4 | | | ab | 1 | 0 | 1 | 0 | 0 | 1 | 0 |
| M197 | A4 | | A4 | | | ac | 1 | 0 | 1 | 0 | 0 | 1 | 0 |
| M198 | A4 | | A4 | | | ad | 1 | 0 | 1 | 0 | 0 | 1 | 0 |
| M199 | A4 | | A4 | | | ae | 1 | 0 | 1 | 0 | 0 | 1 | 0 |
| M200 | | | | | | | | | | | | | |
| M201 | | | | | | | | | | | | | |
| M202 | | | | | | | | | | | | | |
| M203 | A4 | | A4 | | | ai | 1 | 0 | 1 | 0 | 0 | 1 | 0 |
| M204 | A4 | | A4 | | | ai | 1 | 0 | 1 | 0 | 0 | 1 | 0 |
| M205 | A4 | | A4 | | | ak | 1 | 0 | 1 | 0 | 0 | 1 | 0 |
| M206 | A4 | | A4 | | | al | 1 | 0 | 1 | 0 | 0 | 1 | 0 |
| M207 | A4 | | A4 | | | am | 1 | 0 | 1 | 0 | 0 | 1 | 0 |
| M208 | A4 | | A4 | | | an | 1 | 0 | 1 | 0 | 0 | 1 | 0 |
| M209 | A4 | | A4 | | | ao | 1 | 0 | 1 | 0 | 0 | 1 | 0 |
| M210 | A4 | | A4 | | | ap | 1 | 0 | 1 | 0 | 0 | 1 | 0 |
| M211 | A4 | | A4 | | | aq | 1 | 0 | 1 | 0 | 0 | 1 | 0 |
| M212 | A4 | | A4 | | | ar | 1 | 0 | 1 | 0 | 0 | 1 | 0 |
| M213 | A4 | | A4 | | | as | 1 | 0 | 1 | 0 | 0 | 1 | 0 |
| M214 | A4 | | A4 | | | at | 1 | 0 | 1 | 0 | 0 | 1 | 0 |
| M215 | A4 | | A4 | | | au | 1 | 0 | 1 | 0 | 0 | 1 | 0 |
| M216 | A4 | | A4 | | | av | 1 | 0 | 1 | 0 | 0 | 1 | 0 |
| M217 | A4 | | A4 | | | aw | 1 | 0 | 1 | 0 | 0 | 1 | 0 |
| M218 | A4 | | A4 | | | ax | 1 | 0 | 1 | 0 | 0 | 1 | 0 |
| M219 | A4 | | A4 | | | ay | 1 | 0 | 1 | 0 | 0 | 1 | 0 |
| M220 | A4 | | A4 | | | Z | 1 | 0 | 1 | 0 | 0 | 1 | 0 |
| M221 | A4 | | A4 | | | ba | 1 | 0 | 1 | 0 | 0 | 1 | 0 |
| M222 | A4 | | A4 | | | bb | 1 | 0 | 1 | 0 | 0 | 1 | 0 |
| M223 | A4 | | A4 | | | ba | 1 | 0 | 1 | 0 | 0 | 1 | 0 |
| M224 | A4 | | A4 | | | bd | 1 | 0 | 1 | 0 | 0 | 1 | 0 |
| M225 | A5 | | A5 | | | a | 1 | 0 | 1 | 0 | 0 | 1 | 0 |
| M226 | A5 | | A5 | | | b | 1 | 0 | 1 | 0 | 0 | 1 | 0 |
| M227 | A5 | | A5 | | | c | 1 | 0 | 1 | 0 | 0 | 1 | 0 |
| M228 | A5 | | A5 | | | d | 1 | 0 | 1 | 0 | 0 | 1 | 0 |
| M229 | A5 | | A5 | | | e | 1 | 0 | 1 | 0 | 0 | 1 | 0 |
| M230 | A5 | | A5 | | | f | 1 | 0 | 1 | 0 | 0 | 1 | 0 |
| M231 | A5 | | A5 | | | q | 1 | 0 | 1 | 0 | 0 | 1 | 0 |
| M232 | A5 | | A5 | | | h | 1 | 0 | 1 | 0 | 0 | 1 | 0 |
| M233 | A5 | | A5 | | | i | 1 | 0 | 1 | 0 | 0 | 1 | 0 |
| M234 | A5 | | A5 | | | i | 1 | 0 | 1 | 0 | 0 | 1 | 0 |
| M235 | A5 | | A5 | | | k | 1 | 0 | 1 | 0 | 0 | 1 | 0 |
| M236 | A5 | | A5 | | | l | 1 | 0 | 1 | 0 | 0 | 1 | 0 |
| M237 | A5 | | A5 | | | m | 1 | 0 | 1 | 0 | 0 | 1 | 0 |
| M238 | A5 | | A5 | | | n | 1 | 0 | 1 | 0 | 0 | 1 | 0 |
| M239 | A5 | | A5 | | | o | 1 | 0 | 1 | 0 | 0 | 1 | 0 |
| M240 | A5 | | A5 | | | p | 1 | 0 | 1 | 0 | 0 | 1 | 0 |
| M241 | A5 | | A5 | | | q | 1 | 0 | 1 | 0 | 0 | 1 | 0 |
| M242 | A5 | | A5 | | | r | 1 | 0 | 1 | 0 | 0 | 1 | 0 |
| M243 | A5 | | A5 | | | s | 1 | 0 | 1 | 0 | 0 | 1 | 0 |
| M244 | A5 | | A5 | | | t | 1 | 0 | 1 | 0 | 0 | 1 | 0 |
| M245 | A5 | | A5 | | | u | 1 | 0 | 1 | 0 | 0 | 1 | 0 |
| M246 | A5 | | A5 | | | v | 1 | 0 | 1 | 0 | 0 | 1 | 0 |
| M247 | A5 | | A5 | | | w | 1 | 0 | 1 | 0 | 0 | 1 | 0 |
| M248 | A5 | | A5 | | | x | 1 | 0 | 1 | 0 | 0 | 1 | 0 |
| M249 | A5 | | A5 | | | y | 1 | 0 | 1 | 0 | 0 | 1 | 0 |
| M250 | A5 | | A5 | | | Z | 1 | 0 | 1 | 0 | 0 | 1 | 0 |
| M251 | A5 | | A5 | | | aa | 1 | 0 | 1 | 0 | 0 | 1 | 0 |
| M252 | A5 | | A5 | | | ab | 1 | 0 | 1 | 0 | 0 | 1 | 0 |
| M253 | A5 | | A5 | | | ac | 1 | 0 | 1 | 0 | 0 | 1 | 0 |
| M254 | A5 | | A5 | | | ad | 1 | 0 | 1 | 0 | 0 | 1 | 0 |
| M255 | A5 | | A5 | | | ae | 1 | 0 | 1 | 0 | 0 | 1 | 0 |
| M256 | | | | | | | | | | | | | |
| M257 | | | | | | | | | | | | | |
| M258 | | | | | | | | | | | | | |
| M259 | A5 | | A5 | | | ai | 1 | 0 | 1 | 0 | 0 | 1 | 0 |
| M260 | A5 | | A5 | | | ai | 1 | 0 | 1 | 0 | 0 | 1 | 0 |
| M261 | A5 | | A5 | | | ak | 1 | 0 | 1 | 0 | 0 | 1 | 0 |
| M262 | A5 | | A5 | | | al | 1 | 0 | 1 | 0 | 0 | 1 | 0 |
| M263 | A5 | | A5 | | | am | 1 | 0 | 1 | 0 | 0 | 1 | 0 |
| M264 | A5 | | A5 | | | an | 1 | 0 | 1 | 0 | 0 | 1 | 0 |
| M265 | A5 | | A5 | | | ao | 1 | 0 | 1 | 0 | 0 | 1 | 0 |
| M266 | A5 | | A5 | | | ap | 1 | 0 | 1 | 0 | 0 | 1 | 0 |
| M267 | A5 | | A5 | | | aq | 1 | 0 | 1 | 0 | 0 | 1 | 0 |
| M268 | A5 | | A5 | | | ar | 1 | 0 | 1 | 0 | 0 | 1 | 0 |
| M269 | A5 | | A5 | | | as | 1 | 0 | 1 | 0 | 0 | 1 | 0 |
| M270 | A5 | | A5 | | | at | 1 | 0 | 1 | 0 | 0 | 1 | 0 |
| M271 | A5 | | A5 | | | au | 1 | 0 | 1 | 0 | 0 | 1 | 0 |
| M272 | A5 | | A5 | | | av | 1 | 0 | 1 | 0 | 0 | 1 | 0 |
| M273 | A5 | | A5 | | | aw | 1 | 0 | 1 | 0 | 0 | 1 | 0 |
| M274 | A5 | | A5 | | | ax | 1 | 0 | 1 | 0 | 0 | 1 | 0 |
| M275 | A5 | | A5 | | | ay | 1 | 0 | 1 | 0 | 0 | 1 | 0 |
| M276 | A5 | | A5 | | | Z | 1 | 0 | 1 | 0 | 0 | 1 | 0 |
| M277 | A5 | | A5 | | | ba | 1 | 0 | 1 | 0 | 0 | 1 | 0 |
| M278 | A5 | | A5 | | | bb | 1 | 0 | 1 | 0 | 0 | 1 | 0 |
| M279 | A5 | | A5 | | | ba | 1 | 0 | 1 | 0 | 0 | 1 | 0 |
| M280 | A5 | | A5 | | | bd | 1 | 0 | 1 | 0 | 0 | 1 | 0 |
| M281 | A6 | | A6 | | | a | 1 | 0 | 1 | 0 | 0 | 1 | 0 |
| M282 | A6 | | A6 | | | b | 1 | 0 | 1 | 0 | 0 | 1 | 0 |
| M283 | A6 | | A6 | | | c | 1 | 0 | 1 | 0 | 0 | 1 | 0 |
| M284 | A6 | | A6 | | | d | 1 | 0 | 1 | 0 | 0 | 1 | 0 |
| M285 | A6 | | A6 | | | e | 1 | 0 | 1 | 0 | 0 | 1 | 0 |
| M286 | A6 | | A6 | | | f | 1 | 0 | 1 | 0 | 0 | 1 | 0 |
| M287 | A6 | | A6 | | | q | 1 | 0 | 1 | 0 | 0 | 1 | 0 |
| M288 | A6 | | A6 | | | h | 1 | 0 | 1 | 0 | 0 | 1 | 0 |
| M289 | A6 | | A6 | | | i | 1 | 0 | 1 | 0 | 0 | 1 | 0 |
| M290 | A6 | | A6 | | | i | 1 | 0 | 1 | 0 | 0 | 1 | 0 |
| M291 | A6 | | A6 | | | k | 1 | 0 | 1 | 0 | 0 | 1 | 0 |
| M292 | A6 | | A6 | | | l | 1 | 0 | 1 | 0 | 0 | 1 | 0 |
| M293 | A6 | | A6 | | | m | 1 | 0 | 1 | 0 | 0 | 1 | 0 |
| M294 | A6 | | A6 | | | n | 1 | 0 | 1 | 0 | 0 | 1 | 0 |
| M295 | A6 | | A6 | | | o | 1 | 0 | 1 | 0 | 0 | 1 | 0 |
| M296 | A6 | | A6 | | | p | 1 | 0 | 1 | 0 | 0 | 1 | 0 |
| M297 | A6 | | A6 | | | q | 1 | 0 | 1 | 0 | 0 | 1 | 0 |
| M298 | A6 | | A6 | | | r | 1 | 0 | 1 | 0 | 0 | 1 | 0 |
| M299 | A6 | | A6 | | | s | 1 | 0 | 1 | 0 | 0 | 1 | 0 |
| M300 | A6 | | A6 | | | t | 1 | 0 | 1 | 0 | 0 | 1 | 0 |
| M301 | A6 | | A6 | | | u | 1 | 0 | 1 | 0 | 0 | 1 | 0 |
| M302 | A6 | | A6 | | | v | 1 | 0 | 1 | 0 | 0 | 1 | 0 |
| M303 | A6 | | A6 | | | w | 1 | 0 | 1 | 0 | 0 | 1 | 0 |
| M304 | A6 | | A6 | | | x | 1 | 0 | 1 | 0 | 0 | 1 | 0 |
| M305 | A6 | | A6 | | | y | 1 | 0 | 1 | 0 | 0 | 1 | 0 |
| M306 | A6 | | A6 | | | Z | 1 | 0 | 1 | 0 | 0 | 1 | 0 |
| M307 | A6 | | A6 | | | aa | 1 | 0 | 1 | 0 | 0 | 1 | 0 |
| M308 | A6 | | A6 | | | ab | 1 | 0 | 1 | 0 | 0 | 1 | 0 |
| M309 | A6 | | A6 | | | ac | 1 | 0 | 1 | 0 | 0 | 1 | 0 |
| M310 | A6 | | A6 | | | ad | 1 | 0 | 1 | 0 | 0 | 1 | 0 |
| M311 | A6 | | A6 | | | ae | 1 | 0 | 1 | 0 | 0 | 1 | 0 |
| M312 | | | | | | | | | | | | | |
| M313 | | | | | | | | | | | | | |
| M314 | | | | | | | | | | | | | |
| M315 | A6 | | A6 | | | ai | 1 | 0 | 1 | 0 | 0 | 1 | 0 |
| M316 | A6 | | A6 | | | ai | 1 | 0 | 1 | 0 | 0 | 1 | 0 |
| M317 | A6 | | A6 | | | ak | 1 | 0 | 1 | 0 | 0 | 1 | 0 |
| M318 | A6 | | A6 | | | al | 1 | 0 | 1 | 0 | 0 | 1 | 0 |
| M319 | A6 | | A6 | | | am | 1 | 0 | 1 | 0 | 0 | 1 | 0 |
| M320 | A6 | | A6 | | | an | 1 | 0 | 1 | 0 | 0 | 1 | 0 |
| M321 | A6 | | A6 | | | ao | 1 | 0 | 1 | 0 | 0 | 1 | 0 |
| M322 | A6 | | A6 | | | ap | 1 | 0 | 1 | 0 | 0 | 1 | 0 |
| M323 | A6 | | A6 | | | aq | 1 | 0 | 1 | 0 | 0 | 1 | 0 |
| M324 | A6 | | A6 | | | ar | 1 | 0 | 1 | 0 | 0 | 1 | 0 |
| M325 | A6 | | A6 | | | as | 1 | 0 | 1 | 0 | 0 | 1 | 0 |
| M326 | A6 | | A6 | | | at | 1 | 0 | 1 | 0 | 0 | 1 | 0 |
| M327 | A6 | | A6 | | | au | 1 | 0 | 1 | 0 | 0 | 1 | 0 |
| M328 | A6 | | A6 | | | av | 1 | 0 | 1 | 0 | 0 | 1 | 0 |
| M329 | A6 | | A6 | | | aw | 1 | 0 | 1 | 0 | 0 | 1 | 0 |
| M330 | A6 | | A6 | | | ax | 1 | 0 | 1 | 0 | 0 | 1 | 0 |
| M331 | A6 | | A6 | | | ay | 1 | 0 | 1 | 0 | 0 | 1 | 0 |
| M332 | A6 | | A6 | | | Z | 1 | 0 | 1 | 0 | 0 | 1 | 0 |
| M333 | A6 | | A6 | | | ba | 1 | 0 | 1 | 0 | 0 | 1 | 0 |
| M334 | A6 | | A6 | | | bb | 1 | 0 | 1 | 0 | 0 | 1 | 0 |
| M335 | A6 | | A6 | | | ba | 1 | 0 | 1 | 0 | 0 | 1 | 0 |
| M336 | A6 | | A6 | | | bd | 1 | 0 | 1 | 0 | 0 | 1 | 0 |
| M337 | A7 | | A7 | | | a | 1 | 0 | 1 | 0 | 0 | 1 | 0 |
| M338 | A7 | | A7 | | | b | 1 | 0 | 1 | 0 | 0 | 1 | 0 |
| M339 | A7 | | A7 | | | c | 1 | 0 | 1 | 0 | 0 | 1 | 0 |
| M340 | A7 | | A7 | | | d | 1 | 0 | 1 | 0 | 0 | 1 | 0 |
| M341 | A7 | | A7 | | | e | 1 | 0 | 1 | 0 | 0 | 1 | 0 |
| M342 | A7 | | A7 | | | f | 1 | 0 | 1 | 0 | 0 | 1 | 0 |
| M343 | A7 | | A7 | | | q | 1 | 0 | 1 | 0 | 0 | 1 | 0 |
| M344 | A7 | | A7 | | | h | 1 | 0 | 1 | 0 | 0 | 1 | 0 |
| M345 | A7 | | A7 | | | i | 1 | 0 | 1 | 0 | 0 | 1 | 0 |
| M346 | A7 | | A7 | | | i | 1 | 0 | 1 | 0 | 0 | 1 | 0 |
| M347 | A7 | | A7 | | | k | 1 | 0 | 1 | 0 | 0 | 1 | 0 |
| M348 | A7 | | A7 | | | l | 1 | 0 | 1 | 0 | 0 | 1 | 0 |
| M349 | A7 | | A7 | | | m | 1 | 0 | 1 | 0 | 0 | 1 | 0 |
| M350 | A7 | | A7 | | | n | 1 | 0 | 1 | 0 | 0 | 1 | 0 |
| M351 | A7 | | A7 | | | o | 1 | 0 | 1 | 0 | 0 | 1 | 0 |
| M352 | A7 | | A7 | | | p | 1 | 0 | 1 | 0 | 0 | 1 | 0 |
| M353 | A7 | | A7 | | | q | 1 | 0 | 1 | 0 | 0 | 1 | 0 |
| M354 | A7 | | A7 | | | r | 1 | 0 | 1 | 0 | 0 | 1 | 0 |
| M355 | A7 | | A7 | | | s | 1 | 0 | 1 | 0 | 0 | 1 | 0 |
| M356 | A7 | | A7 | | | t | 1 | 0 | 1 | 0 | 0 | 1 | 0 |
| M357 | A7 | | A7 | | | u | 1 | 0 | 1 | 0 | 0 | 1 | 0 |
| M358 | A7 | | A7 | | | v | 1 | 0 | 1 | 0 | 0 | 1 | 0 |
| M359 | A7 | | A7 | | | w | 1 | 0 | 1 | 0 | 0 | 1 | 0 |
| M360 | A7 | | A7 | | | x | 1 | 0 | 1 | 0 | 0 | 1 | 0 |
| M361 | A7 | | A7 | | | y | 1 | 0 | 1 | 0 | 0 | 1 | 0 |
| M362 | A7 | | A7 | | | Z | 1 | 0 | 1 | 0 | 0 | 1 | 0 |
| M363 | A7 | | A7 | | | aa | 1 | 0 | 1 | 0 | 0 | 1 | 0 |
| M364 | A7 | | A7 | | | ab | 1 | 0 | 1 | 0 | 0 | 1 | 0 |
| M365 | A7 | | A7 | | | ac | 1 | 0 | 1 | 0 | 0 | 1 | 0 |
| M366 | A7 | | A7 | | | ad | 1 | 0 | 1 | 0 | 0 | 1 | 0 |
| M367 | A7 | | A7 | | | ae | 1 | 0 | 1 | 0 | 0 | 1 | 0 |
| M368 | | | | | | | | | | | | | |
| M369 | | | | | | | | | | | | | |
| M370 | | | | | | | | | | | | | |
| M371 | A7 | | A7 | | | ai | 1 | 0 | 1 | 0 | 0 | 1 | 0 |
| M372 | A7 | | A7 | | | ai | 1 | 0 | 1 | 0 | 0 | 1 | 0 |
| M373 | A7 | | A7 | | | ak | 1 | 0 | 1 | 0 | 0 | 1 | 0 |
| M374 | A7 | | A7 | | | al | 1 | 0 | 1 | 0 | 0 | 1 | 0 |
| M375 | A7 | | A7 | | | am | 1 | 0 | 1 | 0 | 0 | 1 | 0 |
| M376 | A7 | | A7 | | | an | 1 | 0 | 1 | 0 | 0 | 1 | 0 |
| M377 | A7 | | A7 | | | ao | 1 | 0 | 1 | 0 | 0 | 1 | 0 |
| M378 | A7 | | A7 | | | ap | 1 | 0 | 1 | 0 | 0 | 1 | 0 |
| M379 | A7 | | A7 | | | aq | 1 | 0 | 1 | 0 | 0 | 1 | 0 |
| M380 | A7 | | A7 | | | ar | 1 | 0 | 1 | 0 | 0 | 1 | 0 |
| M381 | A7 | | A7 | | | as | 1 | 0 | 1 | 0 | 0 | 1 | 0 |
| M382 | A7 | | A7 | | | at | 1 | 0 | 1 | 0 | 0 | 1 | 0 |
| M383 | A7 | | A7 | | | au | 1 | 0 | 1 | 0 | 0 | 1 | 0 |
| M384 | A7 | | A7 | | | av | 1 | 0 | 1 | 0 | 0 | 1 | 0 |
| M385 | A7 | | A7 | | | aw | 1 | 0 | 1 | 0 | 0 | 1 | 0 |
| M386 | A7 | | A7 | | | ax | 1 | 0 | 1 | 0 | 0 | 1 | 0 |
| M387 | A7 | | A7 | | | ay | 1 | 0 | 1 | 0 | 0 | 1 | 0 |
| M388 | A7 | | A7 | | | Z | 1 | 0 | 1 | 0 | 0 | 1 | 0 |
| M389 | A7 | | A7 | | | ba | 1 | 0 | 1 | 0 | 0 | 1 | 0 |
| M390 | A7 | | A7 | | | bb | 1 | 0 | 1 | 0 | 0 | 1 | 0 |
| M391 | A7 | | A7 | | | ba | 1 | 0 | 1 | 0 | 0 | 1 | 0 |
| M392 | A7 | | A7 | | | bd | 1 | 0 | 1 | 0 | 0 | 1 | 0 |
| M393 | A8 | | A8 | | | a | 1 | 0 | 1 | 0 | 0 | 1 | 0 |
| M394 | A8 | | A8 | | | b | 1 | 0 | 1 | 0 | 0 | 1 | 0 |
| M395 | A8 | | A8 | | | c | 1 | 0 | 1 | 0 | 0 | 1 | 0 |
| M396 | A8 | | A8 | | | d | 1 | 0 | 1 | 0 | 0 | 1 | 0 |
| M397 | A8 | | A8 | | | e | 1 | 0 | 1 | 0 | 0 | 1 | 0 |
| M398 | A8 | | A8 | | | f | 1 | 0 | 1 | 0 | 0 | 1 | 0 |
| M399 | A8 | | A8 | | | q | 1 | 0 | 1 | 0 | 0 | 1 | 0 |
| M400 | A8 | | A8 | | | h | 1 | 0 | 1 | 0 | 0 | 1 | 0 |
| M401 | A8 | | A8 | | | i | 1 | 0 | 1 | 0 | 0 | 1 | 0 |
| M402 | A8 | | A8 | | | i | 1 | 0 | 1 | 0 | 0 | 1 | 0 |
| M403 | A8 | | A8 | | | k | 1 | 0 | 1 | 0 | 0 | 1 | 0 |
| M404 | A8 | | A8 | | | l | 1 | 0 | 1 | 0 | 0 | 1 | 0 |
| M405 | A8 | | A8 | | | m | 1 | 0 | 1 | 0 | 0 | 1 | 0 |
| M406 | A8 | | A8 | | | n | 1 | 0 | 1 | 0 | 0 | 1 | 0 |
| M407 | A8 | | A8 | | | o | 1 | 0 | 1 | 0 | 0 | 1 | 0 |
| M408 | A8 | | A8 | | | p | 1 | 0 | 1 | 0 | 0 | 1 | 0 |
| M409 | A8 | | A8 | | | q | 1 | 0 | 1 | 0 | 0 | 1 | 0 |
| M410 | A8 | | A8 | | | r | 1 | 0 | 1 | 0 | 0 | 1 | 0 |
| M411 | A8 | | A8 | | | s | 1 | 0 | 1 | 0 | 0 | 1 | 0 |
| M412 | A8 | | A8 | | | t | 1 | 0 | 1 | 0 | 0 | 1 | 0 |
| M413 | A8 | | A8 | | | u | 1 | 0 | 1 | 0 | 0 | 1 | 0 |
| M414 | A8 | | A8 | | | v | 1 | 0 | 1 | 0 | 0 | 1 | 0 |
| M415 | A8 | | A8 | | | w | 1 | 0 | 1 | 0 | 0 | 1 | 0 |
| M416 | A8 | | A8 | | | x | 1 | 0 | 1 | 0 | 0 | 1 | 0 |
| M417 | A8 | | A8 | | | y | 1 | 0 | 1 | 0 | 0 | 1 | 0 |
| M418 | A8 | | A8 | | | z | 1 | 0 | 1 | 0 | 0 | 1 | 0 |
| M419 | A8 | | A8 | | | aa | 1 | 0 | 1 | 0 | 0 | 1 | 0 |
| M420 | A8 | | A8 | | | ab | 1 | 0 | 1 | 0 | 0 | 1 | 0 |
| M421 | A8 | | A8 | | | ac | 1 | 0 | 1 | 0 | 0 | 1 | 0 |
| M422 | A8 | | A8 | | | ad | 1 | 0 | 1 | 0 | 0 | 1 | 0 |
| M423 | A8 | | A8 | | | ae | 1 | 0 | 1 | 0 | 0 | 1 | 0 |
| M424 | | | | | | | | | | | | | |
| M425 | | | | | | | | | | | | | |
| M426 | | | | | | | | | | | | | |
| M427 | A8 | | A8 | | | ai | 1 | 0 | 1 | 0 | 0 | 1 | 0 |
| M428 | A8 | | A8 | | | ai | 1 | 0 | 1 | 0 | 0 | 1 | 0 |
| M429 | A8 | | A8 | | | ak | 1 | 0 | 1 | 0 | 0 | 1 | 0 |
| M430 | A8 | | A8 | | | al | 1 | 0 | 1 | 0 | 0 | 1 | 0 |
| M431 | A8 | | A8 | | | am | 1 | 0 | 1 | 0 | 0 | 1 | 0 |
| M432 | A8 | | A8 | | | an | 1 | 0 | 1 | 0 | 0 | 1 | 0 |
| M433 | A8 | | A8 | | | ao | 1 | 0 | 1 | 0 | 0 | 1 | 0 |
| M434 | A8 | | A8 | | | ap | 1 | 0 | 1 | 0 | 0 | 1 | 0 |
| M435 | A8 | | A8 | | | aq | 1 | 0 | 1 | 0 | 0 | 1 | 0 |
| M436 | A8 | | A8 | | | ar | 1 | 0 | 1 | 0 | 0 | 1 | 0 |
| M437 | A8 | | A8 | | | as | 1 | 0 | 1 | 0 | 0 | 1 | 0 |
| M438 | A8 | | A8 | | | at | 1 | 0 | 1 | 0 | 0 | 1 | 0 |
| M439 | A8 | | A8 | | | au | 1 | 0 | 1 | 0 | 0 | 1 | 0 |
| M440 | A8 | | A8 | | | av | 1 | 0 | 1 | 0 | 0 | 1 | 0 |
| M441 | A8 | | A8 | | | aw | 1 | 0 | 1 | 0 | 0 | 1 | 0 |
| M442 | A8 | | A8 | | | ax | 1 | 0 | 1 | 0 | 0 | 1 | 0 |
| M443 | A8 | | A8 | | | ay | 1 | 0 | 1 | 0 | 0 | 1 | 0 |
| M444 | A8 | | A8 | | | z | 1 | 0 | 1 | 0 | 0 | 1 | 0 |
| M445 | A8 | | A8 | | | ba | 1 | 0 | 1 | 0 | 0 | 1 | 0 |
| M446 | A8 | | A8 | | | bb | 1 | 0 | 1 | 0 | 0 | 1 | 0 |
| M447 | A8 | | A8 | | | ba | 1 | 0 | 1 | 0 | 0 | 1 | 0 |
| M448 | A8 | | A8 | | | bd | 1 | 0 | 1 | 0 | 0 | 1 | 0 |
| M449 | A9 | | A9 | | | a | 1 | 0 | 1 | 0 | 0 | 1 | 0 |
| M450 | A9 | | A9 | | | b | 1 | 0 | 1 | 0 | 0 | 1 | 0 |
| M451 | A9 | | A9 | | | c | 1 | 0 | 1 | 0 | 0 | 1 | 0 |
| M452 | A9 | | A9 | | | d | 1 | 0 | 1 | 0 | 0 | 1 | 0 |
| M453 | A9 | | A9 | | | e | 1 | 0 | 1 | 0 | 0 | 1 | 0 |
| M454 | A9 | | A9 | | | f | 1 | 0 | 1 | 0 | 0 | 1 | 0 |
| M455 | A9 | | A9 | | | q | 1 | 0 | 1 | 0 | 0 | 1 | 0 |
| M456 | A9 | | A9 | | | h | 1 | 0 | 1 | 0 | 0 | 1 | 0 |
| M457 | A9 | | A9 | | | i | 1 | 0 | 1 | 0 | 0 | 1 | 0 |
| M458 | A9 | | A9 | | | i | 1 | 0 | 1 | 0 | 0 | 1 | 0 |
| M459 | A9 | | A9 | | | k | 1 | 0 | 1 | 0 | 0 | 1 | 0 |
| M460 | A9 | | A9 | | | l | 1 | 0 | 1 | 0 | 0 | 1 | 0 |
| M461 | A9 | | A9 | | | m | 1 | 0 | 1 | 0 | 0 | 1 | 0 |
| M462 | A9 | | A9 | | | n | 1 | 0 | 1 | 0 | 0 | 1 | 0 |
| M463 | A9 | | A9 | | | o | 1 | 0 | 1 | 0 | 0 | 1 | 0 |
| M464 | A9 | | A9 | | | p | 1 | 0 | 1 | 0 | 0 | 1 | 0 |
| M465 | A9 | | A9 | | | q | 1 | 0 | 1 | 0 | 0 | 1 | 0 |
| M466 | A9 | | A9 | | | r | 1 | 0 | 1 | 0 | 0 | 1 | 0 |
| M467 | A9 | | A9 | | | s | 1 | 0 | 1 | 0 | 0 | 1 | 0 |
| M468 | A9 | | A9 | | | t | 1 | 0 | 1 | 0 | 0 | 1 | 0 |
| M469 | A9 | | A9 | | | u | 1 | 0 | 1 | 0 | 0 | 1 | 0 |
| M470 | A9 | | A9 | | | v | 1 | 0 | 1 | 0 | 0 | 1 | 0 |
| M471 | A9 | | A9 | | | w | 1 | 0 | 1 | 0 | 0 | 1 | 0 |
| M472 | A9 | | A9 | | | x | 1 | 0 | 1 | 0 | 0 | 1 | 0 |
| M473 | A9 | | A9 | | | y | 1 | 0 | 1 | 0 | 0 | 1 | 0 |
| M474 | A9 | | A9 | | | z | 1 | 0 | 1 | 0 | 0 | 1 | 0 |
| M475 | A9 | | A9 | | | aa | 1 | 0 | 1 | 0 | 0 | 1 | 0 |
| M476 | A9 | | A9 | | | ab | 1 | 0 | 1 | 0 | 0 | 1 | 0 |
| M477 | A9 | | A9 | | | ac | 1 | 0 | 1 | 0 | 0 | 1 | 0 |
| M478 | A9 | | A9 | | | ad | 1 | 0 | 1 | 0 | 0 | 1 | 0 |
| M479 | A9 | | A9 | | | ae | 1 | 0 | 1 | 0 | 0 | 1 | 0 |
| M480 | | | | | | | | | | | | | |
| M481 | | | | | | | | | | | | | |
| M482 | | | | | | | | | | | | | |
| M483 | A9 | | A9 | | | ai | 1 | 0 | 1 | 0 | 0 | 1 | 0 |
| M484 | A9 | | A9 | | | ai | 1 | 0 | 1 | 0 | 0 | 1 | 0 |
| M485 | A9 | | A9 | | | ak | 1 | 0 | 1 | 0 | 0 | 1 | 0 |
| M486 | A9 | | A9 | | | al | 1 | 0 | 1 | 0 | 0 | 1 | 0 |
| M487 | A9 | | A9 | | | am | 1 | 0 | 1 | 0 | 0 | 1 | 0 |
| M488 | A9 | | A9 | | | an | 1 | 0 | 1 | 0 | 0 | 1 | 0 |
| M489 | A9 | | A9 | | | ao | 1 | 0 | 1 | 0 | 0 | 1 | 0 |
| M490 | A9 | | A9 | | | ap | 1 | 0 | 1 | 0 | 0 | 1 | 0 |
| M491 | A9 | | A9 | | | aq | 1 | 0 | 1 | 0 | 0 | 1 | 0 |
| M492 | A9 | | A9 | | | ar | 1 | 0 | 1 | 0 | 0 | 1 | 0 |
| M493 | A9 | | A9 | | | as | 1 | 0 | 1 | 0 | 0 | 1 | 0 |
| M494 | A9 | | A9 | | | at | 1 | 0 | 1 | 0 | 0 | 1 | 0 |
| M495 | A9 | | A9 | | | au | 1 | 0 | 1 | 0 | 0 | 1 | 0 |
| M496 | A9 | | A9 | | | av | 1 | 0 | 1 | 0 | 0 | 1 | 0 |
| M497 | A9 | | A9 | | | aw | 1 | 0 | 1 | 0 | 0 | 1 | 0 |
| M498 | A9 | | A9 | | | ax | 1 | 0 | 1 | 0 | 0 | 1 | 0 |
| M499 | A9 | | A9 | | | ay | 1 | 0 | 1 | 0 | 0 | 1 | 0 |
| M500 | A9 | | A9 | | | z | 1 | 0 | 1 | 0 | 0 | 1 | 0 |
| M501 | A9 | | A9 | | | ba | 1 | 0 | 1 | 0 | 0 | 1 | 0 |
| M502 | A9 | | A9 | | | bb | 1 | 0 | 1 | 0 | 0 | 1 | 0 |
| M503 | A9 | | A9 | | | ba | 1 | 0 | 1 | 0 | 0 | 1 | 0 |
| M504 | A9 | | A9 | | | bd | 1 | 0 | 1 | 0 | 0 | 1 | 0 |
| M505 | A10 | | A10 | | | b | 1 | 0 | 1 | 0 | 0 | 1 | 0 |
| M506 | A1 | | A1 | A1 | | a | 1 | 0 | 1 | 1 | 0 | 1 | 0 |
| M507 | A1 | | A1 | A2 | | a | 1 | 0 | 1 | 1 | 0 | 1 | 0 |
| M508 | A1 | | A1 | A3 | | a | 1 | 0 | 1 | 1 | 0 | 1 | 0 |
| M509 | A1 | | A1 | A4 | | a | 1 | 0 | 1 | 1 | 0 | 1 | 0 |
| M510 | A1 | | A1 | A5 | | a | 1 | 0 | 1 | 1 | 0 | 1 | 0 |
| M511 | A1 | | A1 | A5 | | a | 1 | 0 | 1 | 1 | 0 | 1 | 0 |
| M512 | A1 | | A1 | A6 | | a | 1 | 0 | 1 | 1 | 0 | 1 | 0 |
| M513 | A1 | | A1 | A7 | | a | 1 | 0 | 1 | 1 | 0 | 1 | 0 |
| M514 | A1 | | A1 | A8 | | a | 1 | 0 | 1 | 1 | 0 | 1 | 0 |
| M515 | A1 | | A1 | A9 | | a | 1 | 0 | 1 | 1 | 0 | 1 | 0 |
| M516 | A1 | | A1 | A1 | | b | 1 | 0 | 1 | 1 | 0 | 1 | 0 |
| M517 | A1 | | A1 | A2 | | b | 1 | 0 | 1 | 1 | 0 | 1 | 0 |
| M518 | A1 | | A1 | A3 | | b | 1 | 0 | 1 | 1 | 0 | 1 | 0 |
| M519 | A1 | | A1 | A4 | | b | 1 | 0 | 1 | 1 | 0 | 1 | 0 |
| M520 | A1 | | A1 | A5 | | b | 1 | 0 | 1 | 1 | 0 | 1 | 0 |
| M521 | A1 | | A1 | A5 | | b | 1 | 0 | 1 | 1 | 0 | 1 | 0 |
| M522 | A1 | | A1 | A6 | | b | 1 | 0 | 1 | 1 | 0 | 1 | 0 |
| M523 | A1 | | A1 | A7 | | b | 1 | 0 | 1 | 1 | 0 | 1 | 0 |
| M524 | A1 | | A1 | A8 | | b | 1 | 0 | 1 | 1 | 0 | 1 | 0 |
| M525 | A1 | | A1 | A9 | | b | 1 | 0 | 1 | 1 | 0 | 1 | 0 |
| M526 | A1 | | A1 | A1 | | c | 1 | 0 | 1 | 1 | 0 | 1 | 0 |
| M527 | A1 | | A1 | A2 | | c | 1 | 0 | 1 | 1 | 0 | 1 | 0 |
| M528 | A1 | | A1 | A3 | | c | 1 | 0 | 1 | 1 | 0 | 1 | 0 |
| M529 | A1 | | A1 | A1 | | t | 1 | 0 | 1 | 1 | 0 | 1 | 0 |
| M530 | A1 | | A1 | A2 | | t | 1 | 0 | 1 | 1 | 0 | 1 | 0 |
| M531 | A1 | | A1 | A3 | | t | 1 | 0 | 1 | 1 | 0 | 1 | 0 |
| M532 | A1 | | A1 | A1 | | v | 1 | 0 | 1 | 1 | 0 | 1 | 0 |
| M533 | A1 | | A1 | A2 | | v | 1 | 0 | 1 | 1 | 0 | 1 | 0 |
| M534 | A1 | | A1 | A3 | | v | 1 | 0 | 1 | 1 | 0 | 1 | 0 |
| M535 | A1 | | A1 | A1 | | w | 1 | 0 | 1 | 1 | 0 | 1 | 0 |
| M536 | A1 | | A1 | A2 | | w | 1 | 0 | 1 | 1 | 0 | 1 | 0 |
| M537 | | | | | | | | | | | | | |
| M538 | | | | | | | | | | | | | |
| M539 | A1 | | A1 | A1 | | am | 1 | 0 | 1 | 1 | 0 | 1 | 0 |
| M540 | A1 | | A1 | A2 | | am | 1 | 0 | 1 | 1 | 0 | 1 | 0 |
| M541 | A1 | | A1 | A1 | | an | 1 | 0 | 1 | 1 | 0 | 1 | 0 |
| M542 | A1 | | A1 | A2 | | an | 1 | 0 | 1 | 1 | 0 | 1 | 0 |
| M543 | A1 | | A1 | A1 | | ba | 1 | 0 | 1 | 1 | 0 | 1 | 0 |
| M544 | A1 | | A1 | A2 | | ba | 1 | 0 | 1 | 1 | 0 | 1 | 0 |
| M545 | A1 | | A1 | A1 | | bb | 1 | 0 | 1 | 1 | 0 | 1 | 0 |
| M546 | A1 | | A1 | A2 | | bb | 1 | 0 | 1 | 1 | 0 | 1 | 0 |
| M547 | A1 | | A1 | A1 | | b | 1 | 0 | 1 | 1 | 0 | 2 | 0 |
| M548 | A1 | | A1 | A2 | | b | 1 | 0 | 1 | 1 | 0 | 2 | 0 |
| M549 | A1 | | A1 | A3 | | b | 1 | 0 | 1 | 1 | 0 | 2 | 0 |
| M550 | A1 | | A1 | A1 | | b | 1 | 0 | 1 | 1 | 0 | 3 | 0 |
| M551 | A1 | | A1 | A2 | | b | 1 | 0 | 1 | 1 | 0 | 3 | 0 |
| M552 | A1 | | A1 | A1 | | a | 1 | 0 | 1 | 2 | 0 | 1 | 0 |
| M553 | A1 | | A1 | A2 | | a | 1 | 0 | 1 | 2 | 0 | 1 | 0 |
| M554 | A1 | | A1 | A1 | | b | 1 | 0 | 1 | 2 | 0 | 1 | 0 |
| M555 | A1 | | A1 | A2 | | b | 1 | 0 | 1 | 2 | 0 | 1 | 0 |
| M556 | A2 | | A2 | A1 | | a | 1 | 0 | 1 | 1 | 0 | 1 | 0 |
| M557 | A2 | | A2 | A2 | | a | 1 | 0 | 1 | 1 | 0 | 1 | 0 |
| M558 | A2 | | A2 | A3 | | a | 1 | 0 | 1 | 1 | 0 | 1 | 0 |
| M559 | A2 | | A2 | A4 | | a | 1 | 0 | 1 | 1 | 0 | 1 | 0 |
| M560 | A2 | | A2 | A5 | | a | 1 | 0 | 1 | 1 | 0 | 1 | 0 |
| M561 | A2 | | A2 | A5 | | a | 1 | 0 | 1 | 1 | 0 | 1 | 0 |
| M562 | A2 | | A2 | A6 | | a | 1 | 0 | 1 | 1 | 0 | 1 | 0 |
| M563 | A2 | | A2 | A7 | | a | 1 | 0 | 1 | 1 | 0 | 1 | 0 |
| M564 | A2 | | A2 | A8 | | a | 1 | 0 | 1 | 1 | 0 | 1 | 0 |
| M565 | A2 | | A2 | A9 | | a | 1 | 0 | 1 | 1 | 0 | 1 | 0 |
| M566 | A2 | | A2 | A1 | | b | 1 | 0 | 1 | 1 | 0 | 1 | 0 |
| M567 | A2 | | A2 | A2 | | b | 1 | 0 | 1 | 1 | 0 | 1 | 0 |
| M568 | A2 | | A2 | A3 | | b | 1 | 0 | 1 | 1 | 0 | 1 | 0 |
| M569 | A2 | | A2 | A1 | | v | 1 | 0 | 1 | 1 | 0 | 1 | 0 |
| M570 | A2 | | A2 | A2 | | v | 1 | 0 | 1 | 1 | 0 | 1 | 0 |
| M571 | A2 | | A2 | A3 | | v | 1 | 0 | 1 | 1 | 0 | 1 | 0 |
| M572 | A2 | | A2 | A1 | | b | 1 | 0 | 1 | 1 | 0 | 2 | 0 |
| M573 | A2 | | A2 | A2 | | b | 1 | 0 | 1 | 1 | 0 | 2 | 0 |
| M574 | A2 | | A2 | A3 | | b | 1 | 0 | 1 | 1 | 0 | 2 | 0 |
| M575 | A2 | | A2 | A4 | | b | 1 | 0 | 1 | 1 | 0 | 1 | 0 |
| M576 | A2 | | A2 | A5 | | b | 1 | 0 | 1 | 1 | 0 | 1 | 0 |
| M577 | A2 | | A2 | A5 | | b | 1 | 0 | 1 | 1 | 0 | 1 | 0 |
| M578 | A2 | | A2 | A6 | | b | 1 | 0 | 1 | 1 | 0 | 1 | 0 |
| M579 | A2 | | A2 | A7 | | b | 1 | 0 | 1 | 1 | 0 | 1 | 0 |
| M580 | A2 | | A2 | A8 | | b | 1 | 0 | 1 | 1 | 0 | 1 | 0 |
| M581 | A2 | | A2 | A9 | | b | 1 | 0 | 1 | 1 | 0 | 1 | 0 |
| M582 | A3 | | A3 | A1 | | a | 1 | 0 | 1 | 1 | 0 | 1 | 0 |
| M583 | A3 | | A3 | A2 | | a | 1 | 0 | 1 | 1 | 0 | 1 | 0 |
| M584 | A3 | | A3 | A3 | | a | 1 | 0 | 1 | 1 | 0 | 1 | 0 |
| M585 | A3 | | A3 | A4 | | a | 1 | 0 | 1 | 1 | 0 | 1 | 0 |
| M586 | A3 | | A3 | A5 | | a | 1 | 0 | 1 | 1 | 0 | 1 | 0 |
| M587 | A3 | | A3 | A5 | | a | 1 | 0 | 1 | 1 | 0 | 1 | 0 |
| M588 | A3 | | A3 | A6 | | a | 1 | 0 | 1 | 1 | 0 | 1 | 0 |
| M589 | A3 | | A3 | A7 | | a | 1 | 0 | 1 | 1 | 0 | 1 | 0 |
| M590 | A3 | | A3 | A8 | | a | 1 | 0 | 1 | 1 | 0 | 1 | 0 |
| M591 | A3 | | A3 | A9 | | a | 1 | 0 | 1 | 1 | 0 | 1 | 0 |
| M592 | A3 | | A3 | A1 | | b | 1 | 0 | 1 | 1 | 0 | 1 | 0 |
| M593 | A3 | | A3 | A2 | | b | 1 | 0 | 1 | 1 | 0 | 1 | 0 |
| M594 | A3 | | A3 | A3 | | b | 1 | 0 | 1 | 1 | 0 | 1 | 0 |
| M595 | A3 | | A3 | A4 | | b | 1 | 0 | 1 | 1 | 0 | 1 | 0 |
| M596 | A3 | | A3 | A5 | | b | 1 | 0 | 1 | 1 | 0 | 1 | 0 |
| M597 | A3 | | A3 | A5 | | b | 1 | 0 | 1 | 1 | 0 | 1 | 0 |
| M598 | A3 | | A3 | A6 | | b | 1 | 0 | 1 | 1 | 0 | 1 | 0 |
| M599 | A3 | | A3 | A7 | | b | 1 | 0 | 1 | 1 | 0 | 1 | 0 |
| M600 | A3 | | A3 | A8 | | b | 1 | 0 | 1 | 1 | 0 | 1 | 0 |
| M601 | A3 | | A3 | A9 | | b | 1 | 0 | 1 | 1 | 0 | 1 | 0 |
| M602 | A4 | | A4 | A1 | | a | 1 | 0 | 1 | 1 | 0 | 1 | 0 |
| M603 | A4 | | A4 | A2 | | a | 1 | 0 | 1 | 1 | 0 | 1 | 0 |
| M604 | A4 | | A4 | A3 | | a | 1 | 0 | 1 | 1 | 0 | 1 | 0 |
| M605 | A4 | | A4 | A4 | | a | 1 | 0 | 1 | 1 | 0 | 1 | 0 |
| M606 | A4 | | A4 | A5 | | a | 1 | 0 | 1 | 1 | 0 | 1 | 0 |
| M607 | A4 | | A4 | A5 | | a | 1 | 0 | 1 | 1 | 0 | 1 | 0 |
| M608 | A4 | | A4 | A6 | | a | 1 | 0 | 1 | 1 | 0 | 1 | 0 |
| M609 | A4 | | A4 | A7 | | a | 1 | 0 | 1 | 1 | 0 | 1 | 0 |
| M610 | A4 | | A4 | A8 | | a | 1 | 0 | 1 | 1 | 0 | 1 | 0 |
| M611 | A4 | | A4 | A9 | | a | 1 | 0 | 1 | 1 | 0 | 1 | 0 |
| M612 | A4 | | A4 | A1 | | b | 1 | 0 | 1 | 1 | 0 | 1 | 0 |
| M613 | A4 | | A4 | A2 | | b | 1 | 0 | 1 | 1 | 0 | 1 | 0 |
| M614 | A4 | | A4 | A3 | | b | 1 | 0 | 1 | 1 | 0 | 1 | 0 |
| M615 | A4 | | A4 | A4 | | b | 1 | 0 | 1 | 1 | 0 | 1 | 0 |
| M616 | A4 | | A4 | A5 | | b | 1 | 0 | 1 | 1 | 0 | 1 | 0 |
| M617 | A4 | | A4 | A5 | | b | 1 | 0 | 1 | 1 | 0 | 1 | 0 |
| M618 | A4 | | A4 | A6 | | b | 1 | 0 | 1 | 1 | 0 | 1 | 0 |
| M619 | A4 | | A4 | A7 | | b | 1 | 0 | 1 | 1 | 0 | 1 | 0 |
| M620 | A4 | | A4 | A8 | | b | 1 | 0 | 1 | 1 | 0 | 1 | 0 |
| M621 | A4 | | A4 | A9 | | b | 1 | 0 | 1 | 1 | 0 | 1 | 0 |
| M622 | A5 | | A5 | A1 | | a | 1 | 0 | 1 | 1 | 0 | 1 | 0 |
| M623 | A5 | | A5 | A2 | | a | 1 | 0 | 1 | 1 | 0 | 1 | 0 |
| M624 | A5 | | A5 | A3 | | a | 1 | 0 | 1 | 1 | 0 | 1 | 0 |
| M625 | A5 | | A5 | A4 | | a | 1 | 0 | 1 | 1 | 0 | 1 | 0 |
| M626 | A5 | | A5 | A5 | | a | 1 | 0 | 1 | 1 | 0 | 1 | 0 |
| M627 | A5 | | A5 | A5 | | a | 1 | 0 | 1 | 1 | 0 | 1 | 0 |
| M628 | A5 | | A5 | A6 | | a | 1 | 0 | 1 | 1 | 0 | 1 | 0 |
| M629 | A5 | | A5 | A7 | | a | 1 | 0 | 1 | 1 | 0 | 1 | 0 |
| M630 | A5 | | A5 | A8 | | a | 1 | 0 | 1 | 1 | 0 | 1 | 0 |
| M631 | A5 | | A5 | A9 | | a | 1 | 0 | 1 | 1 | 0 | 1 | 0 |
| M632 | A5 | | A5 | A1 | | b | 1 | 0 | 1 | 1 | 0 | 1 | 0 |
| M633 | A5 | | A5 | A2 | | b | 1 | 0 | 1 | 1 | 0 | 1 | 0 |
| M634 | A5 | | A5 | A3 | | b | 1 | 0 | 1 | 1 | 0 | 1 | 0 |
| M635 | A5 | | A5 | A4 | | b | 1 | 0 | 1 | 1 | 0 | 1 | 0 |
| M636 | A5 | | A5 | A5 | | b | 1 | 0 | 1 | 1 | 0 | 1 | 0 |
| M637 | A5 | | A5 | A5 | | b | 1 | 0 | 1 | 1 | 0 | 1 | 0 |
| M638 | A5 | | A5 | A6 | | b | 1 | 0 | 1 | 1 | 0 | 1 | 0 |
| M639 | A5 | | A5 | A7 | | b | 1 | 0 | 1 | 1 | 0 | 1 | 0 |
| M640 | A5 | | A5 | A8 | | b | 1 | 0 | 1 | 1 | 0 | 1 | 0 |
| M641 | A5 | | A5 | A9 | | b | 1 | 0 | 1 | 1 | 0 | 1 | 0 |
| M642 | A6 | | A6 | A1 | | a | 1 | 0 | 1 | 1 | 0 | 1 | 0 |
| M643 | A6 | | A6 | A2 | | a | 1 | 0 | 1 | 1 | 0 | 1 | 0 |
| M644 | A6 | | A6 | A3 | | a | 1 | 0 | 1 | 1 | 0 | 1 | 0 |
| M645 | A6 | | A6 | A4 | | a | 1 | 0 | 1 | 1 | 0 | 1 | 0 |
| M646 | A6 | | A6 | A5 | | a | 1 | 0 | 1 | 1 | 0 | 1 | 0 |
| M647 | A6 | | A6 | A5 | | a | 1 | 0 | 1 | 1 | 0 | 1 | 0 |
| M648 | A6 | | A6 | A6 | | a | 1 | 0 | 1 | 1 | 0 | 1 | 0 |
| M649 | A6 | | A6 | A7 | | a | 1 | 0 | 1 | 1 | 0 | 1 | 0 |
| M650 | A6 | | A6 | A8 | | a | 1 | 0 | 1 | 1 | 0 | 1 | 0 |
| M651 | A6 | | A6 | A9 | | a | 1 | 0 | 1 | 1 | 0 | 1 | 0 |
| M652 | A6 | | A6 | A1 | | b | 1 | 0 | 1 | 1 | 0 | 1 | 0 |
| M653 | A6 | | A6 | A2 | | b | 1 | 0 | 1 | 1 | 0 | 1 | 0 |
| M654 | A6 | | A6 | A3 | | b | 1 | 0 | 1 | 1 | 0 | 1 | 0 |
| M655 | A6 | | A6 | A4 | | b | 1 | 0 | 1 | 1 | 0 | 1 | 0 |
| M656 | A6 | | A6 | A5 | | b | 1 | 0 | 1 | 1 | 0 | 1 | 0 |
| M657 | A6 | | A6 | A5 | | b | 1 | 0 | 1 | 1 | 0 | 1 | 0 |
| M658 | A6 | | A6 | A6 | | b | 1 | 0 | 1 | 1 | 0 | 1 | 0 |
| M659 | A6 | | A6 | A7 | | b | 1 | 0 | 1 | 1 | 0 | 1 | 0 |
| M660 | A6 | | A6 | A8 | | b | 1 | 0 | 1 | 1 | 0 | 1 | 0 |
| M661 | A6 | | A6 | A9 | | b | 1 | 0 | 1 | 1 | 0 | 1 | 0 |
| M662 | A7 | | A7 | A1 | | a | 1 | 0 | 1 | 1 | 0 | 1 | 0 |
| M663 | A7 | | A7 | A2 | | a | 1 | 0 | 1 | 1 | 0 | 1 | 0 |
| M664 | A7 | | A7 | A3 | | a | 1 | 0 | 1 | 1 | 0 | 1 | 0 |
| M665 | A7 | | A7 | A4 | | a | 1 | 0 | 1 | 1 | 0 | 1 | 0 |
| M666 | A7 | | A7 | A5 | | a | 1 | 0 | 1 | 1 | 0 | 1 | 0 |
| M667 | A7 | | A7 | A5 | | a | 1 | 0 | 1 | 1 | 0 | 1 | 0 |
| M668 | A7 | | A7 | A6 | | a | 1 | 0 | 1 | 1 | 0 | 1 | 0 |
| M669 | A7 | | A7 | A7 | | a | 1 | 0 | 1 | 1 | 0 | 1 | 0 |
| M670 | A7 | | A7 | A8 | | a | 1 | 0 | 1 | 1 | 0 | 1 | 0 |
| M671 | A7 | | A7 | A9 | | a | 1 | 0 | 1 | 1 | 0 | 1 | 0 |
| M672 | A7 | | A7 | A1 | | b | 1 | 0 | 1 | 1 | 0 | 1 | 0 |
| M673 | A7 | | A7 | A2 | | b | 1 | 0 | 1 | 1 | 0 | 1 | 0 |
| M674 | A7 | | A7 | A3 | | b | 1 | 0 | 1 | 1 | 0 | 1 | 0 |
| M675 | A7 | | A7 | A4 | | b | 1 | 0 | 1 | 1 | 0 | 1 | 0 |
| M676 | A7 | | A7 | A5 | | b | 1 | 0 | 1 | 1 | 0 | 1 | 0 |
| M677 | A7 | | A7 | A5 | | b | 1 | 0 | 1 | 1 | 0 | 1 | 0 |
| M678 | A7 | | A7 | A6 | | b | 1 | 0 | 1 | 1 | 0 | 1 | 0 |
| M679 | A7 | | A7 | A7 | | b | 1 | 0 | 1 | 1 | 0 | 1 | 0 |
| M680 | A7 | | A7 | A8 | | b | 1 | 0 | 1 | 1 | 0 | 1 | 0 |
| M681 | A7 | | A7 | A9 | | b | 1 | 0 | 1 | 1 | 0 | 1 | 0 |
| M682 | A8 | | A8 | A1 | | a | 1 | 0 | 1 | 1 | 0 | 1 | 0 |
| M683 | A8 | | A8 | A2 | | a | 1 | 0 | 1 | 1 | 0 | 1 | 0 |
| M684 | A8 | | A8 | A3 | | a | 1 | 0 | 1 | 1 | 0 | 1 | 0 |
| M685 | A8 | | A8 | A4 | | a | 1 | 0 | 1 | 1 | 0 | 1 | 0 |
| M686 | A8 | | A8 | A5 | | a | 1 | 0 | 1 | 1 | 0 | 1 | 0 |
| M687 | A8 | | A8 | A5 | | a | 1 | 0 | 1 | 1 | 0 | 1 | 0 |
| M688 | A8 | | A8 | A6 | | a | 1 | 0 | 1 | 1 | 0 | 1 | 0 |
| M689 | A8 | | A8 | A7 | | a | 1 | 0 | 1 | 1 | 0 | 1 | 0 |
| M690 | A8 | | A8 | A8 | | a | 1 | 0 | 1 | 1 | 0 | 1 | 0 |
| M691 | A8 | | A8 | A9 | | a | 1 | 0 | 1 | 1 | 0 | 1 | 0 |
| M692 | A8 | | A8 | A1 | | b | 1 | 0 | 1 | 1 | 0 | 1 | 0 |
| M693 | A8 | | A8 | A2 | | b | 1 | 0 | 1 | 1 | 0 | 1 | 0 |
| M694 | A8 | | A8 | A3 | | b | 1 | 0 | 1 | 1 | 0 | 1 | 0 |
| M695 | A8 | | A8 | A4 | | b | 1 | 0 | 1 | 1 | 0 | 1 | 0 |
| M696 | A8 | | A8 | A5 | | b | 1 | 0 | 1 | 1 | 0 | 1 | 0 |
| M697 | A8 | | A8 | A5 | | b | 1 | 0 | 1 | 1 | 0 | 1 | 0 |
| M698 | A8 | | A8 | A6 | | b | 1 | 0 | 1 | 1 | 0 | 1 | 0 |
| M699 | A8 | | A8 | A7 | | b | 1 | 0 | 1 | 1 | 0 | 1 | 0 |
| M700 | A8 | | A8 | A8 | | b | 1 | 0 | 1 | 1 | 0 | 1 | 0 |
| M701 | A8 | | A8 | A9 | | b | 1 | 0 | 1 | 1 | 0 | 1 | 0 |
| M702 | A9 | | A9 | A1 | | a | 1 | 0 | 1 | 1 | 0 | 1 | 0 |
| M703 | A9 | | A9 | A2 | | a | 1 | 0 | 1 | 1 | 0 | 1 | 0 |
| M704 | A9 | | A9 | A3 | | a | 1 | 0 | 1 | 1 | 0 | 1 | 0 |
| M705 | A9 | | A9 | A4 | | a | 1 | 0 | 1 | 1 | 0 | 1 | 0 |
| M706 | A9 | | A9 | A5 | | a | 1 | 0 | 1 | 1 | 0 | 1 | 0 |
| M707 | A9 | | A9 | A5 | | a | 1 | 0 | 1 | 1 | 0 | 1 | 0 |
| M708 | A9 | | A9 | A6 | | a | 1 | 0 | 1 | 1 | 0 | 1 | 0 |
| M709 | A9 | | A9 | A7 | | a | 1 | 0 | 1 | 1 | 0 | 1 | 0 |
| M710 | A9 | | A9 | A8 | | a | 1 | 0 | 1 | 1 | 0 | 1 | 0 |
| M711 | A9 | | A9 | A9 | | a | 1 | 0 | 1 | 1 | 0 | 1 | 0 |
| M712 | A9 | | A9 | A1 | | b | 1 | 0 | 1 | 1 | 0 | 1 | 0 |
| M713 | A9 | | A9 | A2 | | b | 1 | 0 | 1 | 1 | 0 | 1 | 0 |
| M714 | A9 | | A9 | A3 | | b | 1 | 0 | 1 | 1 | 0 | 1 | 0 |
| M715 | A9 | | A9 | A4 | | b | 1 | 0 | 1 | 1 | 0 | 1 | 0 |
| M716 | A9 | | A9 | A5 | | b | 1 | 0 | 1 | 1 | 0 | 1 | 0 |
| M717 | A9 | | A9 | A5 | | b | 1 | 0 | 1 | 1 | 0 | 1 | 0 |
| M718 | A9 | | A9 | A6 | | b | 1 | 0 | 1 | 1 | 0 | 1 | 0 |
| M719 | A9 | | A9 | A7 | | b | 1 | 0 | 1 | 1 | 0 | 1 | 0 |
| M720 | A9 | | A9 | A8 | | b | 1 | 0 | 1 | 1 | 0 | 1 | 0 |
| M721 | A9 | | A9 | A9 | | b | 1 | 0 | 1 | 1 | 0 | 1 | 0 |
| M722 | A10 | | A1 | A10 | | b | 1 | 0 | 1 | 1 | 0 | 1 | 0 |
| M723 | A1 | A1 | A1 | | | a | 1 | 1 | 1 | 0 | 0 | 1 | 1 |
| M724 | A1 | A1 | A1 | | | b | 1 | 1 | 1 | 0 | 0 | 1 | 1 |
| M725 | A1 | A2 | A1 | | | a | 1 | 1 | 1 | 0 | 0 | 1 | 1 |
| M726 | A1 | A2 | A1 | | | b | 1 | 1 | 1 | 0 | 0 | 1 | 1 |
| M727 | A1 | A3 | A1 | | | a | 1 | 1 | 1 | 0 | 0 | 1 | 1 |
| M728 | A1 | A3 | A1 | | | b | 1 | 1 | 1 | 0 | 0 | 1 | 1 |
| M729 | A1 | A3 | A1 | | | t | 1 | 1 | 1 | 0 | 0 | 1 | 1 |
| M730 | A1 | A3 | A1 | | | v | 1 | 1 | 1 | 0 | 0 | 1 | 1 |
| M731 | A1 | A3 | A1 | | | av | 1 | 1 | 1 | 0 | 0 | 1 | 1 |
| M732 | A1 | A4 | A1 | | | a | 1 | 1 | 1 | 0 | 0 | 1 | 1 |
| M733 | A1 | A4 | A1 | | | b | 1 | 1 | 1 | 0 | 0 | 1 | 1 |
| M734 | A1 | A5 | A1 | | | a | 1 | 1 | 1 | 0 | 0 | 1 | 1 |
| M735 | A1 | A5 | A1 | | | b | 1 | 1 | 1 | 0 | 0 | 1 | 1 |
| M736 | A1 | A6 | A1 | | | a | 1 | 1 | 1 | 0 | 0 | 1 | 1 |
| M737 | A1 | A6 | A1 | | | b | 1 | 1 | 1 | 0 | 0 | 1 | 1 |
| M738 | A1 | A7 | A1 | | | a | 1 | 1 | 1 | 0 | 0 | 1 | 1 |
| M739 | A1 | A7 | A1 | | | b | 1 | 1 | 1 | 0 | 0 | 1 | 1 |
| M740 | A1 | A8 | A1 | | | a | 1 | 1 | 1 | 0 | 0 | 1 | 1 |
| M741 | A1 | A8 | A1 | | | b | 1 | 1 | 1 | 0 | 0 | 1 | 1 |
| M742 | A1 | A9 | A1 | | | a | 1 | 1 | 1 | 0 | 0 | 1 | 1 |
| M743 | A1 | A9 | A1 | | | b | 1 | 1 | 1 | 0 | 0 | 1 | 1 |
| M744 | A1 | A10 | A1 | | | a | 1 | 1 | 1 | 0 | 0 | 1 | 1 |
| M745 | A1 | A10 | A1 | | | b | 1 | 1 | 1 | 0 | 0 | 1 | 1 |
| M746 | A1 | A9 | A1 | | | w | 1 | 1 | 1 | 0 | 0 | 1 | 1 |
| M747 | A1 | A9 | A1 | | | ae | 1 | 1 | 1 | 0 | 0 | 1 | 1 |
| M748 | A1 | A9 | A1 | | | c | 1 | 1 | 1 | 0 | 0 | 1 | 1 |
| M749 | A2 | A1 | A2 | | | a | 1 | 1 | 1 | 0 | 0 | 1 | 1 |
| M750 | A2 | A1 | A2 | | | b | 1 | 1 | 1 | 0 | 0 | 1 | 1 |
| M751 | A2 | A2 | A2 | | | a | 1 | 1 | 1 | 0 | 0 | 1 | 1 |
| M752 | A2 | A2 | A2 | | | b | 1 | 1 | 1 | 0 | 0 | 1 | 1 |
| M753 | A2 | A3 | A2 | | | a | 1 | 1 | 1 | 0 | 0 | 1 | 1 |
| M754 | A2 | A3 | A2 | | | b | 1 | 1 | 1 | 0 | 0 | 1 | 1 |
| M755 | A2 | A3 | A2 | | | t | 1 | 1 | 1 | 0 | 0 | 1 | 1 |
| M756 | A2 | A3 | A2 | | | v | 1 | 1 | 1 | 0 | 0 | 1 | 1 |
| M757 | A2 | A3 | A2 | | | d | 1 | 1 | 1 | 0 | 0 | 1 | 1 |
| M758 | A2 | A4 | A2 | | | a | 1 | 1 | 1 | 0 | 0 | 1 | 1 |
| M759 | A2 | A4 | A2 | | | b | 1 | 1 | 1 | 0 | 0 | 1 | 1 |
| M760 | A2 | A5 | A2 | | | a | 1 | 1 | 1 | 0 | 0 | 1 | 1 |
| M761 | A2 | A5 | A2 | | | b | 1 | 1 | 1 | 0 | 0 | 1 | 1 |
| M762 | A2 | A6 | A2 | | | a | 1 | 1 | 1 | 0 | 0 | 1 | 1 |
| M763 | A2 | A6 | A2 | | | b | 1 | 1 | 1 | 0 | 0 | 1 | 1 |
| M764 | A2 | A7 | A2 | | | a | 1 | 1 | 1 | 0 | 0 | 1 | 1 |
| M765 | A2 | A7 | A2 | | | b | 1 | 1 | 1 | 0 | 0 | 1 | 1 |
| M766 | A2 | A8 | A2 | | | aq | 1 | 1 | 1 | 0 | 0 | 1 | 1 |
| M767 | A2 | A8 | A2 | | | b | 1 | 1 | 1 | 0 | 0 | 1 | 1 |
| M768 | A2 | A9 | A2 | | | a | 1 | 1 | 1 | 0 | 0 | 1 | 1 |
| M769 | A2 | A9 | A2 | | | b | 1 | 1 | 1 | 0 | 0 | 1 | 1 |
| M770 | A2 | A9 | A2 | | | w | 1 | 1 | 1 | 0 | 0 | 1 | 1 |
| M771 | A2 | A9 | A2 | | | ae | 1 | 1 | 1 | 0 | 0 | 1 | 1 |
| M772 | A2 | A9 | A2 | | | bb | 1 | 1 | 1 | 0 | 0 | 1 | 1 |
| M773 | A2 | A10 | A2 | | | a | 1 | 1 | 1 | 0 | 0 | 1 | 1 |
| M774 | A2 | A10 | A2 | | | b | 1 | 1 | 1 | 0 | 0 | 1 | 1 |
| M775 | A3 | A1 | A3 | | | a | 1 | 1 | 1 | 0 | 0 | 1 | 1 |
| M776 | A3 | A1 | A3 | | | b | 1 | 1 | 1 | 0 | 0 | 1 | 1 |
| M777 | A3 | A2 | A3 | | | a | 1 | 1 | 1 | 0 | 0 | 1 | 1 |
| M778 | A3 | A2 | A3 | | | b | 1 | 1 | 1 | 0 | 0 | 1 | 1 |
| M779 | A3 | A3 | A3 | | | x | 1 | 1 | 1 | 0 | 0 | 1 | 1 |
| M780 | A3 | A3 | A3 | | | b | 1 | 1 | 1 | 0 | 0 | 1 | 1 |
| M781 | A3 | A3 | A3 | | | t | 1 | 1 | 1 | 0 | 0 | 1 | 1 |
| M782 | A3 | A3 | A3 | | | v | 1 | 1 | 1 | 0 | 0 | 1 | 1 |
| M783 | A3 | A3 | A3 | | | d | 1 | 1 | 1 | 0 | 0 | 1 | 1 |
| M784 | A3 | A4 | A3 | | | a | 1 | 1 | 1 | 0 | 0 | 1 | 1 |
| M785 | A3 | A4 | A3 | | | b | 1 | 1 | 1 | 0 | 0 | 1 | 1 |
| M786 | A3 | A5 | A3 | | | s | 1 | 1 | 1 | 0 | 0 | 1 | 1 |
| M787 | A3 | A5 | A3 | | | b | 1 | 1 | 1 | 0 | 0 | 1 | 1 |
| M788 | A3 | A6 | A3 | | | a | 1 | 1 | 1 | 0 | 0 | 1 | 1 |
| M789 | A3 | A6 | A3 | | | b | 1 | 1 | 1 | 0 | 0 | 1 | 1 |
| M790 | A3 | A7 | A3 | | | a | 1 | 1 | 1 | 0 | 0 | 1 | 1 |
| M791 | A3 | A7 | A3 | | | i | 1 | 1 | 1 | 0 | 0 | 1 | 1 |
| M792 | A3 | A8 | A3 | | | a | 1 | 1 | 1 | 0 | 0 | 1 | 1 |
| M793 | A3 | A8 | A3 | | | b | 1 | 1 | 1 | 0 | 0 | 1 | 1 |
| M794 | A3 | A9 | A3 | | | d | 1 | 1 | 1 | 0 | 0 | 1 | 1 |
| M795 | A3 | A9 | A3 | | | b | 1 | 1 | 1 | 0 | 0 | 1 | 1 |
| M796 | A3 | A9 | A3 | | | w | 1 | 1 | 1 | 0 | 0 | 1 | 1 |
| M797 | A3 | A9 | A3 | | | ae | 1 | 1 | 1 | 0 | 0 | 1 | 1 |
| M798 | A3 | A9 | A3 | | | bb | 1 | 1 | 1 | 0 | 0 | 1 | 1 |
| M799 | A3 | A10 | A3 | | | am | 1 | 1 | 1 | 0 | 0 | 1 | 1 |
| M800 | A3 | A10 | A3 | | | b | 1 | 1 | 1 | 0 | 0 | 1 | 1 |
| M801 | A4 | A1 | A4 | | | an | 1 | 1 | 1 | 0 | 0 | 1 | 1 |
| M802 | A4 | A1 | A4 | | | b | 1 | 1 | 1 | 0 | 0 | 1 | 1 |
| M803 | A4 | A2 | A4 | | | as | 1 | 1 | 1 | 0 | 0 | 1 | 1 |
| M804 | A4 | A2 | A4 | | | b | 1 | 1 | 1 | 0 | 0 | 1 | 1 |
| M805 | A4 | A3 | A4 | | | x | 1 | 1 | 1 | 0 | 0 | 1 | 1 |
| M806 | A4 | A3 | A4 | | | b | 1 | 1 | 1 | 0 | 0 | 1 | 1 |
| M807 | A4 | A3 | A4 | | | t | 1 | 1 | 1 | 0 | 0 | 1 | 1 |
| M808 | A4 | A3 | A4 | | | v | 1 | 1 | 1 | 0 | 0 | 1 | 1 |
| M809 | A4 | A3 | A4 | | | d | 1 | 1 | 1 | 0 | 0 | 1 | 1 |
| M810 | A4 | A4 | A4 | | | au | 1 | 1 | 1 | 0 | 0 | 1 | 1 |
| M811 | A4 | A4 | A4 | | | b | 1 | 1 | 1 | 0 | 0 | 1 | 1 |
| M812 | A4 | A5 | A4 | | | s | 1 | 1 | 1 | 0 | 0 | 1 | 1 |
| M813 | A4 | A5 | A4 | | | b | 1 | 1 | 1 | 0 | 0 | 1 | 1 |
| M814 | A4 | A6 | A4 | | | a | 1 | 1 | 1 | 0 | 0 | 1 | 1 |
| M815 | A4 | A6 | A4 | | | b | 1 | 1 | 1 | 0 | 0 | 1 | 1 |
| M816 | A4 | A7 | A4 | | | a | 1 | 1 | 1 | 0 | 0 | 1 | 1 |
| M817 | A4 | A7 | A4 | | | i | 1 | 1 | 1 | 0 | 0 | 1 | 1 |
| M818 | A4 | A8 | A4 | | | a | 1 | 1 | 1 | 0 | 0 | 1 | 1 |
| M819 | A4 | A8 | A4 | | | b | 1 | 1 | 1 | 0 | 0 | 1 | 1 |
| M820 | A4 | A9 | A4 | | | d | 1 | 1 | 1 | 0 | 0 | 1 | 1 |
| M821 | A4 | A9 | A4 | | | b | 1 | 1 | 1 | 0 | 0 | 1 | 1 |
| M822 | A4 | A9 | A4 | | | w | 1 | 1 | 1 | 0 | 0 | 1 | 1 |
| M823 | A4 | A9 | A4 | | | ae | 1 | 1 | 1 | 0 | 0 | 1 | 1 |
| M824 | A4 | A9 | A4 | | | bb | 1 | 1 | 1 | 0 | 0 | 1 | 1 |
| M825 | A4 | A10 | A4 | | | am | 1 | 1 | 1 | 0 | 0 | 1 | 1 |
| M826 | A4 | A10 | A4 | | | b | 1 | 1 | 1 | 0 | 0 | 1 | 1 |
| M827 | A5 | A1 | A5 | | | an | 1 | 1 | 1 | 0 | 0 | 1 | 1 |
| M828 | A5 | A1 | A5 | | | b | 1 | 1 | 1 | 0 | 0 | 1 | 1 |
| M829 | A5 | A2 | A5 | | | as | 1 | 1 | 1 | 0 | 0 | 1 | 1 |
| M830 | A5 | A2 | A5 | | | b | 1 | 1 | 1 | 0 | 0 | 1 | 1 |
| M831 | A5 | A3 | A5 | | | x | 1 | 1 | 1 | 0 | 0 | 1 | 1 |
| M832 | A5 | A3 | A5 | | | b | 1 | 1 | 1 | 0 | 0 | 1 | 1 |
| M833 | A5 | A3 | A5 | | | t | 1 | 1 | 1 | 0 | 0 | 1 | 1 |
| M834 | A5 | A3 | A5 | | | v | 1 | 1 | 1 | 0 | 0 | 1 | 1 |
| M835 | A5 | A3 | A5 | | | d | 1 | 1 | 1 | 0 | 0 | 1 | 1 |
| M836 | A5 | A4 | A5 | | | au | 1 | 1 | 1 | 0 | 0 | 1 | 1 |
| M837 | A5 | A4 | A5 | | | b | 1 | 1 | 1 | 0 | 0 | 1 | 1 |
| M838 | A5 | A5 | A5 | | | s | 1 | 1 | 1 | 0 | 0 | 1 | 1 |
| M839 | A5 | A5 | A5 | | | b | 1 | 1 | 1 | 0 | 0 | 1 | 1 |
| M840 | A5 | A6 | A5 | | | a | 1 | 1 | 1 | 0 | 0 | 1 | 1 |
| M841 | A5 | A6 | A5 | | | b | 1 | 1 | 1 | 0 | 0 | 1 | 1 |
| M842 | A5 | A7 | A5 | | | a | 1 | 1 | 1 | 0 | 0 | 1 | 1 |
| M843 | A5 | A7 | A5 | | | j | 1 | 1 | 1 | 0 | 0 | 1 | 1 |
| M844 | A5 | A8 | A5 | | | a | 1 | 1 | 1 | 0 | 0 | 1 | 1 |
| M845 | A5 | A8 | A5 | | | b | 1 | 1 | 1 | 0 | 0 | 1 | 1 |
| M846 | A5 | A9 | A5 | | | d | 1 | 1 | 1 | 0 | 0 | 1 | 1 |
| M847 | A5 | A9 | A5 | | | b | 1 | 1 | 1 | 0 | 0 | 1 | 1 |
| M848 | A5 | A9 | A5 | | | w | 1 | 1 | 1 | 0 | 0 | 1 | 1 |
| M849 | A5 | A9 | A5 | | | ae | 1 | 1 | 1 | 0 | 0 | 1 | 1 |
| M850 | A5 | A9 | A5 | | | bb | 1 | 1 | 1 | 0 | 0 | 1 | 1 |
| M851 | A5 | A10 | A5 | | | am | 1 | 1 | 1 | 0 | 0 | 1 | 1 |
| M852 | A5 | A10 | A5 | | | b | 1 | 1 | 1 | 0 | 0 | 1 | 1 |
| M853 | A6 | A1 | A6 | | | an | 1 | 1 | 1 | 0 | 0 | 1 | 1 |
| M854 | A6 | A1 | A6 | | | b | 1 | 1 | 1 | 0 | 0 | 1 | 1 |
| M855 | A6 | A2 | A6 | | | as | 1 | 1 | 1 | 0 | 0 | 1 | 1 |
| M856 | A6 | A2 | A6 | | | b | 1 | 1 | 1 | 0 | 0 | 1 | 1 |
| M857 | A6 | A3 | A6 | | | x | 1 | 1 | 1 | 0 | 0 | 1 | 1 |
| M858 | A6 | A3 | A6 | | | b | 1 | 1 | 1 | 0 | 0 | 1 | 1 |
| M859 | A6 | A3 | A6 | | | t | 1 | 1 | 1 | 0 | 0 | 1 | 1 |
| M860 | A6 | A3 | A6 | | | v | 1 | 1 | 1 | 0 | 0 | 1 | 1 |
| M861 | A6 | A3 | A6 | | | d | 1 | 1 | 1 | 0 | 0 | 1 | 1 |
| M862 | A6 | A4 | A6 | | | au | 1 | 1 | 1 | 0 | 0 | 1 | 1 |
| M863 | A6 | A4 | A6 | | | b | 1 | 1 | 1 | 0 | 0 | 1 | 1 |
| M864 | A6 | A5 | A6 | | | s | 1 | 1 | 1 | 0 | 0 | 1 | 1 |
| M865 | A6 | A5 | A6 | | | b | 1 | 1 | 1 | 0 | 0 | 1 | 1 |
| M866 | A6 | A6 | A6 | | | a | 1 | 1 | 1 | 0 | 0 | 1 | 1 |
| M867 | A6 | A6 | A6 | | | b | 1 | 1 | 1 | 0 | 0 | 1 | 1 |
| M868 | A6 | A7 | A6 | | | a | 1 | 1 | 1 | 0 | 0 | 1 | 1 |
| M869 | A6 | A7 | A6 | | | j | 1 | 1 | 1 | 0 | 0 | 1 | 1 |
| M870 | A6 | A8 | A6 | | | a | 1 | 1 | 1 | 0 | 0 | 1 | 1 |
| M871 | A6 | A8 | A6 | | | b | 1 | 1 | 1 | 0 | 0 | 1 | 1 |
| M872 | A6 | A9 | A6 | | | d | 1 | 1 | 1 | 0 | 0 | 1 | 1 |
| M873 | A6 | A9 | A6 | | | b | 1 | 1 | 1 | 0 | 0 | 1 | 1 |
| M874 | A6 | A9 | A6 | | | w | 1 | 1 | 1 | 0 | 0 | 1 | 1 |
| M875 | A6 | A9 | A6 | | | ae | 1 | 1 | 1 | 0 | 0 | 1 | 1 |
| M876 | A6 | A9 | A6 | | | bb | 1 | 1 | 1 | 0 | 0 | 1 | 1 |
| M877 | A6 | A10 | A6 | | | am | 1 | 1 | 1 | 0 | 0 | 1 | 1 |
| M878 | A6 | A10 | A6 | | | b | 1 | 1 | 1 | 0 | 0 | 1 | 1 |
| M879 | A7 | A1 | A7 | | | c | 1 | 1 | 1 | 0 | 0 | 1 | 1 |
| M880 | A7 | A1 | A7 | | | b | 1 | 1 | 1 | 0 | 0 | 1 | 1 |
| M881 | A7 | A2 | A7 | | | l | 1 | 1 | 1 | 0 | 0 | 1 | 1 |
| M882 | A7 | A2 | A7 | | | b | 1 | 1 | 1 | 0 | 0 | 1 | 1 |
| M883 | A7 | A3 | A7 | | | s | 1 | 1 | 1 | 0 | 0 | 1 | 1 |
| M884 | A7 | A3 | A7 | | | b | 1 | 1 | 1 | 0 | 0 | 1 | 1 |
| M885 | A7 | A3 | A7 | | | r | 1 | 1 | 1 | 0 | 0 | 1 | 1 |
| M886 | A7 | A3 | A7 | | | v | 1 | 1 | 1 | 0 | 0 | 1 | 1 |
| M887 | A7 | A3 | A7 | | | d | 1 | 1 | 1 | 0 | 0 | 1 | 1 |
| M888 | A7 | A4 | A7 | | | aa | 1 | 1 | 1 | 0 | 0 | 1 | 1 |
| M889 | A7 | A4 | A7 | | | b | 1 | 1 | 1 | 0 | 0 | 1 | 1 |
| M890 | A7 | A5 | A7 | | | ad | 1 | 1 | 1 | 0 | 0 | 1 | 1 |
| M891 | A7 | A5 | A7 | | | b | 1 | 1 | 1 | 0 | 0 | 1 | 1 |
| M892 | A7 | A6 | A7 | | | aq | 1 | 1 | 1 | 0 | 0 | 1 | 1 |
| M893 | A7 | A6 | A7 | | | b | 1 | 1 | 1 | 0 | 0 | 1 | 1 |
| M894 | A7 | A7 | A7 | | | a | 1 | 1 | 1 | 0 | 0 | 1 | 1 |
| M895 | A7 | A7 | A7 | | | j | 1 | 1 | 1 | 0 | 0 | 1 | 1 |
| M896 | A7 | A8 | A7 | | | a | 1 | 1 | 1 | 0 | 0 | 1 | 1 |
| M897 | A7 | A8 | A7 | | | b | 1 | 1 | 1 | 0 | 0 | 1 | 1 |
| M898 | A7 | A9 | A7 | | | d | 1 | 1 | 1 | 0 | 0 | 1 | 1 |
| M899 | A7 | A9 | A7 | | | b | 1 | 1 | 1 | 0 | 0 | 1 | 1 |
| M900 | A7 | A9 | A7 | | | w | 1 | 1 | 1 | 0 | 0 | 1 | 1 |
| M901 | A7 | A9 | A7 | | | ae | 1 | 1 | 1 | 0 | 0 | 1 | 1 |
| M902 | A7 | A9 | A7 | | | bb | 1 | 1 | 1 | 0 | 0 | 1 | 1 |
| M903 | A7 | A10 | A7 | | | am | 1 | 1 | 1 | 0 | 0 | 1 | 1 |
| M904 | A7 | A10 | A7 | | | b | 1 | 1 | 1 | 0 | 0 | 1 | 1 |
| M905 | A8 | A1 | A8 | | | av | 1 | 1 | 1 | 0 | 0 | 1 | 1 |
| M906 | A8 | A1 | A8 | | | b | 1 | 1 | 1 | 0 | 0 | 1 | 1 |
| M907 | A8 | A2 | A8 | | | az | 1 | 1 | 1 | 0 | 0 | 1 | 1 |
| M908 | A8 | A2 | A8 | | | b | 1 | 1 | 1 | 0 | 0 | 1 | 1 |
| M909 | A8 | A3 | A8 | | | al | 1 | 1 | 1 | 0 | 0 | 1 | 1 |
| M910 | A8 | A3 | A8 | | | b | 1 | 1 | 1 | 0 | 0 | 1 | 1 |
| M911 | A8 | A3 | A8 | | | t | 1 | 1 | 1 | 0 | 0 | 1 | 1 |
| M912 | A8 | A3 | A8 | | | v | 1 | 1 | 1 | 0 | 0 | 1 | 1 |
| M913 | A8 | A3 | A8 | | | d | 1 | 1 | 1 | 0 | 0 | 1 | 1 |
| M914 | A8 | A4 | A8 | | | au | 1 | 1 | 1 | 0 | 0 | 1 | 1 |
| M915 | A8 | A4 | A8 | | | b | 1 | 1 | 1 | 0 | 0 | 1 | 1 |
| M916 | A8 | A5 | A8 | | | bb | 1 | 1 | 1 | 0 | 0 | 1 | 1 |
| M917 | A8 | A5 | A8 | | | b | 1 | 1 | 1 | 0 | 0 | 1 | 1 |
| M918 | A8 | A6 | A8 | | | bd | 1 | 1 | 1 | 0 | 0 | 1 | 1 |
| M919 | A8 | A6 | A8 | | | b | 1 | 1 | 1 | 0 | 0 | 1 | 1 |
| M920 | A8 | A7 | A8 | | | a | 1 | 1 | 1 | 0 | 0 | 1 | 1 |
| M921 | A8 | A7 | A8 | | | bc | 1 | 1 | 1 | 0 | 0 | 1 | 1 |
| M922 | A8 | A8 | A8 | | | a | 1 | 1 | 1 | 0 | 0 | 1 | 1 |
| M923 | A8 | A8 | A8 | | | b | 1 | 1 | 1 | 0 | 0 | 1 | 1 |
| M924 | A8 | A9 | A8 | | | d | 1 | 1 | 1 | 0 | 0 | 1 | 1 |
| M925 | A8 | A9 | A8 | | | b | 1 | 1 | 1 | 0 | 0 | 1 | 1 |
| M926 | A8 | A9 | A8 | | | w | 1 | 1 | 1 | 0 | 0 | 1 | 1 |
| M927 | A8 | A9 | A8 | | | ae | 1 | 1 | 1 | 0 | 0 | 1 | 1 |
| M928 | A8 | A9 | A8 | | | bb | 1 | 1 | 1 | 0 | 0 | 1 | 1 |
| M929 | A8 | A10 | A8 | | | am | 1 | 1 | 1 | 0 | 0 | 1 | 1 |
| M930 | A8 | A10 | A8 | | | b | 1 | 1 | 1 | 0 | 0 | 1 | 1 |
| M931 | A9 | A1 | A9 | | | an | 1 | 1 | 1 | 0 | 0 | 1 | 1 |
| M932 | A9 | A1 | A9 | | | b | 1 | 1 | 1 | 0 | 0 | 1 | 1 |
| M933 | A9 | A2 | A9 | | | as | 1 | 1 | 1 | 0 | 0 | 1 | 1 |
| M934 | A9 | A2 | A9 | | | b | 1 | 1 | 1 | 0 | 0 | 1 | 1 |
| M935 | A9 | A3 | A9 | | | x | 1 | 1 | 1 | 0 | 0 | 1 | 1 |
| M936 | A9 | A3 | A9 | | | b | 1 | 1 | 1 | 0 | 0 | 1 | 1 |
| M937 | A9 | A3 | A9 | | | t | 1 | 1 | 1 | 0 | 0 | 1 | 1 |
| M938 | A9 | A3 | A9 | | | v | 1 | 1 | 1 | 0 | 0 | 1 | 1 |
| M939 | A9 | A3 | A9 | | | d | 1 | 1 | 1 | 0 | 0 | 1 | 1 |
| M940 | A9 | A4 | A9 | | | au | 1 | 1 | 1 | 0 | 0 | 1 | 1 |
| M941 | A9 | A4 | A9 | | | b | 1 | 1 | 1 | 0 | 0 | 1 | 1 |
| M942 | A9 | A5 | A9 | | | s | 1 | 1 | 1 | 0 | 0 | 1 | 1 |
| M943 | A9 | A5 | A9 | | | b | 1 | 1 | 1 | 0 | 0 | 1 | 1 |
| M944 | A9 | A6 | A9 | | | a | 1 | 1 | 1 | 0 | 0 | 1 | 1 |
| M945 | A9 | A6 | A9 | | | b | 1 | 1 | 1 | 0 | 0 | 1 | 1 |
| M946 | A9 | A7 | A9 | | | a | 1 | 1 | 1 | 0 | 0 | 1 | 1 |
| M947 | A9 | A7 | A9 | | | j | 1 | 1 | 1 | 0 | 0 | 1 | 1 |
| M948 | A9 | A8 | A9 | | | a | 1 | 1 | 1 | 0 | 0 | 1 | 1 |
| M949 | A9 | A8 | A9 | | | b | 1 | 1 | 1 | 0 | 0 | 1 | 1 |
| M950 | A9 | A9 | A9 | | | d | 1 | 1 | 1 | 0 | 0 | 1 | 1 |
| M951 | A9 | A9 | A9 | | | b | 1 | 1 | 1 | 0 | 0 | 1 | 1 |
| M952 | A9 | A9 | A9 | | | w | 1 | 1 | 1 | 0 | 0 | 1 | 1 |
| M953 | A9 | A9 | A9 | | | ae | 1 | 1 | 1 | 0 | 0 | 1 | 1 |
| M954 | A9 | A9 | A9 | | | bb | 1 | 1 | 1 | 0 | 0 | 1 | 1 |
| M 955 | A9 | A10 | A9 | | | am | 1 | 1 | 1 | 0 | 0 | 1 | 1 |
| M956 | A9 | A10 | A9 | | | b | 1 | 1 | 1 | 0 | 0 | 1 | 1 |
| M957 | A10 | A1 | A10 | | | b | 1 | 1 | 1 | 0 | 0 | 1 | 1 |
| M958 | A1 | A1 | A2 | | | b | 1 | 1 | 1 | 0 | 0 | 1 | 1 |
| M959 | A2 | A3 | A3 | | | b | 1 | 1 | 1 | 0 | 0 | 1 | 1 |
| M960 | A1 | A3 | A1 | | | b | 2 | 1 | 2 | 0 | 0 | 1 | 1 |
| M961 | A1 | A8 | A1 | | | b | 2 | 1 | 2 | 0 | 0 | 1 | 1 |
| M962 | A1 | A1 | A1 | A1 | A1 | b | 1 | 1 | 1 | 1 | 1 | 1 | 1 |
| M963 | A1 | A2 | A1 | A1 | A1 | b | 1 | 1 | 1 | 1 | 1 | 1 | 1 |
| M964 | A1 | A3 | A1 | A1 | A1 | b | 1 | 1 | 1 | 1 | 1 | 1 | 1 |
| M965 | A1 | A3 | A1 | A1 | A1 | b | 2 | 1 | 2 | 1 | 1 | 1 | 1 |
| M966 | A1 | A3 | A1 | A1 | A1 | v | 1 | 1 | 1 | 1 | 1 | 1 | 1 |
| M967 | A1 | A8 | A1 | A1 | A1 | b | 1 | 1 | 1 | 1 | 1 | 1 | 1 |
| M968 | A1 | A9 | A1 | A1 | A1 | b | 1 | 1 | 1 | 1 | 1 | 1 | 1 |
| M969 | A1 | A1 | A1 | A2 | A2 | b | 1 | 1 | 1 | 1 | 1 | 1 | 1 |
| M970 | A1 | A2 | A1 | A2 | A2 | b | 1 | 1 | 1 | 1 | 1 | 1 | 1 |
| M971 | A1 | A3 | A1 | A2 | A2 | b | 3 | 1 | 3 | 1 | 1 | 1 | 1 |
| M972 | A1 | A3 | A1 | A3 | A3 | b | 1 | 1 | 1 | 1 | 1 | 1 | 1 |
| M973 | A1 | A10 | A1 | A1 | A1 | b | 1 | 1 | 1 | 1 | 1 | 1 | 1 |
| M974 | A1 | A3 | A1 | A1 | A1 | b | 1 | 1 | 1 | 1 | 1 | 2 | 2 |
| M975 | A1 | A3 | A1 | A1 | A1 | t | 1 | 1 | 1 | 1 | 1 | 2 | 2 |
| M976 | A1 | A10 | A1 | A1 | A1 | b | 1 | 1 | 1 | 1 | 1 | 2 | 2 |
| M977 | A2 | A2 | A2 | A2 | A2 | b | 1 | 1 | 1 | 1 | 1 | 1 | 1 |
| M978 | A2 | A1 | A2 | A2 | A2 | b | 1 | 1 | 1 | 1 | 1 | 1 | 1 |
| M979 | A2 | A1 | A2 | A1 | A1 | b | 1 | 1 | 1 | 1 | 1 | 1 | 1 |
| M980 | A2 | A3 | A2 | A1 | A1 | b | 1 | 1 | 1 | 1 | 1 | 1 | 1 |
| M981 | A2 | A8 | A2 | A1 | A1 | b | 1 | 1 | 1 | 1 | 1 | 1 | 1 |
| M982 | A2 | A9 | A2 | A1 | A1 | b | 1 | 1 | 1 | 1 | 1 | 1 | 1 |
| M983 | A2 | A10 | A2 | A1 | A1 | b | 1 | 1 | 1 | 1 | 1 | 1 | 1 |
| M984 | A2 | A3 | A2 | A1 | A1 | b | 1 | 1 | 1 | 1 | 1 | 2 | 2 |
| M985 | A2 | A6 | A2 | A2 | A2 | t | 1 | 1 | 1 | 1 | 1 | 1 | 1 |
| M986 | A3 | A3 | A3 | A3 | A3 | v | 1 | 1 | 1 | 1 | 1 | 1 | 1 |
| M987 | A3 | A2 | A3 | A3 | A3 | b | 1 | 1 | 1 | 1 | 1 | 1 | 1 |
| M988 | A3 | A2 | A3 | A1 | A1 | c | 1 | 1 | 1 | 1 | 1 | 1 | 1 |
| M989 | A3 | A2 | A3 | A3 | A1 | u | 1 | 1 | 1 | 1 | 1 | 1 | 1 |
| M990 | A3 | A2 | A1 | A1 | A1 | b | 1 | 1 | 1 | 1 | 1 | 1 | 1 |
| M991 | A3 | A7 | A3 | A1 | A1 | s | 1 | 1 | 1 | 1 | 1 | 1 | 1 |
| M992 | A3 | A5 | A3 | A1 | A1 | t | 1 | 1 | 1 | 1 | 1 | 1 | 1 |
| M993 | A3 | A10 | A3 | A1 | A1 | b | 1 | 1 | 1 | 1 | 1 | 1 | 1 |
| M994 | A4 | A4 | A4 | A4 | A4 | bc | 1 | 1 | 1 | 1 | 1 | 1 | 1 |
| M995 | A4 | A2 | A4 | A1 | A1 | b | 1 | 1 | 1 | 1 | 1 | 1 | 1 |
| M996 | A6 | A6 | A6 | A6 | A6 | bd | 1 | 1 | 1 | 1 | 1 | 1 | 1 |
| M997 | A6 | A3 | A6 | A1 | A1 | a | 1 | 1 | 1 | 1 | 1 | 2 | 2 |
| M998 | A8 | A2 | A8 | A1 | A1 | b | 1 | 1 | 1 | 1 | 1 | 2 | 2 |
| M999 | A9 | A7 | A9 | A2 | A2 | am | 1 | 1 | 1 | 1 | 1 | 1 | 1 |
| M 1000 | A10 | A2 | A1 | A1 | A1 | u | 1 | 1 | 1 | 1 | 1 | 1 | 1 |
| M1001 | | | | A1 | | b | 0 | 0 | 0 | 1 | 0 | 1 | 0 |
| M 1002 | | | | A1 | | t | 0 | 0 | 0 | 1 | 0 | 1 | 0 |
| M 1003 | | | | A1 | | v | 0 | 0 | 0 | 1 | 0 | 1 | 0 |
| M 1004 | | | | A1 | | am | 0 | 0 | 0 | 1 | 0 | 1 | 0 |
| M 1005 | | | | A1 | | as | 0 | 0 | 0 | 1 | 0 | 1 | 0 |
| M 1006 | | | | A1 | | m | 0 | 0 | 0 | 1 | 0 | 1 | 0 |
| M 1007 | | | | A1 | | b | 0 | 0 | 0 | 1 | 0 | 2 | 0 |
| M 1008 | | | | A2 | | a | 0 | 0 | 0 | 1 | 0 | 1 | 0 |
| M 1009 | | | | A3 | | s | 0 | 0 | 0 | 1 | 0 | 2 | 0 |
| M1010 | | | | A4 | | q | 0 | 0 | 0 | 1 | 0 | 1 | 0 |
| M1011 | | | | A5 | | b | 0 | 0 | 0 | 1 | 0 | 2 | 0 |
| M1012 | | | | A6 | | b | 0 | 0 | 0 | 1 | 0 | 1 | 0 |
| M1013 | | | | A7 | | w | 0 | 0 | 0 | 1 | 0 | 2 | 0 |
| M1014 | | | | A8 | | t | 0 | 0 | 0 | 1 | 0 | 1 | 0 |
| M1015 | | | | A9 | | c | 0 | 0 | 0 | 1 | 0 | 2 | 0 |
| M1016 | | A1 | | A1 | A1 | b | 0 | 1 | 0 | 1 | 1 | 1 | 1 |
| M1017 | | A1 | | A1 | A1 | b | 0 | 1 | 0 | 1 | 1 | 2 | 2 |
| M1018 | | A3 | | A1 | A1 | b | 0 | 1 | 0 | 1 | 1 | 1 | 1 |
| M1019 | | A10 | | A1 | A1 | b | 0 | 1 | 0 | 1 | 1 | 1 | 1 |
| M 1020 | | A3 | | A2 | A2 | b | 0 | 1 | 0 | 1 | 1 | 1 | 1 |
| M1021 | | A1 | | | | | 0 | 1 | 0 | 0 | 0 | 1 | 1 |
| M 1022 | | A2 | | | | | 0 | 1 | 0 | 0 | 0 | 1 | 1 |
| M 1023 | | A3 | | | | | 0 | 1 | 0 | 0 | 0 | 1 | 1 |
| M 1024 | | A4 | | | | | 0 | 1 | 0 | 0 | 0 | 1 | 1 |
| M 1025 | | A5 | | | | | 0 | 1 | 0 | 0 | 0 | 1 | 1 |
| M 1026 | | A6 | | | | | 0 | 1 | 0 | 0 | 0 | 1 | 1 |
| M 1027 | | A7 | | | | | 0 | 1 | 0 | 0 | 0 | 1 | 1 |
| M 1028 | | A8 | | | | | 0 | 1 | 0 | 0 | 0 | 1 | 1 |
| M 1029 | | A9 | | | | | 0 | 1 | 0 | 0 | 0 | 1 | 1 |
| M 1030 | | A10 | | | | | 0 | 1 | 0 | 0 | 0 | 1 | 1 |

Bevorzugte konkrete Ausführungsformen der Struktureinheiten der Formel (I) sind in der folgenden Tabelle gezeigt:

| | |
|---|---|
| 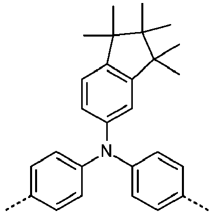 | 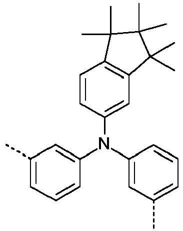 |
| (1) | (2) |
| 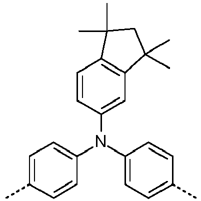 | 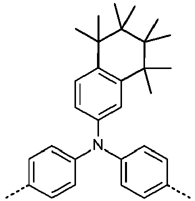 |
| (3) | (4) |
| 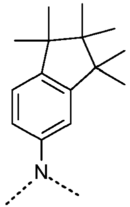 | 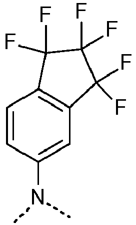 |
| (5) | (6) |
| 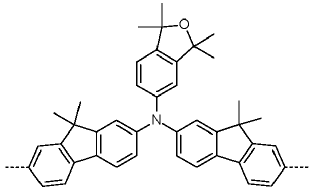 | 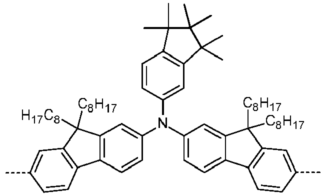 |
| (7) | (8) |
| 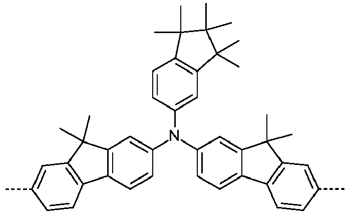 | 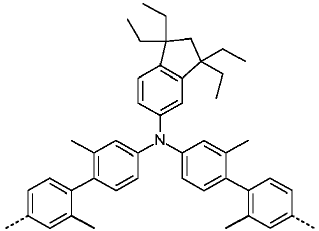 |
| (9) | (10) |
| 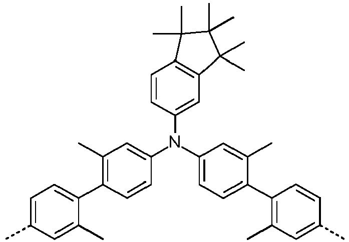 | 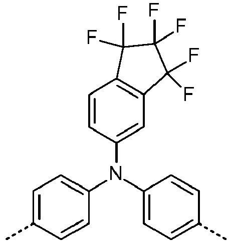 |
| (11) | (12) |
| 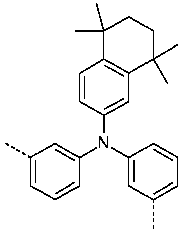 | 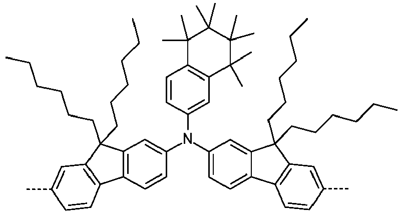 |
| (13) | (14) |
| 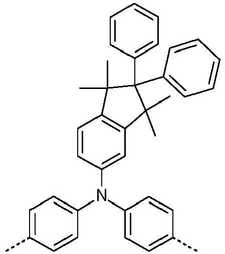 | 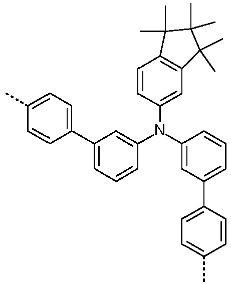 |
| (15) | (16) |
| 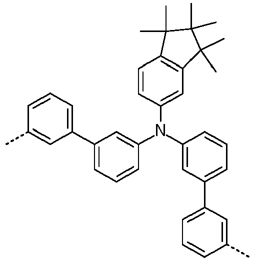 | 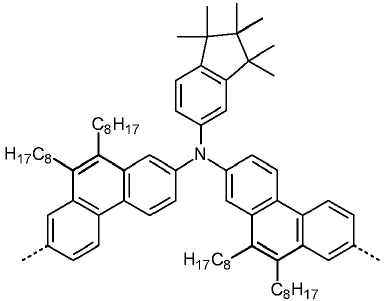 |
| (17) | (18) |
| 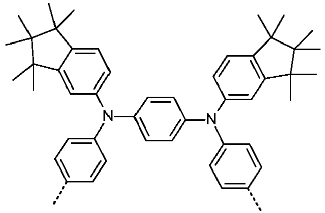 | 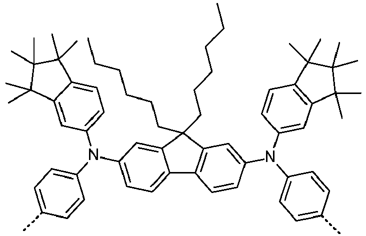 |
| (19) | (20) |
| 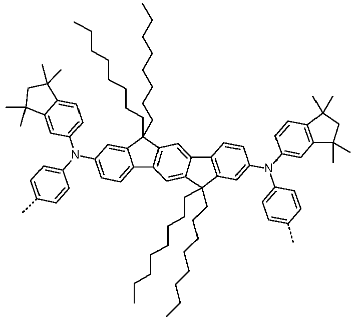 | 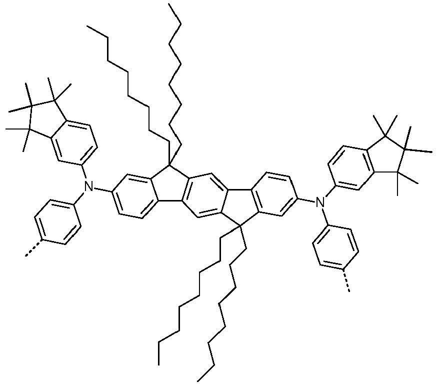 |
| (21) | (22) |
| 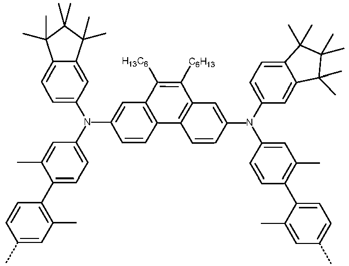 | 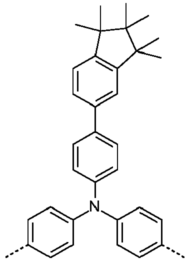 |
| (23) | (24) |
| 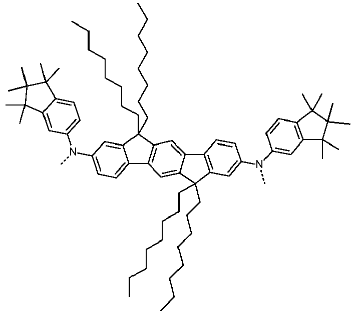 | 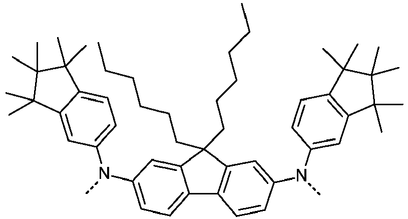 |
| (25) | (26) |
| 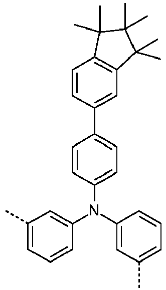 | 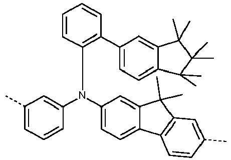 |
| (27) | (28) |
| 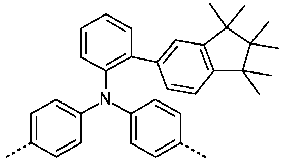 | 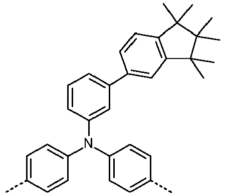 |
| (29) | (30) |
| 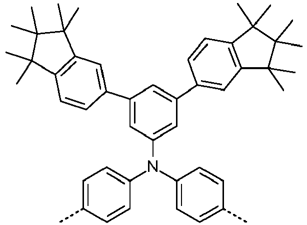 | 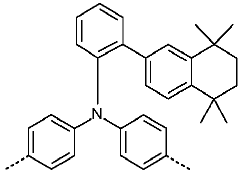 |
| (31) | (32) |
| 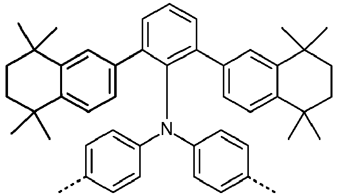 | 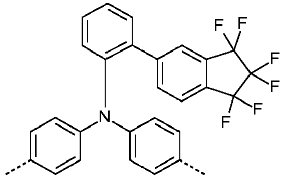 |
| (33) | (34) |
| 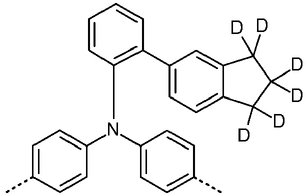 | 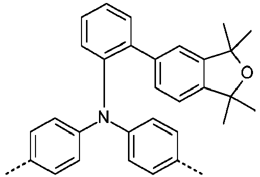 |
| (35) | (36) |
| 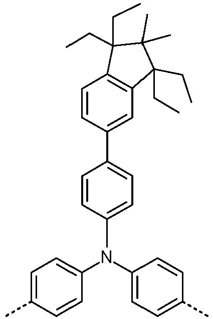 | 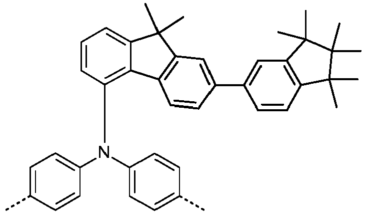 |
| (37) | (38) |
| 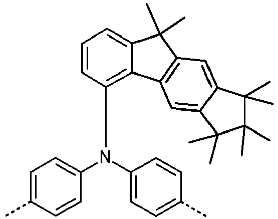 | 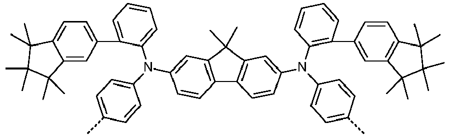 |
| (39) | (40) |
| 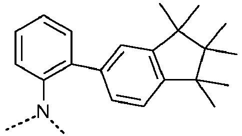 | 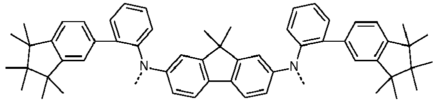 |
| (41) | (42) |

Der Anteil an Struktureinheiten der Formel (I) im Polymer liegt im Bereich von 1 bis 100 mol%. In einer bevorzugten Ausführungsform liegt der Anteil an Struktureinheiten der Formel (I) im Polymer im Bereich von 30 bis 70 mol%, besonders bevorzugt im Bereich von 40 bis 60 mol%, bezogen auf 100 mol% aller copolymerisierbaren Monomere, die im Polymer als Struktureinheiten enthalten sind, d.h., dass das erfindungsgemäße Polymer neben einer oder mehreren Struktureinheiten der Formel (I) noch weitere, von den Struktureinheiten der Formeln (I) verschiedene Struktureinheiten aufweist.

Diese von den Struktureinheiten der Formeln (I) verschiedenen Struktureinheiten sind unter anderem solche, wie sie in der WO 2002/077060 A1, in der WO 2005/014689 A2 und in der WO 2013/156130 offenbart und aufgelistet sind. Diese sind durch Zitat in die Offenbarung der vorliegenden Patentanmeldung einbezogen. Die weiteren Struktureinheiten können beispielsweise aus den folgenden Klassen stammen:

| | |
|---|---|
| Gruppe 1: | Einheiten, welche die Lochinjektions- und/oder Lochtransporteigenschaften der Polymere beeinflussen; |
| Gruppe 2: | Einheiten, welche die Elektroneninjektions- und/oder Elektronentransporteigenschaften der Polymere beeinflussen; |
| Gruppe 3: | Einheiten, die Kombinationen von Einzeleinheiten der Gruppe 1 und Gruppe 2 aufweisen; |
| Gruppe 4: | Einheiten, welche die Emissionscharakteristik insoweit verändern, dass Elektrophosphoreszenz statt Elektrofluoreszenz erhalten werden kann; |
| Gruppe 5: | Einheiten, welche den Übergang vom Singulett- zum Triplettzustand verbessern; |
| Gruppe 6: | Einheiten, welche die Emissionsfarbe der resultierenden Polymere beeinflussen; |
| Gruppe 7: | Einheiten, welche typischerweise als Polymergrundgerüst (Backbone) verwendet werden; |
| Gruppe 8: | Einheiten welche die Delokalisierung der π-Elektronen im Polymer unterbrechen und damit die Konjugationlänge im Polymer verkürzen. |

Bevorzugte erfindungsgemäße Polymere sind solche, bei denen mindestens eine Struktureinheit Ladungstransporteigenschaften aufweist, d.h. die Einheiten aus der Gruppe 1 und/oder 2 enthalten.

Struktureinheiten aus der Gruppe 1, die Lochinjektions- und/oder Lochtransporteigenschaften aufweisen, sind beispielsweise Triarylamin-, Benzidin-, Tetraaryl-para-phenylendiamin-, Triarylphosphin-, Phenothiazin-, Phenoxazin-, Dihydrophenazin-, Thianthren-, Dibenzo-para-dioxin-, Phenoxathiin-, Carbazol-, Azulen-, Thiophen-, Pyrrol- und Furanderivate und weitere O-, S- oder N-haltige Heterocyclen.

Struktureinheiten aus der Gruppe 2, die Elektroneninjektions- und/oder Elektronentransporteigenschaften aufweisen, sind beispielsweise Pyridin-, Pyrimidin-, Pyridazin-, Pyrazin-, Oxadiazol-, Chinolin-, Chinoxalin-, Anthracen-, Benzanthracen-, Pyren-, Perylen-, Benzimidazol-, Triazin-, Keton-, Phosphinoxid- und Phenazinderivate, aber auch Triarylborane und weitere O-, S- oder N-haltige Heterocyclen.

Es kann bevorzugt sein, wenn in den erfindungsgemäßen Polymeren Einheiten aus der Gruppe 3 enthalten sind, in denen Strukturen, welche die Lochmobilität und welche die Elektronenmobilität erhöhen (also Einheiten aus Gruppe 1 und 2), direkt aneinander gebunden sind oder Strukturen enthalten sind, die sowohl die Lochmobilität als auch die Elektronenmobilität erhöhen. Einige dieser Einheiten können als Emitter dienen und verschieben die Emissionsfarbe ins Grüne, Gelbe oder Rote. Ihre Verwendung eignet sich also beispielsweise für die Erzeugung anderer Emissionsfarben aus ursprünglich blau emittierenden Polymeren.

Struktureinheiten der Gruppe 4 sind solche, welche auch bei Raumtemperatur mit hoher Effizienz aus dem Triplettzustand Licht emittieren können, also Elektrophosphoreszenz statt Elektrofluoreszenz zeigen, was häufig eine Steigerung der Energieeffizienz bewirkt. Hierfür eignen sich zunächst Verbindungen, welche Schweratome mit einer Ordnungszahl von mehr als 36 enthalten. Bevorzugt sind Verbindungen, welche d- oder f-Übergangsmetalle enthalten, die die o. g. Bedingung erfüllen. Besonders bevorzugt sind hier entsprechende Struktureinheiten, welche Elemente der Gruppen 8 bis 10 (Ru, Os, Rh, Ir, Pd, Pt) enthalten. Als Struktureinheiten für die erfindungsgemäßen Polymere kommen hier z.B. verschiedene Komplexe in Frage, wie sie z.B. in der WO 02/068435 A1, der WO 02/081488 A1, der EP 1239526 A2 und der WO 2004/026886 A2 beschrieben werden. Entsprechende Monomere werden in der WO 02/068435 A1 und in der WO 2005/042548 A1 beschrieben.

Struktureinheiten der Gruppe 5 sind solche, welche den Übergang vom Singulett- zum Triplettzustand verbessern und welche, unterstützend zu den Strukturelementen der Gruppe 4 eingesetzt, die Phosphoreszenzeigenschaften dieser Strukturelemente verbessern. Hierfür kommen insbesondere Carbazol- und überbrückte Carbazoldimereinheiten in Frage, wie sie z.B. in der WO 2004/070772 A2 und der WO 2004/113468 A1 beschrieben werden. Weiterhin kommen hierfür Ketone, Phosphinoxide, Sulfoxide, Sulfone, Silan-Derivate und ähnliche Verbindungen in Frage, wie sie z.B. in der WO 2005/040302 A1 beschrieben werden.

Struktureinheiten der Gruppe 6 sind neben den oben genannten solche, die mindestens noch eine weitere aromatische oder eine andere konjugierte Struktur aufweisen, welche nicht unter die o. g. Gruppen fallen, d. h. die die Ladungsträgermobilitäten nur wenig beeinflussen, die keine metallorganischen Komplexe sind oder die keinen Einfluss auf den Singulett-Triplett-Übergang haben. Derartige Strukturelemente können die Emissionsfarbe der resultierenden Polymere beeinflussen. Je nach Einheit können sie daher auch als Emitter eingesetzt werden. Bevorzugt sind dabei aromatische Strukturen mit 6 bis 40 C-Atomen oder auch Tolan-, Stilben- oder Bisstyrylarylenderivate, die jeweils mit einem oder mehreren Resten R substituiert sein können. Besonders bevorzugt ist dabei der Einbau von 1,4- oder 9,10-Anthrylen-, 1,6-, 2,7- oder 4,9-Pyrenylen-, 3,9- oder 3,10-Perylenylen-, 4,4'-Tolanylen-, 4,4'-Stilbenylen-, Benzothiadiazol- und ent-sprechenden Sauerstoffderivaten, Chinoxalin-, Phenothiazin-, Phenoxazin-, Dihydrophenazin-, Bis(thiophenyl)arylen-, Oligo(thiophenylen)-, Phenazin-, Rubren-, Pentacen- oder Perylenderivaten, die vorzugsweise substituiert sind, oder vorzugsweise konjugierte Push-Pull-Systeme (Systeme, die mit Donor- und Akzeptorsubstituenten substituiert sind) oder Systeme wie Squarine oder Chinacridone, die vorzugsweise substituiert sind.

Struktureinheiten der Gruppe 7 sind Einheiten, die aromatische Strukturen mit 6 bis 40 C-Atomen beinhalten, welche typischerweise als Polymergrundgerüst (Backbone) verwendet werden. Dies sind beispielsweise 4,5-Dihydropyrenderivate, 4,5,9,10-Tetrahydropyrenderivate, Fluorenderivate, 9,9`-Spirobifluorenderivate, Phenanthrenderivate, 9,10-Dihydrophenanthrenderivate, 5,7-Dihydrodibenzooxepinderivate und cis- und trans-Indenofluorenderivate aber auch 1,2-, 1,3- oder 1,4-Phenylen-, 1,2-, 1,3- oder 1,4-Naphthylen-, 2,2'-, 3,3'- oder 4,4'-Biphenylylen-, 2,2"-, 3,3"- oder 4,4"-Terphenylylen, 2,2'-, 3,3'- oder 4,4'-Bi-1,1'-naphthylylen- oder 2,2"'-, 3,3‴- oder 4,4‴-Quarterphenylylenderivate.

Struktureinheiten der Gruppe 8 sind solche, die konjugationsunterbrechende Eigenschaften besitzen, z.B. durch meta-Verknüpfung, sterische Hinderung oder Verwendung gesättigter Kohlenstoff- oder Siliziumatome. Solche Verbindung werden z.B. in der WO2006/063852, der WO 2012/048778 und der WO 2013/093490 offenbart. Die konjugationsunterbrechenden Eigenschaften der Struktureinheiten der Gruppe 8 zeigen sich unter anderem durch eine Blauverschiebung der Absorptionskante des Polymers.

Bevorzugt sind erfindungsgemäße Polymere, die gleichzeitig neben Struktureinheiten der Formel (I) zusätzlich noch ein oder mehrere Einheiten ausgewählt aus den Gruppen 1 bis 8 enthalten. Besonders bevorzugt sind die Struktureinheiten der Gruppen 1, 7 und 8. Es kann ebenfalls bevorzugt sein, wenn gleichzeitig mehr als eine weitere Struktureinheit aus einer der oben genannten Gruppen vorliegt.

Enthält das erfindungsgemäße Polymer eine oder mehrere Einheiten ausgewählt aus den Gruppen 1 bis 8, so kann eine oder mehrere dieser Einheiten, vorzugsweise eine Einheit aus der Gruppe 1, eine oder mehrere, vorzugsweise eine, vernetzbare Gruppe aufweisen.

Die erfindungsgemäßen Polymere sind entweder Homopolymere aus Struktureinheiten der Formel (I) oder Copolymere. Die erfindungsgemäßen Polymere können linear oder verzweigt sein, vorzugsweise linear. Erfindungsgemäße Copolymere können dabei neben einer oder mehreren Struktureinheiten der Formel (I) potentiell eine oder mehrere weitere Strukturen aus den oben aufgeführten Gruppen 1 bis 8 besitzen.

Die erfindungsgemäßen Copolymere können statistische, alternierende oder blockartige Strukturen aufweisen oder auch mehrere dieser Strukturen abwechselnd besitzen. Besonders bevorzugt weisen die erfindungsgemäßen Copolymere statistische oder alternierende Strukturen auf. Besonders bevorzugt sind die Copolymere statistische oder alternierende Copolymere. Wie Copolymere mit blockartigen Strukturen erhalten werden können und welche weiteren Strukturelemente dafür besonders bevorzugt sind, ist beispielsweise ausführlich in der WO 2005/014688 A2 beschrieben. Diese ist via Zitat Bestandteil der vorliegenden Anmeldung. Ebenso sei an dieser Stelle nochmals hervorgehoben, dass das Polymer auch dendritische Strukturen haben kann.

In einer weiteren Ausführungsform der vorliegenden Erfindung enthalten die erfindungsgemäßen Polymere mindestens eine, vorzugsweise eine Struktureinheit, die eine vernetzbare Gruppe Q enthält.

"Vernetzbare Gruppe Q" im Sinne der vorliegenden Erfindung bedeutet eine funktionelle Gruppe, die in der Lage ist, eine Reaktion einzugehen und so eine unlösliche Verbindung zu bilden. Die Reaktion kann dabei mit einer weiteren, gleichen Gruppe Q, einer weiteren, verschiedenen Gruppe Q oder einem beliebigen anderen Teil derselben oder einer anderen Polymerkette erfolgen. Bei der vernetzbaren Gruppe handelt es sich somit um eine reaktive Gruppe. Dabei erhält man als Ergebnis der Reaktion der vernetzbaren Gruppe ein entsprechend vernetztes Polymer. Die chemische Reaktion kann auch in der Schicht durchgeführt werden, wobei eine unlösliche Schicht entsteht. Die Vernetzung kann gewöhnlich durch Wärme oder durch UV-, Mikrowellen-, Röntgen- oder Elektronenstrahlung unterstützt werden, gegebenenfalls in Gegenwart eines Initiators. "Unlöslich" im Sinne der vorliegenden Erfindung bedeutet vorzugsweise, dass das erfindungsgemäße Polymer nach der Vernetzungsreaktion, also nach der Reaktion der vernetzbaren Gruppen, bei Raumtemperatur in einem organischen Lösungsmittel eine Löslichkeit aufweist, die mindestens einen Faktor 3, vorzugsweise mindestens einen Faktor 10, geringer ist als die des entsprechenden, nicht-vernetzten, erfindungsgemäßen Polymers in demselben organischen Lösungsmittel.

Die vernetzbare Gruppe Q kann als solche über ein entsprechend mit der vernetzbaren Gruppe substituiertes Monomer in das erfindungsgemäße Polymer eingeführt werden. Alternativ und in bestimmten Fällen ebenfalls bevorzugt kann die vernetzbare Gruppe Q über eine Vorläufergruppe Q* (precursor-Gruppe), die Teil eines Monomers ist, in das Polymer eingeführt werden. In diesem Fall trägt das erhaltene Polymer zunächst die Vorläufergruppe Q*. In einer Reaktion am Polymer wird dann die Gruppe Q* in die eigentliche vernetzbare Gruppe Q überführt. Ein Beispiel für eine derartige Vorläufergruppe Q* ist eine endständige Aldehydgruppe, die durch beispielsweise eine Wittig-Reaktion in eine endständige Alkenylgruppe überführt werden kann. Letztere ist dann die eigentliche vernetzbare Gruppe Q.

Die Struktureinheit, die die vernetzbare Gruppe Q trägt, kann dabei in einer ersten Ausführungsform ausgewählt werden aus den Struktureinheiten der Formel (I).

Bevorzugte Struktureinheiten entsprechen dabei einer der folgenden Formeln (I-Q-1) bis (I-Q-6) 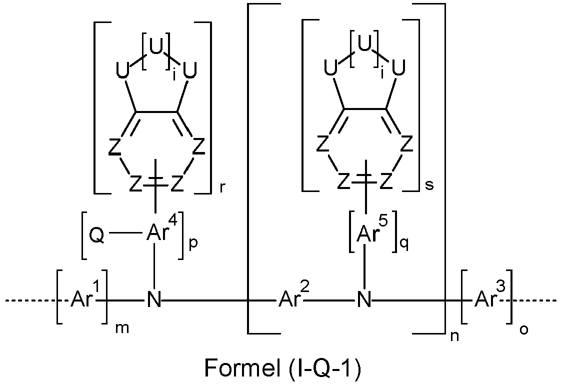 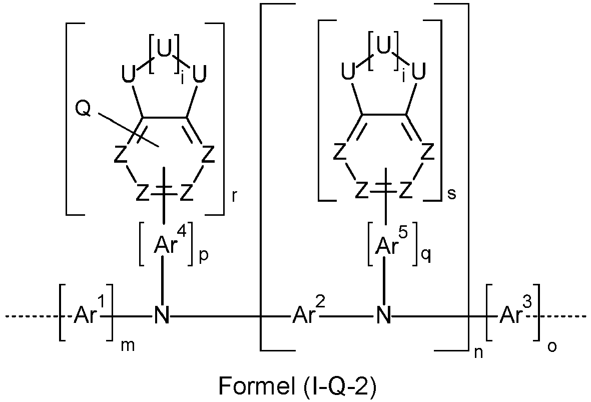 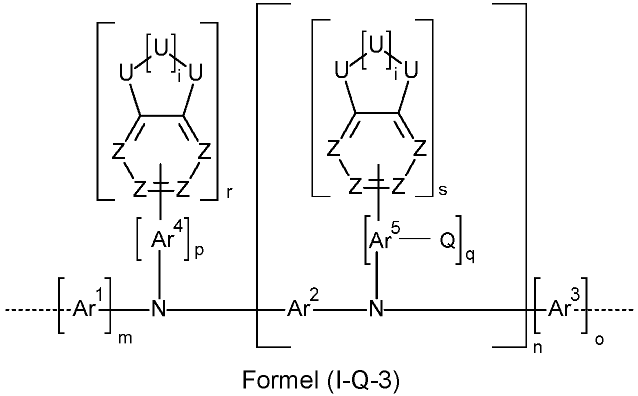 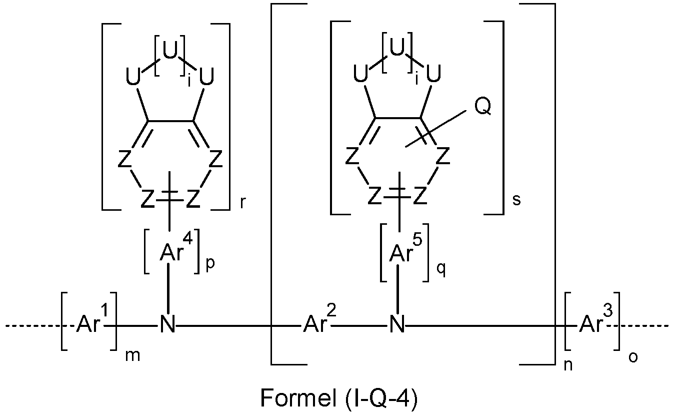 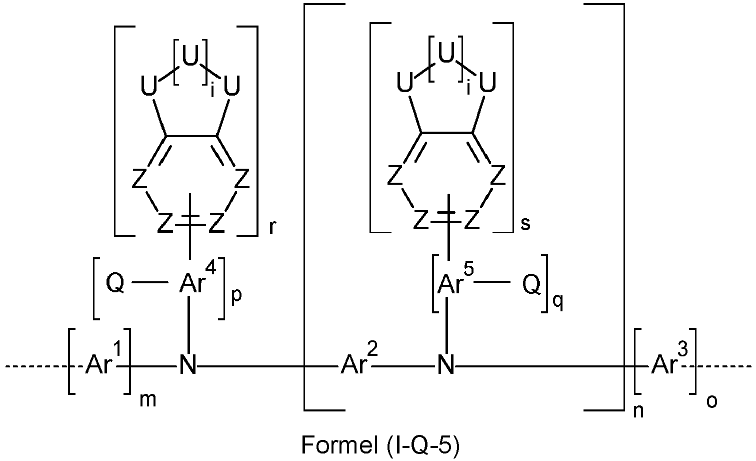 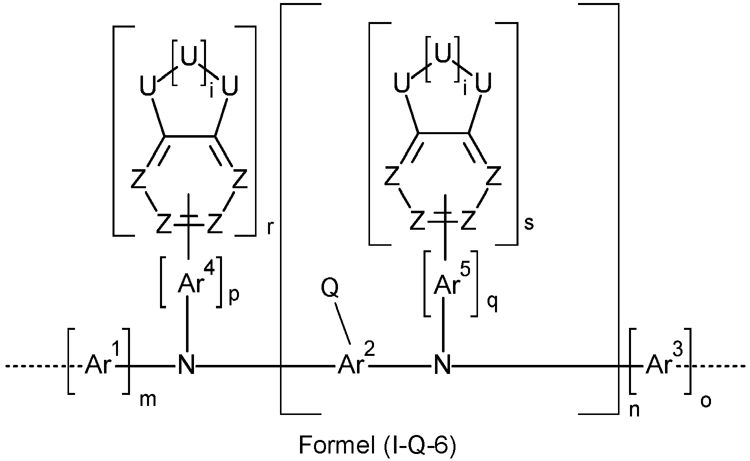 wobei Q eine vernetzbare Gruppe ist und bevorzugt definiert ist wie in den unten angegebenen bevorzugten Ausführungsformen, und wobei die sonstigen Variablen definiert sind wie oben.

Besonders bevorzugte Struktureinheiten der Formel (I), die eine vernetzbare Gruppe Q umfassen, sind dabei die folgenden Struktureinheiten:

| | |
|---|---|
| 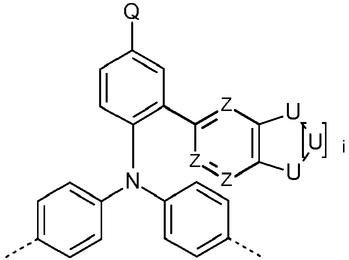 | 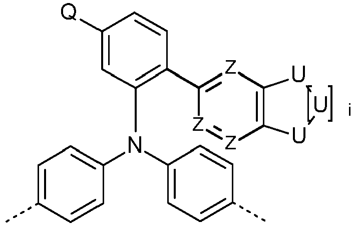 |
| Formel (I-Q-7) | Formel (I-Q-8) |
| 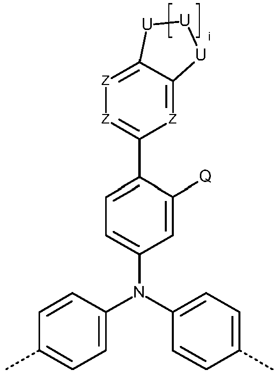 | 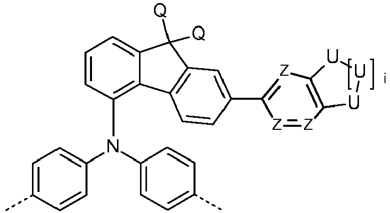 |
| Formel (I-Q-9) | Formel (I-Q-10) |
| 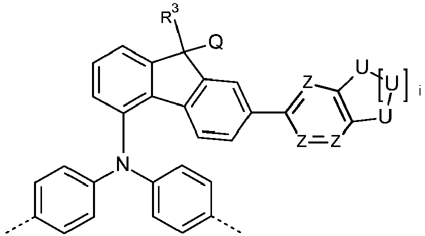 | 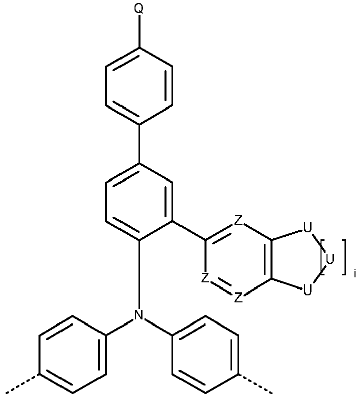 |
| Formel (I-Q-11) | Formel (I-Q-12) |
| 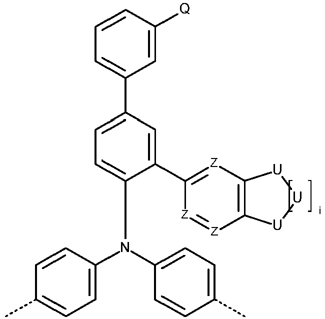 | 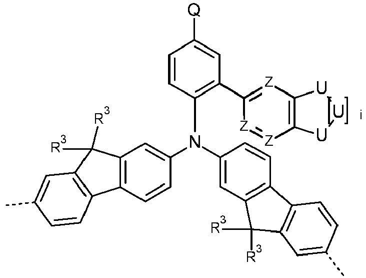 |
| Formel (I-Q-13) | Formel (I-Q-14) |
| 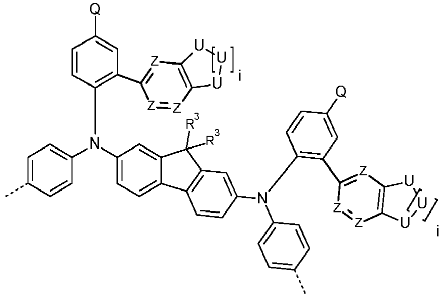 | 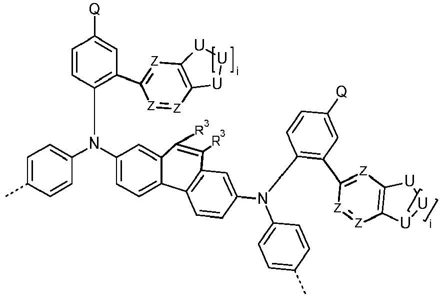 |
| Formel (I-Q-15) | Formel (I-Q-16) |
| 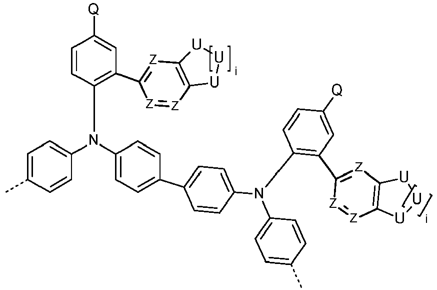 | 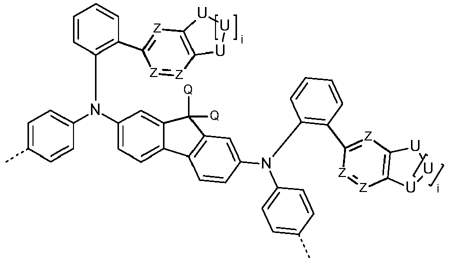 |
| Formel (I-Q-17) | Formel (I-Q-18) |

wobei Q eine vernetzbare Gruppe ist und bevorzugt definiert ist wie in den unten angegebenen bevorzugten Ausführungsformen, und wobei die sonstigen Variablen definiert sind wie oben.

Gemäß einer alternativen Ausführungsform ist die Struktureinheit, die die Gruppe Q trägt, ausgewählt aus Struktureinheiten der oben genannten Gruppen 1 bis 8, bevorzugt aus Struktureinheiten der oben genannten Gruppen 1, 7 und 8, besonders bevorzugt aus Struktureinheiten der oben genannten Gruppe 1.

Erfindungsgemäß bevorzugte vernetzbare Gruppen Q sind die im Folgenden aufgeführten Gruppen:

### a) Endständige oder cyclische Alkenyl- bzw. endständige Dienyl- und Alkinylgruppen:

Geeignet sind Einheiten, die eine endständige oder cyclische Doppelbindung, eine endständige Dienylgruppe oder eine endständige Dreifachbindung enthalten, insbesondere endständige oder cyclische Alkenyl-, endständige Dienyl- bzw. endständige Alkinylgruppen mit 2 bis 40 C-Atomen, vorzugsweise mit 2 bis 10 C-Atomen, wobei auch einzelne CH₂-Gruppen und/oder einzelne H-Atome durch die oben genannten Gruppen R ersetzt sein können.

### b) Alkenyloxy-, Dienyloxy- bzw. Alkinyloxygruppen:

Weiterhin geeignet sind Alkenyloxy-, Dienyloxy- bzw. Alkinyloxygruppen, vorzugsweise Alkenyloxygruppen.

### c) Acrylsäuregruppen:

Weiterhin geeignet sind Acrylsäureeinheiten im weitesten Sinne, vorzugsweise Acrylester, Acrylamide, Methacrylester und Methacrylamide. Besonders bevorzugt sind C₁₋₁₀-Alkylacrylat und C₁₋₁₀-Alkylmethacrylat.

Die Vernetzungsreaktion der oben unter a) bis c) genannten Gruppen kann über einen radikalischen, einen kationischen oder einen anionischen Mechanismus aber auch über Cycloaddition erfolgen.

Es kann sinnvoll sein, einen entsprechenden Initiator für die Vernetzungsreaktion zuzugeben. Geeignete Initiatoren für die radikalische Vernetzung sind beispielsweise Dibenzoylperoxid, AIBN oder TEMPO. Geeignete Initiatoren für die kationische Vernetzung sind beispielsweise AlCl₃, BF₃, Triphenylmethylperchlorat oder Tropyliumhexachlorantimonat. Geeignete Initiatoren für die anionische Vernetzung sind Basen, insbesondere Butyllithium.

In einer bevorzugten Ausführungsform der vorliegenden Erfindung wird die Vernetzung jedoch ohne den Zusatz eines Initiators durchgeführt und ausschließlich thermisch initiiert. Diese Bevorzugung wird dadurch begründet, dass die Abwesenheit des Initiators Verunreinigungen der Schicht verhindert, die zu einer Verschlechterung der Deviceeigenschaften führen könnten.

### d) Oxetane und Oxirane:

Eine weitere geeignete Klasse vernetzbarer Gruppen Q sind Oxetane und Oxirane, die durch Ringöffnung kationisch vernetzen.

Es kann sinnvoll sein, einen entsprechenden Initiator für die Vernetzungsreaktion zuzugeben. Geeignete Initiatoren sind beispielsweise AlCl₃, BF₃, Triphenylmethylperchlorat oder Tropyliumhexachlorantimonat. Ebenso können Photosäuren als Initiatoren zugegeben werden.

### e) Silane:

Weiterhin geeignet als Klasse vernetzbarer Gruppen sind Silangruppen SiRs, wobei mindestens zwei Gruppen R, bevorzugt alle drei Gruppen R für Cl oder eine Alkoxygruppe mit 1 bis 20 C-Atomen stehen. Diese Gruppe reagiert in der Anwesenheit von Wasser zu einem Oligo- oder Polysiloxan.

### f) Cyclobutangruppen

Die oben unter a) bis f) genannten vernetzbaren Gruppen Q sind dem Fachmann generell bekannt, ebenso wie die geeigneten Reaktionsbedingungen, die zur Reaktion dieser Gruppen verwendet werden.

Bevorzugte vernetzbare Gruppen Q umfassen Alkenylgruppen der folgenden Formel Q1, Dienylgruppen der folgenden Formel Q2, Alkinylgruppen der folgenden Formel Q3, Alkenyloxygruppen der folgenden Formel Q4, Dienyloxygruppen der folgenden Formeln Q5, Alkinyloxygruppen der folgenden Formel Q6, Acrylsäuregruppen der folgenden Formeln Q7 und Q8, Oxetangruppen der folgenden Formeln Q9 und Q10, Oxirangruppen der folgenden Formel Q 11, und Cyclobutangruppen der folgenden Formeln Q12, Q13 und Q14:

| | |
|---|---|
| 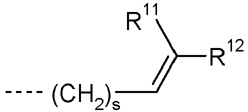 | 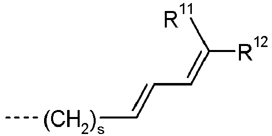 |
| Q1 | Q2 |
| 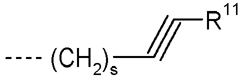 | 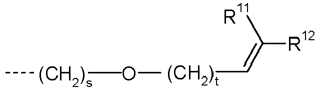 |
| Q3 | Q4 |
| 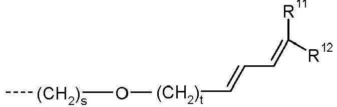 | 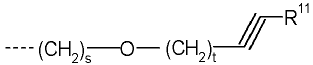 |
| Q5 | Q6 |
| 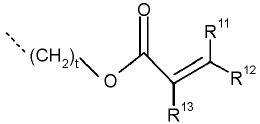 | 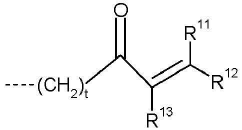 |
| Q7 | Q8 |
| 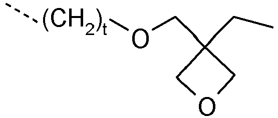 | 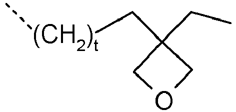 |
| Q9 | Q10 |
| 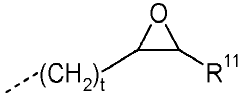 | 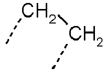 |
| Q11 | Q12 |
| 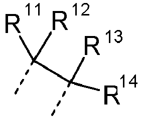 | 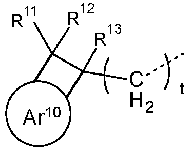 |
| Q13 | Q14 |

Die Reste R¹¹, R¹², R¹³ und R¹⁴ in den Formeln Q1 bis Q8, Q11, Q13 und Q14 sind bei jedem Auftreten, gleich oder verschieden, H, eine geradkettige oder verzweigte Alkylgruppe mit 1 bis 6 C-Atomen, vorzugsweise 1 bis 4 C-Atomen. Besonders bevorzugt sind die R¹¹, R¹², R¹³ und R¹⁴ H, Methyl, Ethyl, n-Propyl, iso-Propyl, n-Butyl, sec-Butyl oder tert-Butyl und ganz besonders bevorzugt H oder Methyl. Die verwendeten Indices haben in den Formeln Q1 bis Q14 die folgende Bedeutung: s = 0 bis 8; und t = 1 bis 8.

Ar¹⁰ in der Formel Q14 ist gewählt aus aromatischen Ringsystemen mit 6 bis 40 aromatischen Ringatomen, die mit einem oder mehreren Resten R¹¹ substituiert sein können, und aus heteroaromatischen Ringsystemen mit 5 bis 40 aromatischen Ringatomen, die mit einem oder mehreren Resten R¹¹ substituiert sein können.

Die gestrichelte Bindung in den Formeln Q1 bis Q11 und Q14 sowie die gestrichelten Bindungen in den Formeln Q12 und Q13 stellen die Anknüpfung der vernetzbaren Gruppe an die Struktureinheiten dar.

Die vernetzbaren Gruppen der Formeln Q1 bis Q14 können dabei direkt mit der Struktureinheit verknüpft sein, oder aber indirekt, über ein weiteres, mono- oder polycyclisches, aromatisches oder heteroaromatisches Ringsystem Ar¹⁰, wie in den folgenden Formeln Q15 bis Q28 dargestellt:

| | |
|---|---|
| 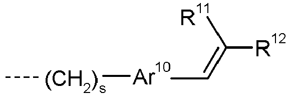 | 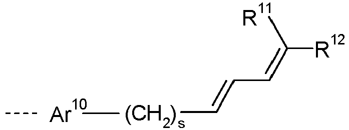 |
| Q15 | Q16 |
| 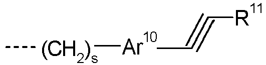 | 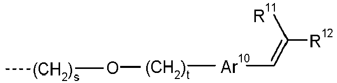 |
| Q17 | Q18 |
| 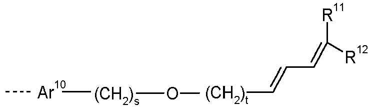 | 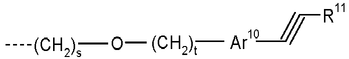 |
| Q19 | Q20 |
| 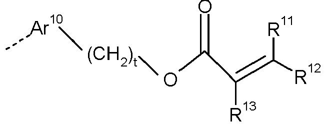 | 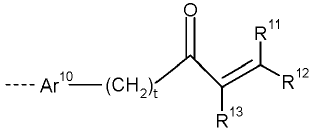 |
| Q21 | Q22 |
| 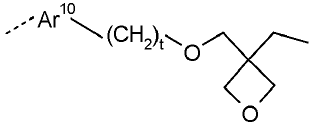 | 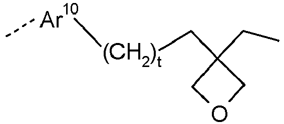 |
| Q23 | Q24 |
| 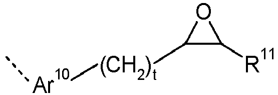 | 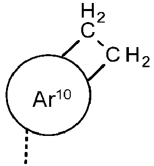 |
| Q25 | Q26 |
| 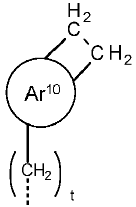 | 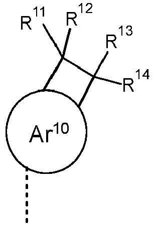 |
| Q27 | Q28 |

wobei Ar¹⁰ gewählt ist aus aromatischen Ringsystemen mit 6 bis 40 aromatischen Ringatomen, die mit einem oder mehreren Resten R¹¹ substituiert sein können, und aus heteroaromatischen Ringsystemen mit 5 bis 40 aromatischen Ringatomen, die mit einem oder mehreren Resten R¹¹ substituiert sein können, und wobei gilt:
Die Reste R¹¹, R¹², R¹³ und R¹⁴ sind bei jedem Auftreten, gleich oder verschieden, H, eine geradkettige oder verzweigte Alkylgruppe mit 1 bis 6 C-Atomen, vorzugsweise 1 bis 4 C-Atomen. Besonders bevorzugt sind die R¹¹, R¹², R¹³ und R¹⁴ H, Methyl, Ethyl, n-Propyl, iso-Propyl, n-Butyl, sec-Butyl oder tert-Butyl und ganz besonders bevorzugt H oder Methyl. Die verwendeten Indices haben in den Formeln Q15 bis Q28 die folgende Bedeutung: s = 0 bis 8; und t = 1 bis 8.

Besonders bevorzugte vernetzbare Gruppen Q sind die folgenden:

| | |
|---|---|
| 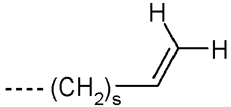 | 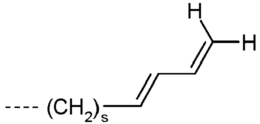 |
| Q1a | Q2a |
| 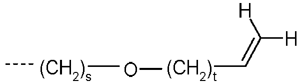 | 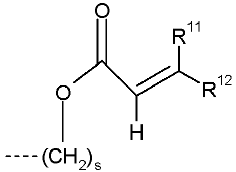 |
| Q4a | Q7a |
| 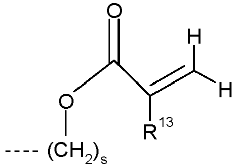 | 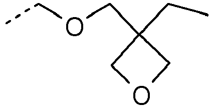 |
| Q7b | Q9a |
| 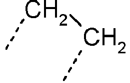 | 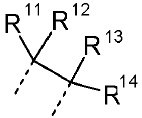 |
| Q12 | Q13 |
| 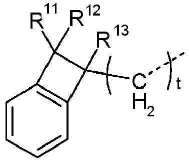 | 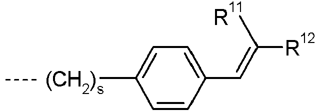 |
| Q14a | Q15a |
| 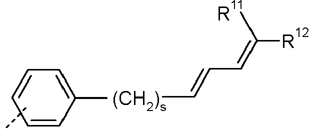 | 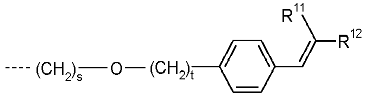 |
| Q16a | Q18a |
| 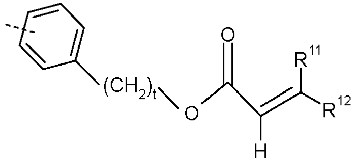 | 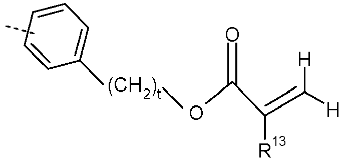 |
| Q21a | Q21b |
| 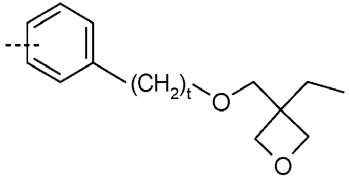 | 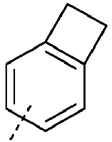 |
| Q23a | Q26a |
| 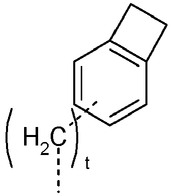 | 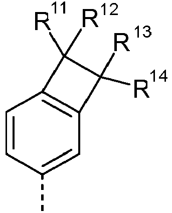 |
| Q27a | Q28a |

Die Reste R¹¹, R¹² ,R¹³ und R¹⁴ sind bei jedem Auftreten, gleich oder verschieden, H oder eine geradkettige oder verzweigte Alkylgruppe mit 1 bis 6 C-Atomen, vorzugsweise 1 bis 4 C-Atomen. Besonders bevorzugt sind die Reste R¹¹, R¹² ,R¹³ und R¹⁴ Methyl, Ethyl, n-Propyl, iso-Propyl, n-Butyl, sec-Butyl oder tert-Butyl und ganz besonders bevorzugt Methyl.

Die verwendeten Indices haben in Formel Q1a bis Q28a die folgende Bedeutung: s = 0 bis 8 und t = 1 bis 8.

Ganz besonders bevorzugte vernetzbare Gruppen Q sind die folgenden:

| | |
|---|---|
| 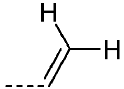 | 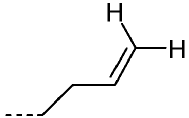 |
| Q1b | Q1c |
| 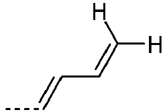 | 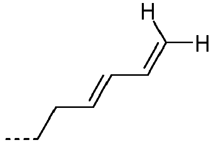 |
| Q2b | Q2c |
| 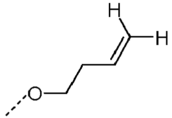 | 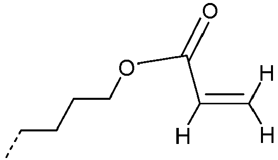 |
| Q4b | Q7c |
| 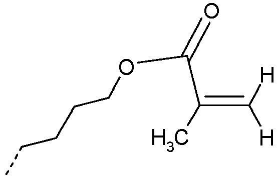 | 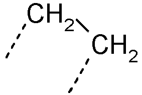 |
| Q7d | Q12b |
| 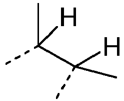 | 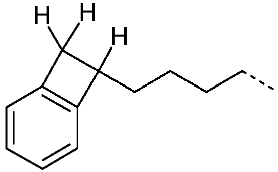 |
| Q13a | Q14b |
| 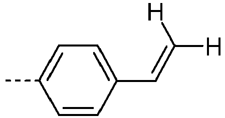 | 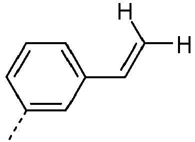 |
| Q15b | Q15c |
| 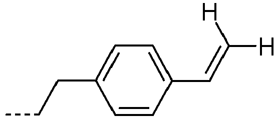 | 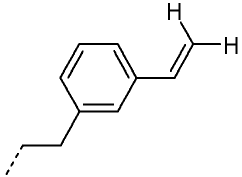 |
| Q15d | Q15e |
| 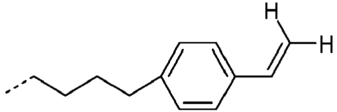 | 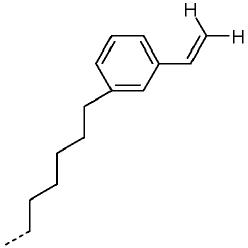 |
| Q15f | Q15q |
| 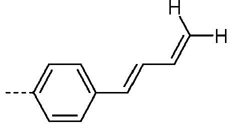 | 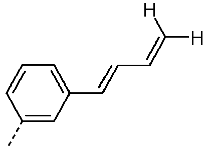 |
| Q16b | Q16c |
| 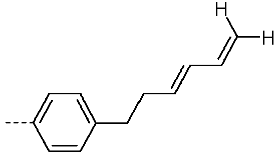 | 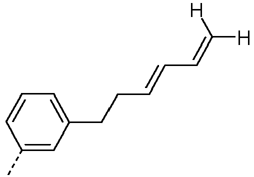 |
| Q16d | Q16e |
| 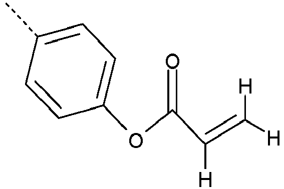 | 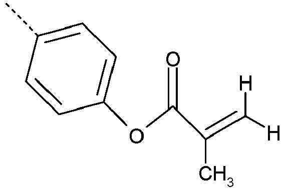 |
| Q21c | Q21d |
| 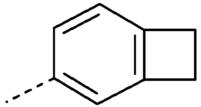 | 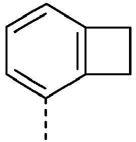 |
| Q26b | Q26c |
| 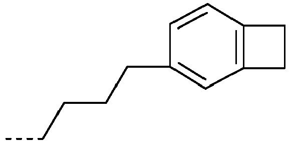 | 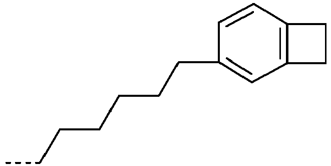 |
| Q27b | Q27c |

Die erfindungsgemäßen Polymere enthaltend Struktureinheiten der Formel (I) werden in der Regel durch Polymerisation von einer oder mehreren Monomersorten hergestellt, von denen mindestens ein Monomer im Polymer zu Struktureinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation;
(D) HECK-Polymerisation;
(E) NEGISHI-Polymerisation;
(F) SONOGASHIRA-Polymerisation;
(G) HIYAMA-Polymerisation; und
(H) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in der WO 03/048225 A2, der WO 2004/037887 A2 und der WO 2004/037887 A2 im Detail beschrieben.

Die C-C-Verknüpfungen sind vorzugsweise ausgewählt aus den Gruppen der SUZUKI-Kupplung, der YAMAMOTO-Kupplung und der STILLE-Kupplung; die C-N-Verknüpfung ist vorzugsweise eine Kupplung gemäß HARTWIG-BUCHWALD.

Gegenstand der vorliegenden Erfindung ist somit auch ein Verfahren zur Herstellung der erfindungsgemäßen Polymere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD, besonders bevorzugt Polymerisation gemäß SUZUKI, hergestellt werden.

Zur Synthese der erfindungsgemäßen Polymere werden Monomerverbindungen benötigt, die Struktureinheiten der Formel (I) in das Polymer einführen.

Weiterer Gegenstand der Erfindung sind somit Monomere gemäß einer Formel (M) 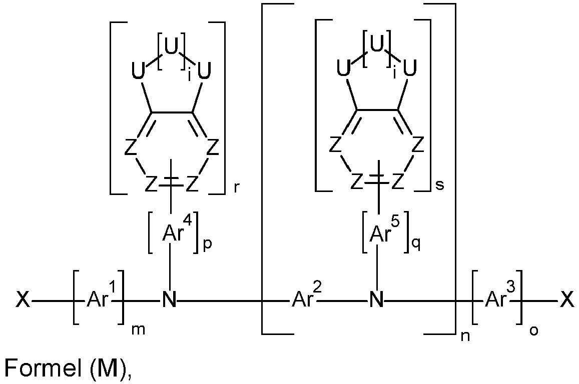 wobei die auftretenden Variablen definiert sind wie oben, und wobei X bei jedem Auftreten gleich oder verschieden eine für eine Polymerisationsreaktion geeignete Abgangsgruppe ist.

Bevorzugt ist X bei jedem Auftreten gleich oder verschieden gewählt aus H, D, Halogenen, bevorzugt Chlor, Brom oder Iod, O-Tosylaten, O-Triflaten, O-Sulfonaten, Boronsäure, Borsäureestern, teilfluorierten Silylgruppen, Diazoniumgruppen und zinnorganischen Verbindungen. Wenn m gleich 1 ist, ist die an der linken Seite gebundene Gruppe X besonders bevorzugt gewählt aus Halogenen, bevorzugt Chlor, Brom oder Iod, Boronsäure und Boronsäureestern. Wenn m gleich 0 ist, ist die an der linken Seite gebundene Gruppe X besonders bevorzugt gleich H. Wenn o gleich 1 ist, ist die an der rechten Seite gebundene Gruppe X besonders bevorzugt gewählt aus Halogenen, bevorzugt Chlor, Brom oder Iod, Boronsäure und Boronsäureestern. Wenn o gleich 0 ist, ist die an der rechten Seite gebundene Gruppe X besonders bevorzugt gleich H.

Für die sonstigen auftretenden Variablen gelten dieselben bevorzugten Ausführungsformen als bevorzugt, wie oben für die Struktureinheit der Formel (I) angegeben.

Die Synthese der Monomere erfolgt bevorzugt unter Verwendung von Buchwald-Kupplungsreaktionen, Suzuki-Kupplungsreaktionen und Bromierungsreaktionen. Gemäß einem bevorzugten Verfahren (Schema 1) wird ein bromiertes Amin mit einem Boronsäurederivat der Formel 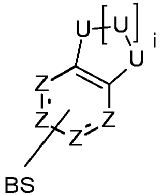 wobei BS ein Boronsäurederivat ist, in einer Suzuki-Kupplungsreaktion umgesetzt. Das erhaltene Kupplungsprodukt wird anschließend bromiert, wodurch eine als Monomer verwendbare Verbindung der Formel (M) erhalten wird.

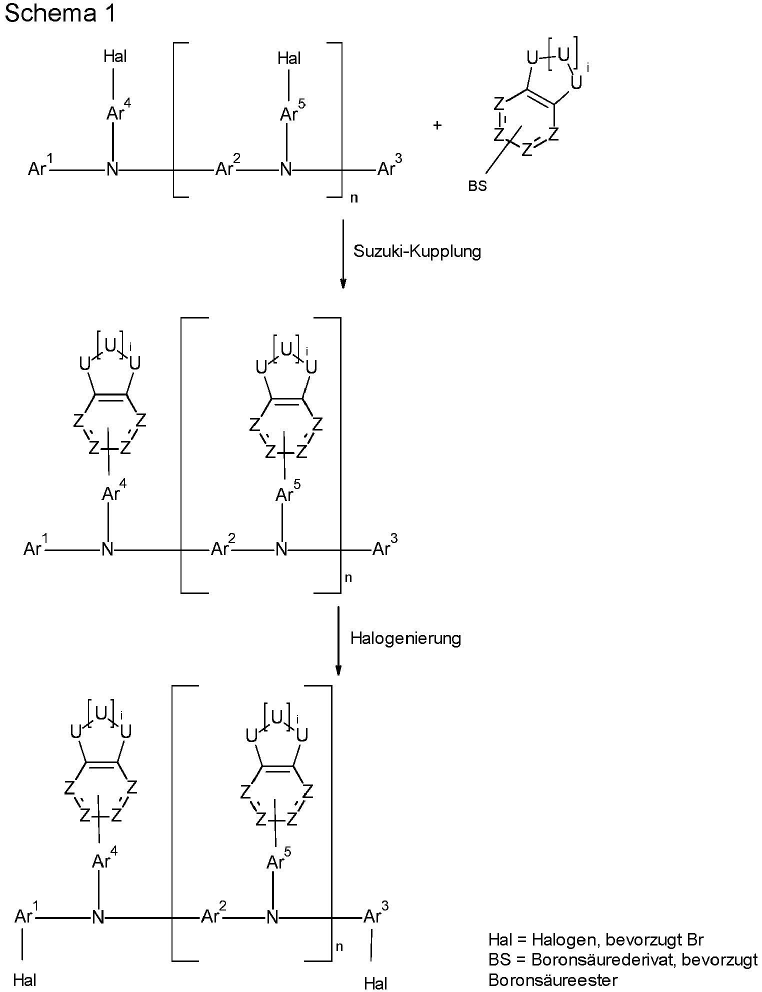

Gemäß einem alternativen bevorzugten Verfahren (Schema 2) wird ein Amin mit einem halogensubstituierten Derivat der folgenden Formel 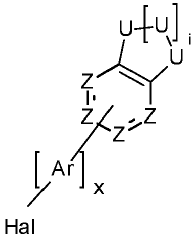 wobei BS ein Boronsäurederivat ist und Ar ein aromatisches oder heteroaromatisches Ringsystem ist, und x gleich 0 oder 1 ist, in einer Buchwald-Kupplungsreaktion umgesetzt. Das erhaltene Kupplungsprodukt wird anschließend bromiert, wodurch eine als Monomer verwendbare Verbindung der Formel (M) erhalten wird.

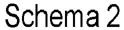

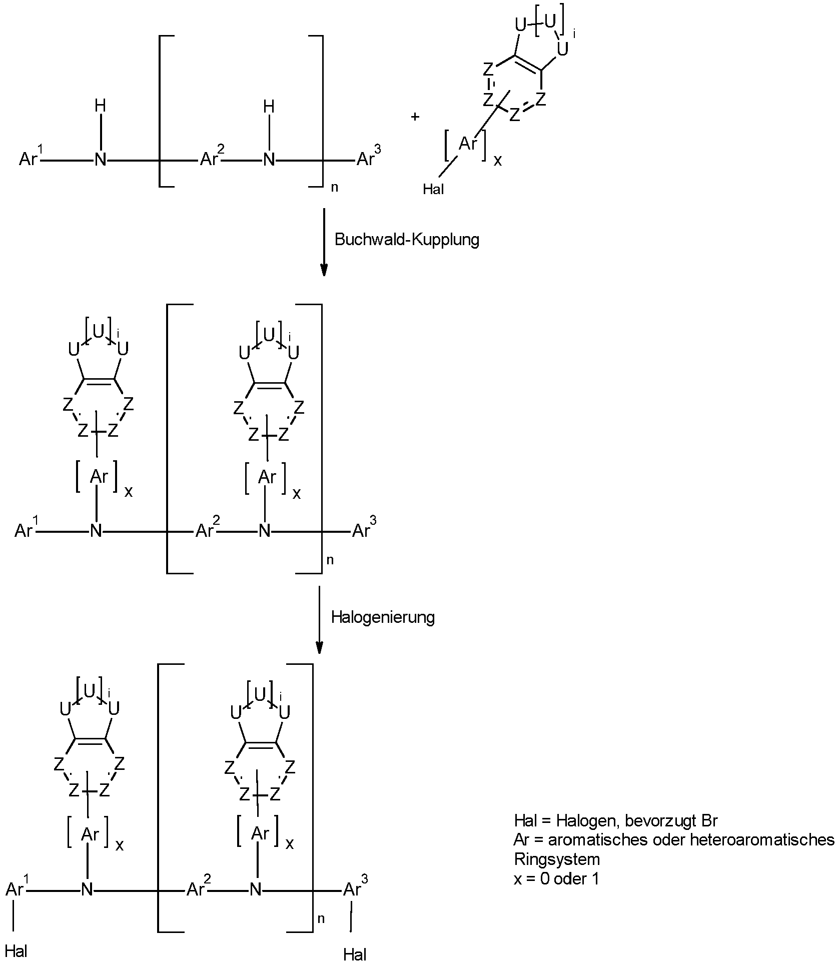

Aus Monomeren der Formel (M) werden erfindungsgemäße Polymere enthaltend mindestens eine Struktureinheit der Formel (I), wie oben definiert, hergestellt.

Die erfindungsgemäßen Polymere können als Reinsubstanz, aber auch als Mischung zusammen mit weiteren beliebigen polymeren, oligomeren, dendritischen oder niedermolekularen Substanzen verwendet werden. Als niedermolekulare Substanz versteht man in der vorliegenden Erfindung Verbindungen mit einem Molekulargewicht im Bereich von 100 bis 3000 g/mol, vorzugsweise 200 bis 2000 g/mol. Diese weiteren Substanzen können z.B. die elektronischen Eigenschaften verbessern oder selbst emittieren. Als Mischung wird vor- und nachstehend eine Mischung enthaltend mindestens eine polymere Komponente bezeichnet. Auf diese Art können eine oder mehrere Polymerschichten bestehend aus einer Mischung (Blend) aus einem oder mehreren erfindungsgemäßen Polymeren mit einer Struktureinheit der Formel (I) und optional einem oder mehreren weiteren Polymeren mit einer oder mehreren niedermolekularen Substanzen hergestellt werden.

Ein weiterer Gegenstand der vorliegenden Erfindung ist somit ein Polymer Blend enthaltend ein oder mehrere erfindungsgemäße Polymere, sowie eine oder mehrere weitere polymere, oligomere, dendritische und/oder niedermolekulare Substanzen.

Gegenstand der Erfindung sind weiterhin Lösungen und Formulierungen aus einem oder mehreren erfindungsgemäßen Polymeren oder einem Polymer Blend in einem oder mehreren Lösungsmitteln. Wie solche Lösungen hergestellt werden können, ist dem Fachmann bekannt und beispielsweise in der WO 02/072714 A1, der WO 03/019694 A2 und der darin zitierten Literatur beschrieben.

Diese Lösungen können verwendet werden, um dünne Polymerschichten herzustellen, zum Beispiel durch Flächenbeschichtungsverfahren (z.B. Spin-coating) oder durch Druckverfahren (z.B. InkJet Printing).

Polymere enthaltend Struktureinheiten, die eine vernetzbare Gruppe Q aufweisen, eignen sich besonders zur Herstellung von Filmen oder Beschichtungen, insbesondere zur Herstellung von strukturierten Beschichtungen, z.B. durch thermische oder lichtinduzierte in-situ-Polymerisation und in-situ-Vernetzung, wie beispielsweise in-situ-UV-Photopolymerisation oder Photopatterning. Dabei können sowohl entsprechende Polymere in Reinsubstanz verwendet werden, es können aber auch Formulierungen oder Mischungen dieser Polymere wie oben beschrieben verwendet werden. Diese können mit oder ohne Zusatz von Lösungsmitteln und/oder Bindemitteln verwendet werden. Geeignete Materialien, Verfahren und Vorrichtungen für die oben beschriebenen Methoden sind z.B. in der WO 2005/083812 A2 beschrieben. Mögliche Bindemittel sind beispielsweise Polystyrol, Polycarbonat, Poly(meth)-acrylate, Polyacrylate, Polyvinylbutyral und ähnliche, optoelektronisch neutrale Polymere.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösungsmittel.

Ein weiterer Gegenstand der vorliegenden Erfindung ist somit die Verwendung eines Polymers, das Struktureinheiten enthält, die eine vernetzbare Gruppe Q aufweisen, zur Herstellung eines vernetzten Polymers. Die vernetzbare Gruppe, die besonders bevorzugt eine Vinylgruppe oder Alkenylgruppe ist, wird vorzugsweise durch die WITTIG-Reaktion oder eine WITTIG-analoge Reaktion in das Polymer eingebaut. Ist die vernetzbare Gruppe eine Vinylgruppe oder Alkenylgruppe, so kann die Vernetzung durch radikalische oder ionische Polymerisation stattfinden, wobei diese thermisch oder durch Strahlung induziert werden kann. Bevorzugt ist die radikalische Polymerisation, die thermisch induziert wird, vorzugsweise bei Temperaturen von weniger als 250°C, besonders bevorzugt bei Temperaturen von weniger als 230°C.

Wahlweise wird während des Vernetzungsverfahrens ein zusätzliches Styrol-Monomer zugesetzt, um einen höheren Grad der Vernetzung zu erzielen. Vorzugsweise ist der Anteil des zugesetzten Styrol-Monomers im Bereich von 0,01 bis 50 mol%, besonders bevorzugt 0,1 bis 30 mol%, bezogen auf 100 mol% aller copolymerisierten Monomere, die im Polymer als Struktureinheiten enthalten sind.

Gegenstand der vorliegenden Erfindung ist somit auch ein Verfahren zur Herstellung eines vernetzten Polymers, das folgende Schritte umfasst:
(a) Bereitstellen von Polymeren, die Struktureinheiten enthalten, die eine oder mehrere vernetzbare Gruppen Q aufweisen; und
(b) Radikalische oder ionische Vernetzung, vorzugsweise radikalische Vernetzung, die sowohl thermisch als auch durch Strahlung, vorzugsweise thermisch, induziert werden kann.

Die durch das erfindungsgemäße Verfahren hergestellten vernetzten Polymere sind in allen gängigen Lösungsmitteln unlöslich. Auf diese Weise lassen sich definierte Schichtdicken herstellen, die auch durch das Aufbringen nachfolgender Schichten nicht wieder gelöst bzw. angelöst werden.

Die vorliegende Erfindung betrifft somit auch ein vernetztes Polymer, das durch das zuvor genannte Verfahren erhältlich ist. Das vernetzte Polymer wird - wie vorstehend beschrieben - vorzugsweise in Form einer vernetzen Polymerschicht hergestellt. Auf die Oberfläche einer solchen vernetzten Polymerschicht kann aufgrund der Unlöslichkeit des vernetzten Polymers in sämtlichen Lösungsmitteln eine weitere Schicht aus einem Lösungsmittel mit den oben beschriebenen Techniken aufgebracht werden.

Die erfindungsgemäßen Polymere können in elektronischen oder optoelektronischen Vorrichtungen bzw. zu deren Herstellung verwendet werden.

Ein weiterer Gegenstand der vorliegenden Anmeldung ist somit die Verwendung der erfindungsgemäßen Polymere in elektronischen oder optoelektronischen Vorrichtungen, vorzugsweise in organischen Elektrolumineszenzvorrichtungen (OLED), organischen Feld-Effekt-Transistoren (OFETs), organischen integrierten Schaltungen (O-ICs), organischen Dünnfilmtransistoren (TFTs), organischen Solarzellen (O-SCs), organischen Laserdioden (O-Laser), organischen photovoltaischen (OPV) Elementen oder Vorrichtungen oder organischen Photorezeptoren (OPCs), besonders bevorzugt in organischen Elektrolumineszenzvorrichtungen (OLED).

Weiterer Gegenstand der vorliegenden Anmeldung ist eine Vorrichtung gewählt aus den oben genannten Vorrichtungen, enthaltend mindestens ein erfindungsgemäßes Polymer. Bevorzugt ist das Polymer dabei in einer lochtransportierenden Schicht enthalten.

Außer Kathode, Anode, emittierender Schicht und lochtransportierender Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend das erfindungsgemäße Polymer ist bevorzugt die folgende: Anode-Lochinjektionsschicht-Lochtransportschicht-wahlweise weitere Lochtransportschicht(en)- emittierende Schicht-wahlweise Lochblockierschicht-Elektronentransportschicht-Kathode. Es können zusätzlich weitere Schichten in der OLED vorhanden sein.

Bevorzugte Ausführungsformen von OLEDs enthaltend das erfindungsgemäße Polymer sind Hybridvorrichtungen, in der eine oder mehrere Schichten, die aus Lösung prozessiert werden, und eine oder mehrere Schichten, die durch Aufdampfen von niedermolekularen Substanzen hergestellt werden, enthalten sind. Diese werden auch als kombinierte PLED/SMOLED (Polymeric Light Emitting Diode/Small Molecule Organic Light Emitting Diode) Systeme bezeichnet. Bevorzugt werden in der erfindungsgemäßen Vorrichtung die Schichten zwischen Anode und emittierender Schicht sowie die emittierende Schicht aus Lösung aufgebracht, und die Schichten zwischen emittierender Schicht und Kathode werden bevorzugt mit einem Sublimationsverfahren aufgebracht.

Schichten aus Lösung werden bevorzugt durch Spincoating, mit einem beliebigen Druckverfahren, wie z. B. Siebdruck, Flexodruck, Nozzle Printing oder Offsetdruck, besonders bevorzugt LITI (Light Induced Thermal Imaging, Thermotransferdruck) oder mit Ink-Jet Druck (Tintenstrahldruck) hergestellt.

Beim Auftragen von Schichten mittels Sublimation werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Gemäß einer alternativen Ausführungsform werden eine oder mehrere Schichten mit dem OVPD (Organic Vapour Phase Deposition) Verfahren oder mit Hilfe einer Trägergassublimation aufgetragen. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Wie OLEDs hergestellt werden können, ist dem Fachmann bekannt und wird beispielsweise als allgemeines Verfahren ausführlich in der WO 2004/070772 A2 beschrieben, das entsprechend für den Einzelfall anzupassen ist.

Die erfindungsgemäßen Polymere eigenen sich insbesondere zur Verwendung in einer lochtransportierenden Schicht einer OLED. Unter einer lochtransportierenden Schicht wird dabei insbesondere eine Schicht verstanden, welche anodenseitig an die emittierende Schicht angrenzt.

Die erfindungsgemäßen Polymere können jedoch auch in einer Lochinjektionsschicht (HIL), einer Lochblockierschicht (HBL), und in einer emittierenden Schicht verwendet werden. Wenn die Polymere in einer emittierenden Schicht verwendet werden, ist dies bevorzugt in der Funktion als Matrixmaterial, insbesondere in der Funktion als lochtransportierendes und/oder als wide bandgap-Matrixmaterial. Unter einer Lochinjektionsschicht wird insbesondere eine Schicht verstanden, die unmittelbar an die Anode angrenzt und die zwischen der Anode und einer Lochtransportschicht angeordnet ist. Unter einer Lochblockierschicht wird insbesondere eine Schicht verstanden, die kathodenseitig direkt an die emittierende Schicht angrenzt, und die zwischen der emittierenden Schicht und einer Elektronentransportschicht angeordnet ist.

Im Folgenden werden bevorzugte Ausführungsformen für die unterschiedlichen Funktionsmaterialien der elektronischen Vorrichtung aufgeführt.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß

WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Besonders bevorzugt sind die in der WO 2014/111269 offenbarten erweiterten Benzoindenofluorene zur Verwendung als fluoreszierende Emitter in der emittierenden Schicht.

Bevorzugte fluoreszierende Emitter, zur Verwendung in der emittierenden Schicht von Vorrichtungen enthaltend die erfindungsgemäßen Polymere, sind im Folgenden gezeigt:

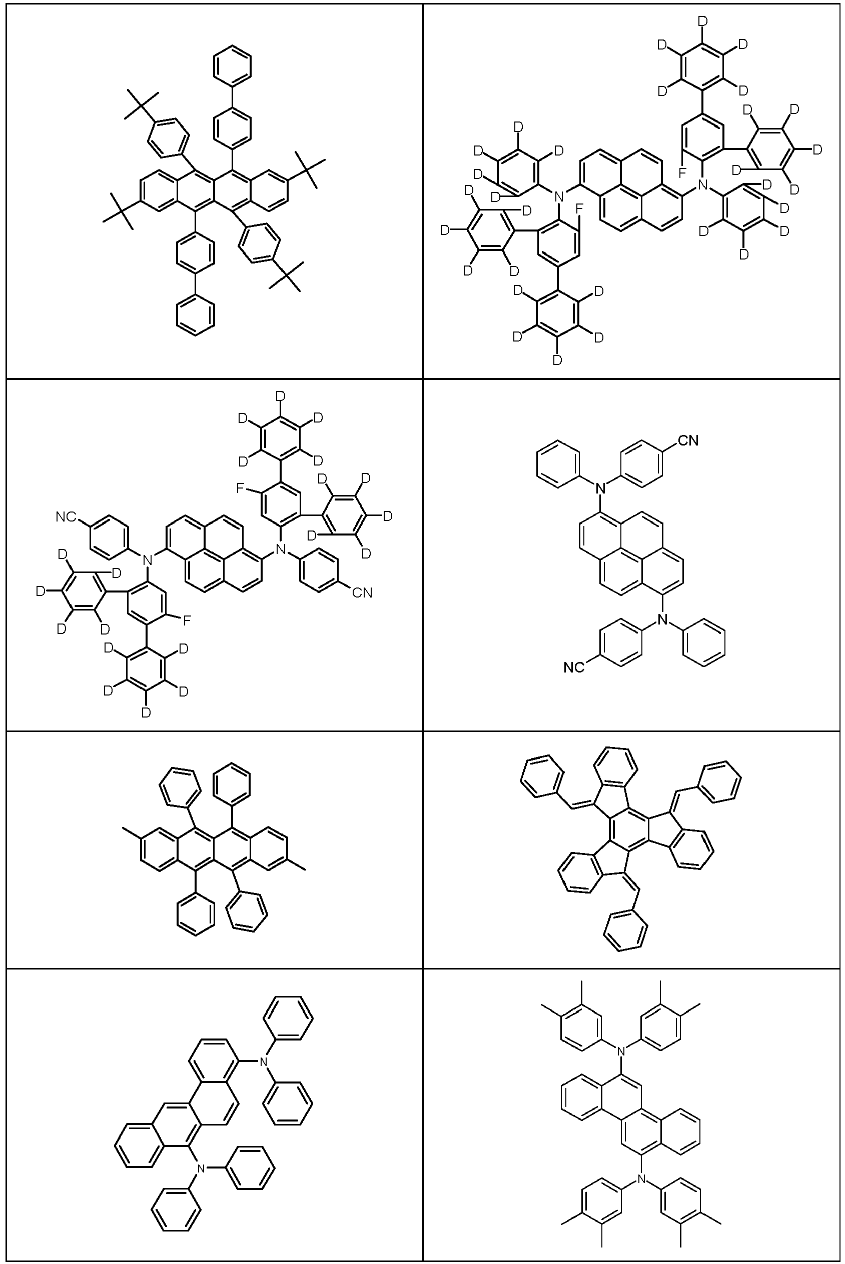

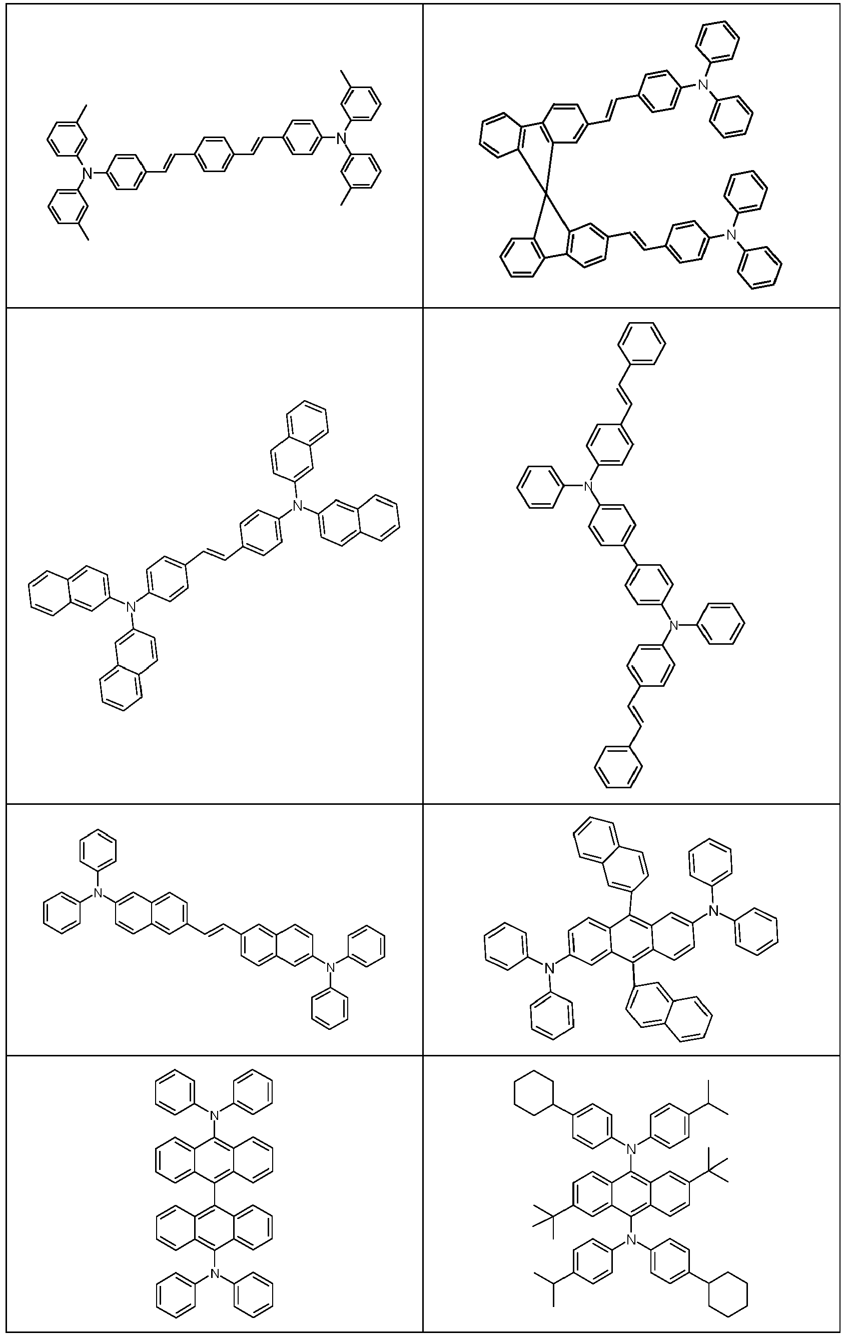

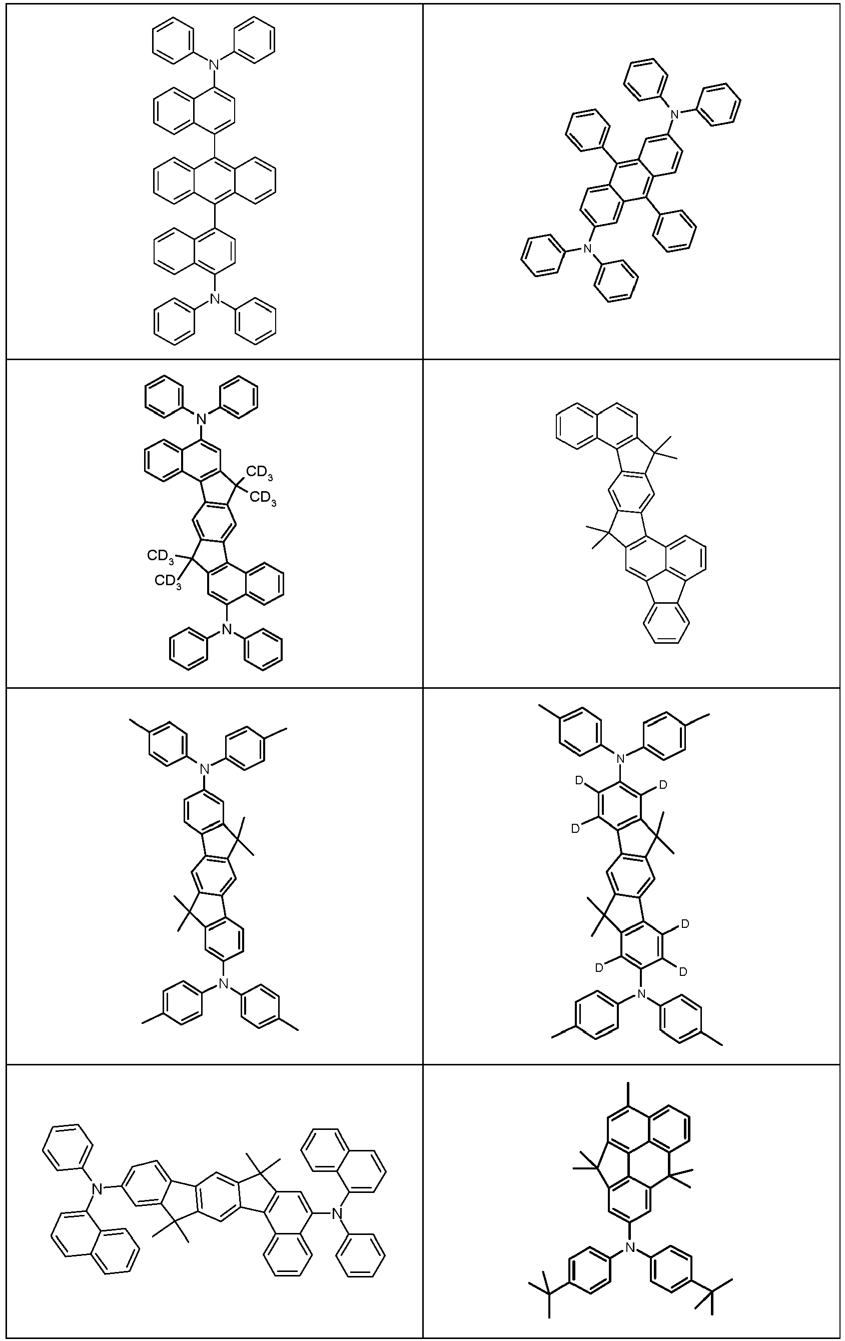

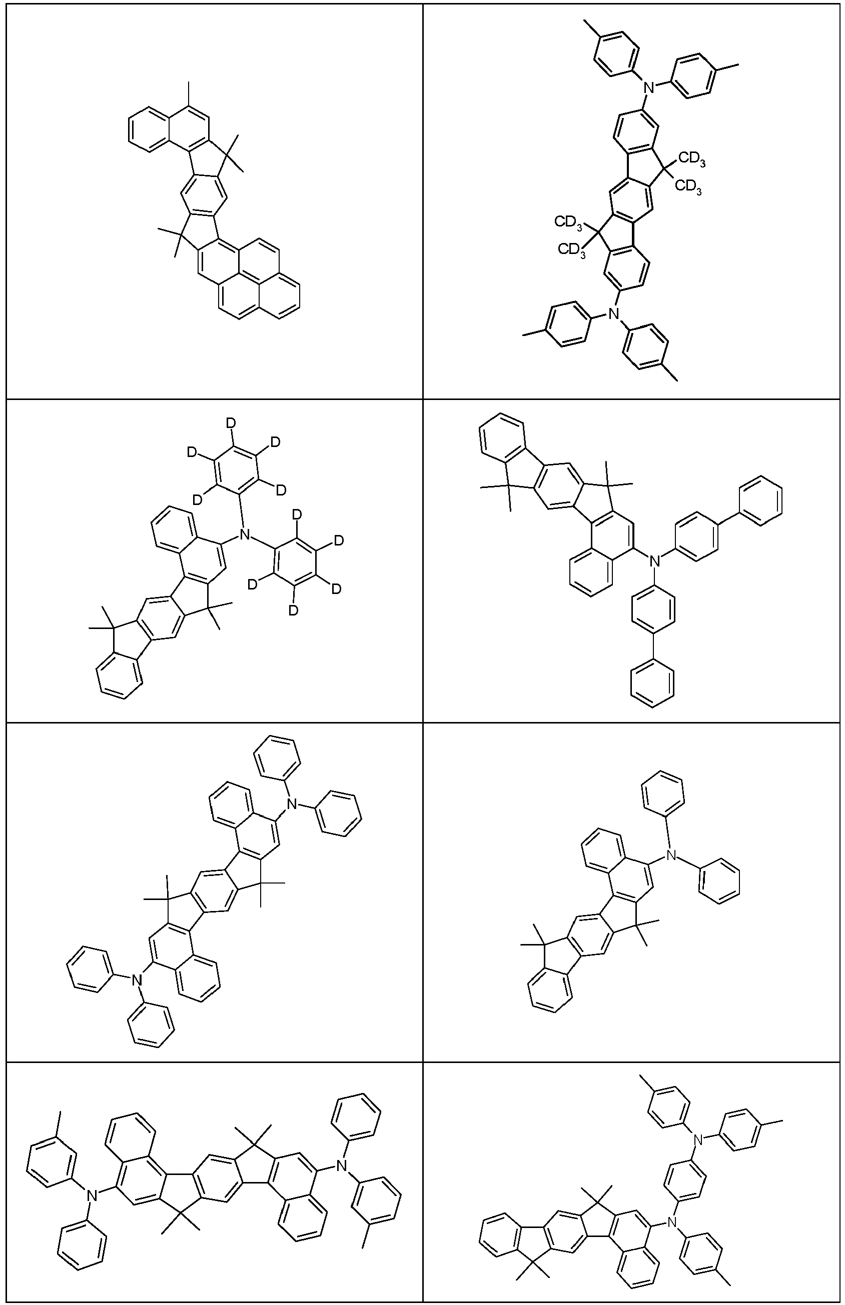

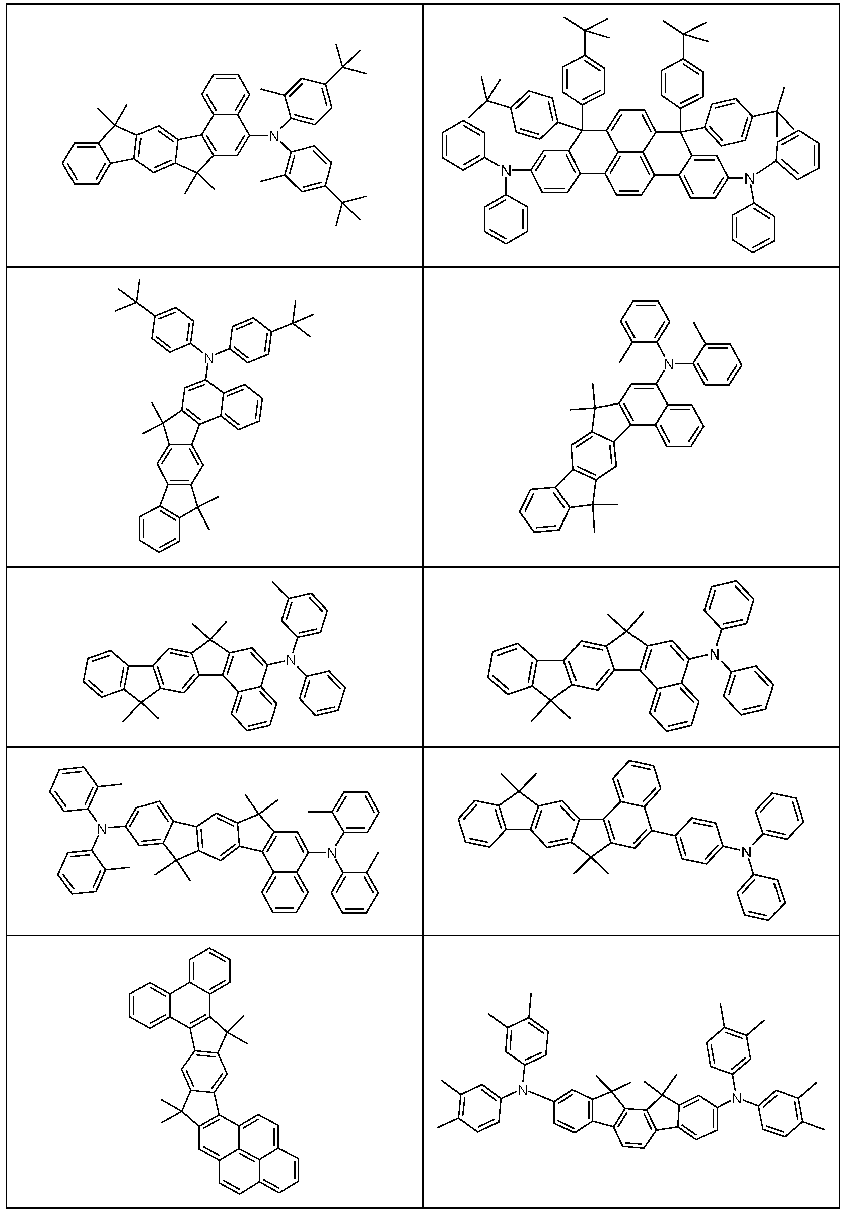

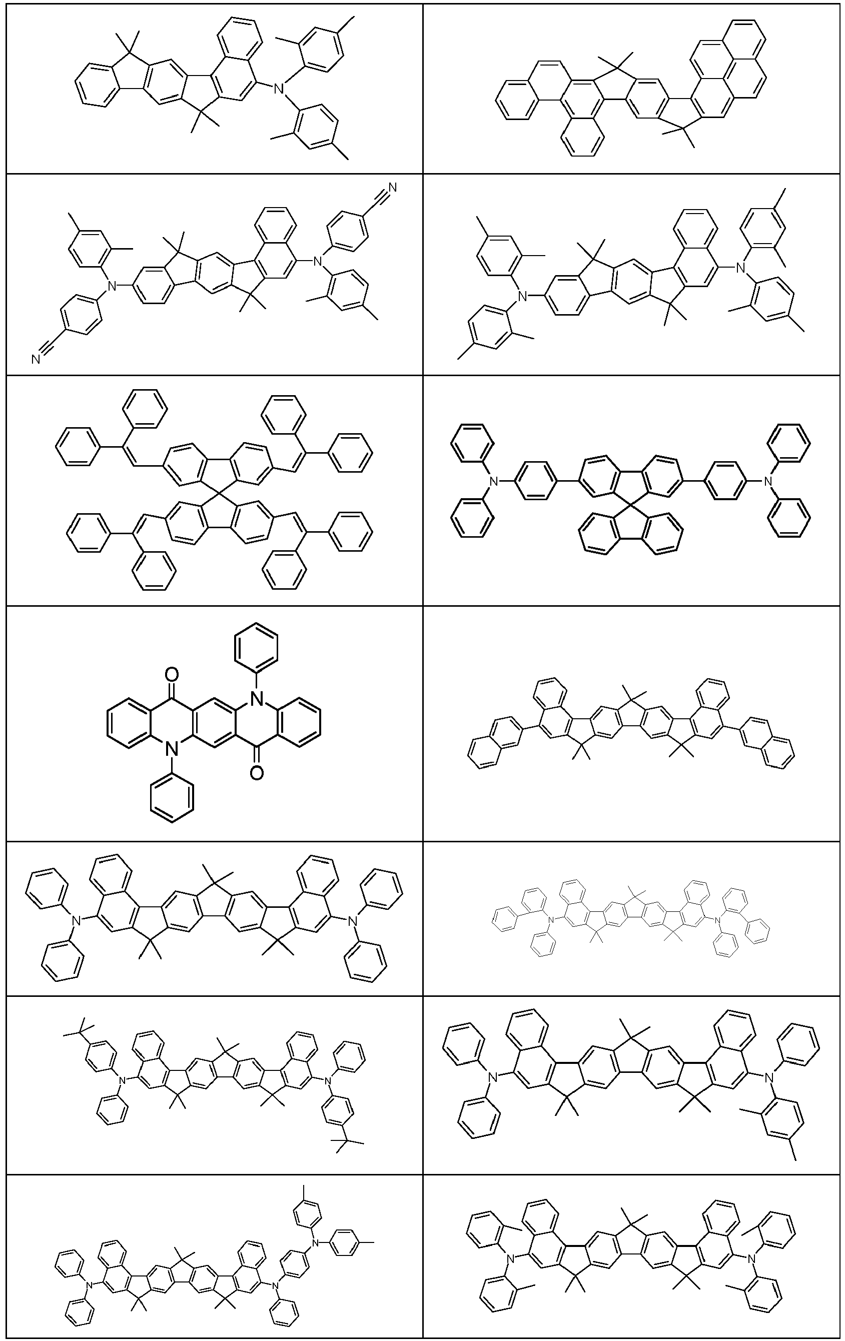

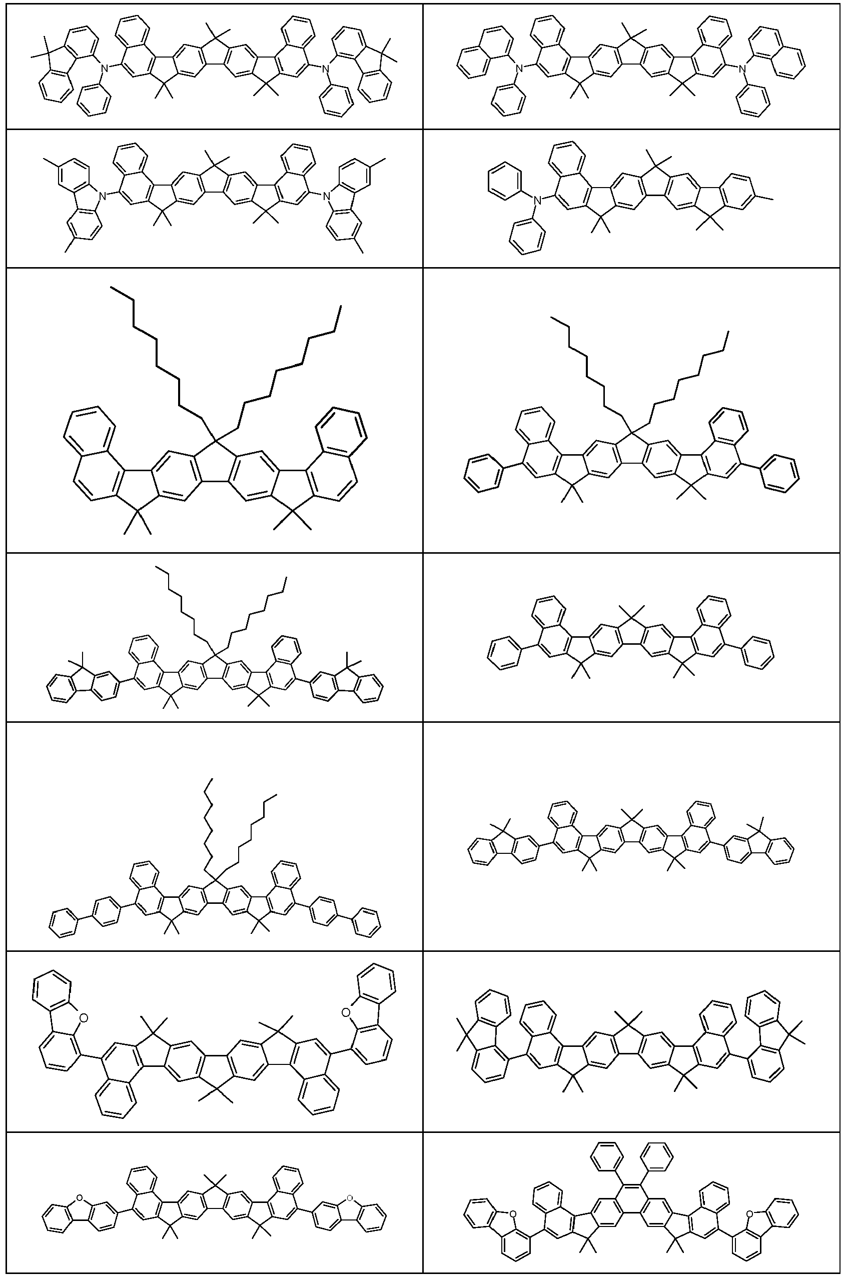

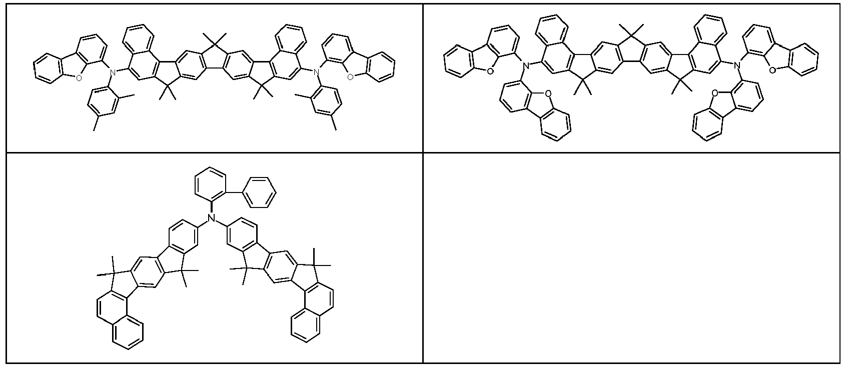

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für fluoreszierende Emitter, zur Verwendung in der emittierenden Schicht von Vorrichtungen enthaltend die erfindungsgemäßen Polymere, sind im Folgenden gezeigt:

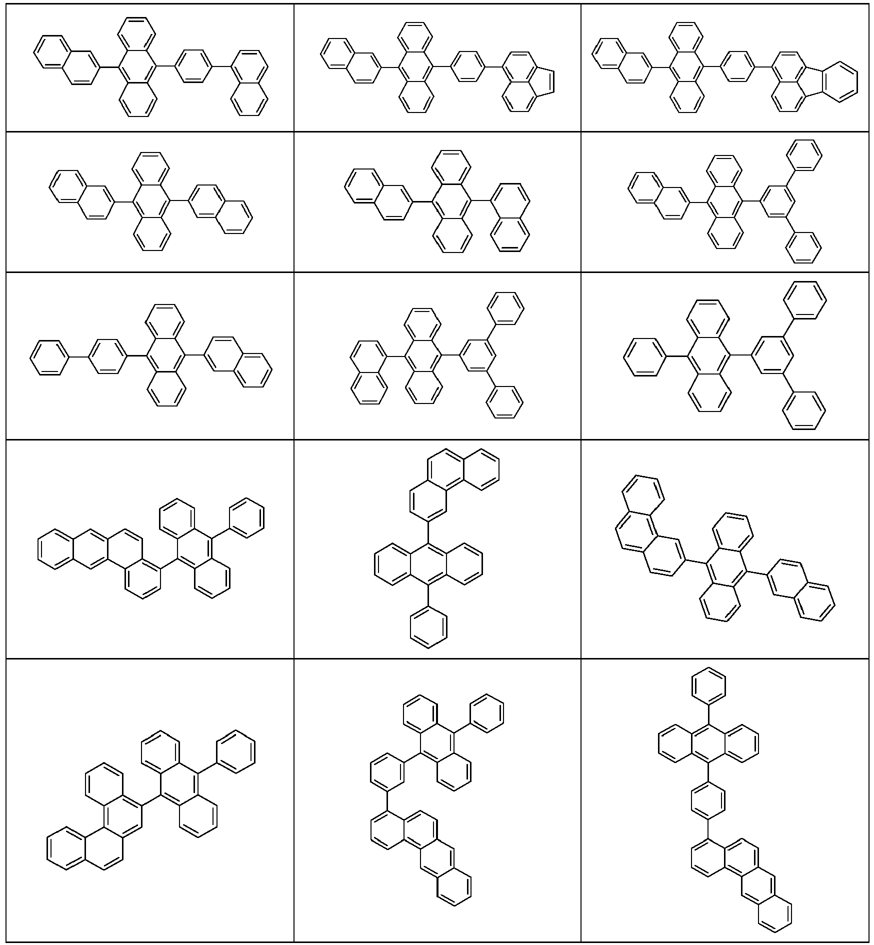

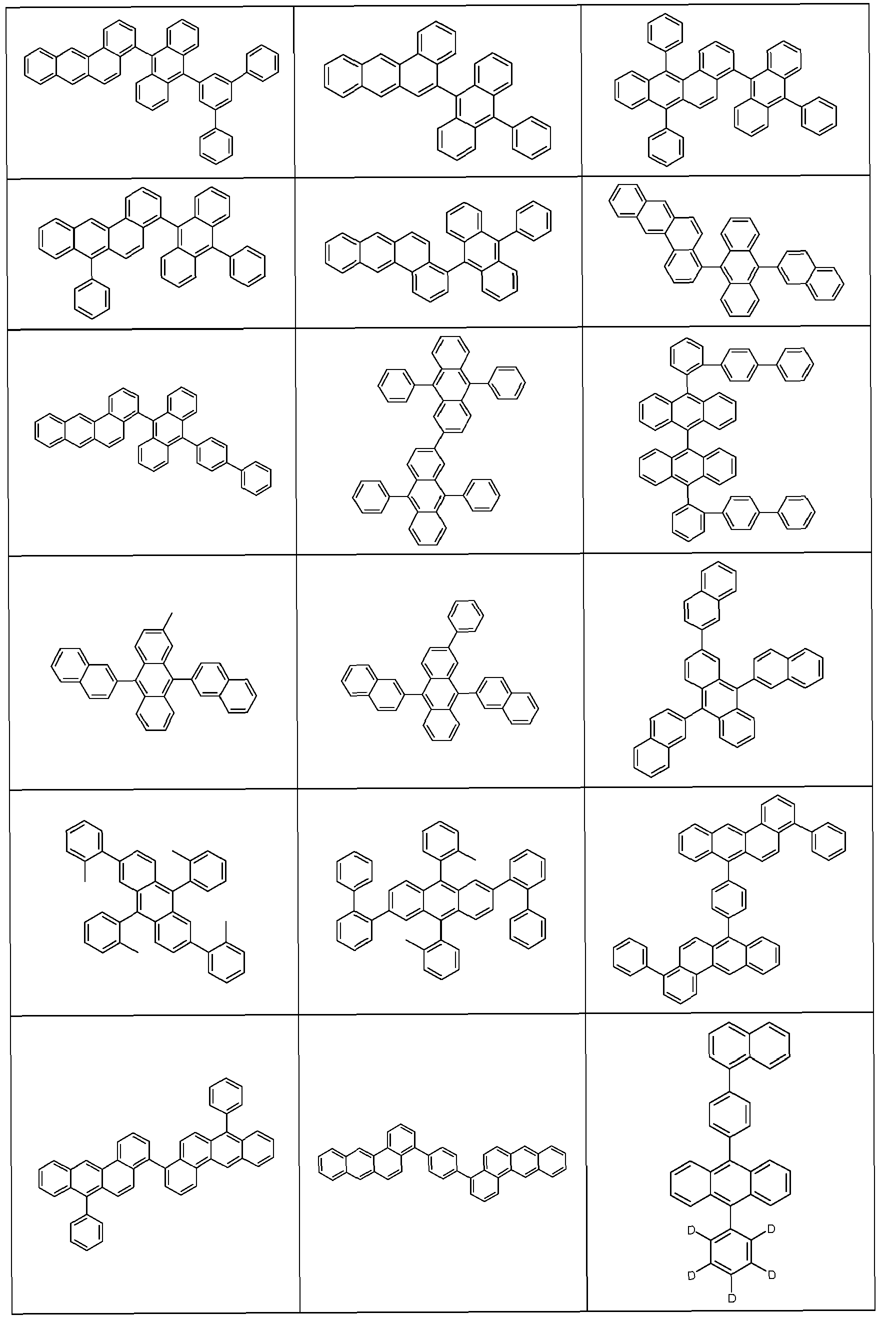

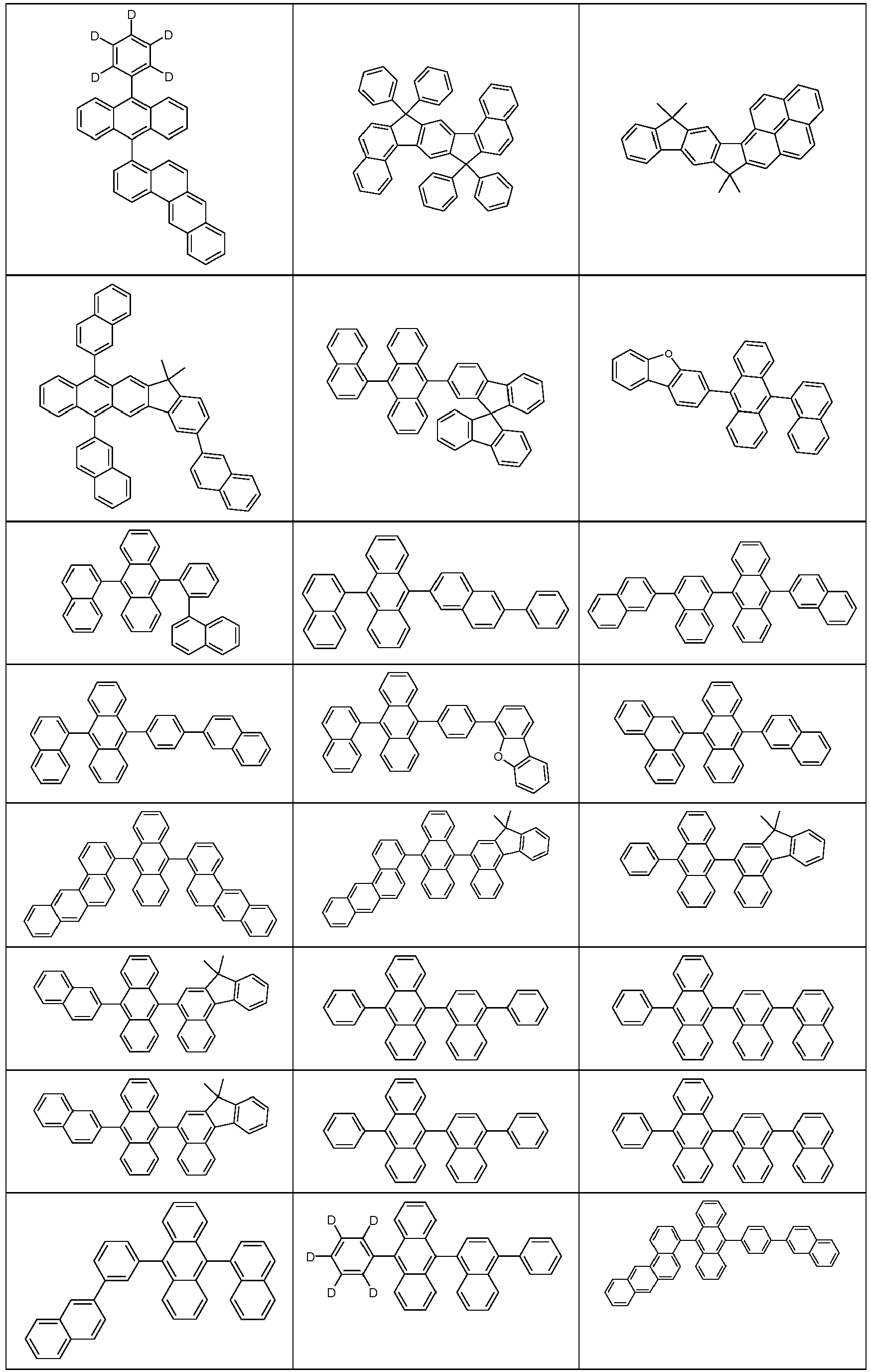

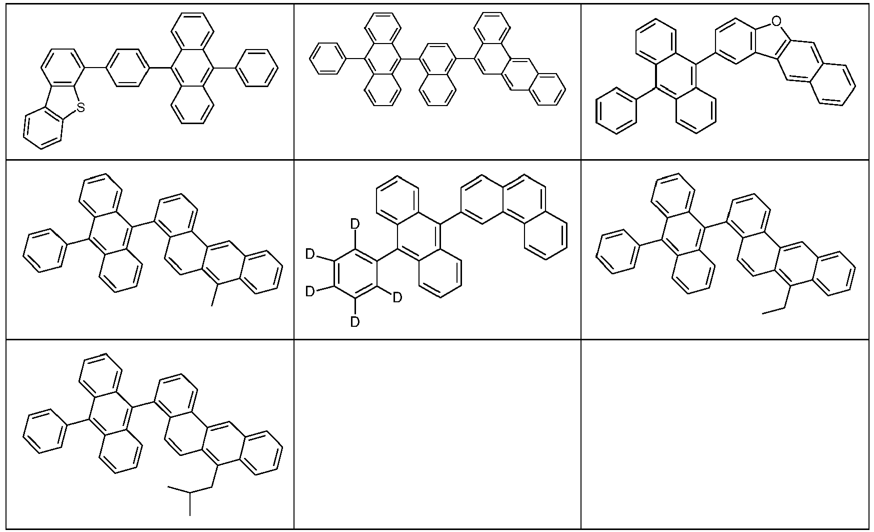

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Bevorzugte Matrixmaterialien für phosphoreszierende emittierende Verbindungen sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den erfindungsgemäßen Polymeren beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können neben den erfindungsgemäßen Verbindungen alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Erfindungsgemäß können die elektronischen Vorrichtungen enthaltend ein oder mehrere erfindungsgemäße Polymere in Displays, als Lichtquellen in Beleuchtungsanwendungen sowie als Lichtquellen in medizinischen und/oder kosmetischen Anwendungen (z.B. Lichttherapie) eingesetzt werden.

### Beispiele

### A) Synthese Beispiele:

### 1) Synthese der erfindungsgemäßen Monomere

### 1-1) Folgende Bausteine (building blocks, BB) werden für die Synthese der Monomere zur Herstellung der erfindungsgemäßen Polymere verwendet:

### a) Tetralin-analoge Bausteine

| | | |
|---|---|---|
| 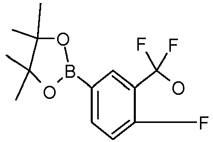 CAS-1562418-39-6 | 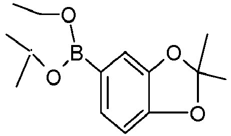 CAS-1562418-43-2 | 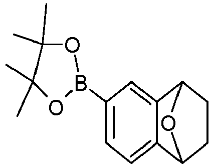 CAS-1562418-44-3 |
| BB-001 | BB-002 | BB-003 |
| 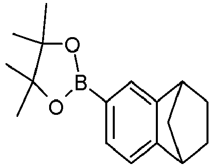 CAS-1562418-27-2 | 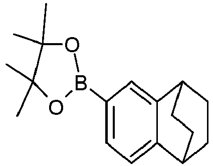 CAS-1801624-60-1 | 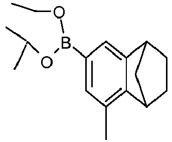 CAS-1562418-29-4 |
| BB-004 | BB-005 | BB-006 |
| 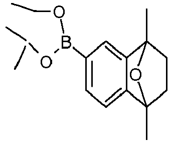 CAS-1562418-47-6 | 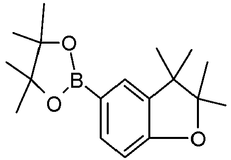 CAS-1562418-41-0 | 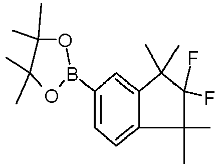 CAS-1562418-25-0 |
| BB-007 | BB-008 | BB-009 |
| 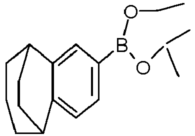 CAS-1801624-62-3 | 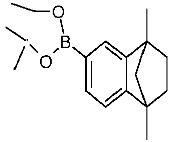 CAS-1562418-31-8 | 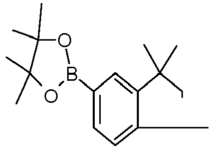 CAS-1312464-73-5 |
| BB-010 | BB-011 | BB-012 |
| 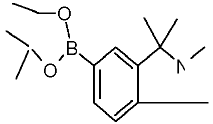 CAS-1562418-49-8 | 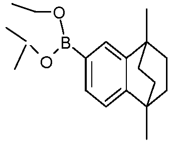 CAS-1801624-61-2 | 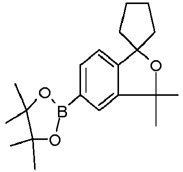 CAS-1562418-37-4 |
| BB-013 | BB-014 | BB-015 |
| 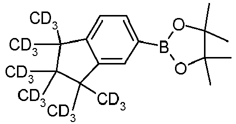 CAS-1562418-17-0 | 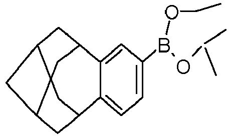 CAS-1801624-63-4 | 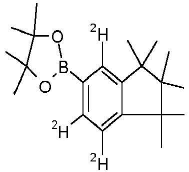 CAS-1562418-21-6 |
| BB-016 | BB-017 | BB-018 |
| 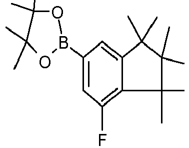 CAS-1562418-23-8 | 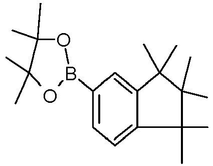 CAS-1562418-16-9 | 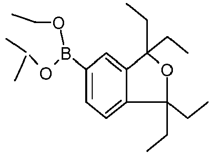 CAS-1562418-35-2 |
| BB-019 | BB-020 | BB-021 |
| 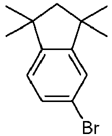 CAS: 169695-24-3 | 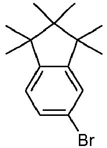 CAS: 1562418-80-7 | 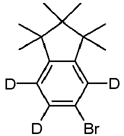 CAS: 1562418-83-0 |
| BB-050 | BB-051 | BB-052 |
| 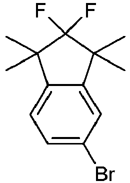 CAS: 1562418-85-2 | 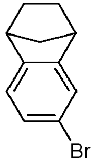 CAS: 16499-72-2 | 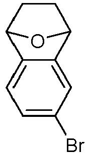 CAS: 1562418-91-0 |
| BB-053 | B B-054 | B B-055 |
| 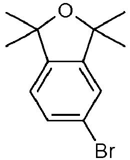 CAS: 179538-76-2 | 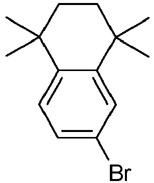 CAS: 27452-17-1 | 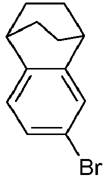 CAS: 1801624-95-2 |
| B B-056 | BB-057 | BB-058 |
| 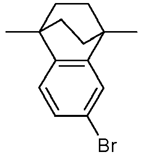 CAS: 1801624-96-3 | 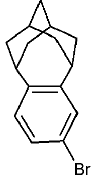 CAS: 1801624-97-4 | 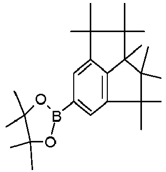 CAS-1562418-51-2 |
| B B-059 | BB-060 | B B-022 |
| 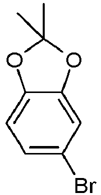 CAS: 73790-19 | 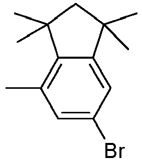 CAS: 1562418-82-9 | 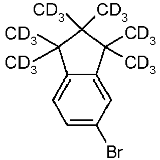 CAS: 1562418-81-8 |
| BB-062 | B B-063 | BB-064 |
| 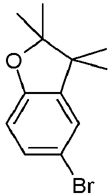 CAS: 1562418-90-9 | 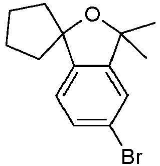 CAS: 1562418-89-6 | 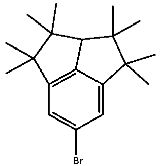 CAS: 1562418-94-3 |
| | BB-066 | BB-067 |
| BB-065 | | |

### b) Amin-Bausteine

| | | |
|---|---|---|
| 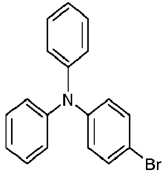 CAS-36809-26-4 | 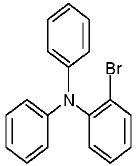 CAS-78600-31-4 | 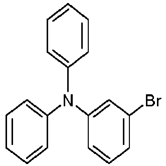 CAS-78600-33-6 |
| BB-500 | BB-501 | BB-502 |
| 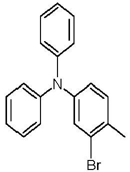 CAS-449153-47-3 | 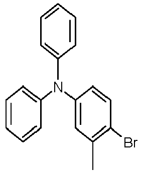 CAS-717880-57-4 | 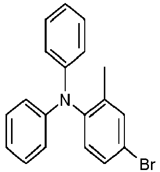 CAS-1458576-37-8 |
| BB-503 | BB-504 | BB-505 |
| 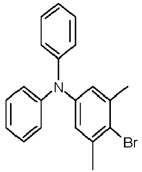 CAS-883550-96-7 | 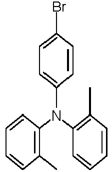 CAS-1373131 -53-3 | 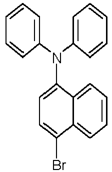 CAS-227314-47-8 |
| BB-506 | B B-507 | BB-508 |
| 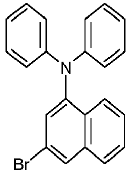 CAS-1807906-06-4 | 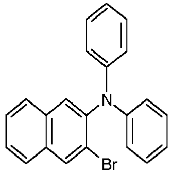 CAS-1807906-07-5 | 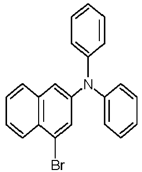 CAS-1877346-08-1 |
| BB-509 | BB-510 | BB-511 |
| 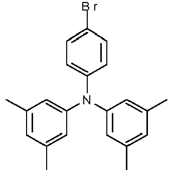 CAS-1372115-87-1 | 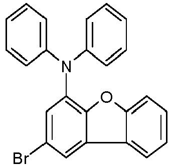 CAS-1801610-95-6 | 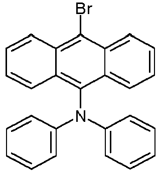 CAS-1801610-95-6 |
| BB-512 | BB-513 | BB-514 |
| 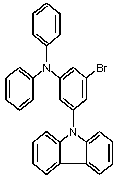 CAS-1686098-89-4 | 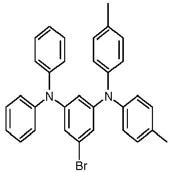 CAS-2007913-44-0 | 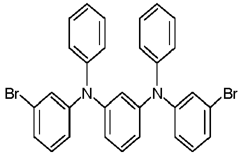 CAS-1365129-09-4 |
| BB-515 | BB-516 | BB-517 |
| 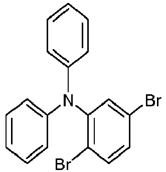 CAS-485372-68-7 | 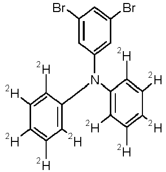 CAS-1291098-54-8 | 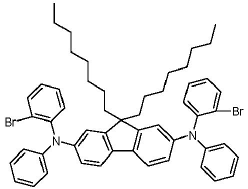 BB-520 |
| BB-518 | BB-519 | |
| 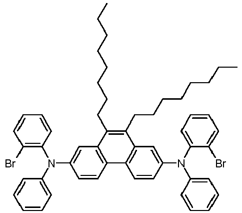 BB-521 | 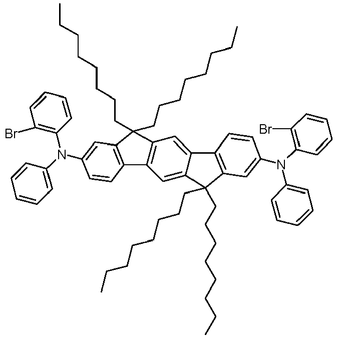 BB-522 | |
| 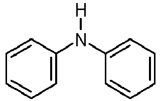 CAS -122-39-4 | 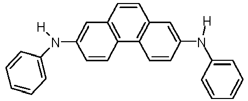 CAS-923020-74-0 | 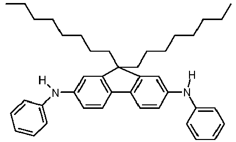 CAS-915031-04-8 |
| BB-750 | BB-751 | BB-752 |

### 1-2) Suzuki-Reaktion der Tetralin-analogen Bausteine und der Amin-Bausteine zu Kupplungsprodukten

### Beispiel-Reaktion

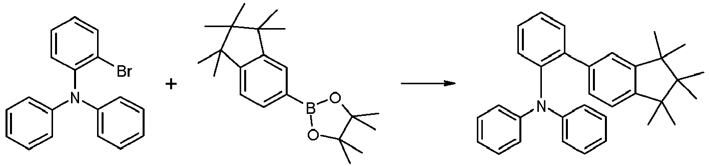

In einen 2-Liter-Vierhalskolben mit KPG-Rührer, Heizbad, Rückflusskühler und Argon-Anschluss werden 57g (176mmol) BB-501, 57,7g (176mmol, 1 eq.) BB-020, 10,16g (9mmol, 0.05eq) Tetrakis(triphenylphosphin)-palladium(0) (CAS: 14221-01-3) und 53,46g (387mmol 2.2eq) Kaliumcarbonat eingewogen und mit Schutzgas inertisiert. Es werden 400ml Toluol, 250ml 1,4-Dioxan und 115ml Wasser zugegeben und die Reaktionsmischung für 24h unter Rückfluss erhitzt. Nach Abkühlen wird mit Wasser verdünnt, die organische Phase abgetrennt und das Lösemittel im Vakuum entfernt. Der Rückstand wird mehrfach aus Heptan umkristallisiert und anschließend sublimiert. Es werden 34,8g (44,42%, 78mmol) des farblosen Feststoffes BB-1031 erhalten.

Folgende Strukturen können mit gleicher Vorschrift und ähnlichen Ausbeuten erhalten werden:

| | | |
|---|---|---|
| 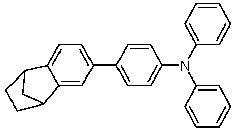 BB-500+BB-004 | 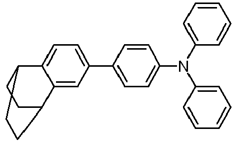 BB-500+BB-005 | 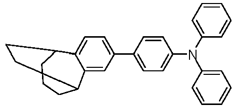 BB-500+BB-010 |
| BB-1000 | BB-1001 | BB-1002 |
| 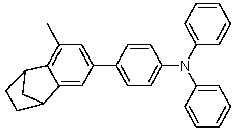 BB-500+BB-006 | 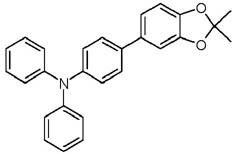 BB-500+BB-002 | 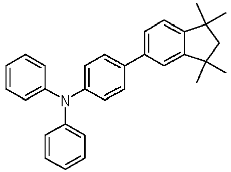 BB-500+BB-012 |
| BB-1003 | BB-1004 | BB-1005 |
| 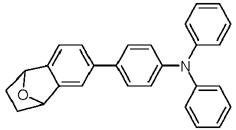 BB-500+BB-003 | 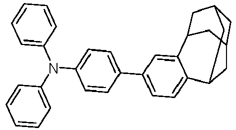 BB-500+BB-017 | 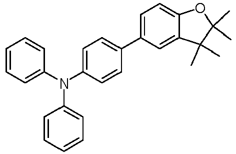 BB-500+BB-008 |
| BB-1006 | BB-1007 | BB-1008 |
| 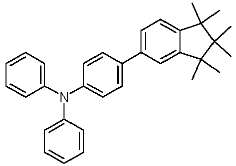 BB-500+BB-020 | 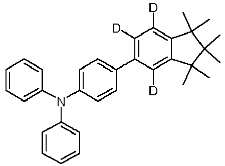 BB-500+BB-018 | 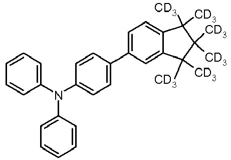 BB-500+BB-016 |
| BB-1009 | BB-1010 | BB-1011 |
| 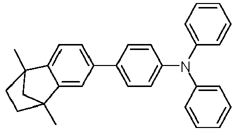 BB-500+BB-011 | 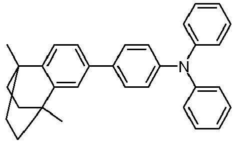 BB-500+BB-014 | 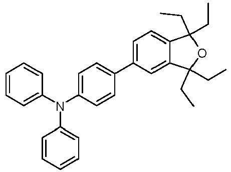 BB-500+BB-021 |
| BB-1012 | BB-1013 | BB-1014 |
| 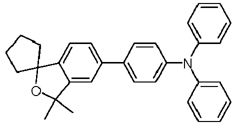 BB-500+BB-015 | 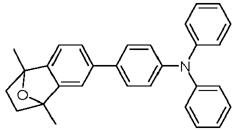 BB-500+BB-007 | 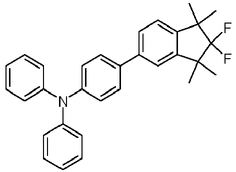 BB-500+BB-009 |
| BB-1015 | BB-1016 | BB-1017 |
| 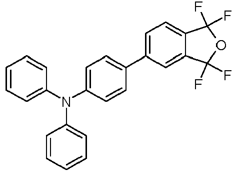 BB-500+BB-001 | 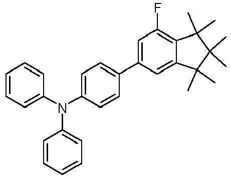 BB-500+BB-019 | 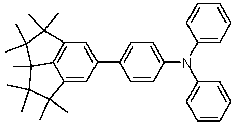 B B-500+ B B-022 |
| BB-1018 | BB-1019 | BB-1020 |
| 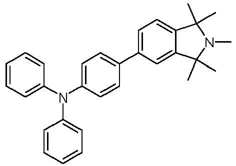 BB-500+BB-013 | 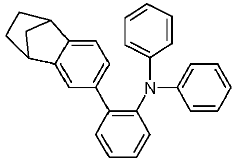 BB-501+BB-004 | 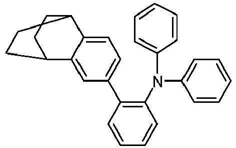 BB-501+BB-005 |
| BB-1021 | BB-1022 | BB-1023 |
| 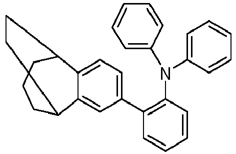 BB-501+BB-010 | 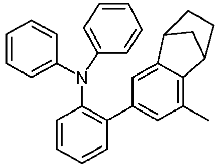 BB-501+BB-006 | 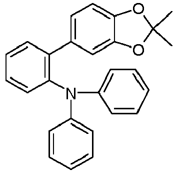 BB-501+BB-002 |
| BB-1024 | B B-1025 | BB-1026 |
| 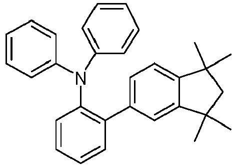 BB-501+BB-012 | 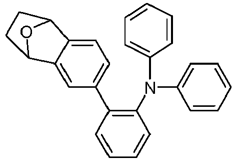 BB-501+BB-003 | 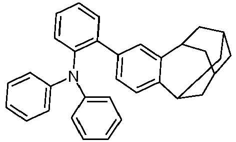 BB-501+BB-017 |
| BB-1027 | BB-1028 | BB-1029 |
| 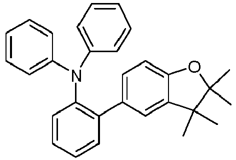 BB-501+BB-008 | 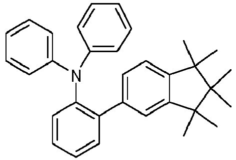 BB-501+BB-020 | 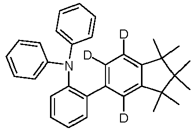 BB-501+BB-018 |
| BB-1030 | BB-1031 | BB-1032 |
| 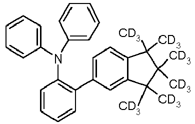 BB-501+BB-016 | 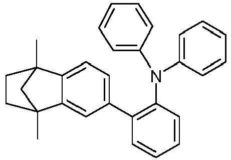 BB-501+BB-011 | 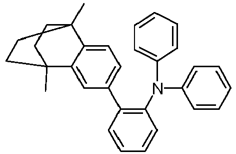 BB-501+BB-014 |
| B B-1033 | BB-1034 | BB-1035 |
| 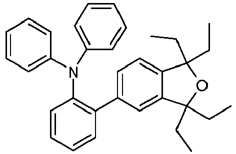 BB-501+BB-021 | 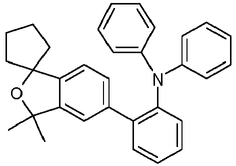 BB-501+BB-015 | 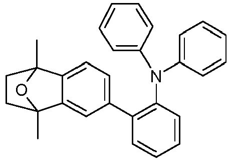 BB-501+BB-007 |
| BB-1036 | BB-1037 | BB-1038 |
| 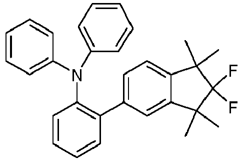 BB-501+BB-009 | 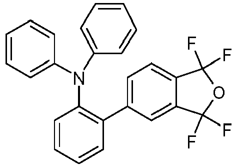 BB-501+BB-001 | 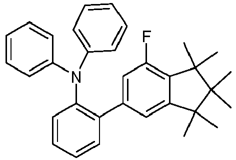 BB-501+BB-019 |
| BB-1039 | BB-1040 | BB-1041 |
| 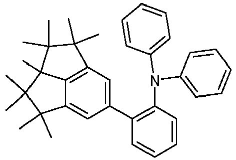 BB-501+BB-022 | 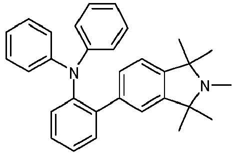 BB-501+BB-013 | 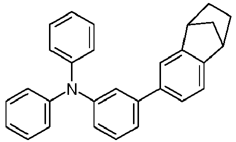 BB-502+BB-004 |
| BB-1042 | BB-1043 | BB-1044 |
| 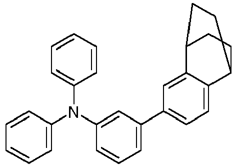 BB-502+BB-005 | 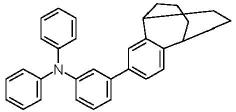 BB-502+BB-010 | 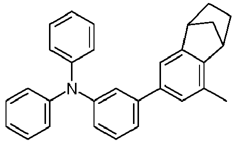 BB-502+BB-006 |
| BB-1045 | BB-1046 | BB-1047 |
| 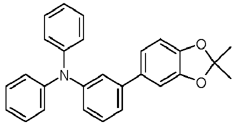 BB-502+BB-002 | 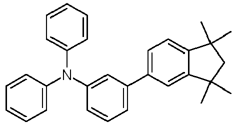 BB-502+BB-012 | 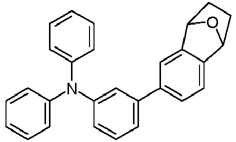 BB-502+BB-003 |
| BB-1048 | BB-1049 | BB-1050 |
| 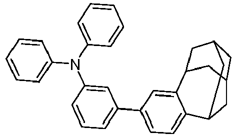 BB-502+BB-017 | 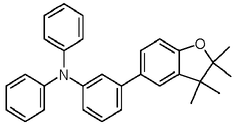 BB-502+BB-008 | 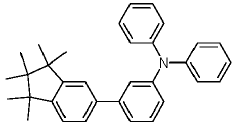 BB-502+BB-020 |
| BB-1051 | BB-1052 | BB-1053 |
| 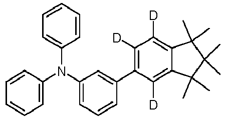 BB-502+BB-018 | 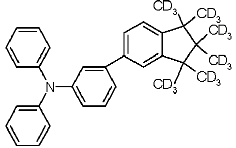 BB-502+BB-016 | 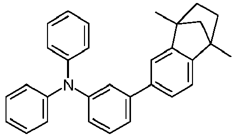 BB-502+BB-011 |
| BB-1054 | BB-1055 | BB-1056 |
| 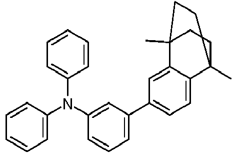 BB-502+BB-014 | 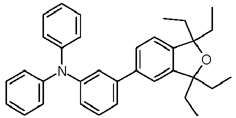 BB-502+BB-021 | 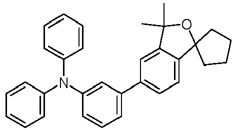 BB-502+BB-015 |
| BB-1057 | BB-1058 | BB-1059 |
| 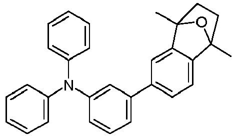 BB-502+BB-007 | 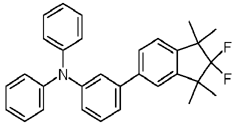 BB-502+BB-009 | 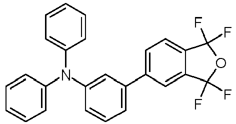 BB-502+BB-001 |
| BB-1060 | BB-1061 | BB-1062 |
| 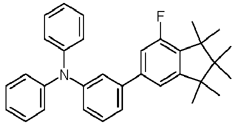 BB-502+BB-019 | 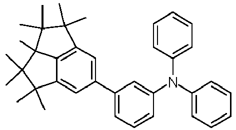 BB-502+BB-022 | 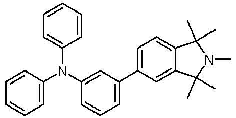 BB-502+BB-013 |
| BB-1063 | BB-1064 | BB-1065 |
| 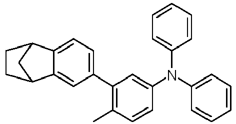 BB-503+BB-004 | 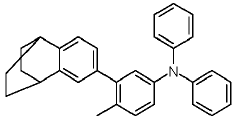 BB-503+BB-005 | 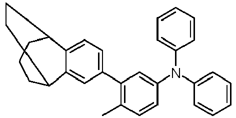 BB-503+BB-010 |
| BB-1066 | BB-1067 | BB-1068 |
| 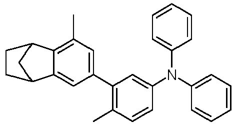 BB-503+BB-006 | 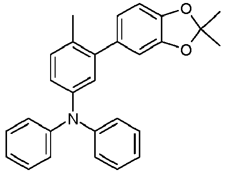 BB-503+BB-002 | 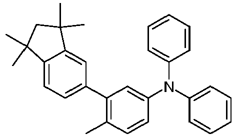 BB-503+BB-012 |
| BB-1069 | BB-1070 | BB-1071 |
| 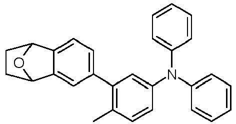 BB-503+BB-003 | 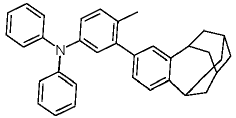 BB-503+BB-017 | 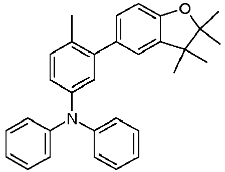 BB-503+BB-008 |
| BB-1072 | BB-1073 | BB-1074 |
| 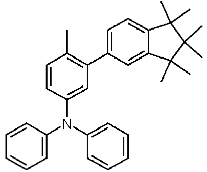 BB-503+BB-020 | 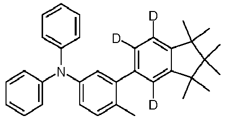 BB-503+BB-018 | 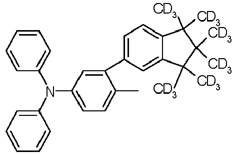 BB-503+BB-016 |
| BB-1075 | BB-1076 | BB-1077 |
| 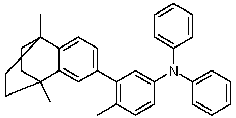 BB-503+BB-014 | 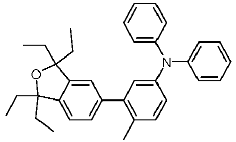 BB-503+BB-021 | 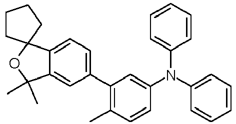 BB-503+BB-015 |
| BB-1079 | BB-1080 | BB-1081 |
| 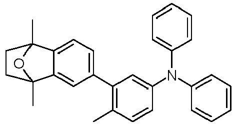 BB-503+BB-007 | 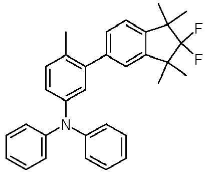 BB-503+BB-009 | 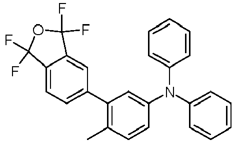 BB-503+BB-001 |
| BB-1082 | BB-1083 | BB-1084 |
| 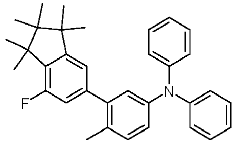 BB-503+BB-019 | 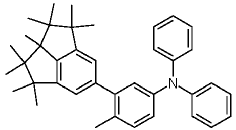 BB-503+BB-022 | 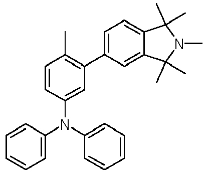 BB-503+BB-013 |
| BB-1085 | BB-1086 | BB-1087 |
| 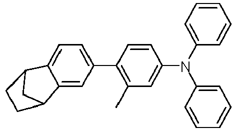 BB-504+BB-004 | 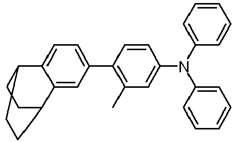 BB-504+BB-005 | 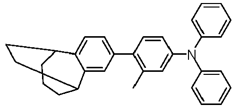 BB-504+BB-010 |
| BB-1088 | BB-1089 | BB-1090 |
| 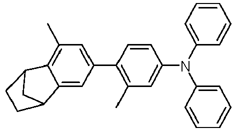 BB-504+BB-006 | 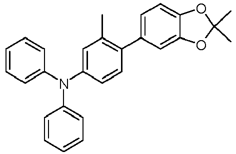 BB-504+BB-002 | 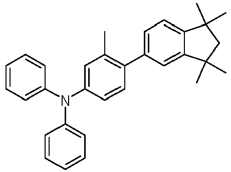 BB-504+BB-012 |
| BB-1091 | BB-1092 | BB-1093 |
| 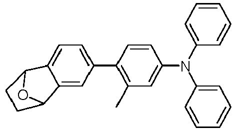 BB-504+BB-003 | 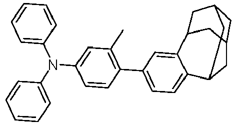 BB-504+BB-017 | 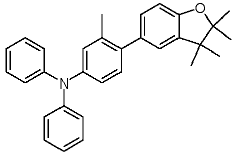 BB-504+BB-008 |
| BB-1094 | BB-1095 | BB-1096 |
| 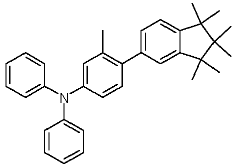 BB-504+BB-020 | 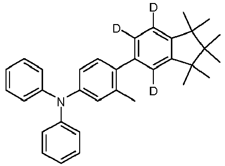 BB-504+BB-018 | 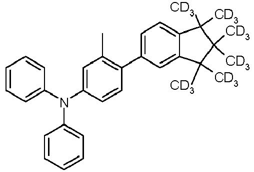 BB-504+BB-016 |
| BB-1097 | BB-1098 | BB-1099 |
| 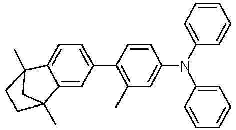 BB-504+BB-011 | 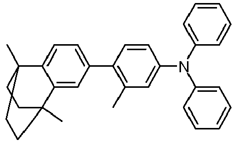 BB-504+BB-014 | 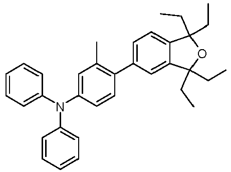 BB-504+BB-021 |
| BB-1100 | BB-1101 | BB-1102 |
| 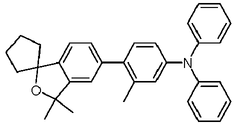 BB-504+BB-015 | 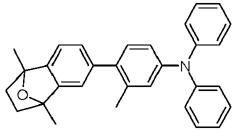 BB-504+BB-007 | 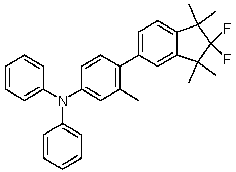 BB-504+BB-009 |
| BB-1103 | BB-1104 | BB-1105 |
| 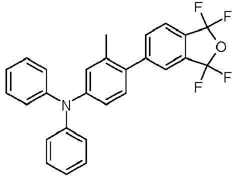 BB-504+BB-001 | 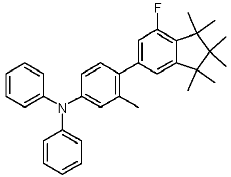 BB-504+BB-019 | 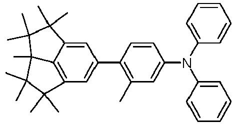 BB-504+BB-022 |
| BB-1106 | BB-1107 | BB-1108 |
| 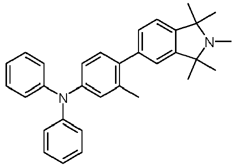 BB-504+BB-013 | 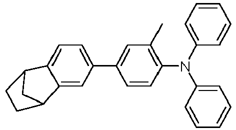 BB-505+BB-004 | 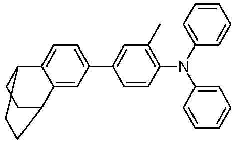 BB-505+BB-005 |
| BB-1109 | BB-1110 | BB-1111 |
| 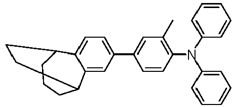 BB-505+BB-010 | 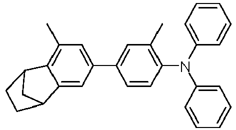 BB-505+BB-006 | 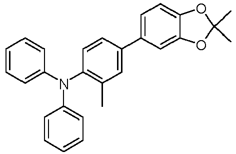 BB-505+BB-002 |
| BB-1112 | BB-1113 | BB-1114 |
| 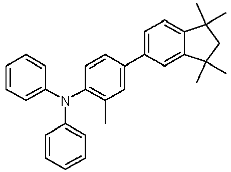 BB-505+BB-012 | 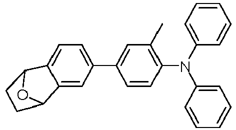 BB-505+BB-003 | 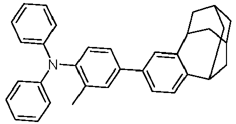 BB-505+BB-017 |
| BB-1115 | BB-1116 | BB-1117 |
| 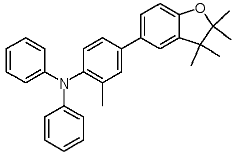 BB-505+BB-008 | 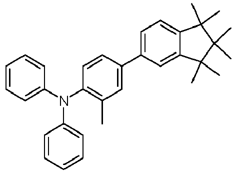 BB-505+BB-020 | 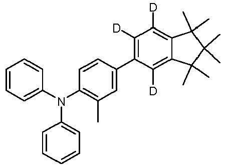 BB-505+BB-018 |
| BB-1118 | BB-1119 | BB-1120 |
| 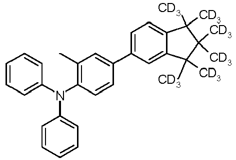 BB-505+BB-016 | 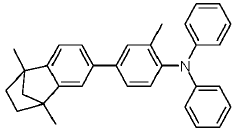 BB-505+BB-011 | 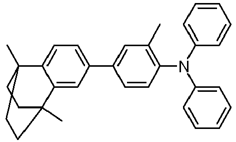 BB-505+BB-014 |
| BB-1121 | BB-1122 | BB-1123 |
| 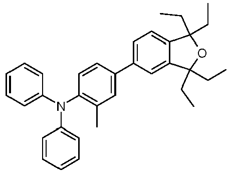 BB-505+BB-021 | 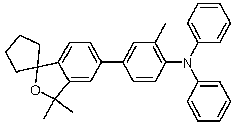 BB-505+BB-015 | 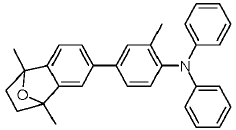 BB-505+BB-007 |
| BB-1124 | BB-1125 | BB-1126 |
| 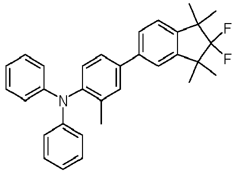 BB-505+BB-009 | 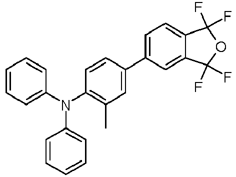 BB-505+BB-001 | 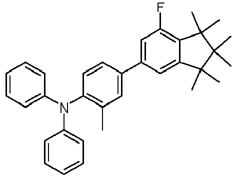 BB-505+BB-019 |
| BB-1127 | BB-1128 | BB-1129 |
| 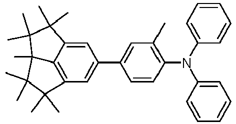 BB-505+BB-022 | 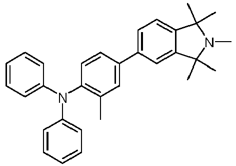 BB-505+BB-013 | 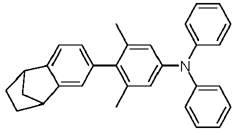 BB-506+BB-004 |
| BB-1130 | BB-1131 | BB-1132 |
| 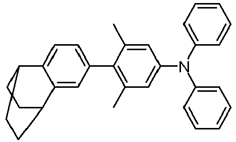 BB-506+BB-005 | 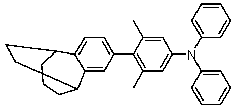 BB-506+BB-010 | 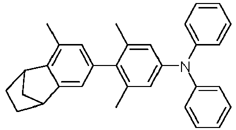 BB-506+BB-006 |
| BB-1133 | BB-1134 | BB-1135 |
| 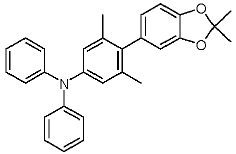 BB-506+BB-002 | 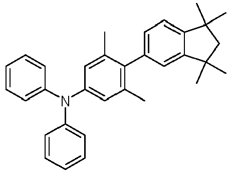 BB-506+BB-012 | 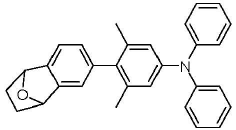 BB-506+BB-003 |
| BB-1136 | BB-1137 | BB-1138 |
| 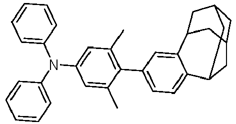 BB-506+BB-017 | 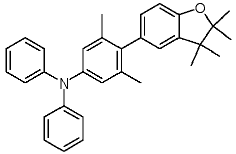 BB-506+BB-008 | 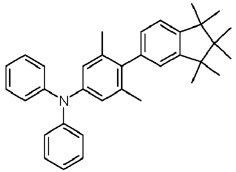 BB-506+BB-020 |
| BB-1139 | BB-1140 | BB-1141 |
| 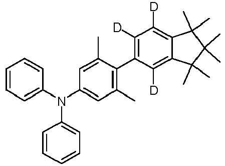 BB-506+BB-018 | 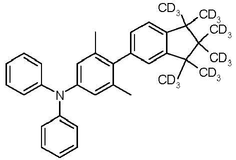 BB-506+BB-016 | 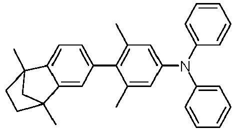 BB-506+BB-011 |
| BB-1142 | BB-1143 | BB-1144 |
| 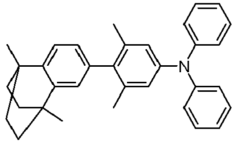 BB-506+BB-014 | 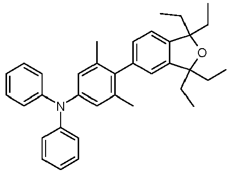 BB-506+BB-021 | 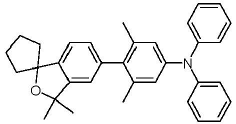 BB-506+BB-015 |
| BB-1145 | BB-1146 | BB-1147 |
| 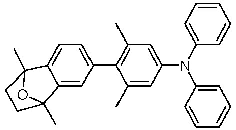 BB-506+BB-007 | 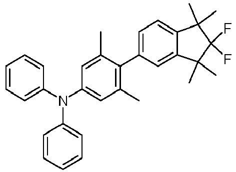 BB-506+BB-009 | 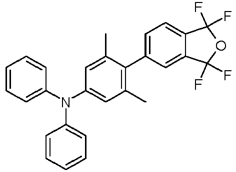 BB-506+BB-001 |
| BB-1148 | BB-1149 | BB-1150 |
| 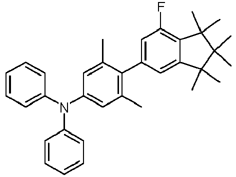 BB-506+BB-019 | 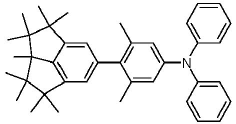 BB-506+BB-022 | 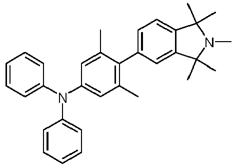 BB-506+BB-013 |
| BB-1151 | BB-1152 | BB-1153 |
| 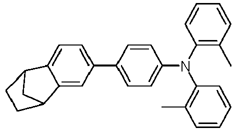 BB-507+BB-004 | 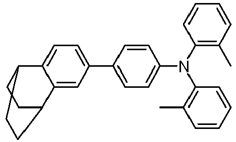 BB-507+BB-005 | 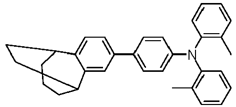 BB-507+BB-010 |
| BB-1154 | BB-1155 | BB-1156 |
| 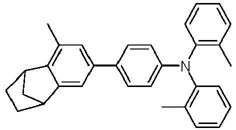 BB-507+BB-006 | 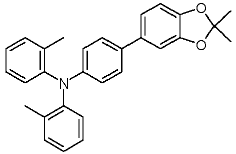 BB-507+BB-002 | 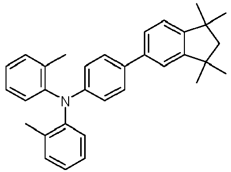 BB-507+BB-012 |
| BB-1157 | BB-1158 | BB-1159 |
| 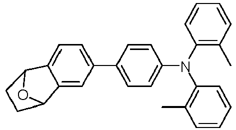 BB-507+BB-003 | 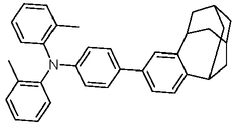 BB-507+BB-017 | 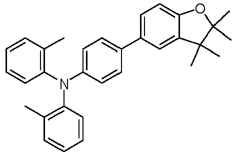 BB-507+BB-008 |
| BB-1160 | BB-1161 | BB-1162 |
| 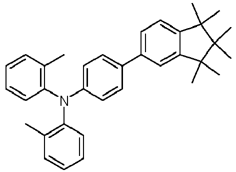 BB-507+BB-020 | 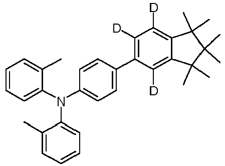 BB-507+BB-018 | 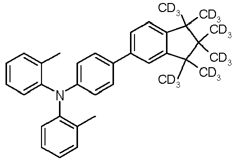 BB-507+BB-016 |
| BB-1163 | BB-1164 | BB-1165 |
| 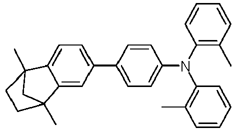 BB-507+BB-011 | 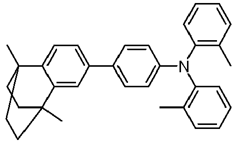 BB-507+BB-014 | 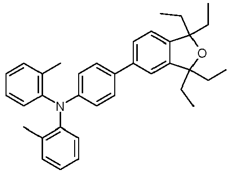 BB-507+BB-021 |
| BB-1166 | BB-1167 | BB-1168 |
| 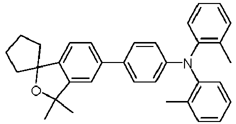 BB-507+BB-015 | 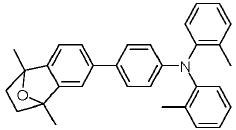 BB-507+BB-007 | 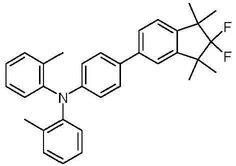 BB-507+BB-009 |
| BB-1169 | BB-1170 | BB-1171 |
| 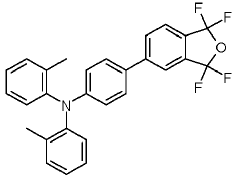 BB-507+BB-001 | 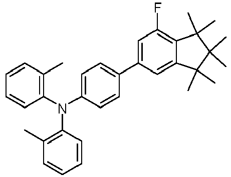 BB-507+BB-019 | 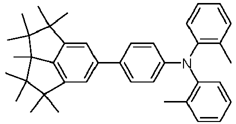 BB-507+BB-022 |
| BB-1172 | BB-1173 | BB-1174 |
| 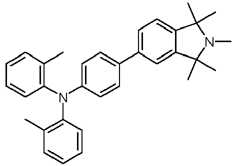 BB-507+BB-013 | 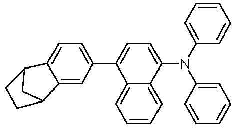 BB-508+BB-004 | 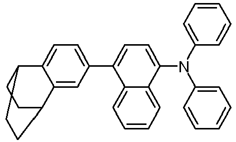 BB-508+BB-005 |
| BB-1175 | BB-1176 | BB-1177 |
| 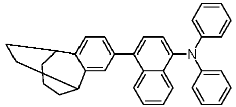 BB-508+BB-010 | 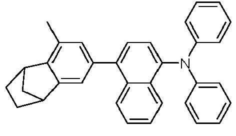 BB-508+BB-006 | 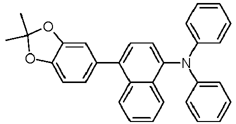 BB-508+BB-002 |
| BB-1178 | BB-1179 | BB-1180 |
| 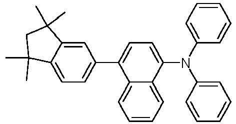 BB-508+BB-012 | 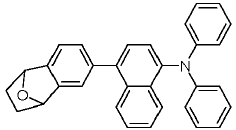 BB-508+BB-003 | 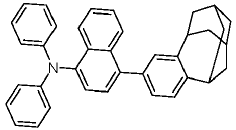 BB-508+BB-017 |
| BB-1181 | BB-1182 | BB-1183 |
| 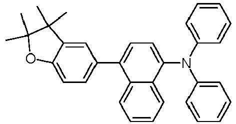 BB-508+BB-008 | 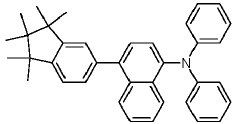 BB-508+BB-020 | 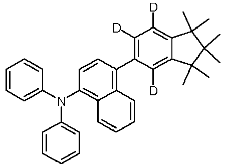 BB-508+BB-018 |
| BB-1184 | BB-1185 | BB-1186 |
| 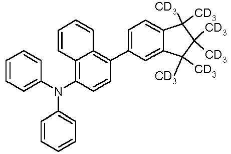 BB-508+BB-016 | 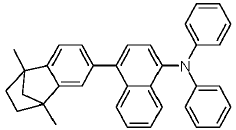 BB-508+BB-011 | 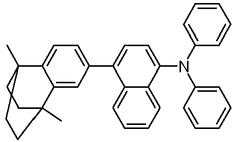 BB-508+BB-014 |
| BB-1187 | BB-1188 | BB-1189 |
| 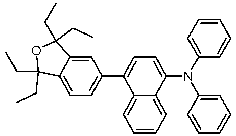 BB-508+BB-021 | 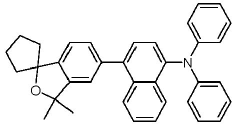 BB-508+BB-015 | 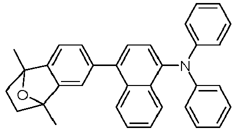 B B-508+ B B-007 |
| BB-1190 | BB-1191 | BB-1192 |
| 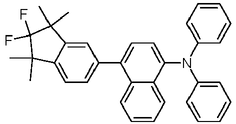 BB-508+BB-009 | 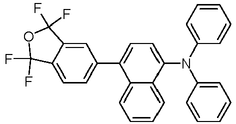 BB-508+BB-001 | 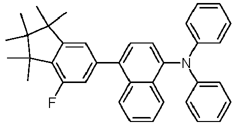 BB-508+BB-019 |
| BB-1193 | BB-1194 | BB-1195 |
| 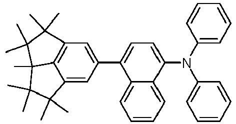 BB-508+BB-022 | 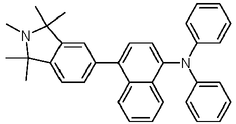 BB-508+BB-013 | 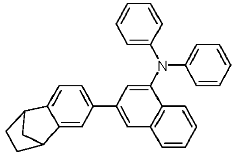 BB-509+BB-004 |
| BB-1196 | BB-1197 | BB-1198 |
| 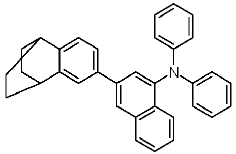 BB-509+BB-005 | 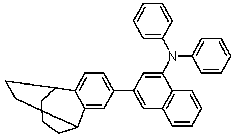 BB-509+BB-010 | 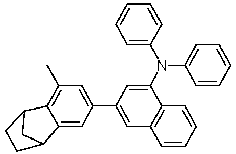 B B-509+ B B-006 |
| BB-1199 | BB-1200 | BB-1201 |
| 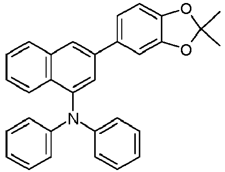 BB-509+BB-002 | 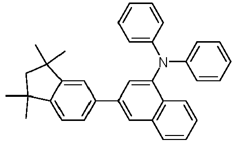 BB-509+BB-012 | 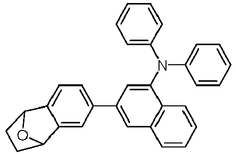 BB-509+BB-003 |
| BB-1202 | BB-1203 | BB-1204 |
| 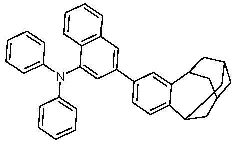 BB-509+BB-017 | 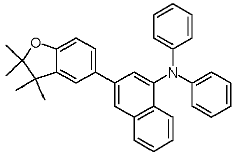 BB-509+BB-008 | 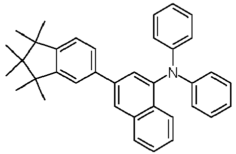 BB-509+BB-020 |
| BB-1205 | BB-1206 | BB-1207 |
| 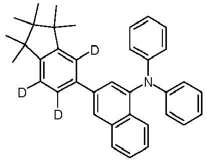 BB-509+BB-018 | 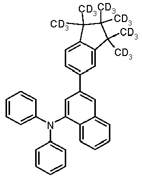 BB-509+BB-016 | 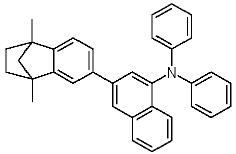 BB-509+BB-011 |
| BB-1208 | BB-1209 | BB-1210 |
| 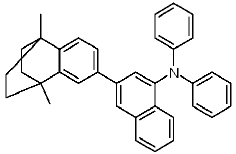 BB-509+BB-014 | 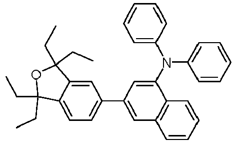 BB-509+BB-021 | 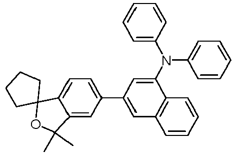 BB-509+BB-015 |
| BB-1211 | BB-1212 | BB-1213 |
| 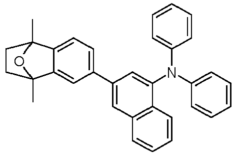 BB-509+BB-007 | 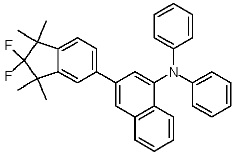 BB-509+BB-009 | 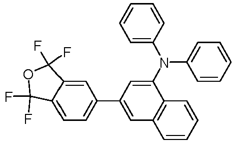 BB-509+BB-001 |
| BB-1214 | BB-1215 | BB-1216 |
| 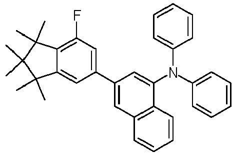 BB-509+BB-019 | 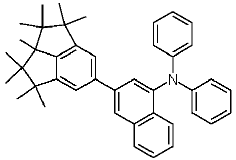 BB-509+BB-022 | 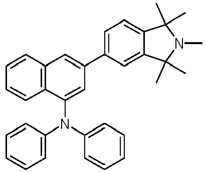 BB-509+BB-013 |
| BB-1217 | BB-1218 | BB-1219 |
| 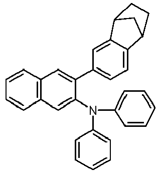 BB-510+BB-004 | 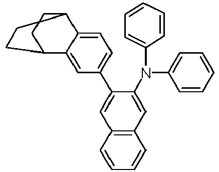 BB-510+BB-005 | 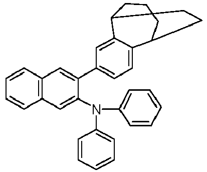 BB-510+BB-010 |
| BB-1220 | BB-1221 | BB-1222 |
| 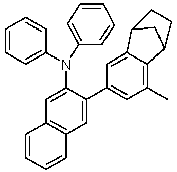 BB-510+BB-006 | 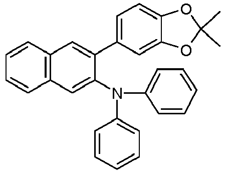 BB-510+BB-002 | 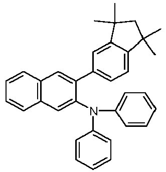 BB-510+BB-012 |
| B B-1223 | BB-1224 | BB-1225 |
| 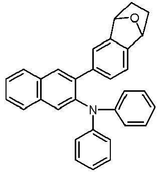 BB-510+BB-003 | 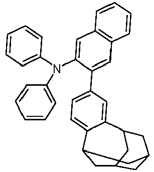 BB-510+BB-017 | 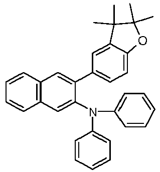 BB-510+BB-008 |
| BB-1226 | BB-1227 | BB-1228 |
| 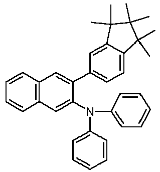 BB-510+BB-020 | 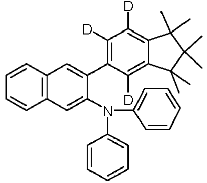 BB-510+BB-018 | 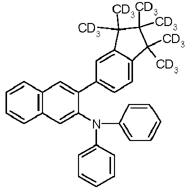 BB-510+BB-016 |
| B B-1229 | BB-1230 | BB-1231 |
| 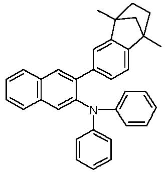 BB-510+BB-011 | 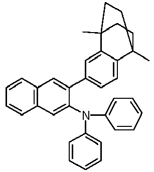 BB-510+BB-014 | 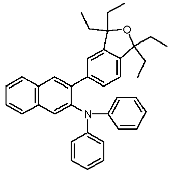 BB-510+BB-021 |
| BB-1232 | BB-1233 | BB-1234 |
| 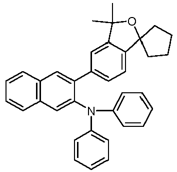 BB-510+BB-015 | 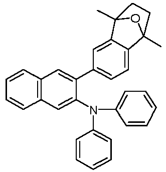 BB-510+BB-007 | 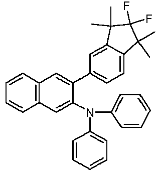 BB-510+BB-009 |
| B B-1235 | BB-1236 | BB-1237 |
| 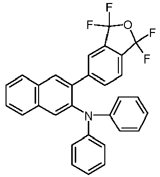 BB-510+BB-001 | 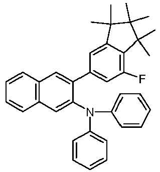 BB-510+BB-019 | 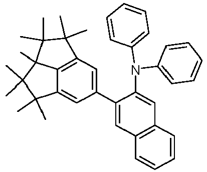 BB-510+BB-022 |
| BB-1238 | BB-1239 | BB-1240 |
| 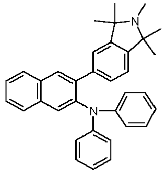 BB-510+BB-013 | 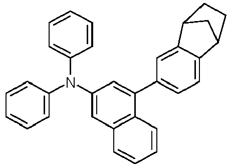 BB-511+BB-004 | 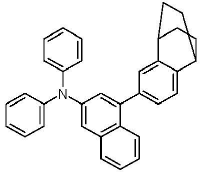 BB-511+BB-005 |
| BB-1241 | BB-1242 | BB-1243 |
| 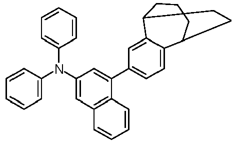 BB-511+BB-010 | 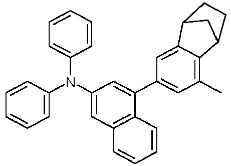 BB-511+BB-006 | 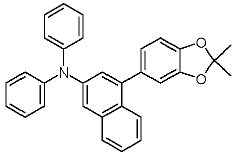 BB-511+BB-002 |
| BB-1244 | BB-1245 | BB-1246 |
| 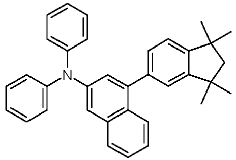 BB-511+BB-012 | 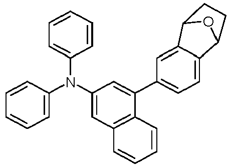 BB-511+BB-003 | 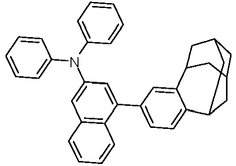 BB-511+BB-017 |
| BB-1247 | BB-1248 | BB-1249 |
| 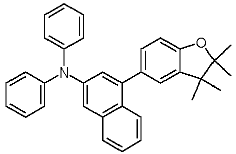 BB-511+BB-008 | 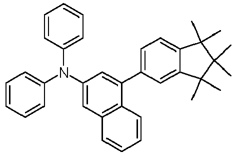 BB-511+BB-020 | 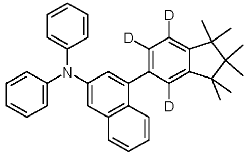 BB-511+BB-018 |
| BB-1250 | BB-1251 | BB-1252 |
| 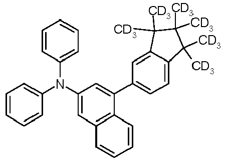 BB-511+BB-016 | 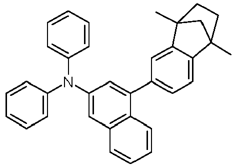 BB-511+BB-011 | 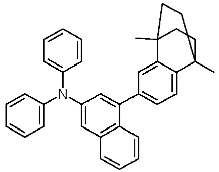 BB-511+BB-014 |
| BB-1253 | BB-1254 | BB-1255 |
| 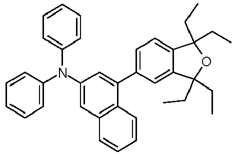 BB-511+BB-021 | 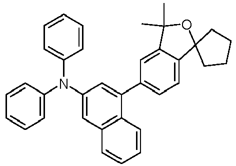 BB-511+BB-015 | 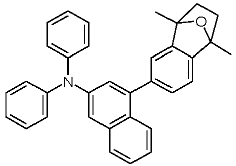 BB-511+BB-007 |
| BB-1256 | BB-1257 | BB-1258 |
| 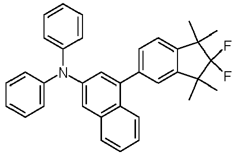 BB-511+BB-009 | 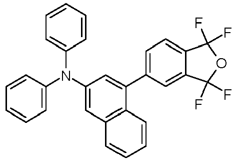 BB-511+BB-001 | 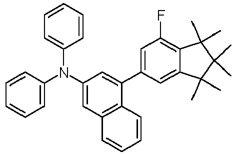 BB-511+BB-019 |
| BB-1259 | BB-1260 | BB-1261 |
| 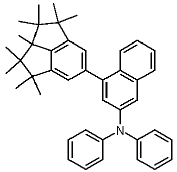 BB-511+BB-022 | 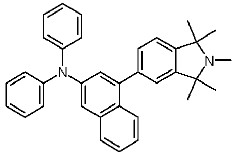 BB-511+BB-013 | 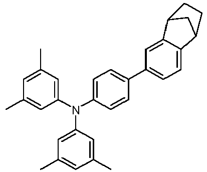 BB-512+BB-004 |
| BB-1262 | BB-1263 | BB-1264 |
| 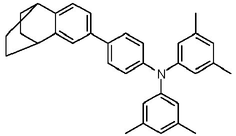 BB-512+BB-005 | 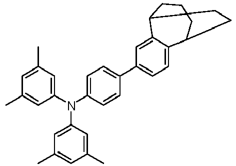 BB-512+BB-010 | 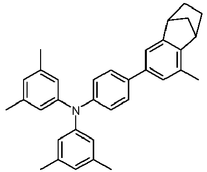 BB-512+BB-006 |
| BB-1265 | BB-1266 | BB-1267 |
| 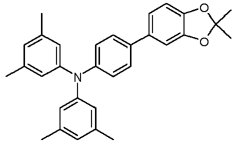 BB-512+BB-002 | 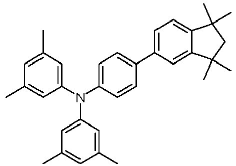 BB-512+BB-012 | 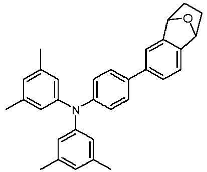 BB-512+BB-003 |
| BB-1268 | BB-1269 | BB-1270 |
| 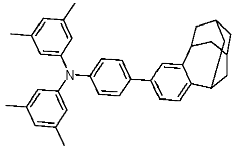 BB-512+BB-017 | 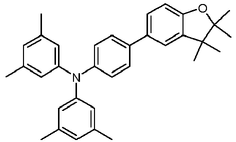 BB-512+BB-008 | 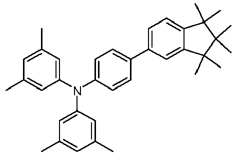 BB-512+BB-020 |
| BB-1271 | BB-1272 | BB-1273 |
| 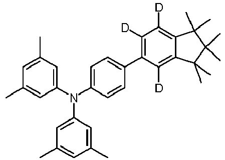 BB-512+BB-018 | 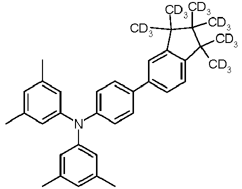 BB-512+BB-016 | 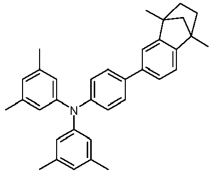 BB-512+BB-011 |
| BB-1274 | BB-1275 | BB-1276 |
| 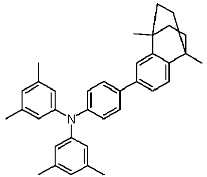 BB-512+BB-014 | 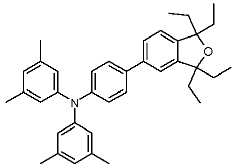 BB-512+BB-021 | 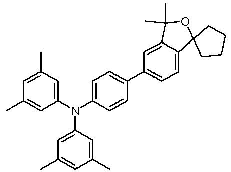 BB-512+BB-015 |
| BB-1277 | BB-1278 | BB-1279 |
| 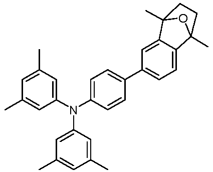 BB-512+BB-007 | 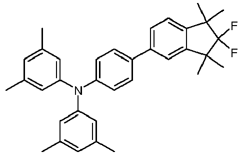 BB-512+BB-009 | 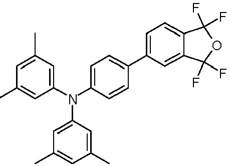 BB-512+BB-001 |
| BB-1280 | BB-1281 | BB-1282 |
| 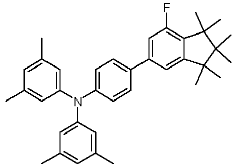 BB-512+BB-019 | 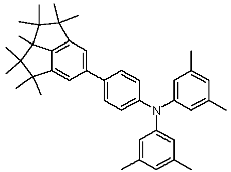 BB-512+BB-022 | 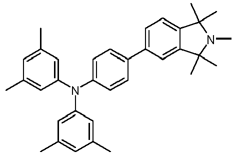 BB-512+BB-013 |
| BB-1283 | BB-1284 | BB-1285 |
| 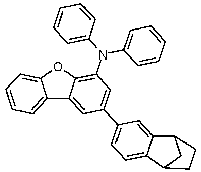 BB-513+BB-004 | 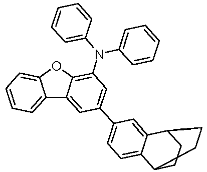 BB-513+BB-005 | 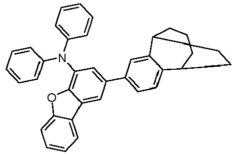 BB-513+BB-010 |
| BB-1286 | BB-1287 | BB-1288 |
| 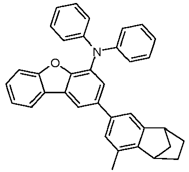 BB-513+BB-006 | 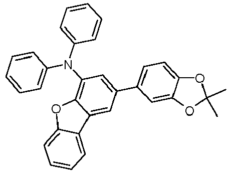 BB-513+BB-002 | 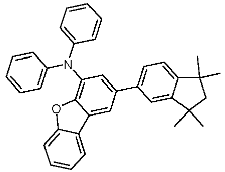 BB-513+BB-012 |
| BB-1289 | BB-1290 | BB-1291 |
| 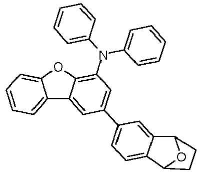 BB-513+BB-003 | 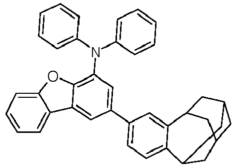 BB-513+BB-017 | 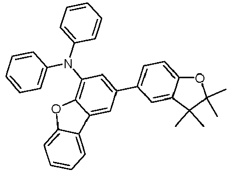 BB-513+BB-008 |
| BB-1292 | BB-1293 | BB-1294 |
| 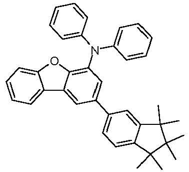 BB-513+BB-020 | 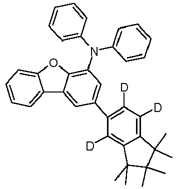 BB-513+BB-018 | 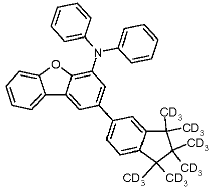 BB-513+BB-016 |
| BB-1295 | B B-1296 | BB-1297 |
| 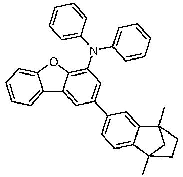 BB-513+BB-011 | 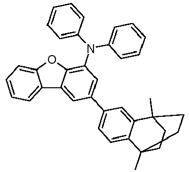 BB-513+BB-014 | 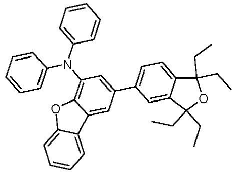 BB-513+BB-021 |
| BB-1298 | BB-1299 | BB-1300 |
| 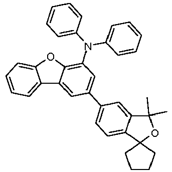 BB-513+BB-015 | 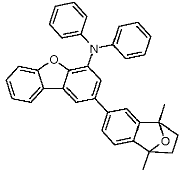 BB-513+BB-007 | 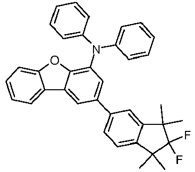 BB-513+BB-009 |
| BB-1301 | BB-1302 | BB-1303 |
| 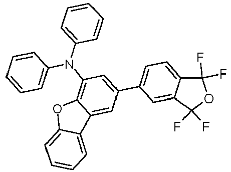 BB-513+BB-001 | 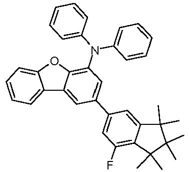 BB-513+BB-019 | 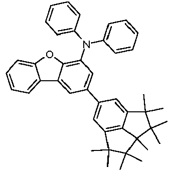 BB-513+BB-022 |
| BB-1304 | BB-1305 | BB-1306 |
| 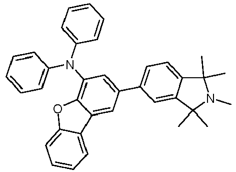 BB-513+BB-013 | 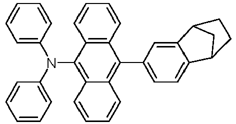 BB-5BB-063+BB-004 | 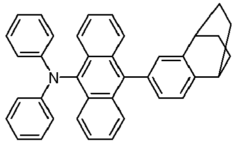 BB-5BB-063+BB-005 |
| BB-1307 | B B-1308 | BB-1309 |
| 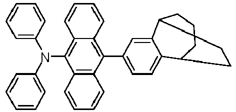 BB-5BB-063+BB-010 | 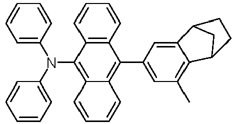 BB-5BB-063+BB-006 | 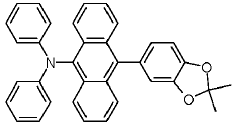 BB-5BB-063+BB-002 |
| BB-1310 | BB-1311 | BB-1312 |
| 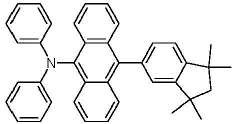 BB-5BB-063+BB-012 | 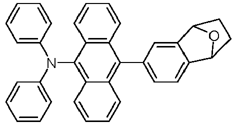 BB-5BB-063+BB-003 | 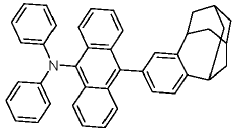 BB-5BB-063+BB-017 |
| BB-1313 | BB-1314 | BB-1315 |
| 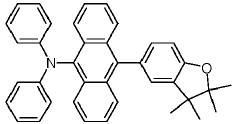 BB-5BB-063+BB-008 | 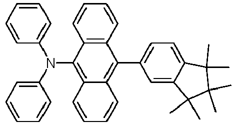 BB-5BB-063+BB-020 | 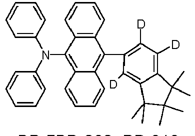 BB-5BB-063+BB-018 |
| BB-1316 | BB-1317 | BB-1318 |
| 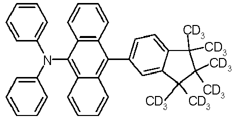 BB-5BB-063+BB-016 | 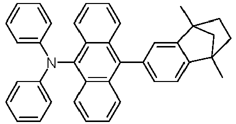 BB-5BB-063+BB-011 | 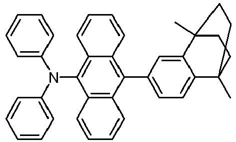 BB-5BB-063+BB-014 |
| BB-1319 | BB-1320 | BB-1321 |
| 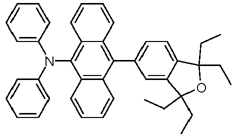 BB-5BB-063+BB-021 | 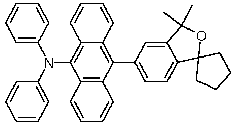 BB-5BB-063+BB-015 | 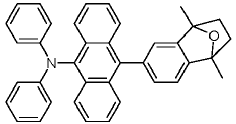 BB-5BB-063+BB-007 |
| BB-1322 | BB-1323 | BB-1324 |
| 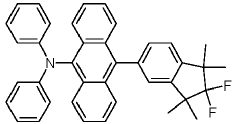 BB-5BB-063+BB-009 | 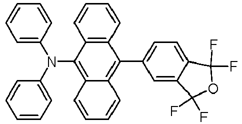 BB-5BB-063+BB-001 | 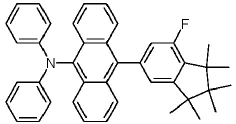 BB-5BB-063+BB-019 |
| BB-1325 | BB-1326 | BB-1327 |
| 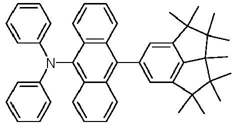 BB-5BB-063+BB-022 | 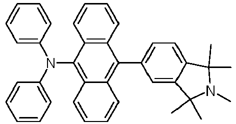 BB-5BB-063+BB-013 | 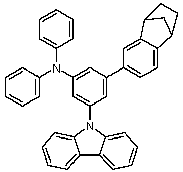 BB-515+BB-004 |
| BB-1328 | BB-1329 | BB-1330 |
| 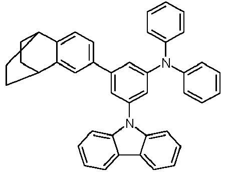 BB-515+BB-005 | 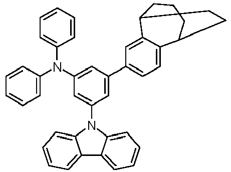 BB-515+BB-010 | 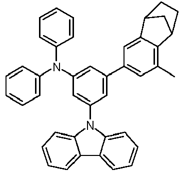 BB-515+BB-006 |
| BB-1331 | BB-1332 | BB-1333 |
| 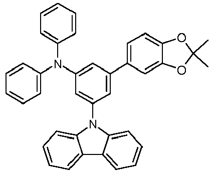 BB-515+BB-002 | 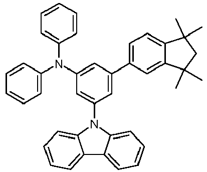 BB-515+BB-012 | 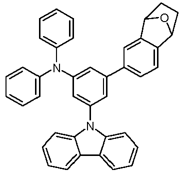 BB-515+BB-003 |
| BB-1334 | BB-1335 | BB-1336 |
| 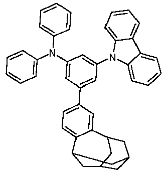 BB-515+BB-017 | 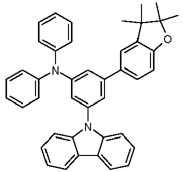 BB-515+BB-008 | 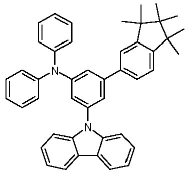 BB-515+BB-020 |
| BB-1337 | BB-1338 | BB-1339 |
| 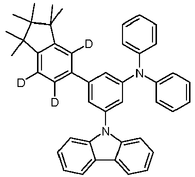 BB-515+BB-018 | 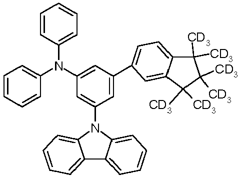 BB-515+BB-016 | 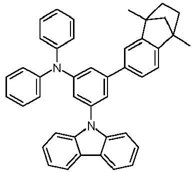 BB-515+BB-011 |
| BB-1340 | BB-1341 | BB-1342 |
| 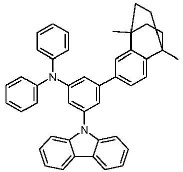 BB-515+BB-014 | 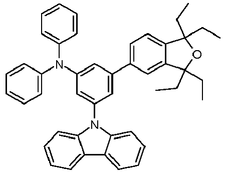 BB-515+BB-021 | 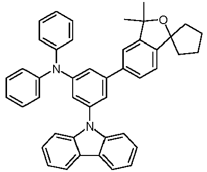 BB-515+BB-015 |
| BB-1343 | BB-1344 | BB-1345 |
| 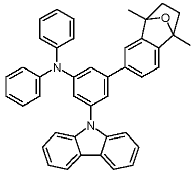 BB-515+BB-007 | 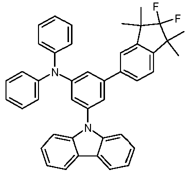 BB-515+BB-009 | 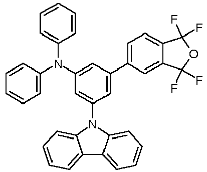 BB-515+BB-001 |
| BB-1346 | BB-1347 | BB-1348 |
| 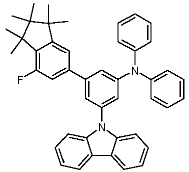 BB-515+BB-019 | 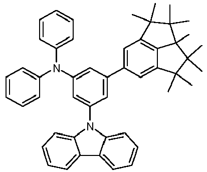 BB-515+BB-022 | 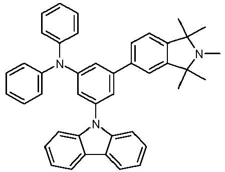 BB-515+BB-013 |
| BB-1349 | BB-1350 | BB-1351 |
| 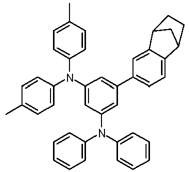 BB-516+BB-004 | 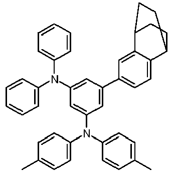 BB-516+BB-005 | 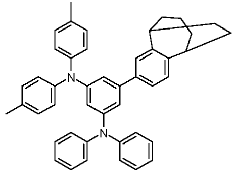 BB-516+BB-010 |
| BB-1352 | BB-1353 | BB-1354 |
| 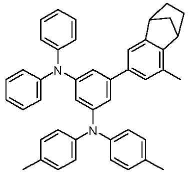 BB-516+BB-006 | 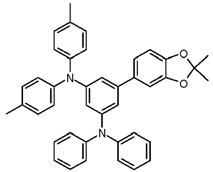 BB-516+BB-002 | 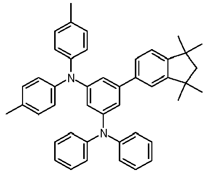 BB-516+BB-012 |
| BB-1355 | BB-1356 | BB-1357 |
| 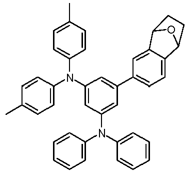 BB-516+BB-003 | 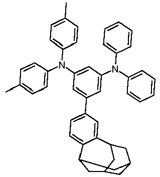 BB-516+BB-017 | 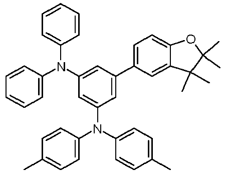 BB-516+BB-008 |
| BB-1358 | BB-1359 | BB-1360 |
| 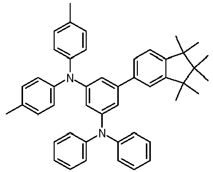 BB-516+BB-020 | 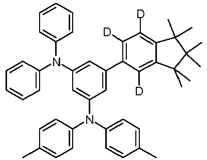 BB-516+BB-018 | 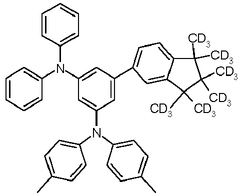 BB-516+BB-016 |
| BB-1361 | BB-1362 | BB-1363 |
| 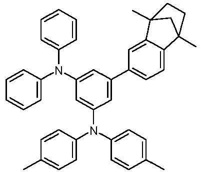 BB-516+BB-011 | 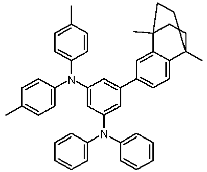 BB-516+BB-014 | 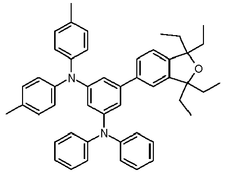 BB-516+BB-021 |
| BB-1364 | BB-1365 | BB-1366 |
| 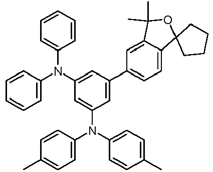 BB-516+BB-015 | 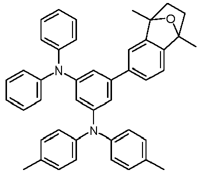 BB-516+BB-007 | 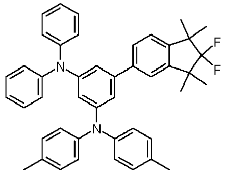 BB-516+BB-009 |
| BB-1367 | BB-1368 | BB-1369 |
| 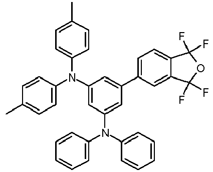 BB-516+BB-001 | 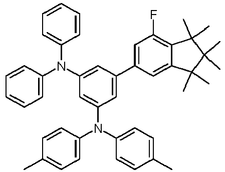 BB-516+BB-019 | 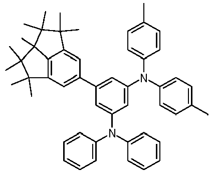 BB-516+BB-022 |
| BB-1370 | BB-1371 | BB-1372 |
| 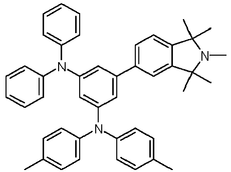 BB-516+BB-013 | 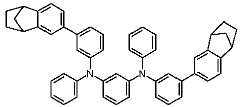 BB-517+BB-004 | 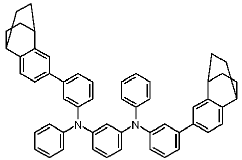 BB-517+BB-005 |
| BB-1373 | BB-1374 | BB-1375 |
| 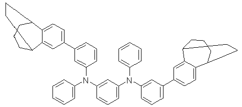 BB-517+BB-010 | 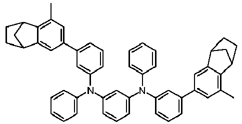 BB-517+BB-006 | 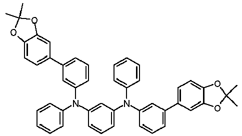 BB-517+BB-002 |
| BB-1376 | BB-1377 | BB-1378 |
| 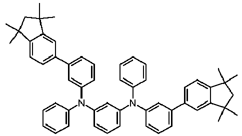 BB-517+BB-012 | 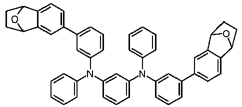 BB-517+BB-003 | 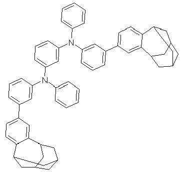 BB-517+BB-017 |
| BB-1379 | BB-1380 | BB-1381 |
| 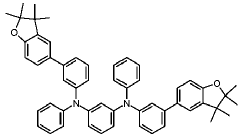 BB-517+BB-008 | 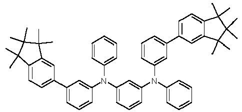 BB-517+BB-020 | 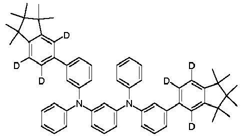 BB-517+BB-018 |
| BB-1382 | BB-1383 | BB-1384 |
| 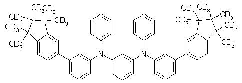 BB-517+BB-016 | 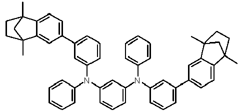 BB-517+BB-011 | 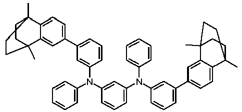 BB-517+BB-014 |
| BB-1385 | BB-1386 | BB-1387 |
| 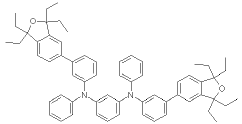 BB-517+BB-021 | 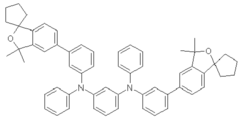 BB-517+BB-015 | 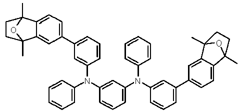 BB-517+BB-007 |
| BB-1388 | BB-1389 | BB-1390 |
| 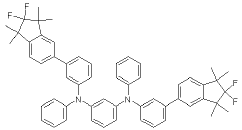 BB-517+BB-009 | 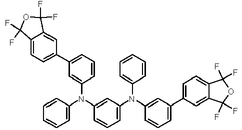 BB-517+BB-001 | 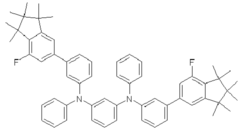 BB-517+BB-019 |
| BB-1391 | BB-1392 | BB-1393 |
| 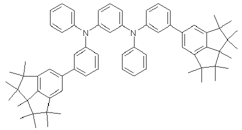 BB-517+BB-022 | 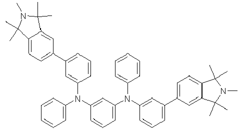 BB-517+BB-013 | 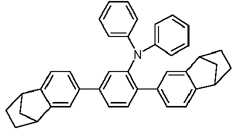 BB-518+BB-004 |
| BB-1394 | BB-1395 | BB-1396 |
| 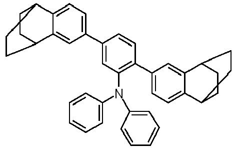 BB-518+BB-005 | 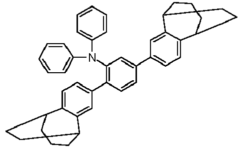 BB-518+BB-010 | 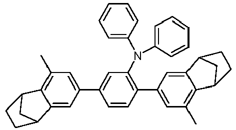 BB-518+BB-006 |
| BB-1397 | BB-1398 | BB-1399 |
| 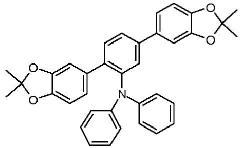 BB-518+BB-002 | 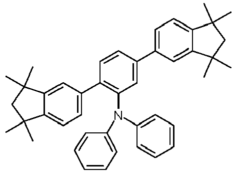 BB-518+BB-012 | 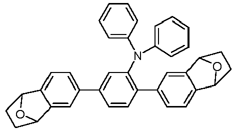 BB-518+BB-003 |
| BB-1400 | BB-1401 | BB-1402 |
| 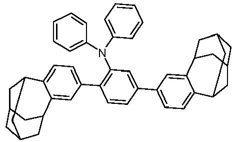 BB-518+BB-017 | 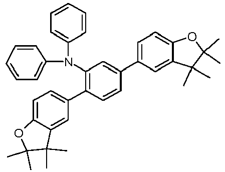 BB-518+BB-008 | 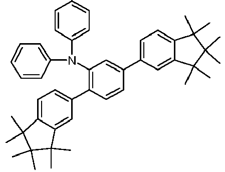 BB-518+BB-020 |
| BB-1403 | BB-1404 | BB-1405 |
| 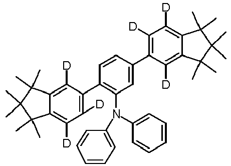 BB-518+BB-018 | 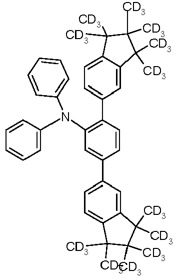 BB-518+BB-016 | 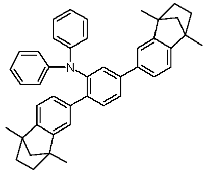 BB-518+BB-011 |
| BB-1406 | BB-1407 | BB-1408 |
| 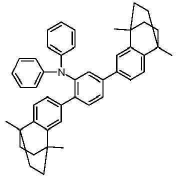 BB-518+BB-014 | 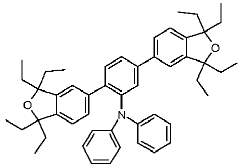 BB-518+BB-021 | 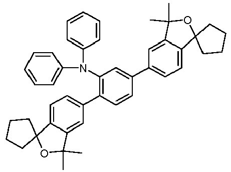 BB-518+BB-015 |
| BB-1409 | BB-1410 | BB-1411 |
| 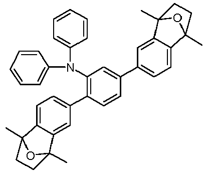 BB-518+BB-007 | 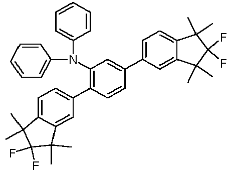 BB-518+BB-009 | 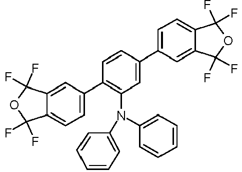 BB-518+BB-001 |
| BB-1412 | BB-1413 | BB-1414 |
| 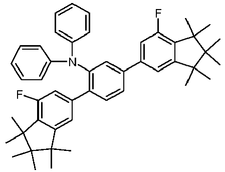 | 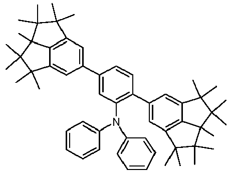 | 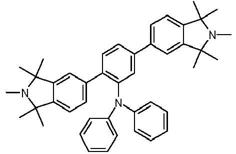 BB-518+BB-013 |
| BB-518+BB-019 | BB-518+BB-022 | BB-1417 |
| BB-1415 | BB-1416 | |
| 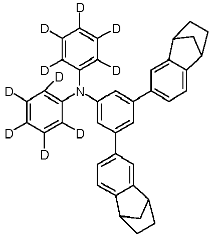 BB-519+BB-004 | 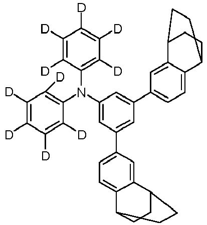 BB-519+BB-005 | 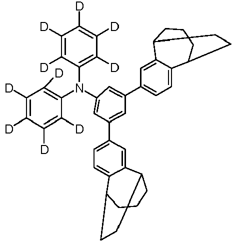 BB-519+BB-010 |
| BB-1418 | BB-1419 | BB-1420 |
| 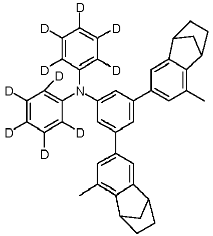 BB-519+BB-006 | 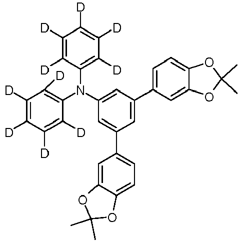 BB-519+BB-002 | 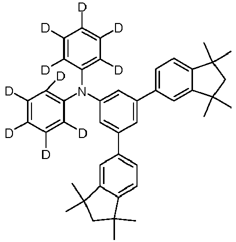 BB-519+BB-012 |
| BB-1421 | B B-1422 | BB-1423 |
| 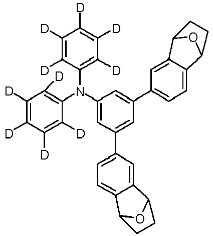 BB-519+BB-003 | 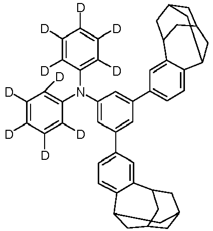 BB-519+BB-017 | 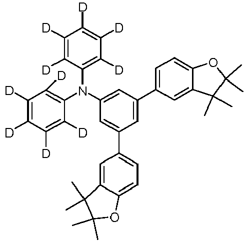 BB-519+BB-008 |
| BB-1424 | BB-1425 | BB-1426 |
| 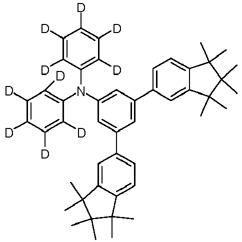 BB-519+BB-020 | 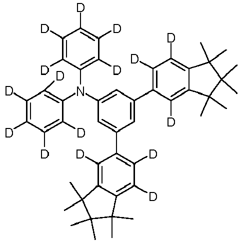 BB-519+BB-018 | 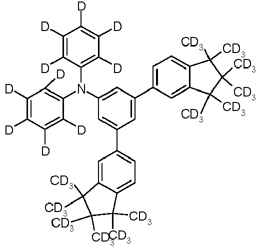 BB-519+BB-016 |
| BB-1427 | BB-1428 | BB-1429 |
| 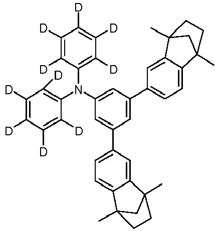 BB-519+BB-011 | 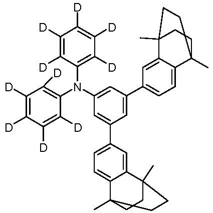 BB-519+BB-014 | 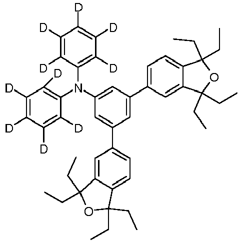 BB-519+BB-021 |
| BB-1430 | BB-1431 | BB-1432 |
| 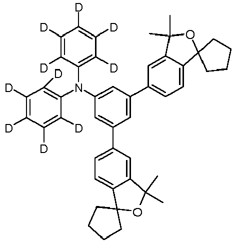 BB-519+BB-015 | 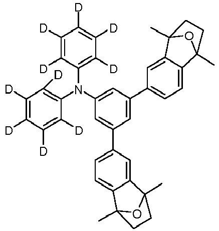 BB-519+BB-007 | 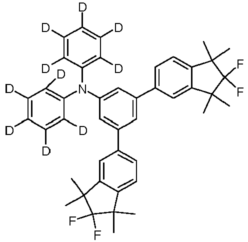 BB-519+BB-009 |
| BB-1433 | BB-1434 | BB-1435 |
| 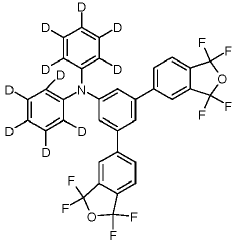 BB-519+BB-001 | 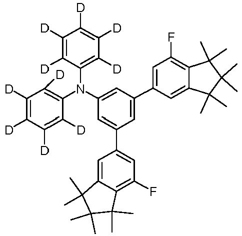 BB-519+BB-019 | 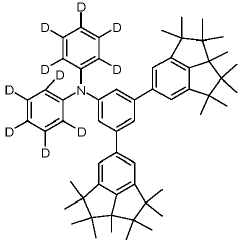 BB-519+BB-022 |
| BB-1436 | BB-1437 | BB-1438 |
| 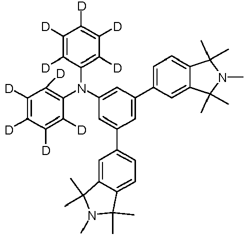 BB-519+BB-013 | 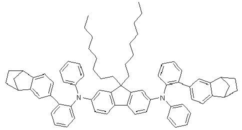 BB520+BB-004 | 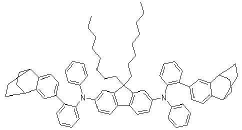 BB520+BB-005 |
| BB-1439 | BB-1440 | BB-1441 |
| 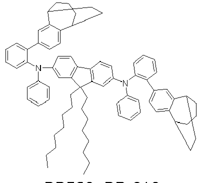 BB520+BB-010 | 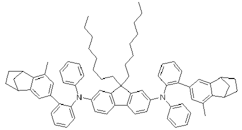 BB520+BB-006 | 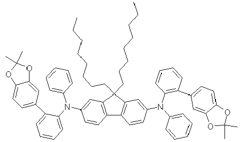 BB520+BB-002 |
| BB-1442 | BB-1443 | BB-1444 |
| 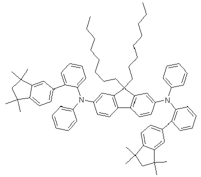 BB520+BB-012 | 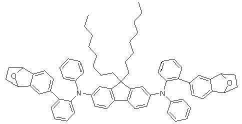 BB520+BB-003 | 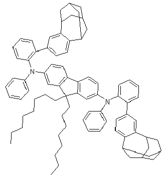 BB520+BB-017 |
| BB-1445 | BB-1446 | BB-1447 |
| 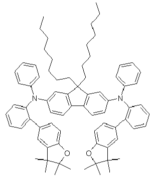 BB520+BB-008 | 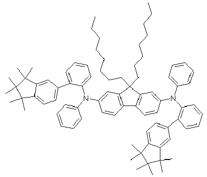 BB520+BB-020 | 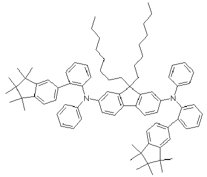 BB520+BB-016 |
| BB-1448 | BB-1449 | BB-1450 |
| 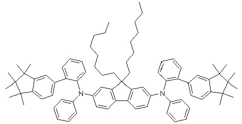 BB520+BB-018 | 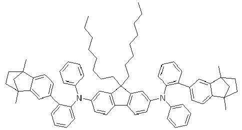 BB520+BB-011 | 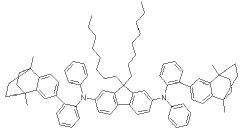 BB520+BB-014 |
| BB-1451 | BB-1452 | BB-1453 |
| 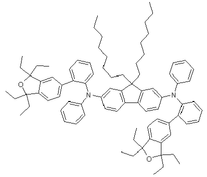 BB520+BB-021 | 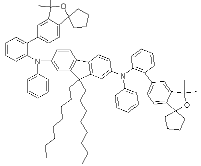 BB520+BB-015 | 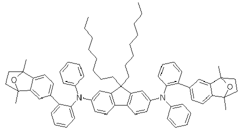 BB520+BB-007 |
| BB-1454 | BB-1455 | BB-1456 |
| 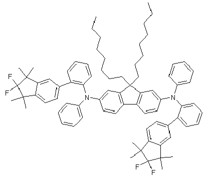 BB520+BB-015 | 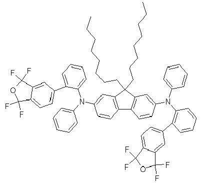 BB520+BB-001 | 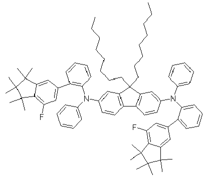 BB520+BB-019 |
| BB-1457 | BB-1458 | BB-1459 |
| 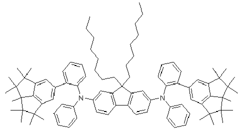 BB520+BB-022 | 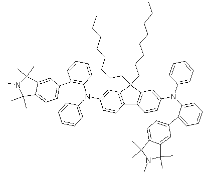 BB520+BB-013 | 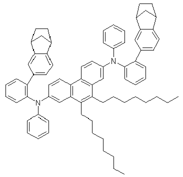 BB521+BB-004 |
| BB-1460 | BB-1461 | BB-1462 |
| 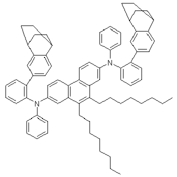 BB521+BB-005 | 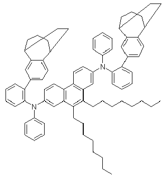 BB521+BB-010 | 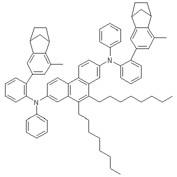 BB521+BB-006 |
| BB-1463 | BB-1464 | BB-1465 |
| 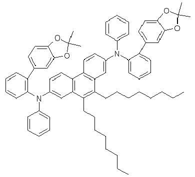 BB521+BB-002 | 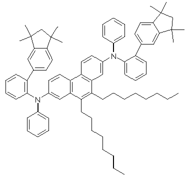 BB521+BB-012 | 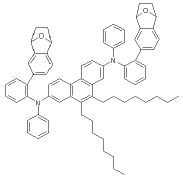 BB521+BB-003 |
| BB-1466 | BB-1467 | BB-1468 |
| 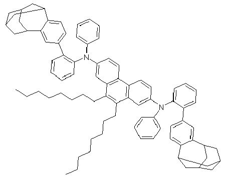 BB521+BB-017 | 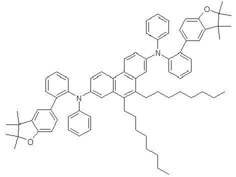 BB521+BB-008 | 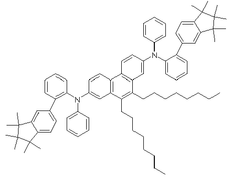 BB521+BB-020 |
| BB-1469 | BB-1470 | BB-1471 |
| 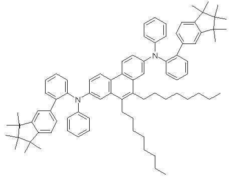 BB521+BB-016 | 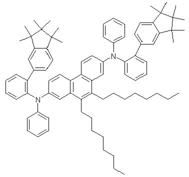 BB521+BB-018 | 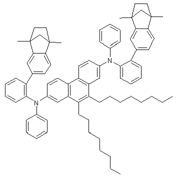 BB521+BB-011 |
| BB-1472 | BB-1473 | BB-1474 |
| 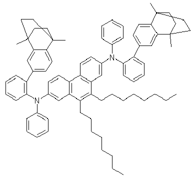 BB521+BB-014 | 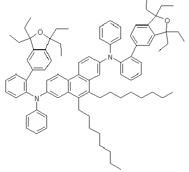 BB521+BB-021 | 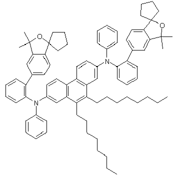 BB521+BB-015 |
| BB-1475 | BB-1476 | BB-1477 |
| 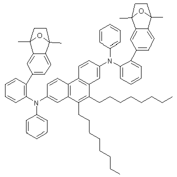 BB521+BB-007 | 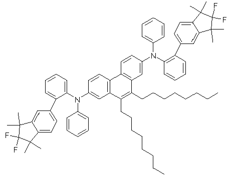 BB521+BB-015 | 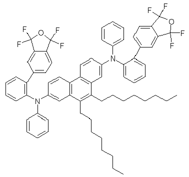 BB521+BB-001 |
| BB-1478 | BB-1479 | BB-1480 |
| 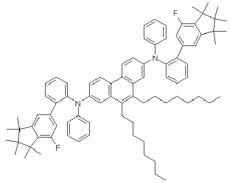 BB521+BB-019 | 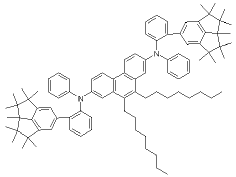 BB521+BB-022 | 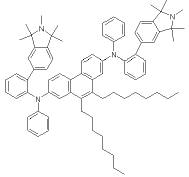 BB521+BB-013 |
| BB-1481 | BB-1482 | BB-1483 |
| 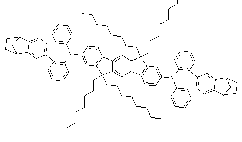 BB522+BB-004 | 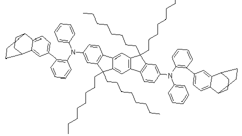 BB522+BB-005 | 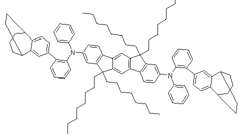 BB522+BB-010 |
| BB-1484 | BB-1485 | BB-1486 |
| 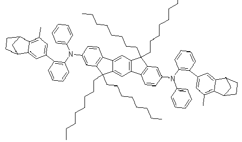 BB522+BB-006 | 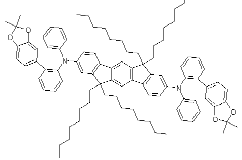 BB522+BB-002 | 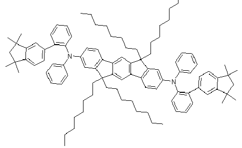 BB522+BB-012 |
| BB-1487 | BB-1488 | BB-1489 |
| 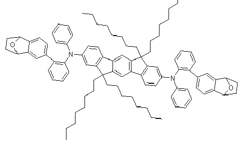 BB522+BB-003 | 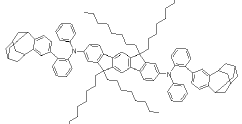 BB522+BB-017 | 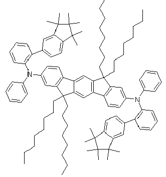 BB522+BB-020 |
| BB-1490 | BB-1491 | BB-1493 |
| 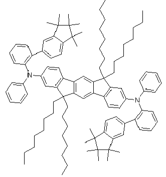 BB522+BB-016 | 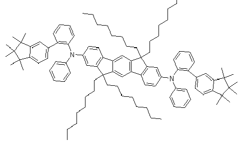 BB522+BB-018 | 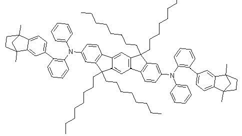 BB522+BB-011 |
| BB-1494 | BB-1495 | BB-1496 |
| 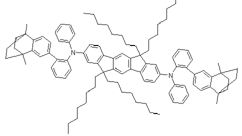 BB522+BB-014 | 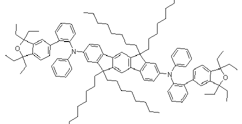 BB522+BB-021 | 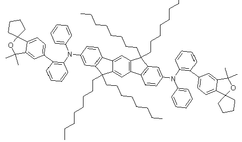 BB522+BB-015 |
| BB-1497 | BB-1498 | BB-1499 |
| 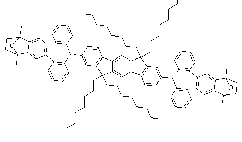 BB522+BB-007 | 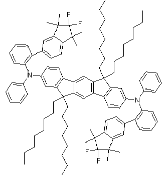 BB522+BB-015 | 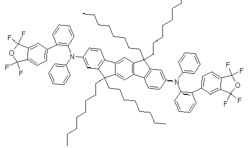 BB522+BB-001 |
| BB-1500 | BB-1501 | BB-1502 |
| 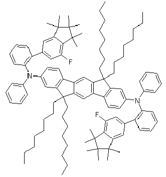 BB522+BB-019 | 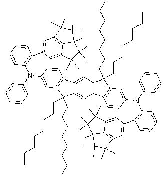 BB522+BB-022 | 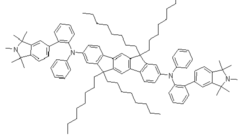 BB522+BB-013 |
| BB-1503 | BB-1504 | BB-1505 |

### 1-3) Buchwald-Reaktion der Tetralin-analogen Bausteine mit Amin-Bausteinen zu Kupplungsprodukten

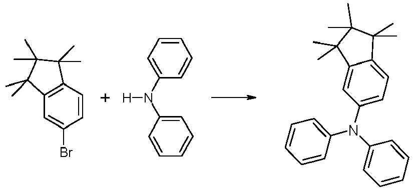

In einen 2Liter-Vierhalskolben mit KPG-Rührer, Rückflusskühler und Argonanschluss werden 81 g (288mmol) BB-051, 53,61g (317mmol, 1,1eq) BB-750, 41,5g (432mmol), 1,5 eq) Natrium-*tert*.-butylat, 2,36g (5,76mmol, 0,02eq) 2-Dicyclohexylphosphino-2',6'-methoxybiphenyl (SPhos), 647mg (2,88mmol, 0,01eq) Palladium(II)-acetat eingewogen und mit 600ml Toluol, 500ml Ethanol und 350ml Wasser versetzt. Die Reaktionsmischung wird für 48 Stunden unter Rückfluss gekocht, abkühlen gelassen, mit 500ml Wasser und 500ml Toluol verdünnt und die Phasen getrennt. Die organische Phase wird über neutralem Aluminiumoxid filtriert und das Lösemittel wird im Vakuum entfernt. Der Rückstand wird mit 500ml Ethanol versetzt und bei 50°C über Nacht gerührt. Der Feststoff wird abgesaugt und im Vakuum getrocknet. Es werden 65,4g (177mmol, 61 % Ausbeute) eines farblosen Feststoffes BB-2013 erhalten.

Folgende Strukturen können mit gleicher Vorschrift und ähnlichen Ausbeuten erhalten werden:

| | | |
|---|---|---|
| 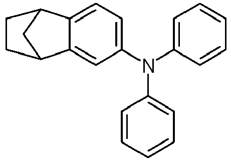 BB-058+BB-750 | | 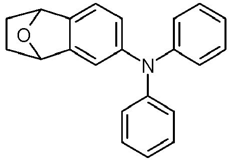 BB-055+BB-750 |
| BB-2001 | | BB-2002 |
| 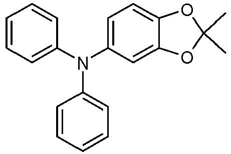 BB-062+BB-750 | 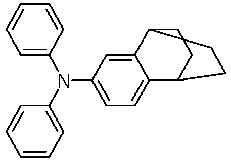 BB-058+BB-750 | 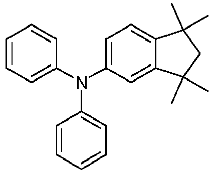 BB-050+BB-750 |
| BB-2003 | BB-2004 | BB-2005 |
| 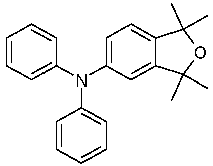 BB-056+BB-750 | 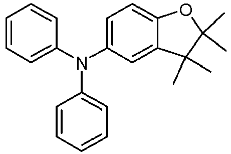 BB-065+BB-750 | 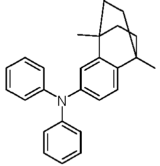 BB-059+BB-750 |
| BB-2006 | BB-2007 | BB-2008 |
| 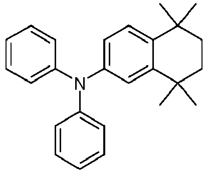 BB-057+BB-750 | 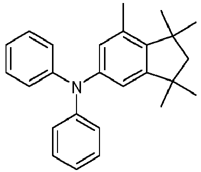 BB-063+BB-750 | 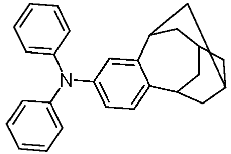 BB-060+BB-750 |
| BB-2009 | BB-2010 | BB-2011 |
| 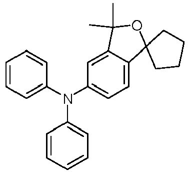 BB-066+BB-750 | 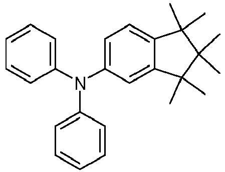 BB-051+BB-750 | 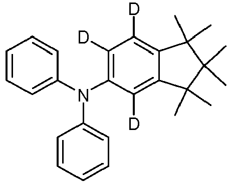 BB-052+BB-750 |
| BB-2012 | BB-2013 | BB-2014 |
| 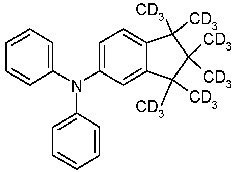 BB-064+BB-750 | 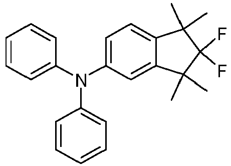 BB-053+BB-750 | 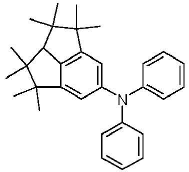 BB-067+BB-750 |
| BB-2015 | BB-2016 | BB-2017 |
| 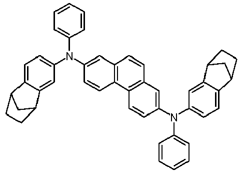 BB-058+BB-751 | | 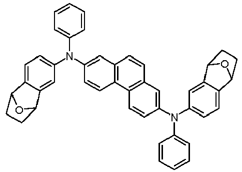 BB-055+BB-751 |
| BB-2019 | | BB-2020 |
| 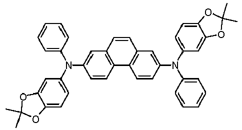 BB-062+BB-751 | 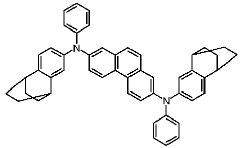 BB-058+BB-751 | 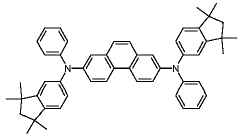 BB-050+BB-751 |
| BB-2021 | BB-2022 | BB-2023 |
| 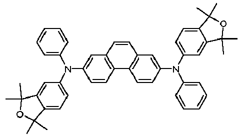 BB-056+BB-751 | 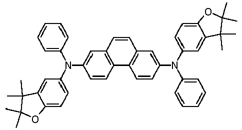 BB-065+BB-751 | 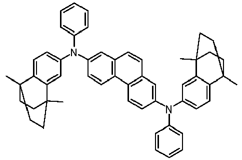 BB-059+BB-751 |
| BB-2024 | BB-2025 | BB-2026 |
| 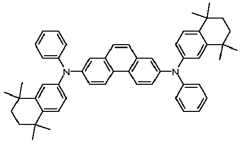 BB-057+BB-751 | 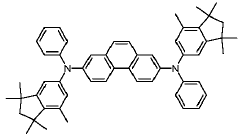 BB-063+BB-751 | 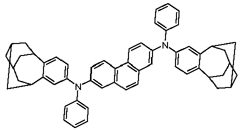 BB-060+BB-751 |
| BB-2027 | BB-2028 | BB-2029 |
| 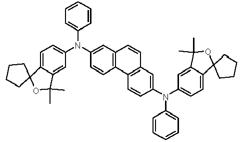 BB-066+BB-751 | 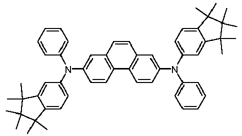 BB-051+BB-751 | 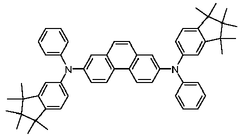 BB-052+BB-751 |
| BB-2030 | BB-2031 | BB-2032 |
| 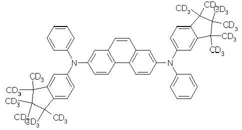 BB-064+BB-751 | 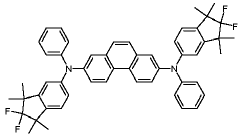 BB-053+BB-751 | |
| BB-2033 | BB-2034 | |
| 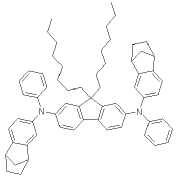 BB-058+BB-752 | 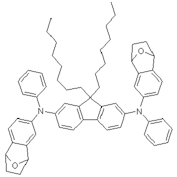 BB-055+BB-752 | 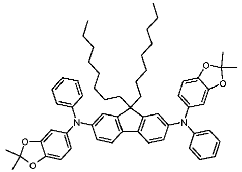 BB-062+BB-752 |
| BB-2036 | BB-2037 | BB-2038 |
| 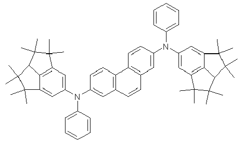 BB-067+BB-751 | 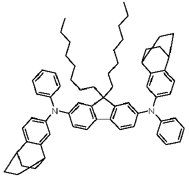 BB-058+BB-752 | 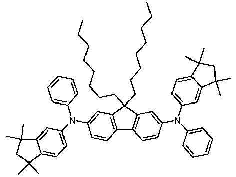 BB-050+BB-752 |
| BB-2039 | BB-2040 | BB-2041 |
| 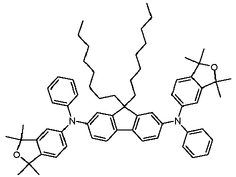 BB-056+BB-752 | 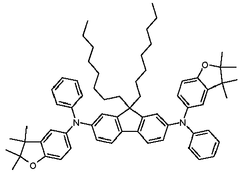 BB-065+BB-752 | 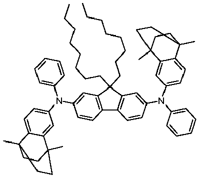 BB-059+BB-752 |
| BB-2042 | BB-2043 | BB-2044 |
| 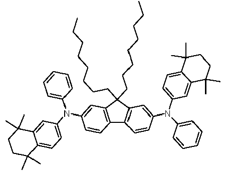 BB-057+BB-752 | 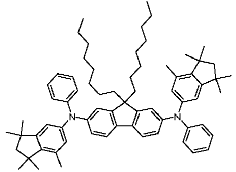 BB-063+BB-752 | 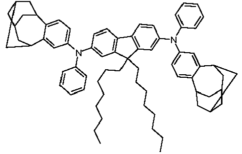 BB-060+BB-752 |
| BB-2045 | BB-2046 | BB-2047 |
| 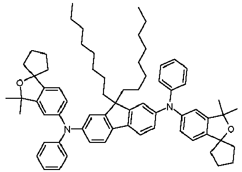 BB-066+BB-752 | 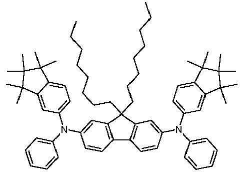 BB-051+BB-752 | 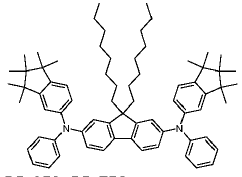 BB-052+BB-752 |
| BB-2048 | | BB-2049 |
| 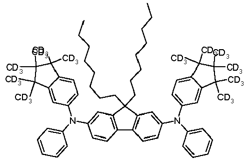 BB-064+BB-752 | 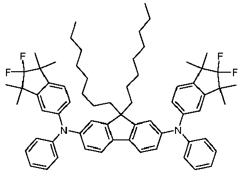 BB-053+BB-752 | 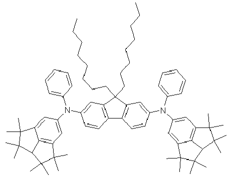 BB-067+BB-752 |
| BB-2050 | BB-2051 | BB-2052 |

### 1-4) Bromierung der Kupplungsprodukte zu den Monomerverbindungen

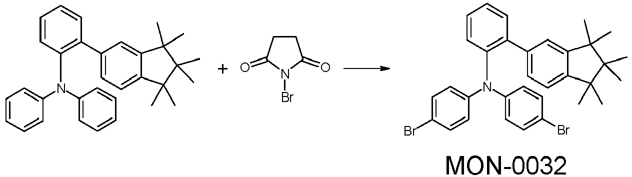

In einen 4Liter-Vierhalskolben mit Rückflusskühler, Argon-Anschluss, KPG-Rührer und Heizbad werden 130,4g (292,6mmol) BB-1031 in 2500ml Dichlormethan gelöst, 0,5ml Eisessig zugegeben und 104,2g (585,2mmol, 2eq) N-Bromsuccinimid (CAS: 128-08-5) portionsweise zugegeben. Die Reaktionsmischung wird für 24h unter Lichtausschluss bei Raumtemperatur gerührt, mit Wasser extrahiert und das Lösemittel im Vakuum entfernt. Der Rückstand wird in 1500ml Ethanol aufgekocht, der Feststoff abgesaugt und mehrfach aus Methylethylketon und Heptan umkristallisiert. Es werden 115,9g (192mmol, 65,6% Ausbeute) des erfindungsgemäßen Monomers MON-0032 als farbloser Feststoff erhalten.

Folgende erfindungsgemäßen Monomere können mit gleicher Vorschrift und ähnlichen Ausbeuten erhalten werden:

| | | |
|---|---|---|
| 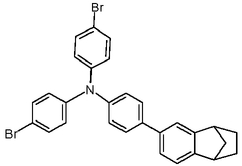 BB-1000 | 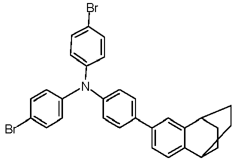 BB-1001 | 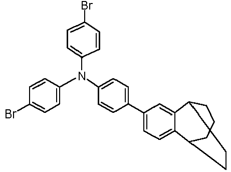 BB-1002 |
| Mon-0001 | Mon-0002 | Mon-0003 |
| 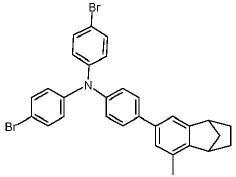 BB-1003 | 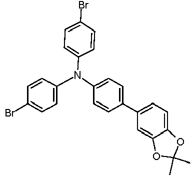 BB-1004 | 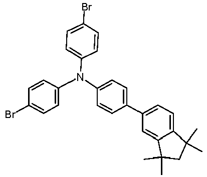 BB-1005 |
| Mon-0004 | Mon-0005 | Mon-0006 |
| 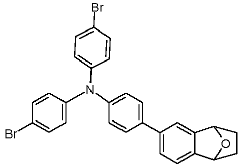 BB-1006 | 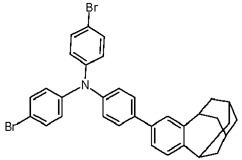 BB-1007 | 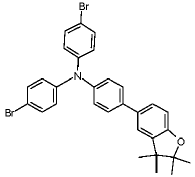 BB-1008 |
| Mon-0007 | Mon-0008 | Mon-0009 |
| 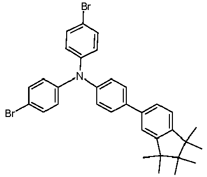 BB-1009 | 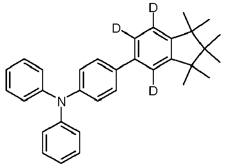 BB-1010 | 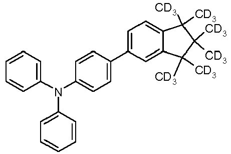 BB-1011 |
| Mon-0010 | Mon-0011 | Mon-0012 |
| 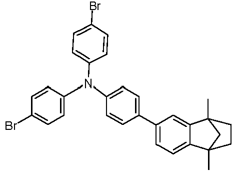 BB-1012 | 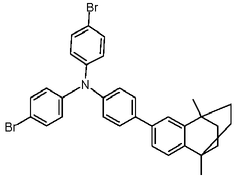 BB-1013 | 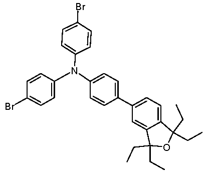 BB-1014 |
| Mon-0013 | Mon-0014 | Mon-0015 |
| 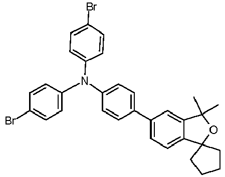 BB-1015 | 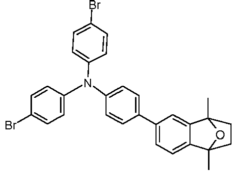 BB-1016 | 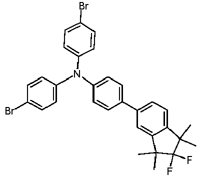 BB-1017 |
| Mon-0016 | Mon-0017 | Mon-0018 |
| 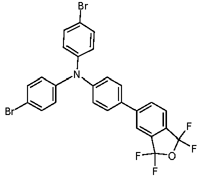 BB-1018 | 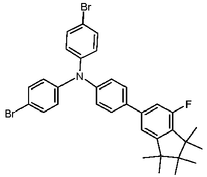 BB-1019 | 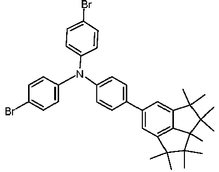 BB-1020 |
| Mon-0019 | Mon-0020 | Mon-0021 |
| 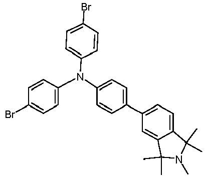 BB-1021 | 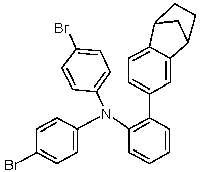 BB-1022 | 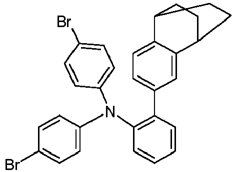 BB-1023 |
| Mon-0022 | Mon-0023 | Mon-0024 |
| 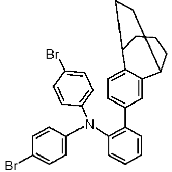 BB-1024 | 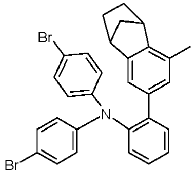 BB-1025 | 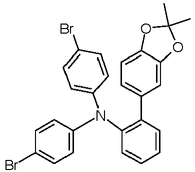 BB-1026 |
| Mon-0025 | Mon-0026 | Mon-0027 |
| 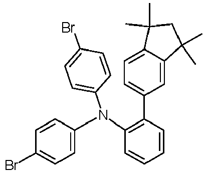 BB-1027 | 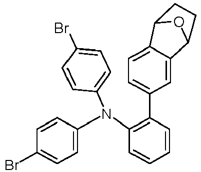 BB-1028 | 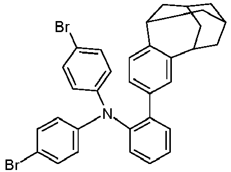 BB-1029 |
| Mon-0028 | Mon-0029 | Mon-0030 |
| 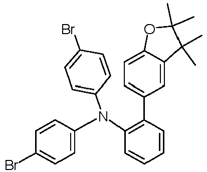 BB-1030 | 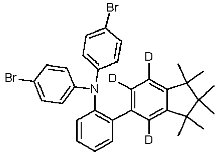 BB-1032 | |
| Mon-0031 | Mon-0033 | |
| 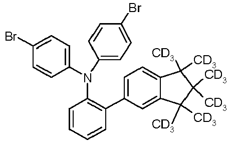 BB-1033 | 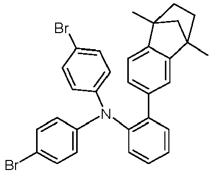 BB-1034 | 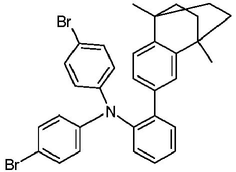 BB-1035 |
| Mon-0034 | Mon-0035 | Mon-0036 |
| 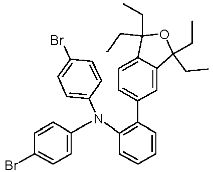 BB-1036 | 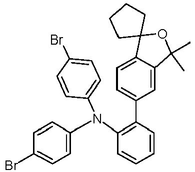 BB-1037 | 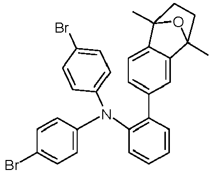 BB-1038 |
| Mon-0037 | Mon-0038 | Mon-0039 |
| 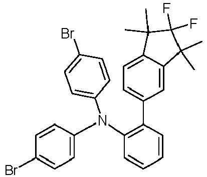 BB-1039 | 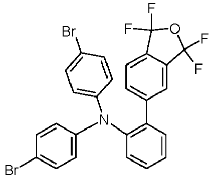 BB-1040 | 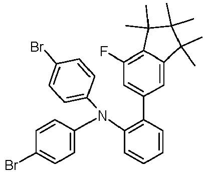 BB-1041 |
| Mon-0040 | Mon-0041 | Mon-0042 |
| 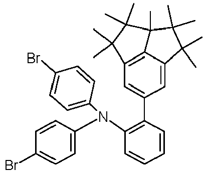 BB-1042 | 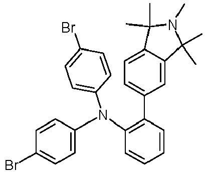 BB-1043 | 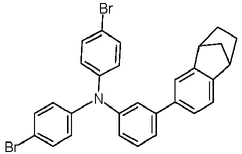 BB-1044 |
| Mon-0043 | Mon-0044 | Mon-0045 |
| 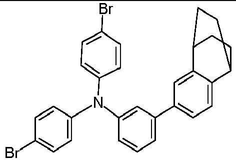 BB-1045 | 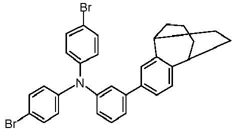 BB-1046 | 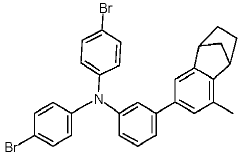 BB-1047 |
| Mon-0046 | Mon-0047 | Mon-0048 |
| 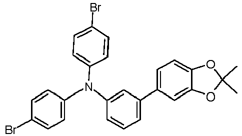 BB-1048 | 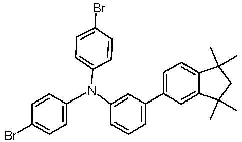 BB-1049 | 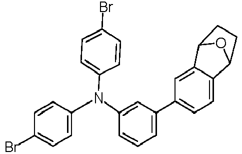 BB-1050 |
| Mon-0049 | Mon-0050 | Mon-0051 |
| 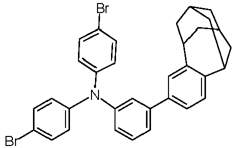 BB-1051 | 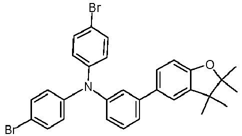 BB-1052 | 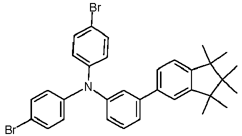 BB-1053 |
| Mon-0052 | Mon-0053 | Mon-0054 |
| 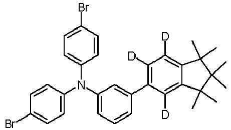 BB-1054 | 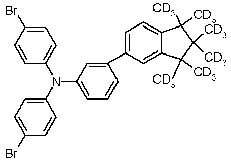 BB-1055 | 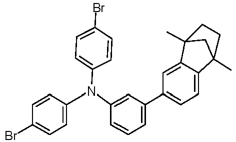 BB-1056 |
| Mon-0055 | Mon-0056 | Mon-0057 |
| 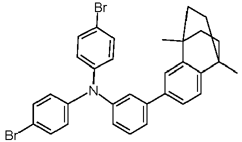 BB-1057 | 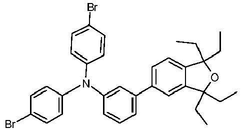 BB-1058 | 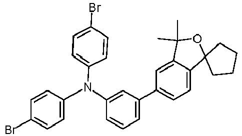 BB-1059 |
| Mon-0058 | Mon-0059 | Mon-0069 |
| 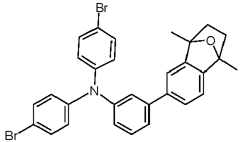 BB-1060 | 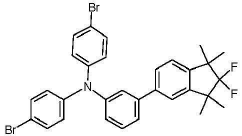 BB-1061 | 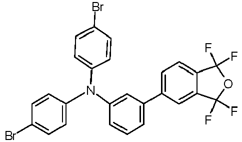 BB-1062 |
| Mon-0061 | Mon-0062 | Mon-0063 |
| 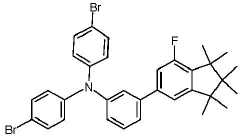 BB-1063 | 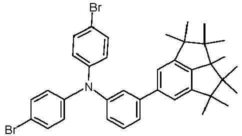 BB-1064 | 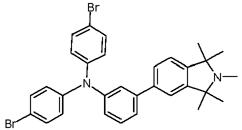 BB-1065 |
| Mon-0064 | Mon-0065 | Mon-0066 |
| 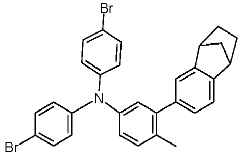 BB-1066 | 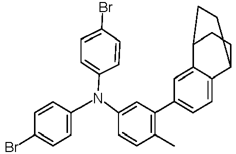 BB-1067 | 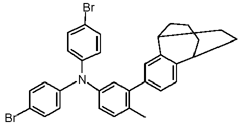 BB-1068 |
| Mon-0067 | Mon-0068 | Mon-0069 |
| 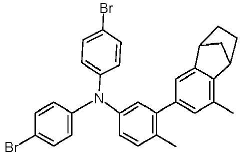 BB-1069 | 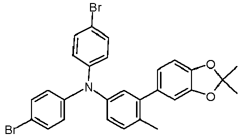 BB-1070 | 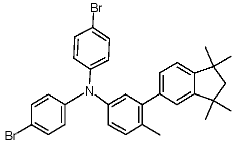 BB-1071 |
| Mon-0070 | Mon-0071 | Mon-0072 |
| 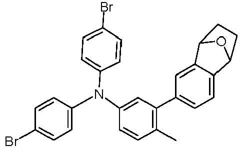 BB-1072 | 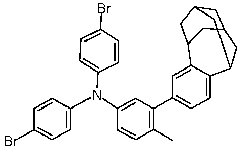 BB-1073 | 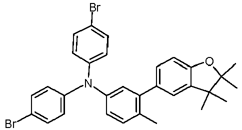 BB-1074 |
| Mon-0073 | Mon-0074 | Mon-0075 |
| 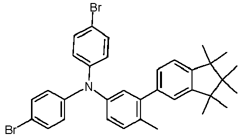 BB-1075 | 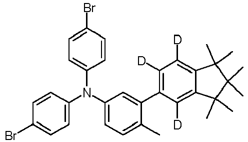 BB-1076 | 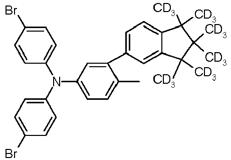 BB-1077 |
| Mon-0076 | Mon-0077 | Mon-0078 |
| 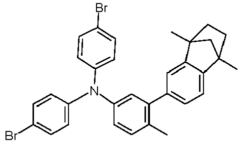 BB-1078 | 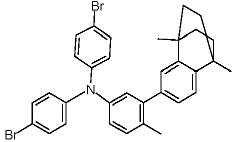 BB-1079 | 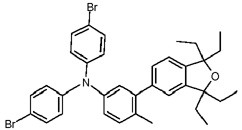 BB-1080 |
| Mon-0079 | Mon-0080 | Mon-0081 |
| 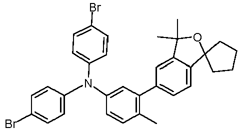 BB-1081 | 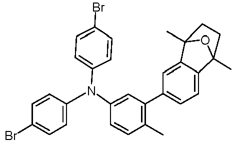 BB-1082 | 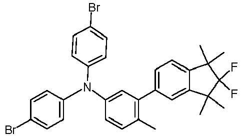 BB-1083 |
| Mon-0082 | Mon-0083 | Mon-0084 |
| 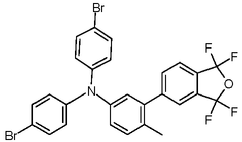 BB-1084 | 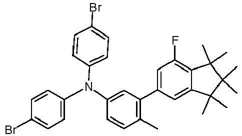 BB-1085 | 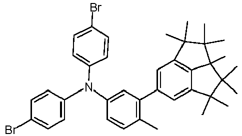 BB-1086 |
| Mon-0085 | Mon-0086 | Mon-0087 |
| 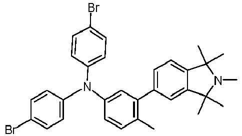 BB-1087 | 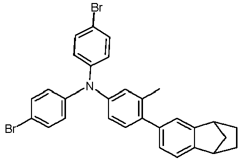 BB-1088 | 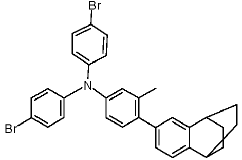 BB-1089 |
| Mon-0088 | Mon-0089 | Mon-0090 |
| 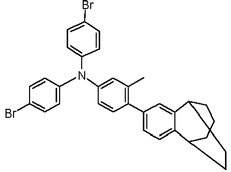 BB-1090 | 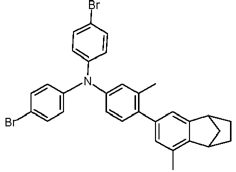 BB-1091 | 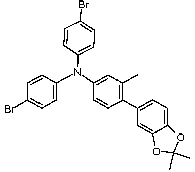 BB-1092 |
| Mon-0091 | Mon-0092 | Mon-0093 |
| 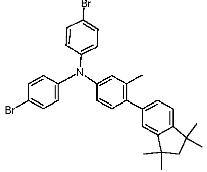 BB-1093 | 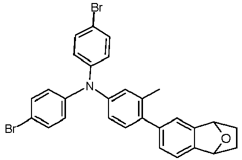 BB-1094 | 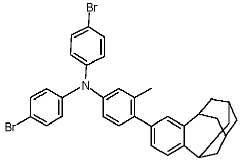 BB-1095 |
| Mon-0094 | Mon-0095 | Mon-0096 |
| 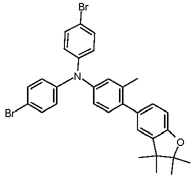 BB-1096 | 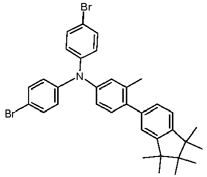 BB-1097 | 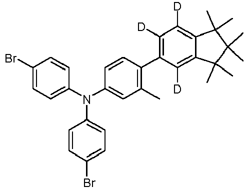 BB-1098 |
| Mon-0097 | Mon-0098 | Mon-0099 |
| 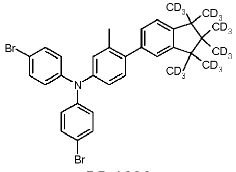 BB-1099 | 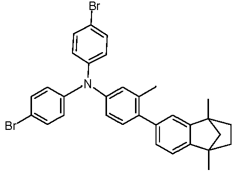 BB-1100 | 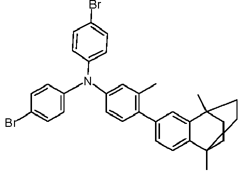 BB-1101 |
| Mon-0100 | Mon-0101 | Mon-0102 |
| 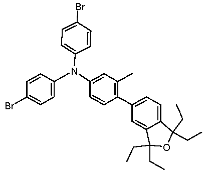 BB-1102 | 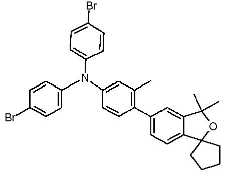 BB-1103 | 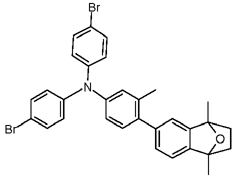 BB-1104 |
| Mon-0103 | Mon-0104 | Mon-0105 |
| 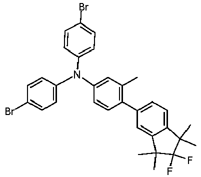 BB-1105 | 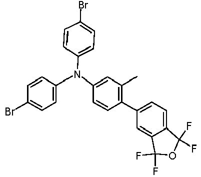 BB-1106 | 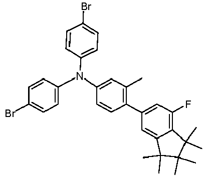 BB-1107 |
| Mon-0106 | Mon-0107 | Mon-0108 |
| 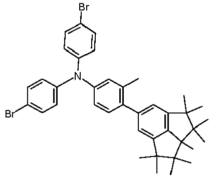 BB-1108 | 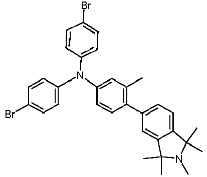 BB-1109 | 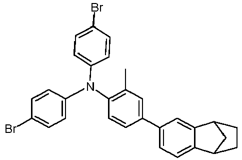 BB-1110 |
| Mon-0109 | Mon-0110 | Mon-0111 |
| 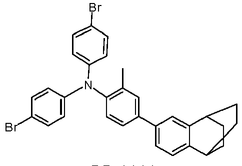 BB-1111 | 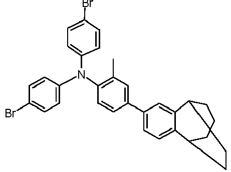 BB-1112 | 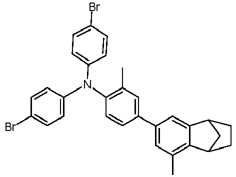 BB-1113 |
| Mon-0112 | Mon-0113 | Mon-0114 |
| 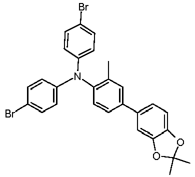 BB-1114 | 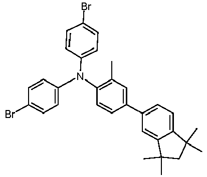 BB-1115 | 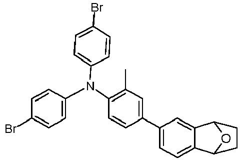 BB-1116 |
| Mon-0115 | Mon-0116 | Mon-0117 |
| 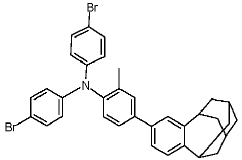 BB-1117 | 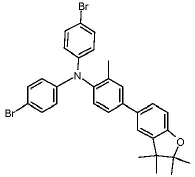 BB-1118 | 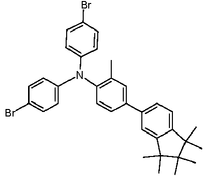 BB-1119 |
| Mon-0118 | Mon-0119 | Mon-0120 |
| 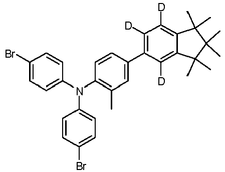 BB-1120 | 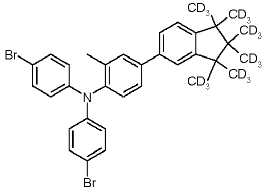 BB-1121 | 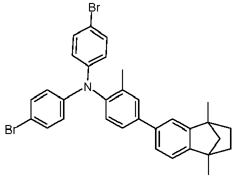 BB-1122 |
| Mon-0121 | Mon-0122 | Mon-0123 |
| 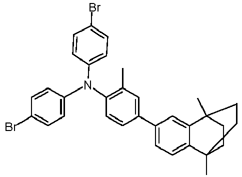 BB-1123 | 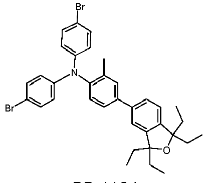 BB-1124 | 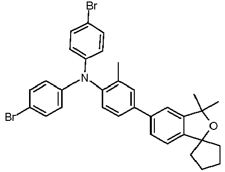 BB-1125 |
| Mon-0124 | Mon-0125 | Mon-0126 |
| 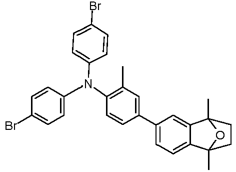 BB-1126 | 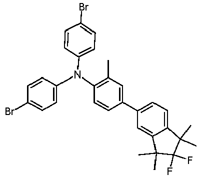 BB-1127 | 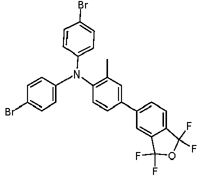 BB-1128 |
| Mon-0127 | Mon-0128 | Mon-0129 |
| 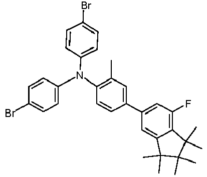 BB-1129 | 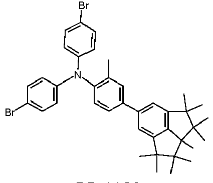 BB-1130 | 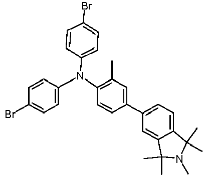 BB-1131 |
| Mon-0130 | Mon-0131 | Mon-0132 |
| 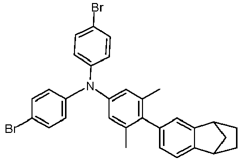 BB-1132 | 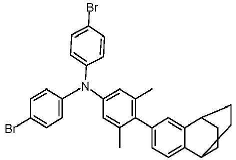 BB-1133 | 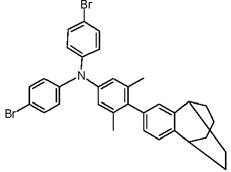 BB-1134 |
| Mon-0133 | Mon-0134 | Mon-0135 |
| 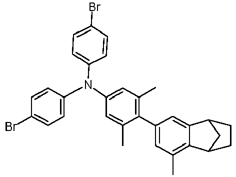 BB-1135 | 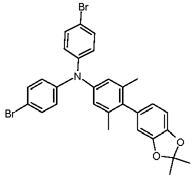 BB-1136 | 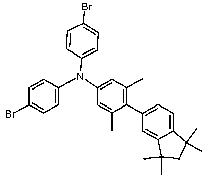 BB-1137 |
| Mon-0136 | Mon-0137 | Mon-0138 |
| 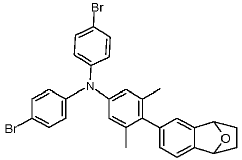 BB-1138 | 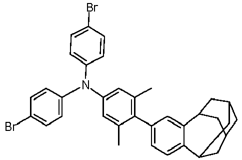 BB-1139 | 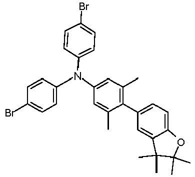 BB-1140 |
| Mon-0139 | Mon-0140 | Mon-0141 |
| 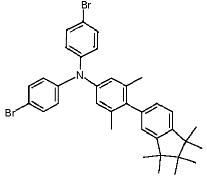 BB-1141 | 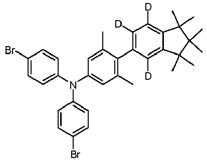 BB-1142 | 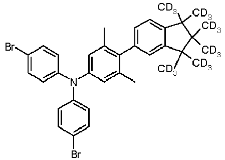 BB-1143 |
| Mon-0142 | Mon-0143 | Mon-0144 |
| 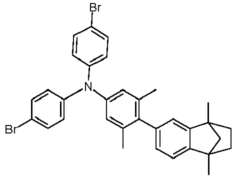 BB-1144 | 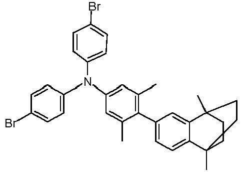 BB-1145 | 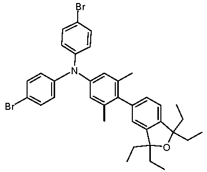 BB-1146 |
| Mon-0145 | Mon-0146 | Mon-0147 |
| 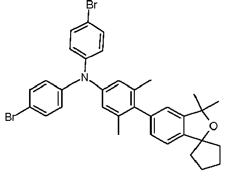 BB-1147 | 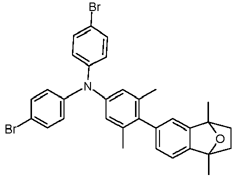 BB-1148 | 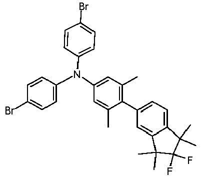 BB-1149 |
| Mon-0148 | Mon-0149 | Mon-0150 |
| 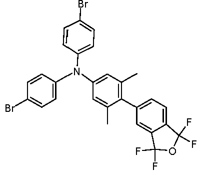 BB-1150 | 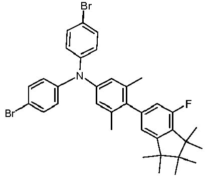 BB-1151 | 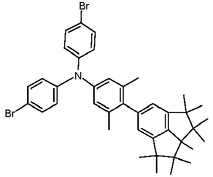 BB-1152 |
| Mon-0151 | Mon-0152 | Mon-0153 |
| 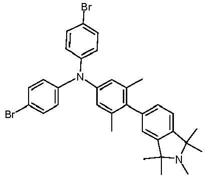 BB-1153 | 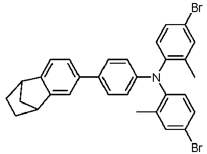 BB-1154 | 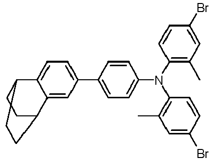 BB-1155 |
| Mon-0154 | Mon-0155 | Mon-0156 |
| 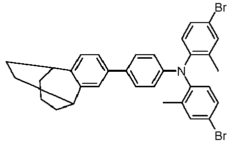 BB-1156 | 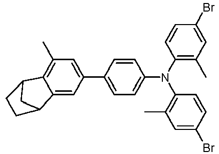 BB-1157 | 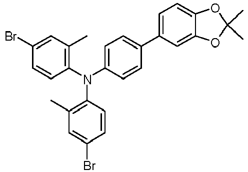 BB-1158 |
| Mon-0157 | Mon-0158 | Mon-0159 |
| 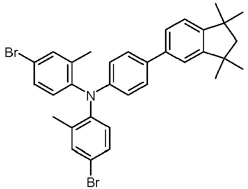 BB-1159 | 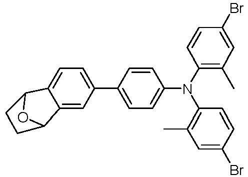 BB-1160 | 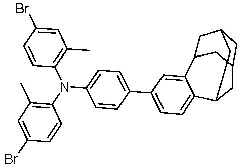 BB-1161 |
| Mon-0160 | Mon-0161 | Mon-0162 |
| 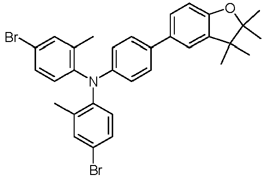 BB-1162 | 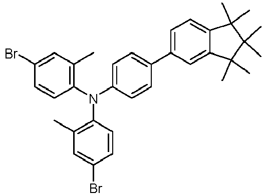 BB-1163 | 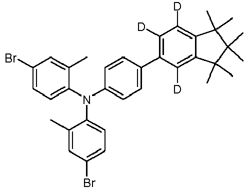 BB-1164 |
| Mon-0163 | Mon-0164 | Mon-0165 |
| 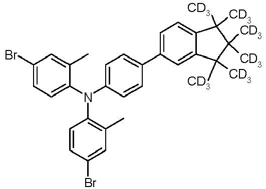 BB-1165 | 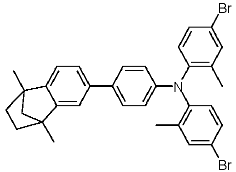 BB-1166 | 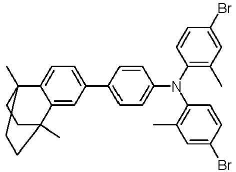 BB-1167 |
| Mon-0166 | Mon-0167 | Mon-0168 |
| 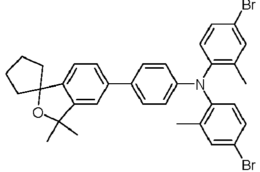 BB-1168 | 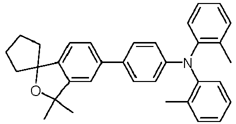 BB-1169 | 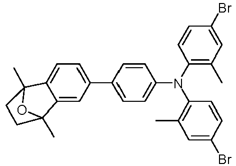 BB-1170 |
| Mon-0169 | Mon-0170 | Mon-0171 |
| 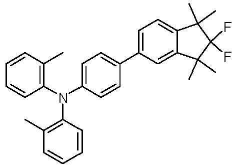 BB-1171 | 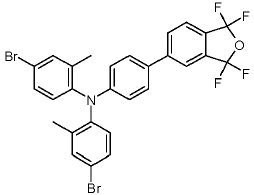 BB-1172 | 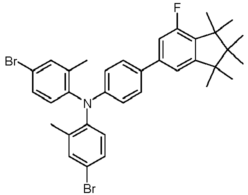 BB-1173 |
| Mon-0172 | Mon-0173 | Mon-0174 |
| 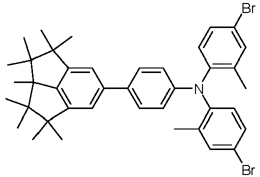 BB-1174 | 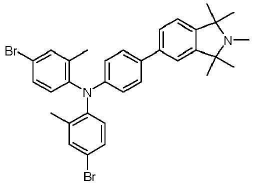 BB-1175 | 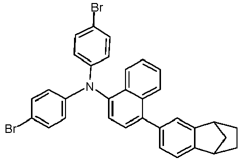 BB-1176 |
| Mon-0175 | Mon-0176 | Mon-0177 |
| 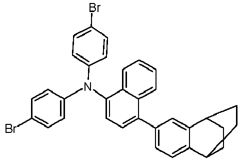 BB-1177 | 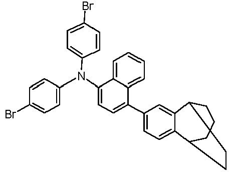 BB-1178 | 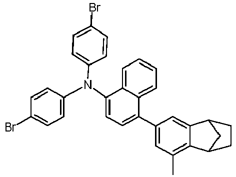 BB-1179 |
| Mon-0178 | Mon-0179 | Mon-0180 |
| 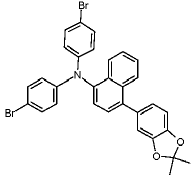 BB-1180 | 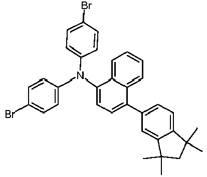 BB-1181 | 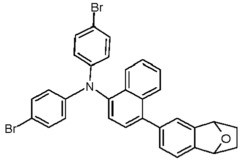 BB-1182 |
| Mon-0181 | Mon-0182 | Mon-0183 |
| 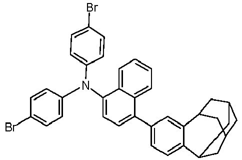 BB-1183 | 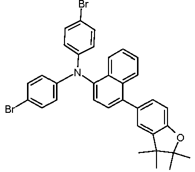 BB-1184 | 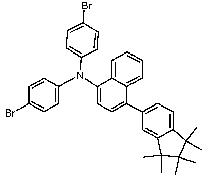 BB-1185 |
| Mon-0184 | Mon-0185 | Mon-0186 |
| 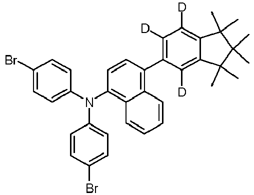 BB-1186 | 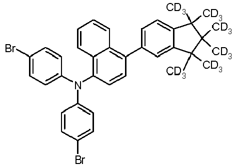 BB-1187 | 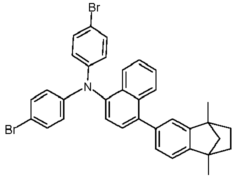 BB-1188 |
| Mon-0187 | Mon-0188 | Mon-0189 |
| 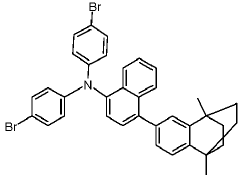 BB-1189 | 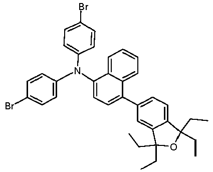 BB-1190 | 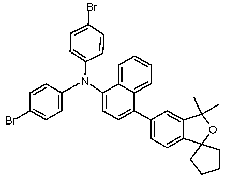 BB-1191 |
| Mon-0190 | Mon-0191 | Mon-0192 |
| 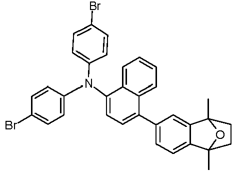 BB-1192 | 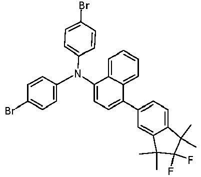 BB-1193 | 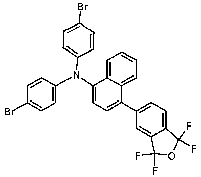 BB-1194 |
| Mon-0193 | Mon-0194 | Mon-0195 |
| 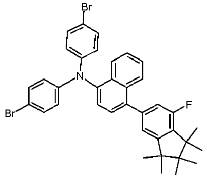 BB-1195 | 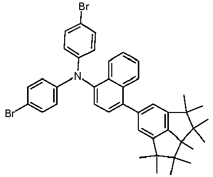 BB-1196 | 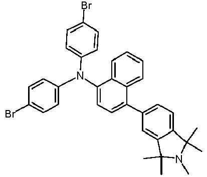 BB-1197 |
| Mon-0196 | Mon-0197 | Mon-0198 |
| 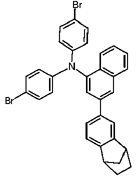 BB-1198 | 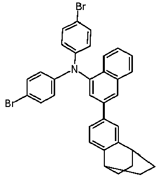 BB-1199 | 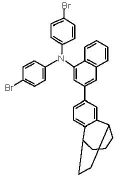 BB-1200 |
| Mon-0199 | Mon-0200 | Mon-0201 |
| 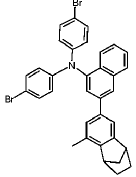 BB-1201 | 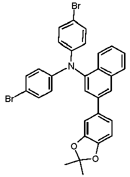 BB-1202 | 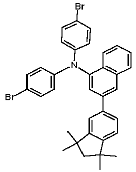 BB-1203 |
| Mon-0202 | Mon-0203 | Mon-0204 |
| 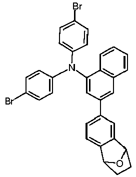 BB-1204 | 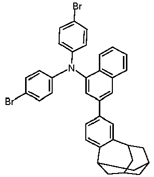 BB-1205 | 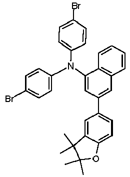 BB-1206 |
| Mon-0205 | Mon-0206 | Mon-0207 |
| 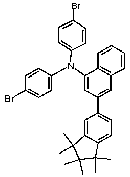 BB-1207 | 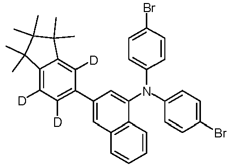 BB-1208 | 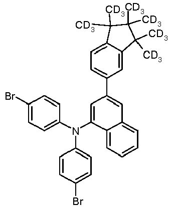 BB-1209 |
| Mon-0208 | Mon-0209 | Mon-0210 |
| 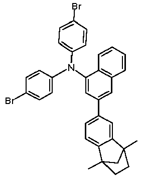 BB-1210 | 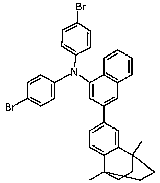 BB-1211 | 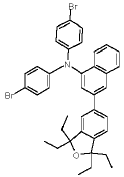 BB-1212 |
| Mon-0211 | Mon-0212 | Mon-0213 |
| 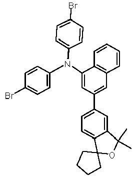 BB-1213 | 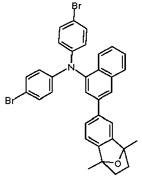 BB-1214 | 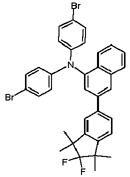 BB-1215 |
| Mon-0214 | Mon-0215 | Mon-0216 |
| 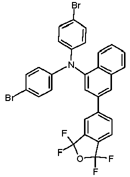 BB-1216 | 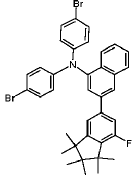 BB-1217 | 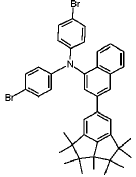 BB-1218 |
| Mon-0217 | Mon-0218 | Mon-0219 |
| 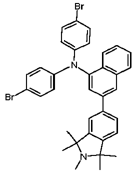 BB-1219 | 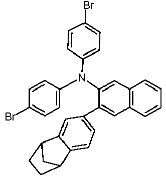 BB-1220 | 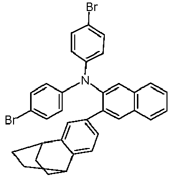 BB-1221 |
| Mon-0220 | Mon-0221 | Mon-0222 |
| 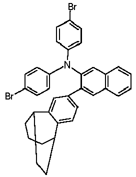 BB-1222 | 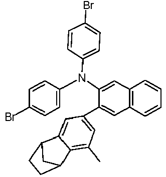 BB-1223 | 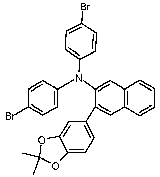 BB-1224 |
| Mon-0223 | Mon-0224 | Mon-0225 |
| 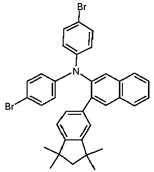 BB-1225 | 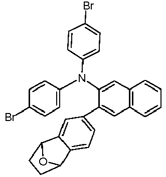 BB-1226 | 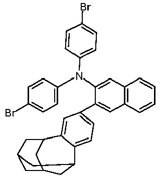 BB-1227 |
| Mon-0226 | Mon-0227 | Mon-0228 |
| 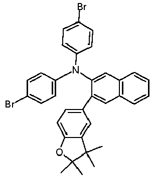 BB-1228 | 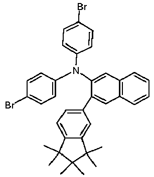 BB-1229 | 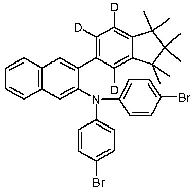 BB-1230 |
| Mon-0229 | Mon-0230 | Mon-0231 |
| 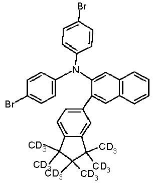 BB-1231 | 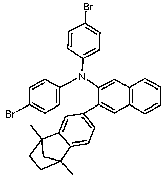 BB-1232 | 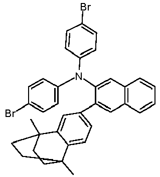 BB-1233 |
| Mon-0232 | Mon-0233 | Mon-0234 |
| 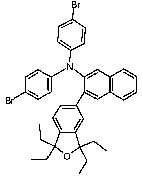 BB-1234 | 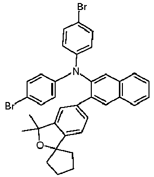 BB-1235 | 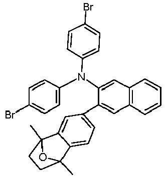 BB-1236 |
| Mon-0235 | Mon-0236 | Mon-0237 |
| 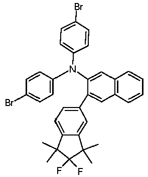 BB-1237 | 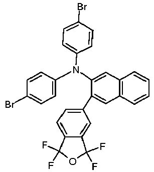 BB-1238 | 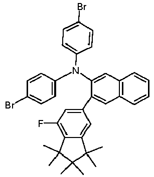 BB-1239 |
| Mon-0238 | Mon-0239 | Mon-0240 |
| 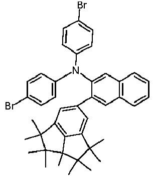 BB-1240 | 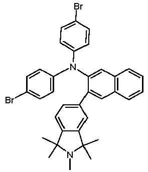 BB-1241 | 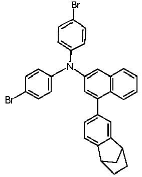 BB-1242 |
| Mon-0241 | Mon-0242 | Mon-0243 |
| 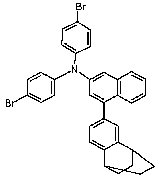 BB-1243 | 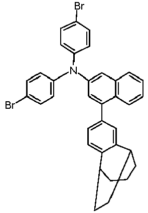 BB-1244 | 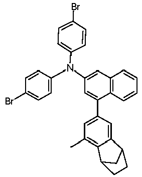 BB-1245 |
| Mon-0244 | Mon-0245 | Mon-0246 |
| 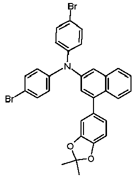 BB-1246 | 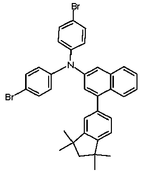 BB-1247 | 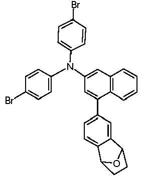 BB-1248 |
| Mon-0247 | Mon-0248 | Mon-0249 |
| 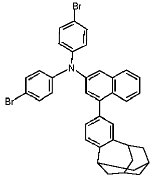 BB-1249 | 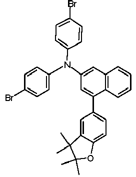 BB-1250 | 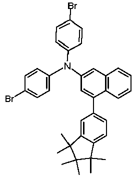 BB-1251 |
| Mon-0250 | Mon-0251 | Mon-0252 |
| 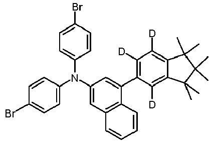 BB-1252 | 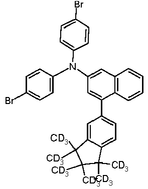 BB-1253 | 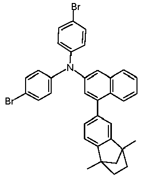 BB-1254 |
| Mon-0253 | Mon-0254 | Mon-0255 |
| 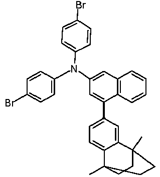 BB-1255 | 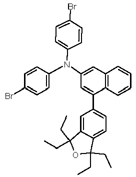 BB-1256 | 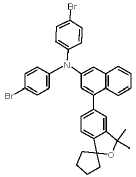 BB-1257 |
| Mon-0256 | Mon-0257 | Mon-0258 |
| 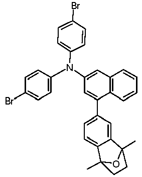 BB-1258 | 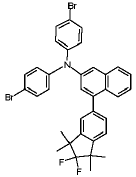 BB-1259 | 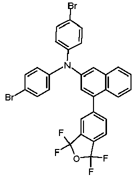 BB-1260 |
| Mon-0259 | Mon-0260 | Mon-0261 |
| 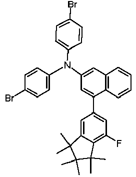 BB-1261 | 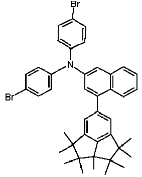 BB-1262 | 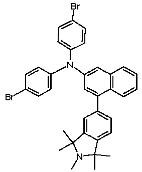 BB-1263 |
| Mon-0262 | Mon-0263 | Mon-0264 |
| 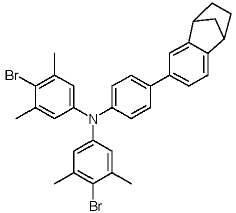 BB-1264 | 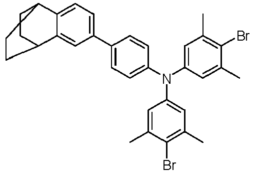 BB-1265 | 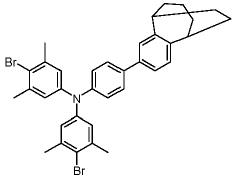 BB-1266 |
| Mon-0265 | Mon-0266 | Mon-0267 |
| 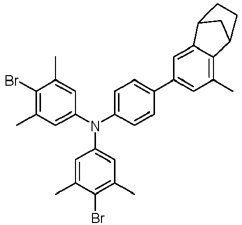 BB-1267 | 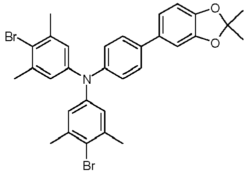 BB-1268 | 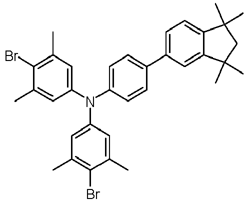 BB-1269 |
| Mon-0268 | Mon-0269 | Mon-0270 |
| 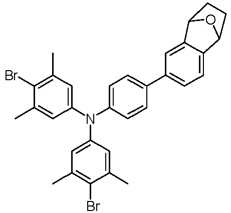 BB-1270 | 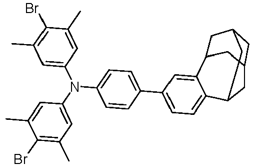 BB-1271 | 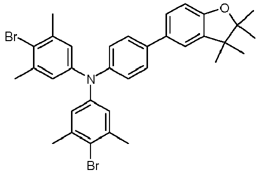 BB-1272 |
| Mon-0271 | Mon-0272 | Mon-0273 |
| 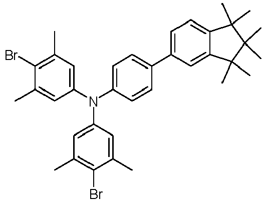 BB-1273 | 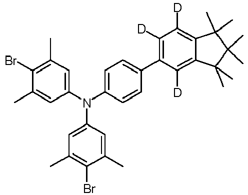 BB-1274 | 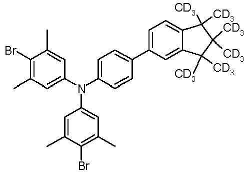 BB-1275 |
| Mon-0274 | Mon-0275 | Mon-0276 |
| 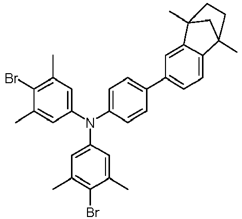 BB-1276 | 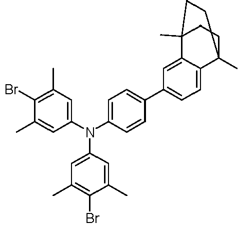 BB-1277 | 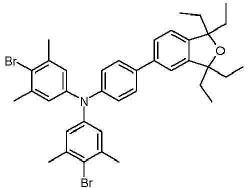 BB-1278 |
| Mon-0277 | Mon-0278 | Mon-0279 |
| 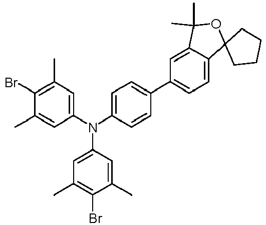 BB-1279 | 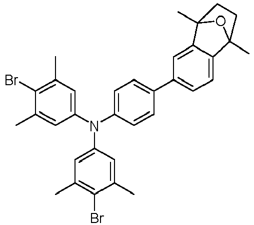 BB-1280 | 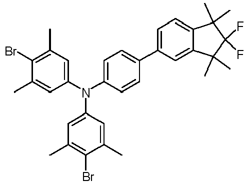 BB-1281 |
| Mon-0280 | Mon-0281 | Mon-0282 |
| 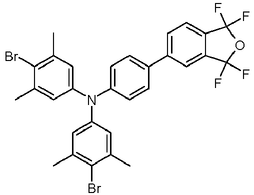 BB-1282 | 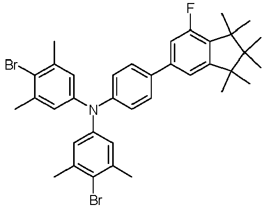 BB-1283 | 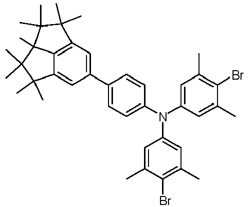 BB-1284 |
| Mon-0283 | Mon-0284 | Mon-0285 |
| 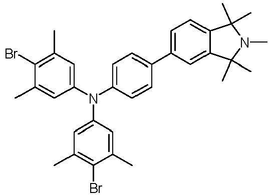 BB-1285 | 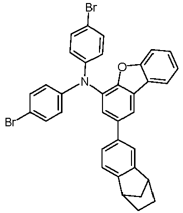 BB-1286 | 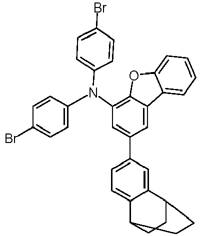 BB-1287 |
| Mon-0286 | Mon-0287 | Mon-0288 |
| 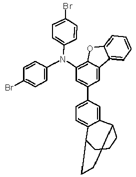 BB-1288 | 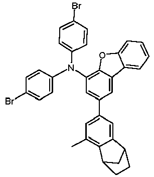 BB-1289 | 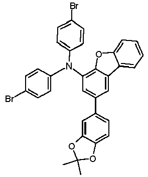 BB-1290 |
| Mon-0289 | Mon-0290 | Mon-0291 |
| 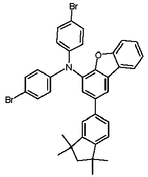 BB-1291 | 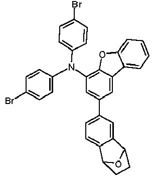 BB-1292 | 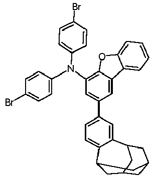 BB-1293 |
| Mon-0292 | Mon-0293 | Mon-0294 |
| 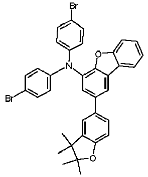 BB-1294 | 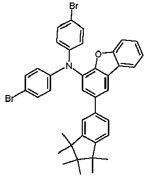 BB-1295 | 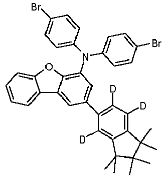 BB-1296 |
| Mon-0295 | Mon-0296 | Mon-0297 |
| 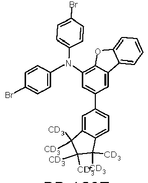 BB-1297 | 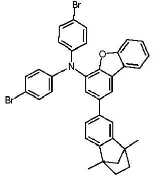 BB-1298 | 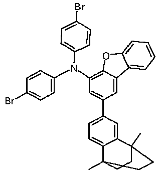 BB-1299 |
| Mon-0298 | Mon-0299 | Mon-0300 |
| 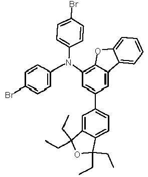 BB-1300 | 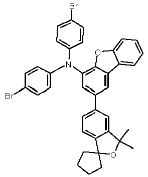 BB-1301 | 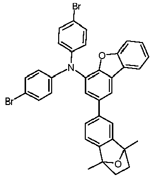 BB-1302 |
| Mon-0301 | Mon-0302 | Mon-0303 |
| 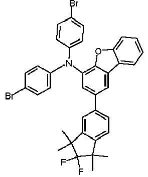 BB-1303 | 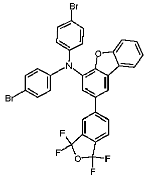 BB-1304 | 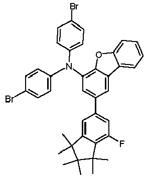 BB-1305 |
| Mon-0304 | Mon-0305 | Mon-0306 |
| 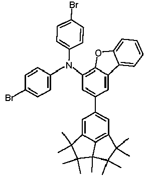 BB-1306 | 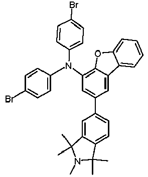 BB-1307 | 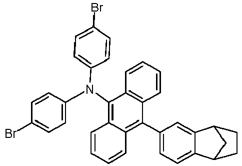 BB-1308 |
| Mon-0307 | Mon-0308 | Mon-0309 |
| 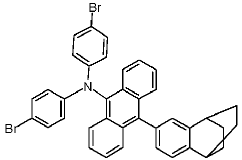 BB-1309 | 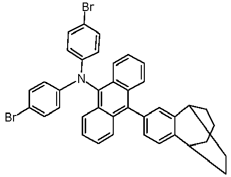 BB-1310 | 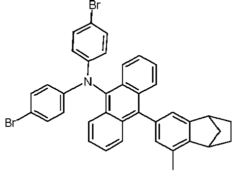 BB-1311 |
| Mon-0310 | Mon-0311 | Mon-0312 |
| 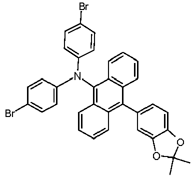 BB-1312 | 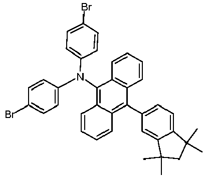 BB-1313 | 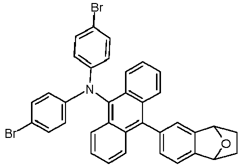 BB-1314 |
| Mon-0313 | Mon-0314 | Mon-0315 |
| 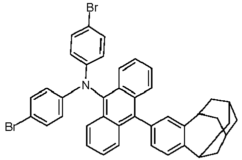 BB-1315 | 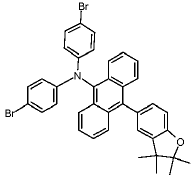 BB-1316 | 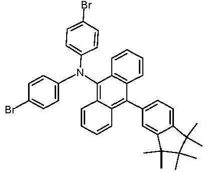 BB-1317 |
| Mon-0316 | Mon-0317 | Mon-0318 |
| 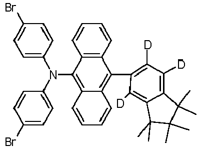 BB-1318 | 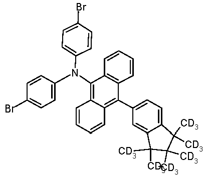 BB-1319 | 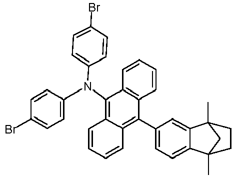 BB-1320 |
| Mon-0319 | Mon-0320 | Mon-0321 |
| 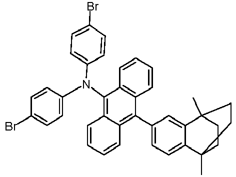 BB-1321 | 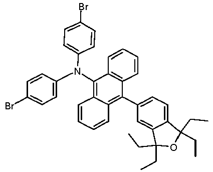 BB-1322 | 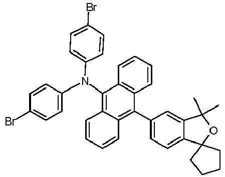 BB-1323 |
| Mon-0322 | Mon-0323 | Mon-0324 |
| 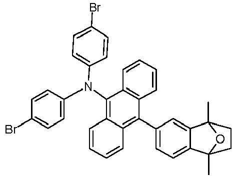 BB-1324 | 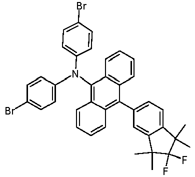 BB-1325 | 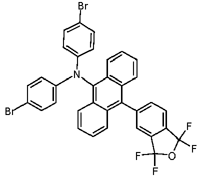 BB-1326 |
| Mon-0325 | Mon-0326 | Mon-0327 |
| 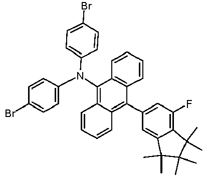 BB-1327 | 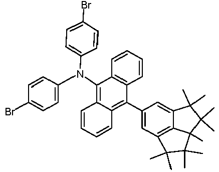 BB-1328 | 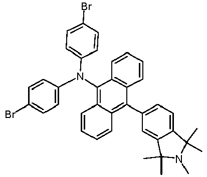 BB-1329 |
| Mon-0328 | Mon-0329 | Mon-0330 |
| 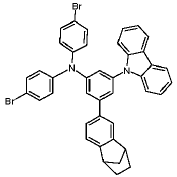 BB-1330 | 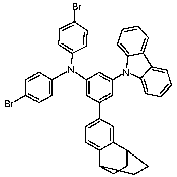 BB-1331 | 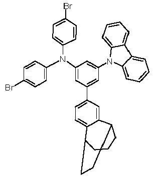 BB-1332 |
| Mon-0331 | Mon-0332 | Mon-0333 |
| 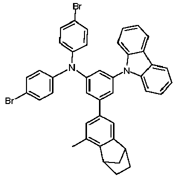 BB-1333 | 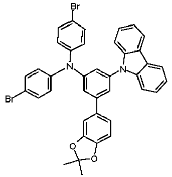 BB-1334 | 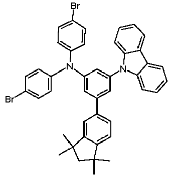 BB-1335 |
| Mon-0334 | Mon-0335 | Mon-0336 |
| 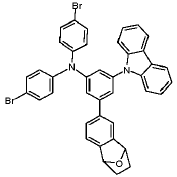 BB-1336 | 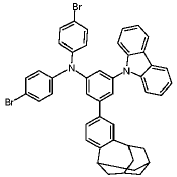 BB-1337 | 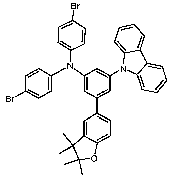 BB-1338 |
| Mon-0337 | Mon-0338 | Mon-0339 |
| 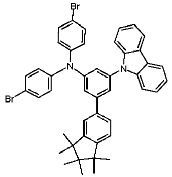 BB-1339 | 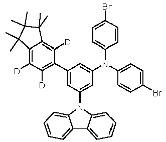 BB-1340 | 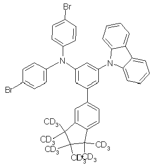 BB-1341 |
| Mon-0340 | Mon-0341 | Mon-0342 |
| 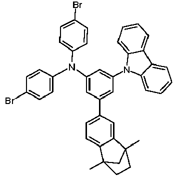 BB-1342 | 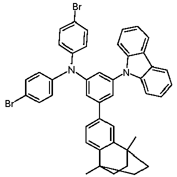 BB-1343 | 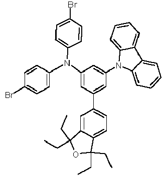 BB-1344 |
| Mon-0343 | Mon-0344 | Mon-0345 |
| 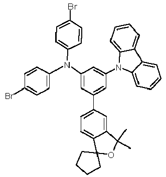 BB-1345 | 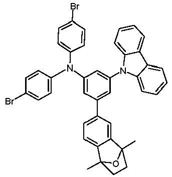 BB-1346 | 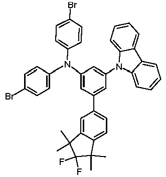 BB-1347 |
| Mon-0346 | Mon-0347 | Mon-0348 |
| 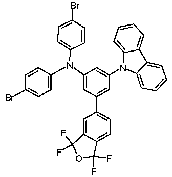 BB-1348 | 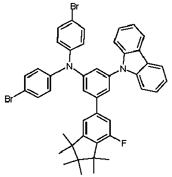 BB-1349 | 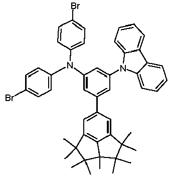 BB-1350 |
| Mon-0349 | Mon-0350 | Mon-0351 |
| 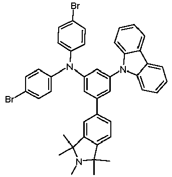 BB-1351 | 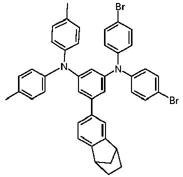 BB-1352 | 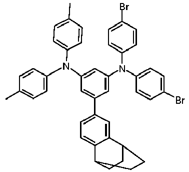 BB-1353 |
| Mon-0352 | Mon-0353 | Mon-0354 |
| 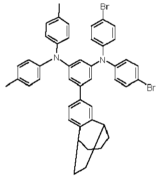 BB-1354 | 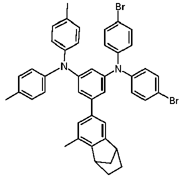 BB-1355 | 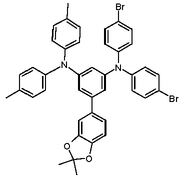 BB-1356 |
| Mon-0355 | Mon-0356 | Mon-0357 |
| 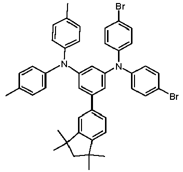 BB-1357 | 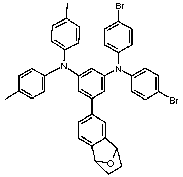 BB-1358 | 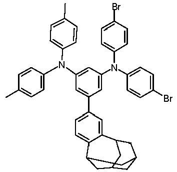 BB-1359 |
| Mon-0358 | Mon-0359 | Mon-0360 |
| 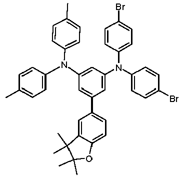 BB-1360 | 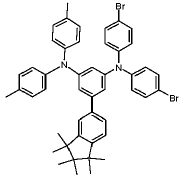 BB-1361 | 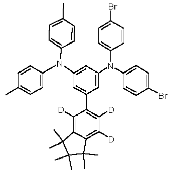 BB-1362 |
| Mon-0361 | Mon-0362 | Mon-0363 |
| 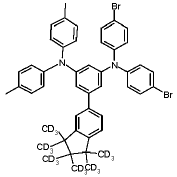 BB-1363 | 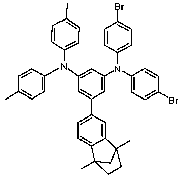 BB-1364 | 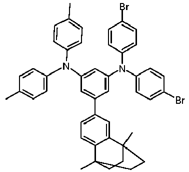 BB-1365 |
| Mon-0364 | Mon-0365 | Mon-0366 |
| 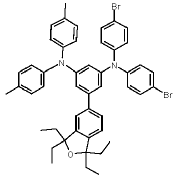 BB-1366 | 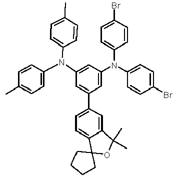 BB-1367 | 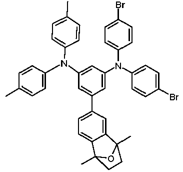 BB-1368 |
| Mon-0367 | Mon-0368 | Mon-0369 |
| 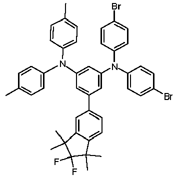 BB-1369 | 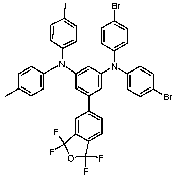 BB-1370 | 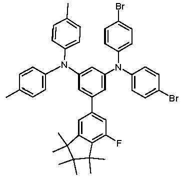 BB-1371 |
| Mon-0370 | Mon-0371 | Mon-0372 |
| 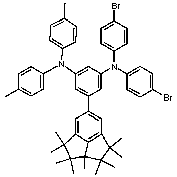 BB-1372 | 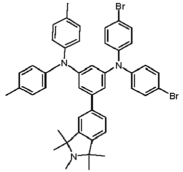 BB-1373 | 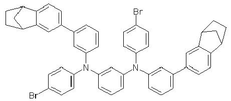 BB-1374 |
| Mon-0373 | Mon-0374 | Mon-0375 |
| 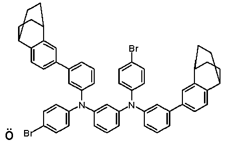 BB-1375 | 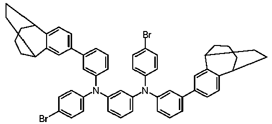 BB-1376 | 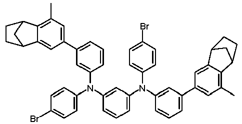 BB-1377 |
| Mon-0376 | Mon-0377 | Mon-0378 |
| 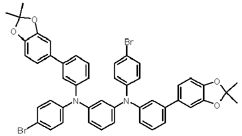 BB-1378 | 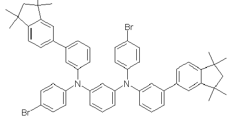 BB-1379 | 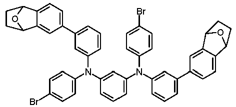 BB-1380 |
| Mon-0379 | Mon-0380 | Mon-0381 |
| 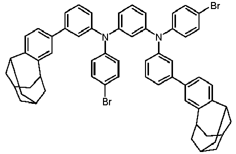 BB-1381 | 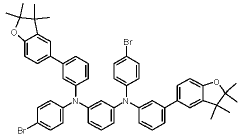 BB-1382 | 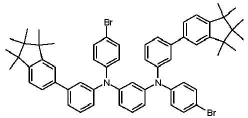 BB-1383 |
| Mon-0382 | Mon-0383 | Mon-0384 |
| 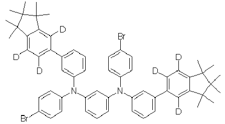 BB-1384 | 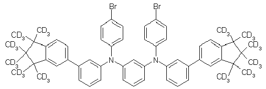 BB-1385 | 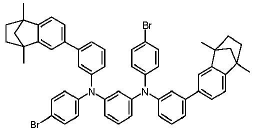 BB-1386 |
| Mon-0385 | Mon-0386 | Mon-0387 |
| 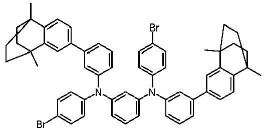 BB-1387 | 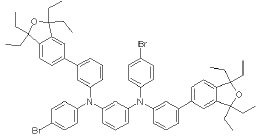 BB-1388 | 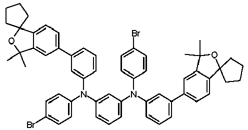 BB-1389 |
| Mon-0388 | Mon-0389 | Mon-0390 |
| 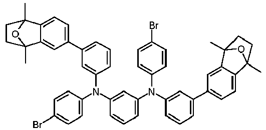 BB-1390 | 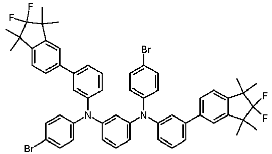 BB-1391 | 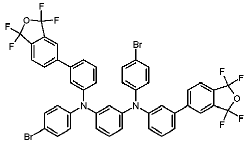 BB-1392 |
| Mon-0391 | Mon-0392 | Mon-0393 |
| 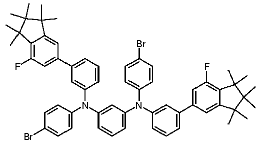 BB-1393 | 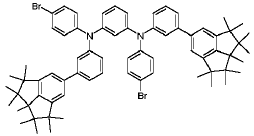 BB-1394 | 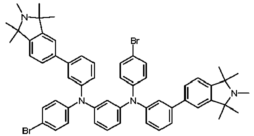 BB-1395 |
| Mon-0394 | Mon-0395 | Mon-0396 |
| 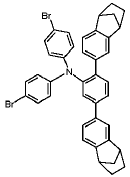 BB-1396 | 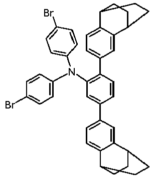 BB-1397 | 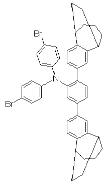 BB-1398 |
| Mon-0397 | Mon-0398 | Mon-0399 |
| 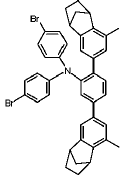 BB-1399 | 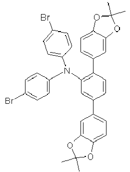 BB-1400 | 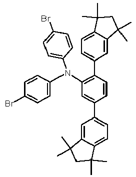 BB-1401 |
| Mon-0400 | Mon-0401 | Mon-0402 |
| 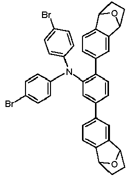 BB-1402 | 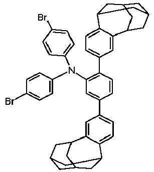 BB-1403 | 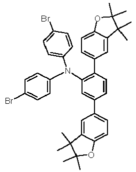 BB-1404 |
| Mon-0403 | Mon-0404 | Mon-0405 |
| 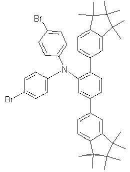 BB-1405 | 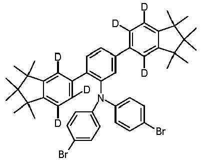 BB-1406 | 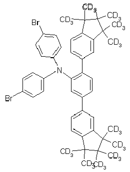 BB-1407 |
| Mon-0406 | Mon-0407 | Mon-0408 |
| 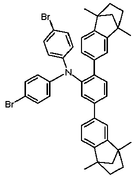 BB-1408 | 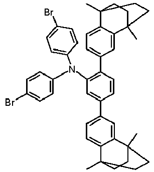 BB-1409 | 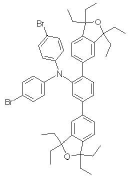 BB-1410 |
| Mon-0409 | Mon-0410 | Mon-0411 |
| 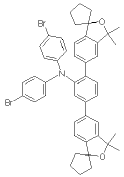 BB-1411 | 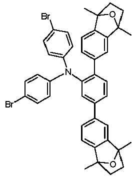 BB-1412 | 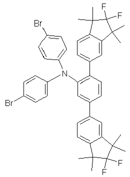 BB-1413 |
| Mon-0412 | Mon-0413 | Mon-0414 |
| 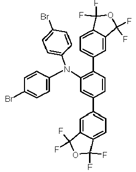 BB-1414 | 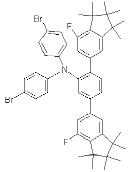 BB-1415 | 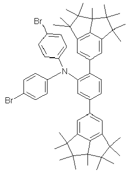 BB-1416 |
| Mon-0415 | Mon-0416 | Mon-0417 |
| 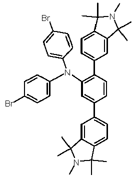 BB-1417 | 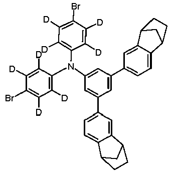 BB-1418 | 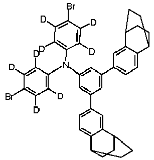 BB-1420 |
| Mon-0418 | Mon-0419 | Mon-0421 |
| 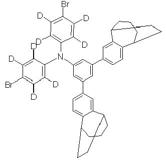 BB-1421 | 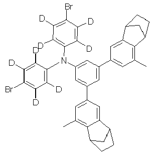 BB-1422 | 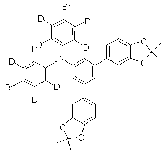 BB-1423 |
| Mon-0422 | Mon-0423 | Mon-0424 |
| 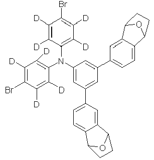 BB-1424 | 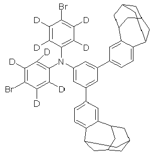 BB-1425 | 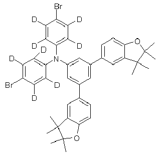 BB-1426 |
| Mon-0425 | Mon-0426 | Mon-0427 |
| 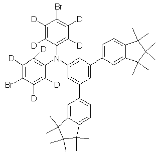 BB-1427 | 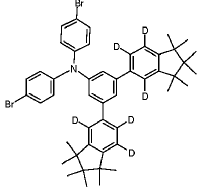 BB-1428 | 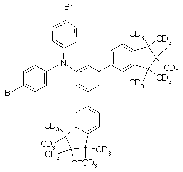 BB-1429 |
| Mon-0428 | Mon-0429 | Mon-0430 |
| 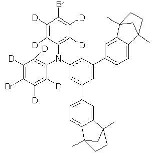 BB-1430 | 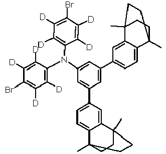 BB-1431 | 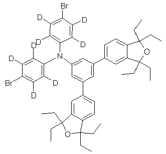 BB-1432 |
| Mon-0431 | Mon-0432 | Mon-0433 |
| 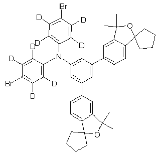 BB-1433 | 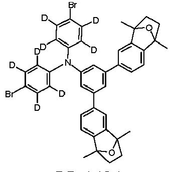 BB-1434 | 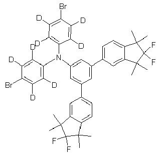 BB-1435 |
| Mon-0434 | Mon-0435 | Mon-0436 |
| 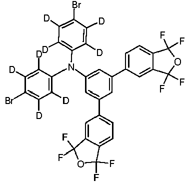 BB-1436 | 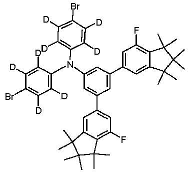 BB-1437 | 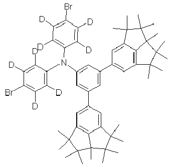 BB-1438 |
| Mon-0437 | Mon-0438 | Mon-0439 |
| 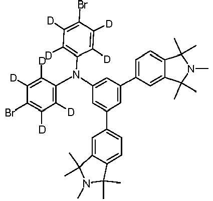 BB-1439 | 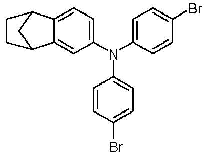 BB-2001 | 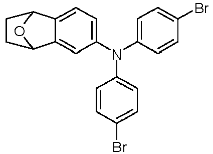 BB-2002 |
| Mon-0440 | Mon-1001 | Mon-1002 |
| 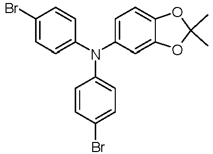 BB-2003 | 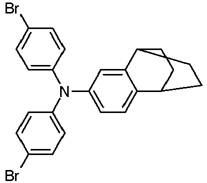 BB-2004 | 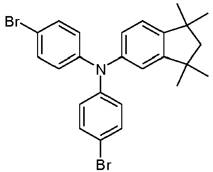 BB-2005 |
| Mon-1003 | Mon-1004 | Mon-1005 |
| 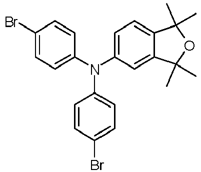 BB-2006 | 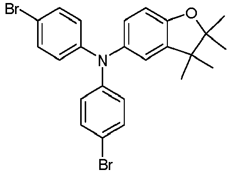 BB-2007 | 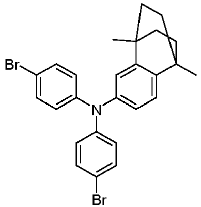 BB-2008 |
| Mon-1006 | Mon-1007 | Mon-1008 |
| 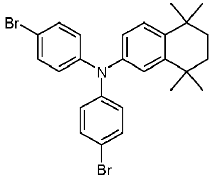 BB-2009 | 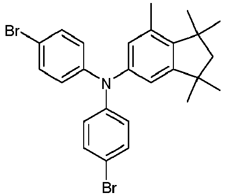 BB-2010 | 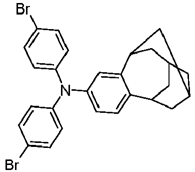 BB-2011 |
| Mon-1009 | Mon-1010 | Mon-1011 |
| 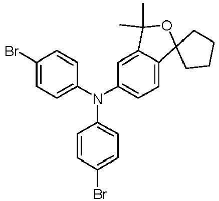 BB-2012 | 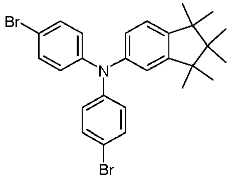 BB-2013 | 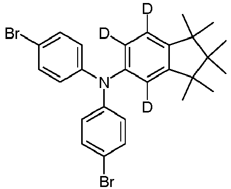 BB-2014 |
| Mon-1012 | Mon-1013 | Mon-1014 |
| 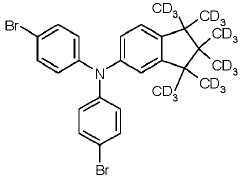 BB-2015 | 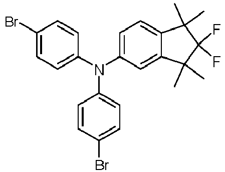 BB-2016 | 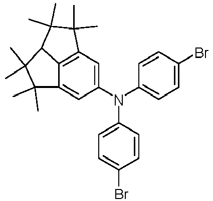 BB-2017 |
| Mon-1015 | Mon-1016 | Mon-1017 |
| 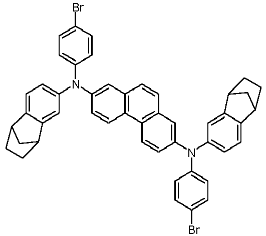 BB-2019 | | 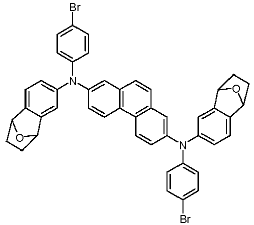 BB-2020 |
| Mon-1019 | | Mon-1020 |
| 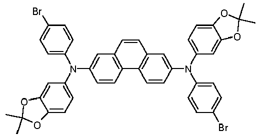 BB-2021 | 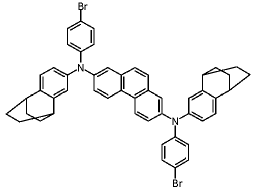 BB-2022 | 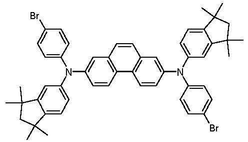 BB-2023 |
| Mon-1021 | Mon-1022 | Mon-1023 |
| 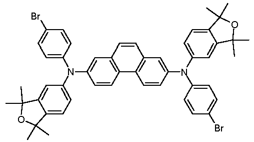 BB-2024 | 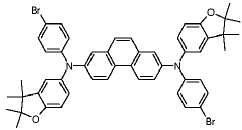 BB-2025 | 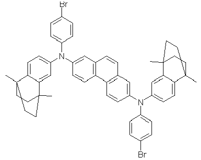 BB-2026 |
| Mon-1024 | Mon-1025 | Mon-1026 |
| 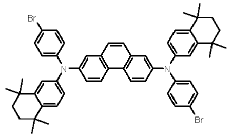 BB-2027 | 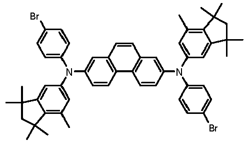 BB-2028 | 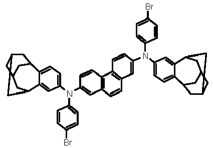 BB-2029 |
| Mon-1027 | Mon-1028 | Mon-1029 |
| 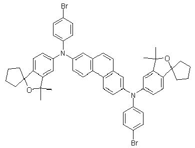 BB-2030 | 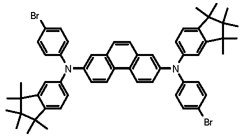 BB-2031 | 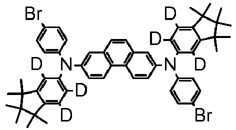 BB-2032 |
| Mon-1030 | Mon-1031 | Mon-1032 |
| 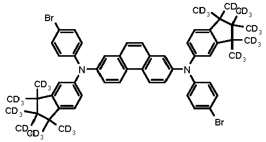 BB-2033 | 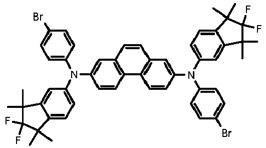 BB-2034 | |
| Mon-1033 | Mon-1034 | |
| 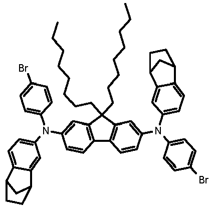 BB-2036 | 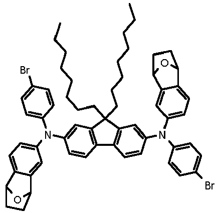 BB-2037 | 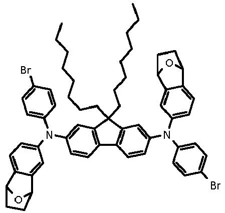 BB-2038 |
| Mon-1036 | Mon-1037 | Mon-1038 |
| 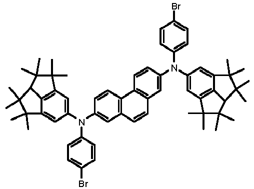 BB-2039 | 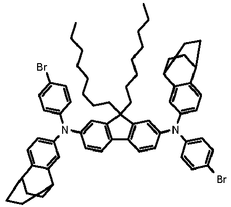 BB-2040 | 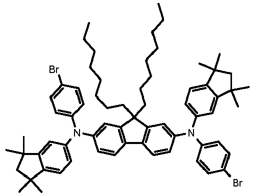 BB-2041 |
| Mon-1039 | Mon-1040 | Mon-1041 |
| 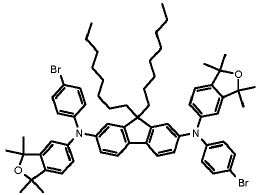 BB-2042 | 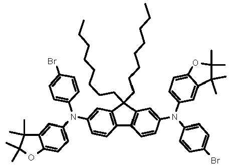 BB-2043 | 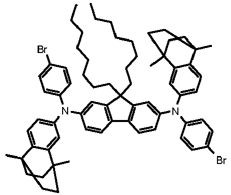 BB-2044 |
| Mon-1042 | Mon-1043 | Mon-1044 |
| 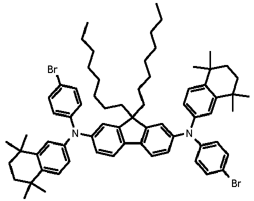 BB-2045 | 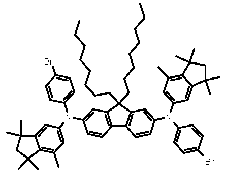 BB-2046 | 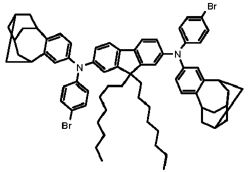 BB-2047 |
| Mon-1045 | Mon-1046 | Mon-1047 |
| 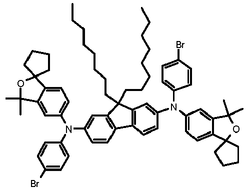 BB-2048 | 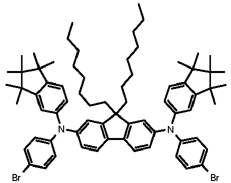 B-2049 | 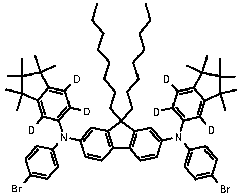 BB-2050 |
| Mon-1048 | Mon-1049 | Mon-1050 |
| 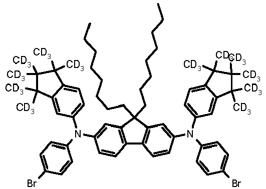 BB-2051 | 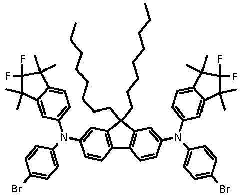 BB-2052 | 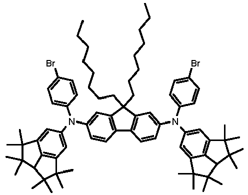 BB-2053 |
| Mon-1051 | Mon-1052 | Mon-1053 |

### 2) Weitere verwendete Monomere:

| Monomer | Struktur | Synthese nach Publikation / CAS-Nummer |
|---|---|---|
| MON-01-Br | 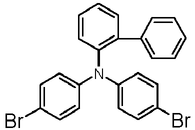 | WO 2013/156130 |
| MON-01-BE | 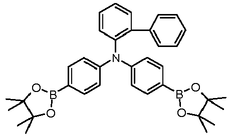 | WO 2013/156130 |
| Mon-02-BE | 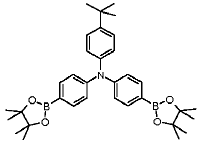 | WO 99/048160 A1 |
| MON-20-BE | 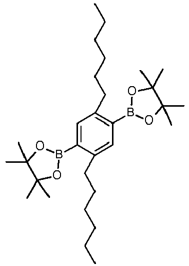 | CAS 374934-77-7 |
| MON-21-BE | 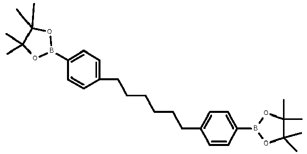 | CAS 1257064-91-7 |
| Mon-22-BE | 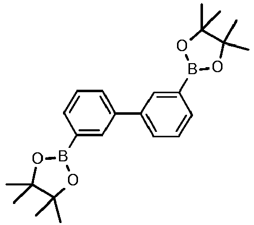 | CAS 850264-92-5 |
| MON-30-Br | 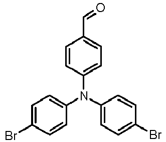 | WO 2010/097155 |
| MON-30-BE | 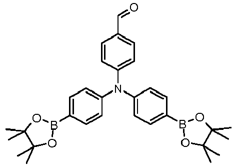 | WO 2010/097155 |
| MON-31-Br | 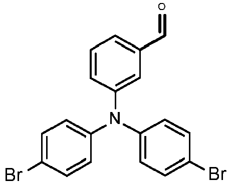 | WO 2013/156130 |
| MON-31-BE | 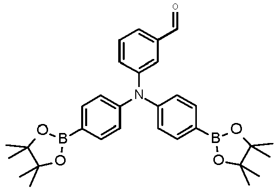 | WO 2013/156130 |
| MON-32-BE | 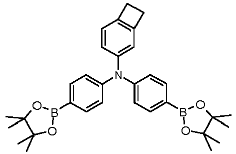 | WO 2009/102027 |
| MON-32-Br | 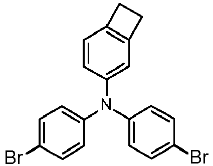 | CAS 852534-20-4 |
| Mon-40-Br | 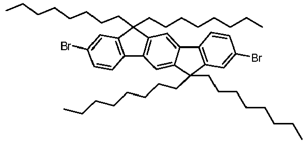 | Macromolecules 2000, 33, 2016-2020 |
| Mon-40-BE | 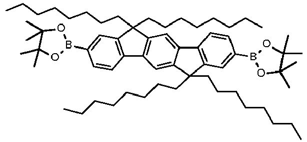 | CAS 628303-20-8 |
| Mon-41-BE | 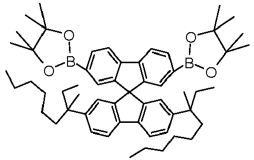 | WO 2003/020790 |
| Mon-42-BE | 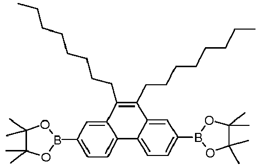 | WO 2005/104264 |

### 3) Synthese der Polymere

Die Vergleichspolymere V1 und V2 sowie die erfindungsgemäßen Polymere Po1 bis Po22 werden durch SUZUKI-Kupplung gemäß dem in der WO 2003/048225 beschriebenen Verfahren aus den obenstehend offenbarten Monomeren hergestellt.

Bei der Herstellung der Polymere werden die unten angegeben Monomere in den entsprechenden prozentualen Anteilen, wie unten angegeben, in der Reaktionsmischung eingesetzt. Die auf diese Weise hergestellten Polymere V1 und V2 sowie Po1 bis Po22 enthalten die Struktureinheiten nach Abspaltung der Abgangsgruppen in den in der untenstehenden Tabelle angegebenen prozentualen Anteilen (Prozentangaben = mol%).

Bei den Polymeren, die aus Monomeren hergestellt werden, die Aldehydgruppen aufweisen, werden diese nach der Polymerisation durch WITTIG Reaktion gemäß dem in der WO 2010/097155 beschriebenen Verfahren in vernetzbare Vinylgruppen überführt (Beispiele mit Synthesevorschrift auf Seite 36/37) . Die entsprechend in der untenstehenden Tabelle aufgeführten Polymere weisen somit vernetzbare Vinylgruppen anstelle der ursprünglich vorhandenen Aldehydgruppen auf.

Die Palladium- und Bromgehalte der Polymeren werden per ICP-MS bestimmt. Die ermittelten Werte liegen unter 10 ppm.

Die Molekulargewichte M_{w} sowie die Polydispersitäten D werden mittels Gelpermeationschromatographie (GPC) (Model: Agilent HPLC System Series 1100) ermittelt (Säule: PL-RapidH von Polymer Laboratories; Lösungsmittel: THF mit 0,12 Vol% o-Dichlorbenzol; Detektion: UV und Brechungsindex; Temperatur: 40°C). Kalibriert wird mit Polystyrolstandards.

### Direktvergleiche:

| **Polymer** | **MON A** | **%** | **MON B** | **%** | **MON C** | **%** | **Mw/D** |
|---|---|---|---|---|---|---|---|
| V1 | MON-40-Br | 50 | MON-01-BE | 40 | MON-30-BE | 10 | 125K/2.3 |
| V2 | MON-01-Br | 30 | MON-20-BE | 50 | MON-30-Br | 20 | 65K/2.7 |
| Po1 | MON-40-BE | 50 | Mon-0032 | 40 | MON-30-Br | 10 | 135K/2.4 |
| Po2 | Mon-0032 | 30 | MON-20-BE | 50 | MON-30-Br | 20 | 70K/3.1 |

Zusätzlich werden die folgenden erfindungsgemäßen Polymere hergestellt, unter Verwendung der Monomerbausteine Mon-0032, Mon-0033, Mon-0036, Mon-0039, Mon-0042, Mon-0043, Mon-0054, Mon-0084, Mon-0164, Mon-0406, Mon-0410, Mon-0429, Mon-1006, Mon-1013, Mon-1031, Mon-1040, Mon-1041 und Mon-1049 (Strukturen s. Tabelle oben).

| **Poly-mer** | **MON A** | **%** | **MON B** | **%** | **MON C** | **%** | **MON D** | **%** | **Mw/D** |
|---|---|---|---|---|---|---|---|---|---|
| Po3 | MON-40-BE | 50 | Mon-0042 | 40 | MON-30-Br | 10 | | | 150K/2.3 |
| Po4 | Mon-0054 | 30 | MON-01-BE | 50 | MON-32-BE | 20 | | | 55K/3.2 |
| Po5 | Mon-0032 | 30 | MON-20-BE | 50 | MON-32-Br | 20 | | | 80K/4.1 |
| Po6 | MON-041-BE | 40 | Mon-1031 | 50 | MON-31-BE | 20 | | | 180K/2.5 |
| Po7 | Mon-1013 | 20 | MON-21-BE | 50 | MON-01-Br | 20 | MON-30-Br | 10 | 45K/3.6 |
| Po8 | MON-40-Br | 25 | MON-22-BE | 50 | Mon-0406 | 25 | | | 120K/4.5 |
| Po9 | MON-40-BE | 50 | Mon-0429 | 40 | MON-30-Br | 10 | | | 125K/2.1 |
| Po10 | MON-40-BE | 50 | Mon-0164 | 40 | MON-30-Br | 10 | | | 95K/2.3 |
| Po11 | MON-41-BE | 50 | Mon-0084 | 40 | MON-032-Br | 10 | | | 90K/2.6 |
| Po12 | MON-40-BE | 50 | Mon-0036 | 40 | MON-31-Br | 10 | | | 105K/3.0 |
| Po13 | Mon-1041 | 50 | MON-30-BE | 50 | | | | | 80K/2.9 |
| Po14 | Mon-1049 | 50 | MON-01-BE | 30 | MON-30-BE | 20 | | | 65K/2.8 |
| Po15 | Mon-1040 | 50 | MON-02-BE | 30 | MON-32-BE | 20 | | | 55K/4.2 |
| Po16 | MON-042-BE | 50 | Mon-1006 | 30 | MON-31-Br | 20 | | | 85K/2.5 |
| Po17 | Mon-0406 | 30 | Mon-0410 | 20 | MON-01-BE | 30 | MON-30-BE | 20 | 50K/3.1 |
| Po18 | MON-40-BE | 50 | Mon-0043 | 40 | MON-032-Br | 10 | | | 105K/3.0 |
| Po19 | MON-42-BE | 50 | Mon-0033 | 30 | MON-032-Br | 20 | | | 95K/2.4 |
| Po20 | MON-041-BE | 40 | Mon-0039 | 50 | MON-30-BE | 10 | | | 75K/2.6 |
| Po21 | Mon-0032 | 40 | MON-20-BE | 50 | MON-30-Br | 10 | | | 85K/2.6 |
| Po22 | Mon-0032 | 30 | MON-01-BE | 50 | MON-30-Br | 20 | | | 50K/3.8 |

### B) Beispiele für verbessertes Lösungsverhalten

### Konzentration der Lösungen: 7 mg/ml, Lösungsmittel: 3-Phenoxytoluol

| | V1 | Po1 | V2 | Po2 |
|---|---|---|---|---|
| Benötigte Zeit für vollständige Auflösung (min) | 47 | 38 | 52 | 42 |
| Δ (min) | 9 | | 10 | |

Die Zeit bis die gesamte Polymermenge in Lösung gegangen ist, ist für die erfindungsgemäßen Polymere ca. 20% kürzer.

### C) Device-Beispiele

Die erfindungsgemäßen Polymere können aus Lösung verarbeitet werden. Löslich prozessierte OLEDs sind verglichen mit vakuumprozessierten OLEDs wesentlich einfacher herstellbar und haben dennoch gute Eigenschaften.

Die Herstellung solcher lösungsbasierter OLEDs ist in der Literatur bereits vielfach beschrieben, z.B. in der WO 2004/037887 und der WO 2010/097155. Das Verfahren wird auf die im Folgenden beschriebenen Gegebenheiten (Schichtdickenvariation, Materialien) angepasst.

Die erfindungsgemäßen Polymere werden in zwei verschiedenen Schichtabfolgen verwendet:
Aufbau A ist wie folgt:
   - Substrat,
   - ITO (50 nm),
   - Lochinjektionsschicht (HIL) (20 nm),
   - Lochtransportschicht (HTL) (20 nm),
   - Emissionsschicht (EML) (60 nm),
   - Lochblockierschicht (HBL) (10 nm)
   - Elektronentransportschicht (ETL) (40 nm),
   - Kathode (Al) (100 nm).
Aufbau B ist wie folgt:
   - Substrat,
   - ITO (50 nm),
   - Lochinjektionsschicht (HIL) (20 nm),
   - Lochtransportschicht (HTL) (20 nm),
   - Emissionsschicht (EML) (30 nm),
   - Lochblockierschicht (HBL) (10 nm)
   - Elektronentransportschicht (ETL) (40 nm),
   - Kathode (Al) (100 nm).

Als Substrat dienen Glasplättchen, die mit strukturiertem ITO (Indium-ZinnOxid) der Dicke 50 nm beschichtet sind. Die Lochinjektionsschicht wird mittels Spin-Coating in inerter Atmosphäre aufgebracht. Hierzu werden ein lochtransportierendes, vernetzbares Polymer und ein p-dotierendes Salz in Toluol gelöst. Entsprechende Materialien wurden u.a. in WO 2016/107668, WO 2013/081052 und EP2325190 beschrieben. Für eine resultierende Schichtdicke von 20 nm wird ein Feststoffgehalt von 6 mg/ml verwendet. Die Schicht wird anschließend auf einer Heizplatte für 30 Minuten bei 200°C in Inertgasatmosphäre ausgeheizt.

Auf diese beschichteten Glasplättchen werden nun die Lochtransport- und die Emissionsschicht aufgebracht.

Als Lochtransportschicht werden die erfindungsgemäßen Verbindungen sowie Vergleichsverbindungen verwendet, jeweils in Toluol gelöst. Der Feststoffgehalt dieser Lösungen liegt bei 5 mg/ml, da mittels Spincoating Schichtdicken von 20 nm erzielt werden sollen. Die Schichten werden in Inertgasatmosphäre aufgeschleudert und für 30 Minuten bei 220°C auf einer Heizplatte ausgeheizt.

Die Emissionsschicht für Aufbau A setzt sich aus den Hostmaterialien H2 und H3 und dem emittierenden Dotierstoff D2 zusammen. Alle drei Materialien liegen in einem Gewichtsanteil von 30% H2, 55% H3 und 15% D2 in der Emissionsschicht vor. Die Mischung für die Emissionsschicht wird in Toluol gelöst. Der Feststoffgehalt dieser Lösung liegt bei 18 mg/ml, da mittels Spincoating Schichtdicken von 60 nm erzielt werden sollen. Die Schichten werden in Inertgasatmosphäre aufgeschleudert und für 10 Minuten bei 150°C ausgeheizt.

Die Emissionsschicht für Aufbau B setzt sich aus dem Hostmaterial H1 und dem emittierenden Dotierstoff D1 zusammen. Beide Materialien liegen in einem Gewichtsanteil von 92% H1 und 8% D1 in der Emissionsschicht vor. Die Mischung für die Emissionsschicht wird in Toluol gelöst. Der Feststoffgehalt dieser Lösung liegt bei 9 mg/ml, da mittels Spincoating Schichtdicken von 30 nm erzielt werden sollen. Die Schichten werden in Inertgasatmosphäre aufgeschleudert und für 10 Minuten bei 150°C ausgeheizt.

Die im vorliegenden Fall verwendeten Materialien sind in Tabelle C1 gezeigt.

**Tabelle C1:**

| Strukturformeln der in der Emissionsschicht verwendeten Materialien | |
|---|---|
| 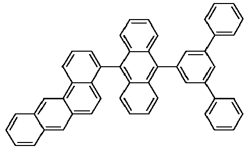 | 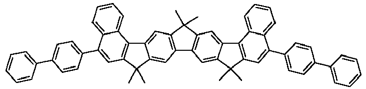 |
| H1 | D1 |
| 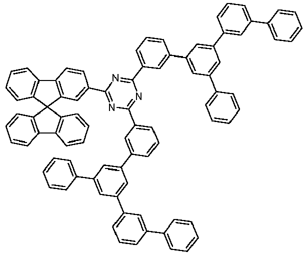 | 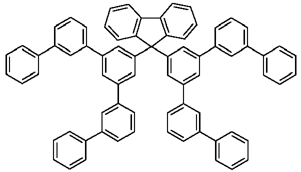 |
| H2 | H3 |
| 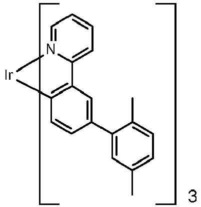 | |
| D2 | |

Die Materialien für die Lochblockierschicht und Elektronentransportschicht werden in einer Vakuumkammer thermisch aufgedampft und sind in Tabelle C2 gezeigt. Die Lochblockierschicht besteht aus ETM1. Die Elektronentransportschicht besteht aus den beiden Materialien ETM1 und ETM2, die einander durch Co-Verdampfung in einem Volumenanteil von jeweils 50% beigemischt werden.

**Tabelle C2:**

| Verwendete HBL- und ETL-Materialien | |
|---|---|
| 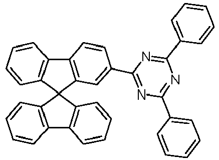 | 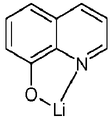 |
| ETM1 | ETM2 |

Die Kathode wird durch die thermische Verdampfung einer 100 nm dicken Aluminiumschicht gebildet.

Die OLEDs werden standardmäßig charakterisiert. Hierfür werden die Elektrolumineszenzspektren, Strom-Spannungs-Leuchtdichte-Kennlinien (IUL-Kennlinien) unter Annahme einer Lambert'schen Abstrahlcharakteristik sowie die (Betriebs-)Lebensdauer bestimmt. Aus den IUL-Kennlinien werden Kennzahlen bestimmt wie z.B. die externe Quanteneffizienz (in %) bei einer bestimmten Helligkeit. LD80 @ 1000cd/m² ist die Lebensdauer, bis die OLED bei einer Starthelligkeit von 1000 cd/m² auf 80 % der Anfangsintensität, also auf 800 cd/m², abgefallen ist.

Die Eigenschaften der verschiedenen OLEDs sind in den Tabellen C3a, C3b und C3c zusammengefasst.

Die Beispiele C01 und C02 sind Vergleichsbeispiele, alle anderen Beispiele zeigen Eigenschaften von OLEDs mit erfindungsgemäßen Lochtransport-Polymeren. Es werden blau und grün emittierende OLEDs mit den erfindungsgemäßen Materialien als HTL erzeugt.

**Tabelle C3a:**

| Eigenschaften der OLEDs | | | | | |
|---|---|---|---|---|---|
| Beispiel | HTL Polymer | Bauteil Aufbau | Effizienz bei 1000 cd/m² | Spannung bei 1000 cd/m² | LD80 bei 10.000 cd/m² |
| | | | % EQE | [V] | [h] |
| C01 | V1 | A | 18.2 | 4.8 | 552 |
| C03 | Po1 | A | 18.6 | 4.8 | 764 |

**Tabelle C3b:**

| Eigenschaften der OLEDs | | | | |
|---|---|---|---|---|
| Beispiel | HTL Polymer | Bauteil Aufbau | Effizienz bei 1000 cd/m² | LD80 bei 1.000 cd/m² |
| | | | % EQE | [h] |
| C02 | V2 | B | 7.0 | 204 |
| C04 | Po2 | B | 7.1 | 223 |

Wie die Ergebnisse der Tabellen C3a und C3b zeigen, ergeben die erfindungsgemäßen Polymere bei Einsatz als Lochtransportschicht in grün phosphoreszierenden und blau fluoreszierenden OLEDs Verbesserungen gegenüber dem Stand der Technik, vor allem in Bezug auf Lebensdauer, Effizienz und Spannung.

**Tabelle C3c:**

| Eigenschaften der OLEDs | | | | | | |
|---|---|---|---|---|---|---|
| Beispiel | HTL Polymer | Bauteil Aufbau | Effizienz bei 1000 cd/m² | Spannung bei 1000 cd/m² | LD80 bei 10.000 cd/m² | LD80 bei 1.000 cd/m² |
| | | | % EQE | [V] | [h] | [h] |
| C05 | Po21 | B | 7.5 | -- | -- | 236 |
| C06 | Po5 | B | 7.6 | -- | -- | 210 |
| C07 | Po22 | A | 17.7 | 3.2 | 537 | -- |

Tabelle 3c zeigt die Effizienz und Lebensdauer von OLEDs enthaltend die erfindungsgemäßen Polymere Po5, Po21 und Po22. Mit den genannten Polymeren werden gute Ergebnisse für diese Parameter erreicht.

Auch mit den weiteren Polymeren Po3, Po4, und Po6-Po20 können auf demselben Weg wie oben gezeigt blau fluoreszierende bzw. grün phosphoreszierende OLEDs hergestellt werden. Auch diese weisen gute Eingeschaften auf, insbesondere gute Lebensdauer und Effizienz.

Weiterhin wird gefunden, dass mit Polymeren enthaltend Struktureinheiten, welche eine oder mehrere Gruppen R¹, insbesondere Alkylgruppen, als Substituenten am Ringsystem bestehend aus den Gruppen U aufweisen, bessere Eigenschaften der OLEDs erzielt werden als mit Polymeren enthaltend Struktureinheiten, die am Ringsystem bestehend aus den Gruppen U unsubstituiert sind.

## Patentansprüche

1. Polymer, enthaltend mindestens eine Struktureinheit der Formel (I) in einem Anteil von 1 bis 100% bezogen auf 100 mol% aller copolymerisierbaren Monomere, die im Polymer als Struktureinheiten enthalten sind 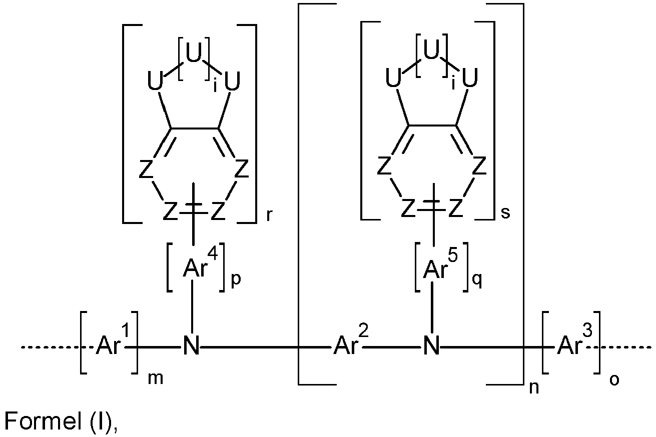 wobei für die auftretenden Variablen gilt:
U ist bei jedem Auftreten gleich oder verschieden C(R¹)₂, CR¹=CR¹, Si(R¹)₂, O oder S, wobei Gruppen gewählt aus CR¹=CR¹, O und S nicht direkt miteinander verbunden sind;
Z ist gleich CR², wenn keine Gruppe daran gebunden ist, und ist gleich C, wenn eine Gruppe daran gebunden ist;
Ar¹, Ar², Ar³, Ar⁴ und Ar⁵ sind gleich oder verschieden gewählt aus heteroaromatischen Ringsystemen mit 5 bis 40 aromatischen Ringatomen, die mit einem oder mehreren Resten R³ substituiert sein können, und aus aromatischen Ringsystemen mit 6 bis 40 aromatischen Ringatomen, die mit einem oder mehreren Resten R³ substituiert sein können;
R¹ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch - R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R², R³ sind bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, C(=O)R⁵, CN, Si(R⁵)₃, N(R⁵)₂, P(=O)(R⁵)₂, OR⁵, S(=O)R⁵, S(=O)₂R⁵, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁴ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁵ substituiert sein können; und wobei eine oder mehrere CH₂-Gruppen in den genannten Alkyl-, Alkoxy-, Alkenyl- und Alkinylgruppen durch -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO oder SO₂ ersetzt sein können;
R⁵ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁵ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
r ist gleich 1, 2, oder 3, wenn p gleich 1 ist, und ist gleich 1, wenn p gleich 0 ist;
s ist gleich 0, 1, 2, oder 3, wenn q gleich 1 ist, und ist gleich 1, wenn q gleich 0 ist;
p ist gleich 0 oder 1; wobei wenn p gleich 0 ist, die Gruppen, die an die in eckigen Klammern mit Index p stehende Einheit gebunden sind, direkt miteinander verbunden sind;
q ist gleich 0 oder 1; wobei wenn q gleich 0 ist, die Gruppen, die an die in eckigen Klammern mit Index q stehende Einheit gebunden sind, direkt miteinander verbunden sind;
n ist gleich 0 oder 1, wobei wenn n gleich 0 ist, die Gruppen, die an die in eckigen Klammern mit Index n stehende Einheit gebunden sind, direkt miteinander verbunden sind;
m ist gleich 0 oder 1, wobei wenn m gleich 0 ist, die Gruppen, die an die in eckigen Klammern mit Index m stehende Einheit gebunden sind, direkt miteinander verbunden sind;
o ist gleich 0 oder 1, wobei wenn o gleich 0 ist, die Gruppen, die an die in eckigen Klammern mit Index o stehende Einheit gebunden sind, direkt miteinander verbunden sind;
i ist bei jedem Auftreten gleich oder verschieden 1 oder 2;
wobei mindestens eine Gruppe U vorhanden ist, die eine oder mehrere Gruppen R¹ enthält, die gewählt sind aus geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² bzw. R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können; und
wobei diejenigen beiden Gruppen U, die sich direkt benachbart zum Brückenkopf-C-Atom befinden, das heißt in benzylischer Position befinden, Reste R¹ tragen, die gewählt sind aus F, CN, Si(R⁴)₃, OR⁴, geradkettigen Alkyl- und Alkoxygruppen mit 1 bis 10 C-Atomen, verzweigten Alkyl- und Alkoxygruppen mit 3 bis 10 C-Atomen, und aromatischen Ringsystemen mit 6 bis 20 aromatischen Ringatomen, wobei zwei oder mehr Reste R¹ bzw. R² bzw. R³ miteinander verknüpft sein können und einen Ring bilden können; und wobei die genannten Alkyl- und Alkoxygruppen und die genannten aromatischen Ringsysteme jeweils mit einem oder mehreren Resten R⁴ substituiert sein können.

2. Polymer nach Anspruch 1, **dadurch gekennzeichnet, dass** Ar¹, Ar², Ar³, Ar⁴ und Ar⁵ bei jedem Auftreten gleich oder verschieden gewählt sind aus Benzol, Biphenyl, Terphenyl, Fluoren, Naphthalin, Phenanthren, Indenofluoren, Spirobifluoren, Dibenzofuran, Dibenzothiophen, Carbazol, Indenocarbazol und Indolocarbazol, die jeweils mit einem oder mehreren Resten R¹ substituiert sein können.

3. Polymer nach Anspruch 1 oder 2, **dadurch gekennzeichnet, dass** R¹ bei jedem Auftreten gleich oder verschieden gewählt ist aus H, D, F, geradkettigen Alkylgruppen mit 1 bis 10 C-Atomen und verzweigten Alkylgruppen mit 3 bis 10 C-Atomen.

4. Polymer nach einem oder mehreren der Ansprüche 1 bis 3, **dadurch gekennzeichnet, dass** die Einheiten 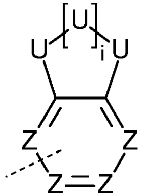 in der Struktureinheit der Formel (I) gewählt sind aus Einheiten der Formel (E-1) 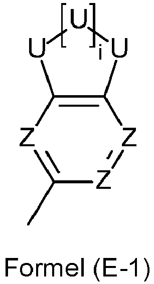 wobei die freie Bindung die Bindung an den Rest der Struktureinheit der Formel (I) darstellt, und wobei die auftretenden Variablen definiert sind wie in einem oder mehreren der Ansprüche 1 bis 3.

5. Polymer nach einem oder mehreren der Ansprüche 1 bis 4, **dadurch gekennzeichnet, dass** die Einheiten 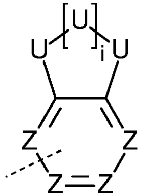 in der Struktureinheit der Formel (I) gewählt sind aus den folgenden Einheiten:
| | | |
|---|---|---|
| 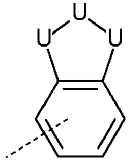 | 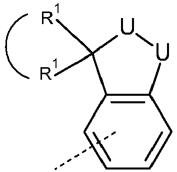 | 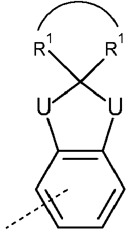 |
| E-a-1 | E-a-2 | E-a-3 |
| 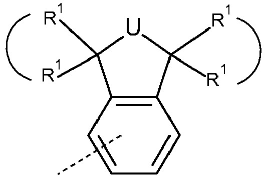 | 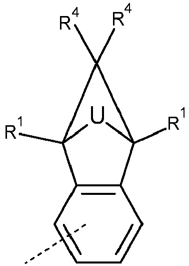 | |
| E-a-4 | E-a-5 | |
| 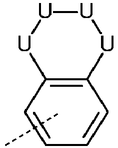 | 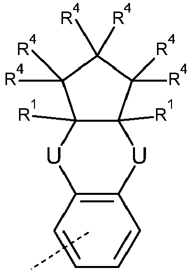 | 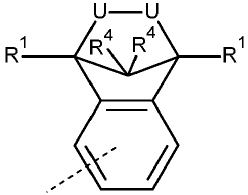 |
| E-b-1 | E-b-2 | E-b-3 |
| 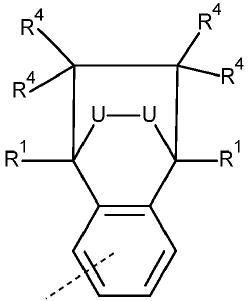 | 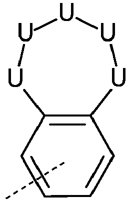 | 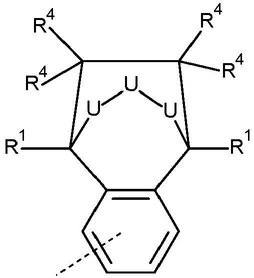 |
| E-b-4 | E-c-1 | E-c-2 |
wobei die gestrichelte Linie die Bindung an den Rest der Struktureinheit der Formel (I) darstellt, und wobei die halbkreisförmige Bindung bedeutet, dass die beiden daran beteiligten Gruppen R¹ miteinander verknüpft sind und einen Ring bilden, und wobei die auftretenden Variablen definiert sind wie in einem oder mehreren der Ansprüche 1 bis 4.

6. Polymer nach einem oder mehreren der Ansprüche 1 bis 5, **dadurch gekennzeichnet, dass** das Strukturelement der Formel (I) einer der Formeln (I-1) bis (I-6) entspricht
| |
|---|
| 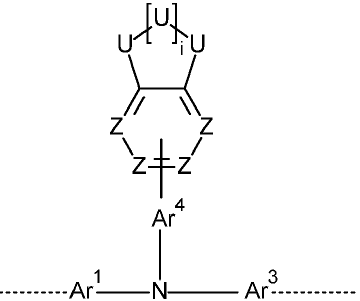 |
| (I-1) |
| 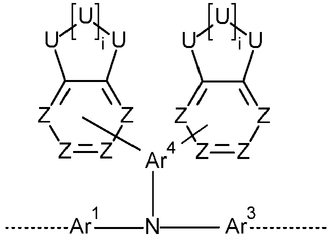 |
| (I-2) |
| 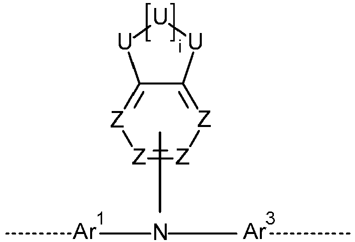 |
| (I-3) |
| 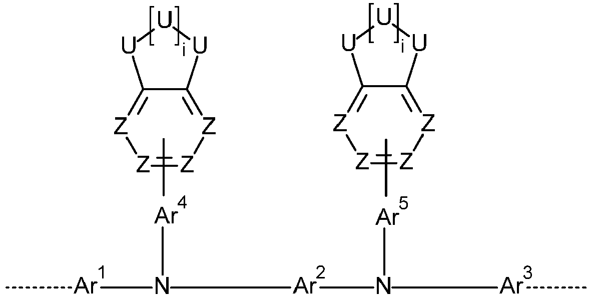 |
| (I-4) |
| 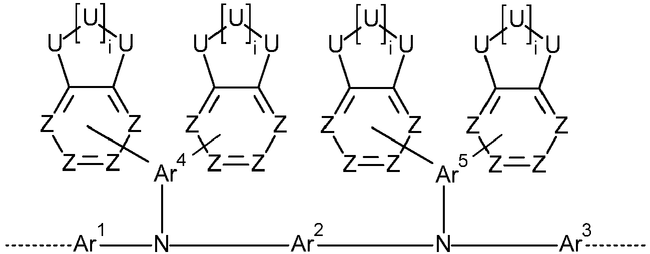 |
| (I-5) |
| 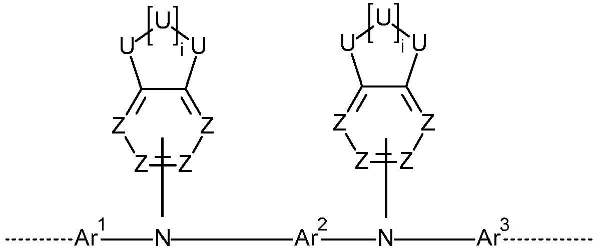 |
| (I-6) |
wobei die auftretenden Variablen definiert sind wie in einem oder mehreren der Ansprüche 1 bis 5.

7. Polymer nach einem oder mehreren der Ansprüche 1 bis 6, **dadurch gekennzeichnet, dass** der Anteil an Struktureinheiten der Formel (I) im Polymer im Bereich von 30 bis 70 mol%, bezogen auf 100 mol% aller copolymerisierbaren Monomere, die im Polymer als Struktureinheiten enthalten sind, liegt.

8. Polymer nach einem oder mehreren der Ansprüche 1 bis 7, **dadurch gekennzeichnet, dass** es mindestens eine Struktureinheit aufweist, die eine vernetzbare Gruppe Q enthält.

9. Polymer nach Anspruch 8, **dadurch gekennzeichnet, dass** die vernetzbare Gruppe Q gewählt ist aus den folgenden Formeln
| | |
|---|---|
| 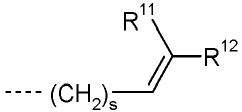 | 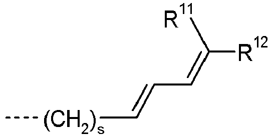 |
| Q1 | Q2 |
| 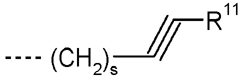 | 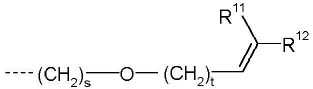 |
| Q3 | Q4 |
| 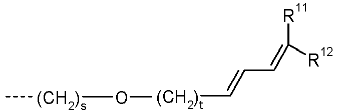 | 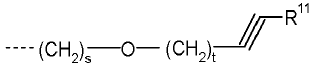 |
| Q5 | Q6 |
| 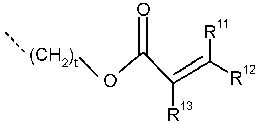 | 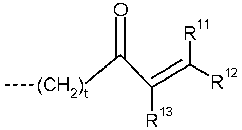 |
| Q7 | Q8 |
| 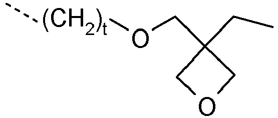 | 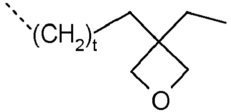 |
| Q9 | Q10 |
| 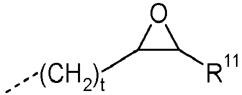 | 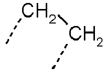 |
| Q11 | Q12 |
| 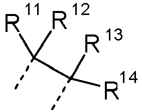 | 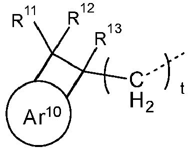 |
| Q13 | Q14 |
wobei R¹¹, R¹², R¹³ und R¹⁴ bei jedem Auftreten gleich oder verschieden gewählt sind aus H und geradkettigen oder verzweigten Alkylgruppen mit 1 bis 6 C-Atomen, s= 0 bis 8 ist, t = 1 bis 8 ist, und Ar¹⁰ gewählt ist aus aromatischen Ringsystemen mit 6 bis 40 aromatischen Ringatomen, die mit einem oder mehreren Resten R¹¹ substituiert sein können, und aus heteroaromatischen Ringsystemen mit 5 bis 40 aromatischen Ringatomen, die mit einem oder mehreren Resten R¹¹ substituiert sein können, und wobei die gestrichelte Bindung die Bindung an den Rest der Formel kennzeichnet.

10. Polymer, erhältlich durch Vernetzungsreaktion eines Polymers nach Anspruch 8 oder 9.

11. Mischung enthaltend ein oder mehrere Polymere nach einem oder mehreren der Ansprüche 1 bis 10, sowie eine oder mehrere weitere polymere, oligomere, dendritische und/oder niedermolekulare Substanzen.

12. Lösung, enthaltend ein oder mehrere Polymere nach einem oder mehreren der Ansprüche 1 bis 10 sowie ein oder mehrere Lösungsmittel.

13. Verwendung eines Polymers nach einem oder mehreren der Ansprüche 1 bis 10 in einer elektronischen Vorrichtung.

14. Elektronische Vorrichtung, enthaltend mindestens ein Polymer nach einem oder mehreren der Ansprüche 1 bis 10.

15. Elektronische Vorrichtung nach Anspruch 14, **dadurch gekennzeichnet, dass** das Polymer nach einem oder mehreren der Ansprüche 1 bis 10 in einer Schicht gewählt aus lochtransportierender Schicht, Lochinjektionsschicht, Elektronenblockierschicht und emittierender Schicht enthalten ist.

16. Verfahren zur Herstellung eines Polymers nach einem oder mehreren der Ansprüche 1 bis 10, **dadurch gekennzeichnet, dass** eine Polymerisation gewählt aus Polymerisation gemäß Suzuki, Polymerisation gemäß Yamamoto, Polymerisation gemäß Stille, Polymerisation gemäß Heck, Polymerisation gemäß Negishi, Polymerisation gemäß Sonogashira, Polymerisation gemäß Hiyama, und Polymerisation gemäß Hartwig-Buchwald durchgeführt wird.

17. Monomer gemäß einer Formel (M) 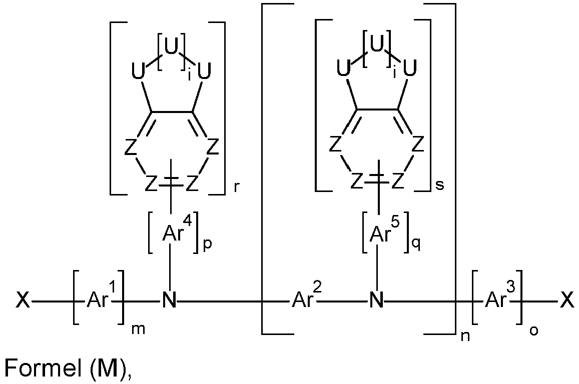 wobei die auftretenden Variablen definiert sind wie in einem oder mehreren der Ansprüche 1 bis 5, und wobei X bei jedem Auftreten gleich oder verschieden eine für eine Polymerisationsreaktion geeignete Abgangsgruppe ist.

## Claims

1. Polymer containing at least one structural unit of the formula (I) in a proportion of 1 to 100%, based on 100 mol% of all copolymerisable monomers present as structural units in the polymer, 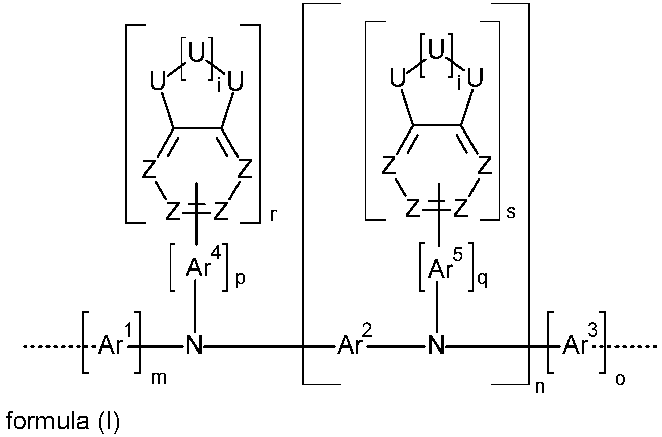 where the following applies to the variables occurring:
U is on each occurrence, identically or differently, C(R¹)₂, CR¹=CR¹, Si(R¹)₂, O or S, where groups selected from CR¹=CR¹, O and S are not connected directly to one another;
Z is equal to CR² if no group is bonded thereto, and is equal to C if a group is bonded thereto;
Ar¹, Ar², Ar³, Ar⁴ and Ar⁵ are selected, identically or differently, from heteroaromatic ring systems having 5 to 40 aromatic ring atoms, which may be substituted by one or more radicals R³, and from aromatic ring systems having 6 to 40 aromatic ring atoms, which may be substituted by one or more radicals R³;
R¹ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R² or R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R², R³ are selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R² or R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, C(=O)R⁵, CN, Si(R⁵)₃, N(R⁵)₂, P(=O)(R⁵)₂, OR⁵, S(=O)R⁵, S(=O)₂R⁵, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁴ may be linked to one another and may form a ring; where the said alkyl, alkoxy, alkenyl and alkynyl groups and the said aromatic ring systems and heteroaromatic ring systems may in each case be substituted by one or more radicals R⁵; and where one or more CH₂ groups in the said alkyl, alkoxy, alkenyl and alkynyl groups may be replaced by -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO or SO₂;
R⁵ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁵ may be linked to one another and may form a ring; and where the said alkyl, alkoxy, alkenyl and alkynyl groups, aromatic ring systems and heteroaromatic ring systems may be substituted by F or CN;
r is equal to 1, 2 or 3 if p is equal to 1, and is equal to 1 if p is equal to 0;
s is equal to 0, 1, 2 or 3 if q is equal to 1, and is equal to 1 if q is equal to 0;
p is equal to 0 or 1; where, if p is equal to 0, the groups that are bonded to the unit in square brackets with index p are connected directly to one another;
q is equal to 0 or 1; where, if q is equal to 0, the groups that are bonded to the unit in square brackets with index q are connected directly to one another;
n is equal to 0 or 1, where, if n is equal to 0, the groups that are bonded to the unit in square brackets with index n are connected directly to one another;
m is equal to 0 or 1, where, if m is equal to 0, the groups that are bonded to the unit in square brackets with index m are connected directly to one another;
o is equal to 0 or 1, where, if o is equal to 0, the groups that are bonded to the unit in square brackets with index o are connected directly to one another;
i is on each occurrence, identically or differently, 1 or 2;
where at least one group U is present that contains one or more groups R¹ selected from straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R² or R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂; and
where the two groups U that are located directly adjacent to the bridgehead C atom, i.e. located in the benzylic position, carry radicals R¹ selected from F, CN, Si(R⁴)₃, OR⁴, straight-chain alkyl and alkoxy groups having 1 to 10 C atoms, branched alkyl and alkoxy groups having 3 to 10 C atoms, and aromatic ring systems having 6 to 20 aromatic ring atoms, where two or more radicals R¹ or R² or R³ may be linked to one another and may form a ring; and where the said alkyl and alkoxy groups and the said aromatic ring systems may in each case be substituted by one or more radicals R⁴.

2. Polymer according to Claim 1, **characterised in that** Ar¹, Ar², Ar³, Ar⁴ and Ar⁵ are selected on each occurrence, identically or differently, from benzene, biphenyl, terphenyl, fluorene, naphthalene, phenanthrene, indeno-fluorene, spirobifluorene, dibenzofuran, dibenzothiophene, carbazole, indenocarbazole and indolocarbazole, which may in each case be substituted by one or more radicals R¹.

3. Polymer according to Claim 1 or 2, **characterised in that** R¹ is selected on each occurrence, identically or differently, from H, D, F, straight-chain alkyl groups having 1 to 10 C atoms and branched alkyl groups having 3 to 10 C atoms.

4. Polymer according to one or more of Claims 1 to 3, **characterised in that** the units 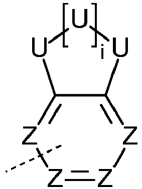 in the structural unit of the formula (I) are selected from units of the formula (E-1) 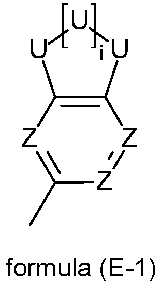 where the free bond represents the bond to the remainder of the structural unit of the formula (I), and where the variables occurring are defined as in one or more of Claims 1 to 3.

5. Polymer according to one or more of Claims 1 to 4, **characterised in that** the units 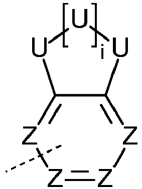 in the structural unit of the formula (I) are selected from the following units:
| | | |
|---|---|---|
| 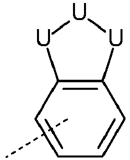 | 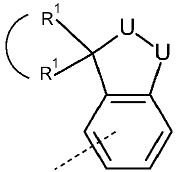 | 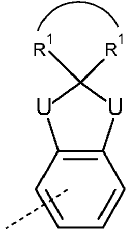 |
| E-a-1 | E-a-2 | E-a-3 |
| 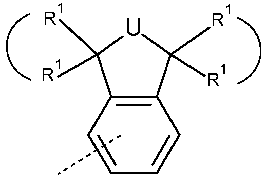 | 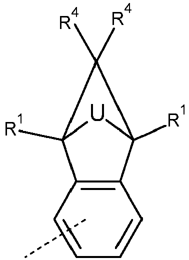 | |
| E-a-4 | E-a-5 | |
| 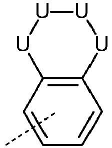 | 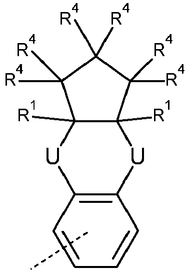 | 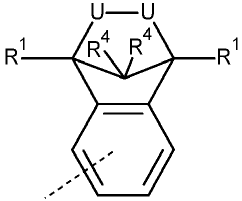 |
| E-b-1 | E-b-2 | E-b-3 |
| 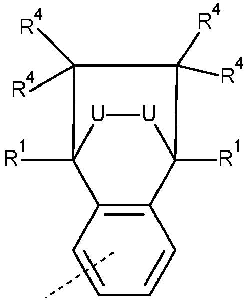 | 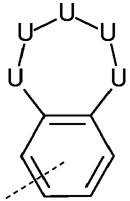 | 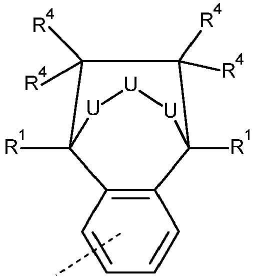 |
| E-b-4 | E-c-1 | E-c-2 |
where the dashed line represents the bond to the remainder of the structural unit of the formula (I), and where the semicircular bond means that the two groups R¹ participating therein are linked to one another and form a ring, and where the variables occurring are defined as in one or more of Claims 1 to 4.

6. Polymer according to one or more of Claims 1 to 5, **characterised in that** the structural element of the formula (I) corresponds to one of the formulae (I-1) to (I-6)
| |
|---|
| 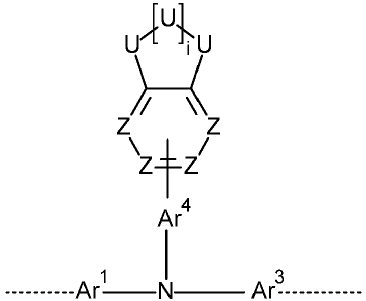 |
| (I-1) |
| 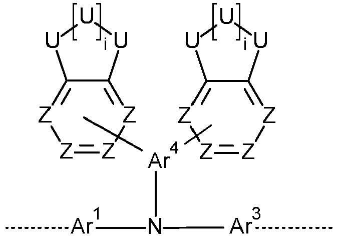 |
| (I-2) |
| 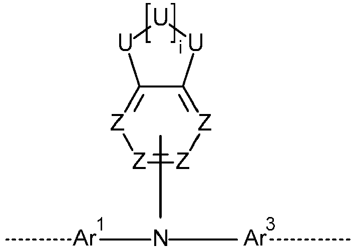 |
| (I-3) |
| 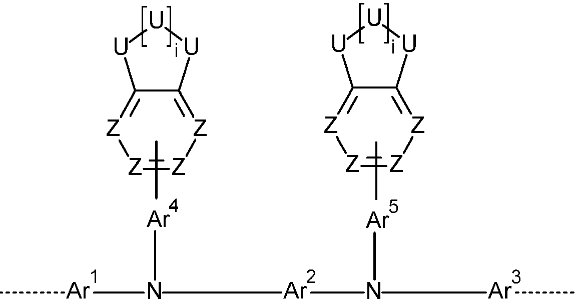 |
| (I-4) |
| 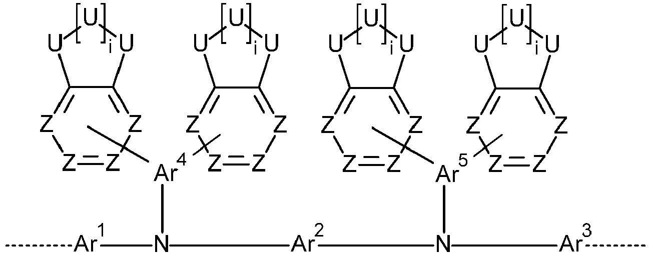 |
| (I-5) |
| 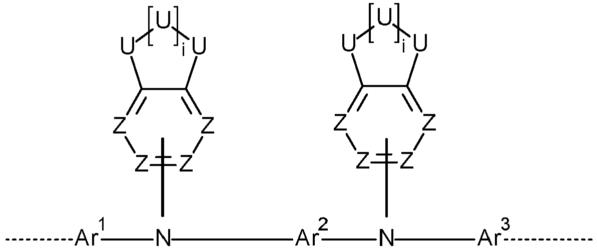 |
| (I-6) |
where the variables occurring are defined as in one or more of Claims 1 to 5.

7. Polymer according to one or more of Claims 1 to 6, **characterised in that** the proportion of structural units of the formula (I) in the polymer is in the range from 30 to 70 mol%, based on 100 mol% of all copolymerisable monomers present as structural units in the polymer.

8. Polymer according to one or more of Claims 1 to 7, **characterised in that** it has at least one structural unit that contains a crosslinkable group Q.

9. Polymer according to Claim 8, **characterised in that** the crosslinkable group Q is selected from the following formulae:
| | |
|---|---|
| 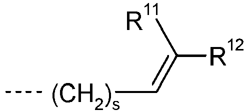 | 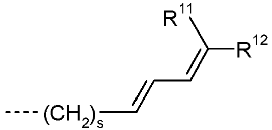 |
| Q1 | Q2 |
| 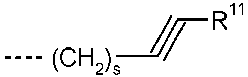 | 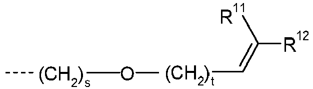 |
| Q3 | Q4 |
| 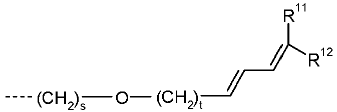 | 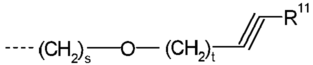 |
| Q5 | Q6 |
| 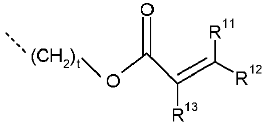 | 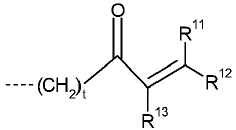 |
| Q7 | Q8 |
| 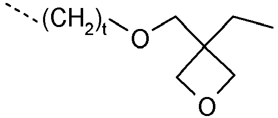 | 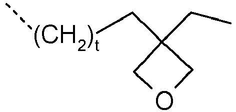 |
| Q9 | Q10 |
| 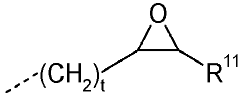 | 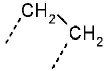 |
| Q11 | Q12 |
| 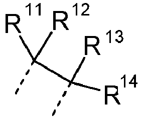 | 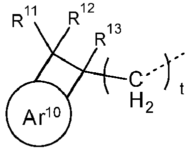 |
| Q13 | Q14 |
where R¹¹, R¹², R¹³ and R¹⁴ are selected on each occurrence, identically or differently, from H and straight-chain or branched alkyl groups having 1 to 6 C atoms, s = 0 to 8, t = 1 to 8, and Ar¹⁰ is selected from aromatic ring systems having 6 to 40 aromatic ring atoms, which may be substituted by one or more radicals R¹¹, and from heteroaromatic ring systems having 5 to 40 aromatic ring atoms, which may be substituted by one or more radicals R¹¹, and where the dashed bond denotes the bond to the remainder of the formula.

10. Polymer obtainable by crosslinking reaction of a polymer according to Claim 8 or 9.

11. Mixture comprising one or more polymers according to one or more of Claims 1 to 10 and one or more further polymeric, oligomeric, dendritic and/or low-molecular-weight substances.

12. Solution comprising one or more polymers according to one or more of Claims 1 to 10 and one or more solvents.

13. Use of a polymer according to one or more of Claims 1 to 10 in an electronic device.

14. Electronic device containing at least one polymer according to one or more of Claims 1 to 10.

15. Electronic device according to Claim 14, **characterised in that** the polymer according to one or more of Claims 1 to 10 is present in a layer selected from hole-transporting layer, hole-injection layer, electron-blocking layer and emitting layer.

16. Process for the preparation of a polymer according to one or more of Claims 1 to 10, **characterised in that** a polymerisation selected from Suzuki polymerisation, Yamamoto polymerisation, Stille polymerisation, Heck polymerisation, Negishi polymerisation, Sonogashira polymerisation, Hiyama polymerisation and Hartwig-Buchwald polymerisation is carried out.

17. Monomer of a formula (M) 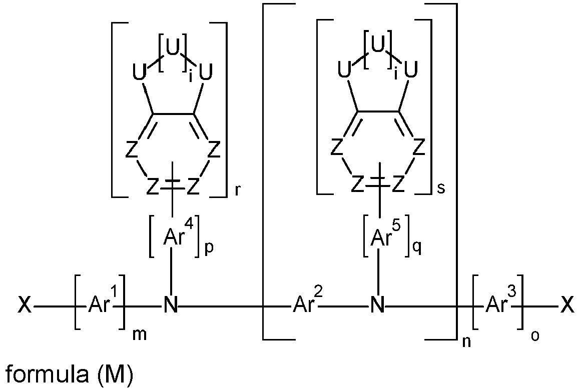 where the variables occurring are defined as in one or more of Claims 1 to 5, and where X is on each occurrence, identically or differently, a leaving group that is suitable for a polymerisation reaction.

## Revendications

1. Polymère contenant au moins un motif structurel de formule (I) selon une proportion allant de 1 à 100%, sur la base de 100% molaire de tous les monomères copolymérisables présents comme motifs structurels dans le polymère, 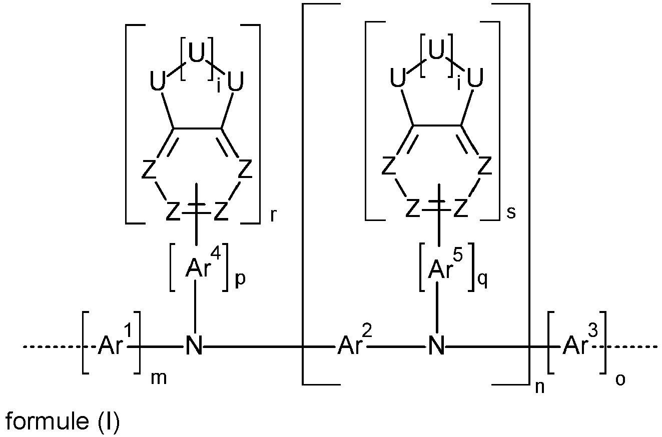 dans laquelle ce qui suit s'applique aux variables présentes :
U est à chaque occurrence, de manière identique ou différente, C(R¹)₂, CR¹=CR¹, Si(R¹)₂, O ou S, où les groupements choisis parmi CR¹=CR¹, O et S ne sont pas reliés directement les uns aux autres ;
Z est égal à CR² si aucun groupement n'y est lié, et est égal à C si un groupement y est lié ;
Ar¹, Ar², Ar³, Ar⁴ et Ar⁵ sont choisis, de manière identique ou différente, parmi des noyaux hétéroaromatiques ayant de 5 à 40 atomes de cycle aromatique, qui peuvent être substitués par un ou plusieurs radicaux R³, et parmi des noyaux aromatiques ayant de 6 à 40 atomes de cycle aromatique, qui peuvent être substitués par un ou plusieurs radicaux R³ ;
R¹ est choisi à chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ou R² ou R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R², R³ sont choisis à chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ou R² ou R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R⁴ est choisi à chaque occurrence, de manière identique ou différente, parmi H, D, F, C(=O)R⁵, CN, Si(R⁵)₃, N(R⁵)₂, P(=O)(R⁵)₂, OR⁵, S(=O)R⁵, S(=O)₂R⁵, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁴ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁵ ; et où un ou plusieurs groupements CH₂ dans lesdits groupements alkyle, alcoxy, alcényle et alcynyle peuvent être remplacés par -R⁵C=CR⁵-, -C=C-, Si(R⁵)₂, C=O, C=NR⁵, -C(=O)O-, -C(=O)NR⁵-, NR⁵, P(=O)(R⁵), -O-, -S-, SO ou SO₂ ;
R⁵ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁵ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
r est égal à 1, 2 ou 3 si p est égal à 1, et est égal à 1 si p est égal à 0 ;
s est égal à 0, 1, 2 ou 3 si q est égal à 1, et est égal à 1 si q est égal à 0 ;
p est égal à 0 ou 1 ; où, si p est égal à 0, les groupements qui sont liés au motif entre crochets avec l'indice p sont reliés directement les uns aux autres ;
q est égal à 0 ou 1 ; où, si q est égal à 0, les groupements qui sont liés au motif entre crochets avec l'indice q sont reliés directement les uns aux autres ;
n est égal à 0 ou 1, où, si n est égal à 0, les groupements qui sont liés au motif entre crochets avec l'indice n sont reliés directement les uns aux autres ;
m est égal à 0 ou 1, où, si m est égal à 0, les groupements qui sont liés au motif entre crochets avec l'indice m sont reliés directement les uns aux autres ;
o est égal à 0 ou 1, où, si o est égal à 0, les groupements qui sont liés au motif entre crochets avec l'indice o sont reliés directement les uns aux autres ;
i vaut à chaque occurrence, de manière identique ou différente, 1 ou 2 ;
où au moins un groupement U est présent et contient un ou plusieurs groupements R¹ choisis parmi des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ou R² ou R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ; et
où les deux groupements U qui sont situés en position directement adjacente à l'atome de C tête de pont, c'est-à-dire situés en position benzy-lique, portent des radicaux R¹ choisis parmi F, CN, Si(R⁴)₃, OR⁴, des groupements alkyle et alcoxy à chaîne linéaire ayant de 1 à 10 atomes de C, des groupements alkyle et alcoxy ramifiés ayant de 3 à 10 atomes de C, et des noyaux aromatiques ayant de 6 à 20 atomes de cycle aromatique, où deux, ou plus, radicaux R¹ ou R² ou R³ peuvent être liés les uns aux autres et peuvent former un cycle ; et où lesdits groupements alkyle et alcoxy et lesdits noyaux aromatiques peuvent dans chaque cas être substitués par un ou plusieurs radicaux R⁴.

2. Polymère selon la revendication 1, **caractérisé en ce que** Ar¹, Ar², Ar³, Ar⁴ et Ar⁵ sont choisis à chaque occurrence, de manière identique ou différente, parmi benzène, biphényle, terphényle, fluorène, naphtalène, phénanthrène, indénofluorène, spirobifluorène, dibenzofurane, dibenzothio-phène, carbazole, indénocarbazole et indolocarbazole, qui peuvent dans chaque cas être substitués par un ou plusieurs radicaux R¹.

3. Polymère selon la revendication 1 ou 2, **caractérisé en ce que** R¹ est choisi à chaque occurrence, de manière identique ou différente, parmi H, D, F, des groupements alkyle à chaîne linéaire ayant de 1 à 10 atomes de C et des groupements alkyle ramifiés ayant de 3 à 10 atomes de C.

4. Polymère selon l'une ou plusieurs parmi les revendications 1 à 3, **caractérisé en ce que** les motifs 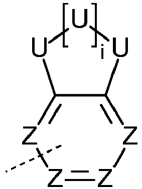 dans le motif structurel de formule (I) sont choisis parmi les motifs de formule (E-1) 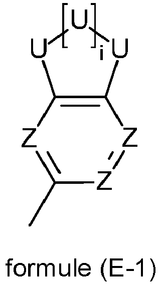 dans laquelle la liaison libre représente la liaison au reste du motif structurel de formule (I), et où les variables présentes sont telles que définies selon l'une ou plusieurs parmi les revendications 1 à 3.

5. Polymère selon l'une ou plusieurs parmi les revendications 1 à 4, **caractérisé en ce que** les motifs 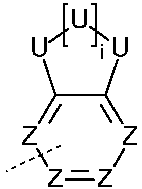 dans le motif structurel de formule (I) sont choisis parmi les motifs suivants :
| | | |
|---|---|---|
| 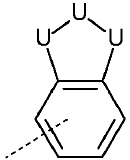 | 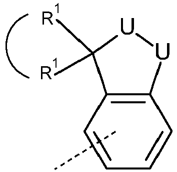 | 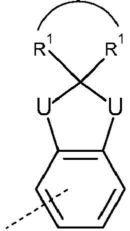 |
| E-a-1 | E-a-2 | E-a-3 |
| 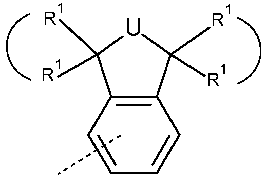 | 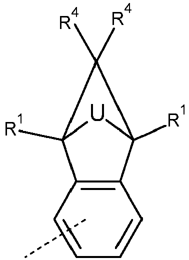 | |
| E-a-4 | E-a-5 | |
| 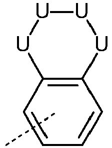 | 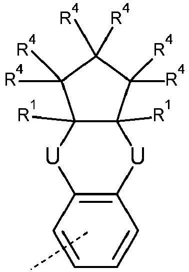 | 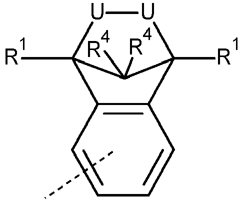 |
| E-b-1 | E-b-2 | E-b-3 |
| 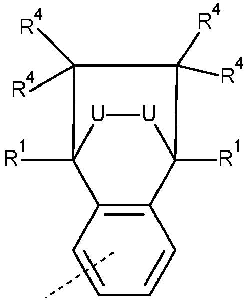 | 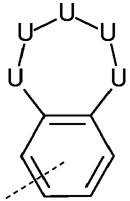 | 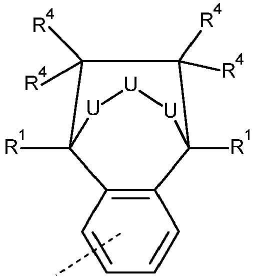 |
| E-b-4 | E-c-1 | E-c-2 |
dans lesquels la ligne en pointillés représente la liaison au reste du motif structurel de formule (I), et où la liaison semi-circulaire signifie que les deux groupements R¹ y participant sont liés l'un à l'autre et forment un cycle, et où les variables présentes sont telles que définies selon l'une ou plusieurs parmi les revendications 1 à 4.

6. Polymère selon l'une ou plusieurs parmi les revendications 1 à 5, **caractérisé en ce que** l'élément structurel de formule (I) correspond à l'une parmi les formules (I-1) à (I-6)
| |
|---|
| 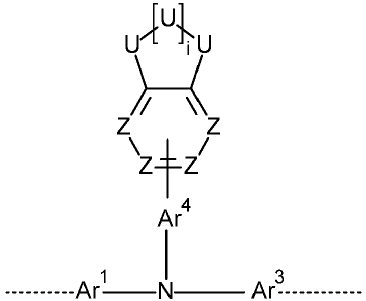 |
| (I-1) |
| 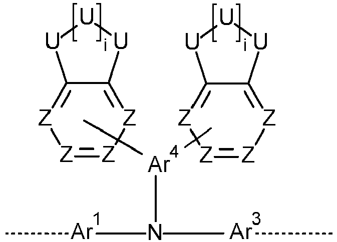 |
| (I-2) |
| 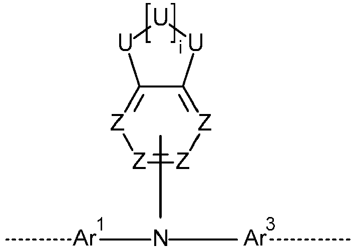 |
| (I-3) |
| 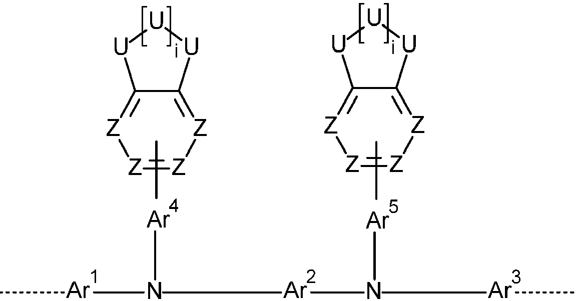 |
| (I-4) |
| 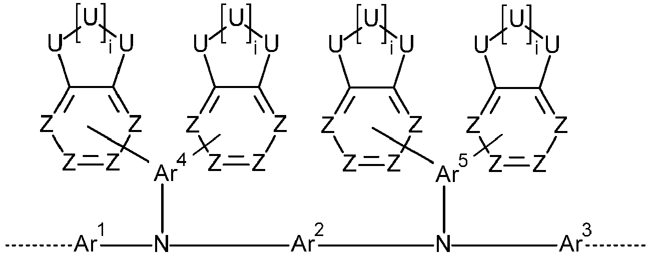 |
| (I-5) |
| 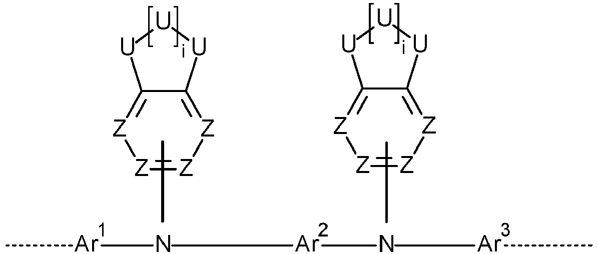 |
| (I-6) |
dans lesquelles les variables présentes sont telles que définies selon l'une ou plusieurs parmi les revendications 1 à 5.

7. Polymère selon l'une ou plusieurs parmi les revendications 1 à 6, **caractérisé en ce que** la proportion de motifs structurels de formule (I) dans le polymère se trouve dans la plage allant de 30 à 70% molaire, sur la base de 100% molaire de tous les monomères copolymérisables présents comme motifs structurels dans le polymère.

8. Polymère selon l'une ou plusieurs parmi les revendications 1 à 7, **caractérisé en ce qu'**il possède au moins un motif structurel qui contient un groupement réticulable Q.

9. Polymère selon la revendication 8, **caractérisé en ce que** le groupement réticulable Q est choisi parmi les formules suivantes :
| | |
|---|---|
| 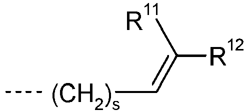 | 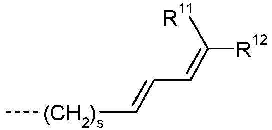 |
| Q1 | Q2 |
| 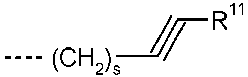 | 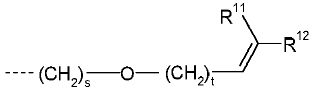 |
| Q3 | Q4 |
| 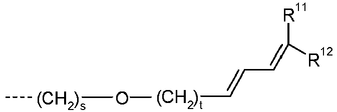 | 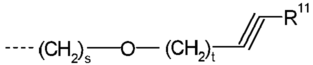 |
| Q5 | Q6 |
| 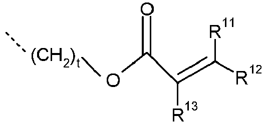 | 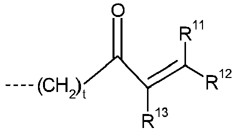 |
| Q7 | Q8 |
| 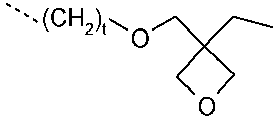 | 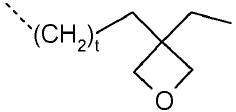 |
| Q9 | Q10 |
| 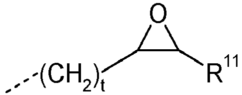 | 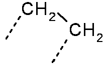 |
| Q11 | Q12 |
| 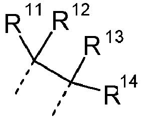 | 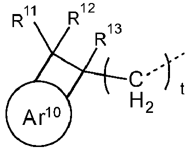 |
| Q13 | Q14 |
dans lesquelles R¹¹, R¹², R¹³ et R¹⁴ sont choisis à chaque occurrence, de manière identique ou différente, parmi H et des groupements alkyle à chaîne linéaire ou ramifiée ayant de 1 à 6 atomes de C, s = 0 à 8, t = 1 à 8, et Ar¹⁰ est choisi parmi des noyaux aromatiques ayant de 6 à 40 atomes de cycle aromatique, qui peuvent être substitués par un ou plusieurs radicaux R¹¹, et parmi des noyaux hétéroaromatiques ayant de 5 à 40 atomes de cycle aromatique, qui peuvent être substitués par un ou plusieurs radicaux R¹¹, et où la liaison en pointillés désigne la liaison au reste de la formule.

10. Polymère pouvant être obtenu par une réaction de réticulation d'un polymère selon la revendication 8 ou 9.

11. Mélange comprenant un ou plusieurs polymères selon l'une ou plusieurs parmi les revendications 1 à 10, et une ou plusieurs autres substances polymères, oligomères, dendritiques et/ou de bas poids moléculaire.

12. Solution comprenant un ou plusieurs polymères selon l'une ou plusieurs parmi les revendications 1 à 10, et un ou plusieurs solvants.

13. Utilisation d'un polymère selon l'une ou plusieurs parmi les revendications 1 à 10, dans un dispositif électronique.

14. Dispositif électronique contenant au moins un polymère selon l'une ou plusieurs parmi les revendications 1 à 10.

15. Dispositif électronique selon la revendication 14, **caractérisé en ce que** le polymère selon l'une ou plusieurs parmi les revendications 1 à 10 est présent dans une couche choisie parmi une couche de transport de trous, une couche d'injection de trous, une couche de blocage d'électrons et une couche émettrice.

16. Procédé de préparation d'un polymère selon l'une ou plusieurs parmi les revendications 1 à 10, **caractérisé en ce qu'**une polymérisation choisie parmi la polymérisation de Suzuki, la polymérisation de Yamamoto, la polymérisation de Stille, la polymérisation de Heck, la polymérisation de Negishi, la polymérisation de Sonogashira, la polymérisation de Hiyama et la polymérisation de Hartwig-Buchwald est mise en oeuvre.

17. Monomère de formule (M) 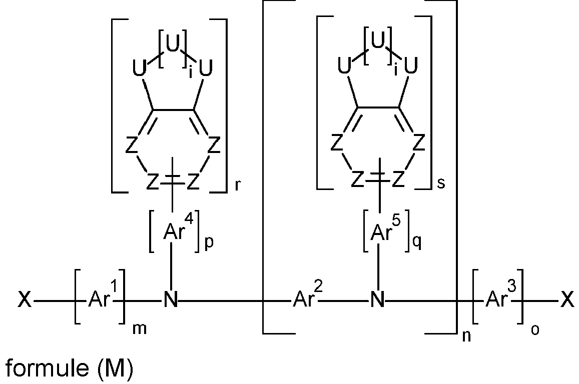 dans laquelle les variables présentes sont telles que définies selon l'une ou plusieurs parmi les revendications 1 à 5, et où X est à chaque occurrence, de manière identique ou différente, un groupement partant qui est convenable pour une réaction de polymérisation.
